(19) Europäisches Patentamt
European Patent Office
Office européen des brevets

(11) EP 4 631 938 A1

(12) EUROPEAN PATENT APPLICATION
published in accordance with Art. 153(4) EPC

(43) Date of publication:
**15.10.2025 Bulletin 2025/42**

(21) Application number: **23900729.7**

(22) Date of filing: **07.12.2023**

(51) International Patent Classification (IPC):
***C07D 401/12*** *(2006.01)* ***A61K 31/44*** *(2006.01)*
***A61K 31/415*** *(2006.01)* ***A61K 31/423*** *(2006.01)*
***A61K 31/427*** *(2006.01)* ***A61K 31/428*** *(2006.01)*
***A61K 31/433*** *(2006.01)* ***A61K 31/437*** *(2006.01)*
***A61K 31/444*** *(2006.01)* ***A61K 31/497*** *(2006.01)*
***A61K 31/501*** *(2006.01)* ***A61K 31/506*** *(2006.01)*
***A61K 31/4184*** *(2006.01)* ***A61K 31/4375*** *(2006.01)*
***A61K 31/4439*** *(2006.01)* ***A61K 31/4725*** *(2006.01)*
***A61K 31/4985*** *(2006.01)* ***A61P 43/00*** *(2006.01)*
***C07D 213/74*** *(2006.01)*

(52) Cooperative Patent Classification (CPC):
**A61K 31/415; A61K 31/4184; A61K 31/423; A61K 31/427; A61K 31/428; A61K 31/433; A61K 31/437; A61K 31/4375; A61K 31/44; A61K 31/4439; A61K 31/444; A61K 31/4725; A61K 31/497; A61K 31/4985; A61K 31/501;**
(Cont.)

(86) International application number:
**PCT/JP2023/043865**

(87) International publication number:
**WO 2024/122617 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.12.2022 JP 2022196048**

(71) Applicant: **Shionogi & Co., Ltd**
**Osaka-shi, Osaka 541-0045 (JP)**

(72) Inventors:
- **OGUMA, Takuya**
**Osaka-shi, Osaka 541-0045 (JP)**
- **YONEZAWA, Shuji**
**Osaka-shi, Osaka 541-0045 (JP)**
- **OKAMOTO, Ryuji**
**Osaka-shi, Osaka 541-0045 (JP)**
- **HATA, Kayoko**
**Osaka-shi, Osaka 541-0045 (JP)**

(74) Representative: **Vossius & Partner Patentanwälte Rechtsanwälte mbB**
**Siebertstrasse 3**
**81675 München (DE)**

# (54) NITROGEN-CONTAINING HETEROCYCLE WITH SEROTONIN RECEPTOR-BINDING ACTIVITY AND CARBOCYCLIC DERIVATIVE

(57) Provided are a compound having 5-HT2A receptor antagonism and/or inverse agonism and 5-HT2C receptor antagonism and/or inverse agonism and a pharmaceutically acceptable salt thereof, and a pharmaceutical composition comprising them.

A compound represented by Formula (I):

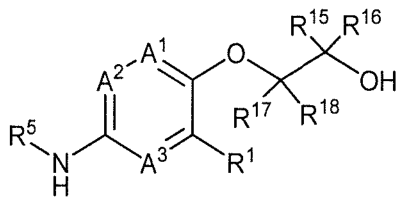

( I )

wherein $R^1$ is substituted or unsubstituted 6-membered aromatic heterocyclyl or the like, $A^1$ is $CR^2$ or the like, $A^2$

EP 4 631 938 A1

is $CR^3$ or the like, $A^3$ is $CR^4$ or the like, $R^2$, $R^3$, and $R^4$ are each independently a hydrogen atom or the like, $R^5$ is substituted or unsubstituted aromatic heterocyclyl or the like, $R^{15}$ and $R^{16}$ are each independently a hydrogen atom or the like, and $R^{17}$ and $R^{18}$ are each independently a hydrogen atom or the like, or a pharmaceutically acceptable salt thereof.

(52) Cooperative Patent Classification (CPC): (Cont.)
**A61K 31/506; A61P 43/00; C07D 213/74; C07D 231/16; C07D 401/04; C07D 401/12; C07D 401/14; C07D 403/04; C07D 403/12; C07D 405/14; C07D 407/14; C07D 413/12; C07D 413/14; C07D 417/12; C07D 417/14; C07D 471/04; C07D 487/04; C07D 513/04**

**Description**

TECHNICAL FIELD

The present invention relates to a compound having serotonin 5-HT2A receptor antagonism and/or inverse agonism and serotonin 5-HT2C receptor antagonism and/or inverse agonism or a pharmaceutically acceptable salt thereof, and a pharmaceutical composition comprising thereof.

The present invention further relates to a compound useful in the treatment and/or prevention of a disease caused by a serotonin 5-HT2A receptor and/or serotonin 5-HT2C receptor or a pharmaceutically acceptable salt thereof, and a pharmaceutical composition comprising thereof.

BACKGROUND ART

Neurodegenerative disorders (ND) are a group of related human diseases that exhibit common pathophysiological characteristics, i.e., progressive degeneration of a selective neuronal population that occurs over time. These neurodegenerative diseases include, for example, Alzheimer's disease and associated dementia, Parkinson's disease, Huntington's disease, Lewy body disease and associated motor disorders, and the like; however, they are not limited to these. Each of these disorders has unique clinical aspects including age of onset, time course of progression, neurosigns and neurosymptoms, neuropsychiatric symptoms, sensitivity to known therapeutic agents, and the like. Also, the pathophysiological basis of each of these disorders is caused by a genetic mechanism specific to each disease (Non Patent Literature 1).

Although the clarification of the genetic causes underlying these essentially different disorders has been significantly advanced, relatively little is known about biochemical mechanisms that cause selective neuronal degeneration common to all of them. In addition, although genetic factors have been identified that cause rare familial forms of the most common of these disorders, including Parkinson's disease and Alzheimer's disease, the pathophysiological basis of the majority of sporadic cases has not yet been known. Thus, there is no specific therapeutic agent that can directly alter disease progression and completely prevent onset. Instead, clinicians have utilized a variety of existing agents to achieve symptomatic relief of the motor, cognitive, and neuropsychiatric expressions that characterize these disorders (Non Patent Literatures 2 and 3).

Of the various neurological symptoms that characterize ND, the appearance of motor abnormalities, including bradykinesia, dyskinesia, and chorea, as well as neuropsychiatric symptoms, including psycosis and emotional symptoms such as anxiety and depression, are common symptoms, that seriously affects the functional status and quality of life of patients (Non Patent Literatures 4 and 5). Although most existing therapeutic agents, including antipsychotics and antidepressants, are often efficacious in these patients, their tolerability is significantly low (Non Patent Literature 6). Also, available Parkinson's disease therapeutic agents, including L-dopa and dopamine agonists, are generally effective, but cause the emergence of treatment-restricting side effects that are currently too severe to be addressed by drug therapy.

For a long time, there was no approved drug specifically for ND. However, in 2016, the 5-HT2A receptor inverse agonist pimavanserin was first approved in the United States for the treatment of hallucinations and delusions associated with Parkinson's disease (Non Patent Literature 7). This drug has not been reported to cause adverse effects of worsening motor symptoms or cognitive decline, as is the case with existing antipsychotic drugs. Pimavanserin's primary pharmacological action is serotonin 5-HT2A receptor inverse agonism/antagonism, but it also has serotonin 5-HT2C receptor inverse agonism (Non Patent Literature 8). The results of 5-HT2A occupancy measured in human PET study of pimavanserin and the results of clinical trials of pimavanserin suggest that pimavanserin exerts its pharmacological effects via 5-HT2A and 2C (Non Patent Literature 9). In addition, pimavanserin has a significant adverse effect on the cardiovascular system, and its dosage is restricted.

These findings make it clear that the development of novel therapeutic agents is required which are specifically designed to be not only effective for these specific symptoms which cause physical disability, but also tolerated in these specific patient populations. This can be achieved by improving the selectivity of drug-target interactions of new therapeutic agents. Specifically, it can be achieved by having strong activity and selectivity for target 5-HT2A and 5-HT2C, and reducing adverse effects on the cardiovascular system.

Patent Documents 1 to 16 and Non-Patent Documents 10 to 13 describe compounds having serotonin 5-HT2A receptor antagonism and/or inverse agonism, but none of the documents describes or suggests the compounds related to the present invention. A benzamide derivative having interleukin-6 inhibitory activity is disclosed in Patent Document 17, but serotonin 5-HT2A receptor antagonism and/or inverse agonism and therapeutic effects on hallucinations and delusions are not described, and the compounds related to the present invention are neither described nor suggested.

A pyrimidine derivative having protein kinase C inhibitor is disclosed in Patent Documents 18 to 20, but serotonin 5-HT2A receptor antagonism and/or inverse agonism and therapeutic effects on hallucinations and delusions are not described, and the compounds related to the present invention are neither described nor suggested.

Prior Art Document

Patent Documents

Patent Document 1: WO 2005/012254
Patent Document 2: WO 2006/078610
Patent Document 3: WO 2007/136703
Patent Document 4: WO 2007/136680
Patent Document 5: WO 2022/093850
Patent Document 6: WO 2022/093849
Patent Document 7: WO 2007/120600
Patent Document 8: WO 2006/055734
Patent Document 9: WO 2004/058722
Patent Document 10: WO 2004/028450
Patent Document 11: WO 01/29008
Patent Document 12: WO 2013/171641
Patent Document 13: WO 2008/027483
Patent Document 14: WO 2007/136875
Patent Document 15: WO 99/52927
Patent Document 16: WO 03/062206
Patent Document 17: WO 2019/165158
Patent Document 18: WO 2013/152198
Patent Document 19: WO 2014/089112
Patent Document 20: WO 2014/151900

Non Patent Documents

Non-Patent Document 1: Nature Reviews Neurology, vol. 10, pp. 620-633, 2014
Non-Patent Document 2: Progress in Neurology and Psychiatry, vol. 22, No. 1, pp. 30-35, 2018
NON PATENT LITERATURE 3: Movement Disorders, vol. 24, No. 11, pp. 1641-1649, 2009
Non-Patent Document 4: Parkisonism and Related Disorders, vol. 15, Supplement 3, pp. S105-S109, 2009
Non-Patent Document 5: Neurology, 2004; vol. 63, No. 2, pp. 293-300, 2004
Non-Patent Document 6: JAMA Neurology, vol. 73, No. 5, pp. 535-541, 2016
Non-Patent Document 7: The Lancet, vol. 383, pp. 533-540, 2014
Non-Patent Document 8: Journal of Pharmacology and Experimental Therapeutics, vol. 317, No. 2, pp. 910-918, 2006
Non-Patent Document 9: CNS Spectrums, vol. 21, pp. 271-275, 2016
Non-Patent Document 10: Bioorganic & Medicinal Chemistry Letters, vol. 19, pp. 5486-5489, 2009
Non-Patent Document 11: Journal of Medicinal Chemistry, vol. 53, pp. 4412-4421, 2010
Non-Patent Document 12: Journal of Medicinal Chemistry, vol. 53, pp. 1923-1936, 2010
Non-Patent Document 13: Journal of Medicinal Chemistry, vol. 53, pp. 5696-5706, 2010

SUMMARY OF THE INVENTION

TECHNICAL PROBLEM TO BE SOLVED BY THE INVENTION

An object of the present invention is to provide a novel compound having serotonin 5-HT2A receptor antagonism and/or inverse agonism and 5-HT2C receptor antagonism and/or inverse agonism. More preferably, the present invention is to provide a novel compound or a pharmaceutically acceptable salt thereof, having an effect on serotonin-related disease such as Parkinson's disease- and/or dementia-related hallucinations and delusions by having serotonin 5-HT2A receptor antagonism and/or inverse agonism and 5-HT2C receptor antagonism and/or inverse agonism, and a pharmaceutical comprising them.

MEANS FOR SOLVING THE PROBLEM

The present invention relates to the following items (1) to (24) and (1') to (26').

(1) A compound represented by Formula (I-1):

[Chemical formula 1]

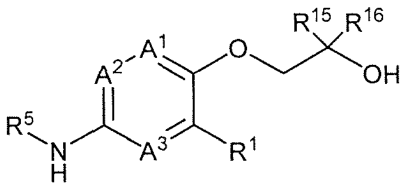

( I-1 )

wherein

$R^1$ is substituted or unsubstituted 6-membered aromatic carbocyclyl; substituted or unsubstituted 6-membered aromatic heterocyclyl, or substituted or unsubstituted 5-membered aromatic heterocyclyl;
$A^1$ is $CR^2$ or N;
$A^2$ is $CR^3$ or N;
$A^3$ is $CR^4$ or N;
$R^2$, $R^3$, and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy; or $R^2$ and $R^3$ may be taken together with a carbon atom to which $R^2$ and $R^3$ are bonded to form a substituted or unsubstituted aromatic carbocycle, a substituted or unsubstituted non-aromatic carbocycle, a substituted or unsubstituted aromatic heterocycle, or a substituted or unsubstituted non-aromatic heterocycle;
$R^5$ is substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl;
$R^{15}$ and $R^{16}$ are each independently a hydrogen atom or substituted or unsubstituted alkyl; or
$R^{15}$ and $R^{16}$ may be taken together with a carbon atom to which $R^{15}$ and $R^{16}$ are bonded to form a substituted or unsubstituted non-aromatic carbocycle, provided that excluded are the following compound:

(i) a compound wherein $R^1$ is substituted or unsubstituted tetrazolyl and (ii) a compound wherein $R^1$ is substituted or unsubstituted tetrazolonyl,
or a pharmaceutically acceptable salt thereof.

(2) The compound according to the above item (1) or a pharmaceutically acceptable salt, wherein $R^{15}$ and $R^{16}$ are each independently a hydrogen atom or alkyl.
(3) The compound according to the above item (1), wherein $R^{15}$ and $R^{16}$ are hydrogen atoms, or a pharmaceutically acceptable salt thereof.
(4) The compound according to any one of the above items (1) to (3), wherein $R^1$ is a group represented by Formula:

[Chemical formula 2]

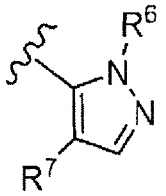 , 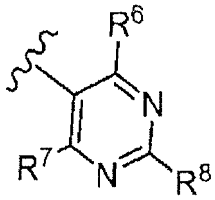 , 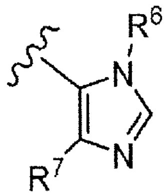 , 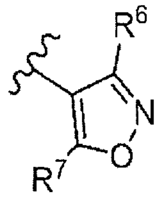 , 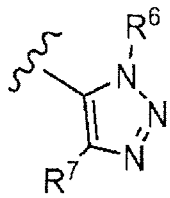 ,

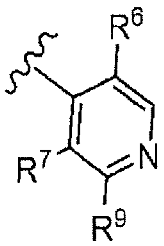 , 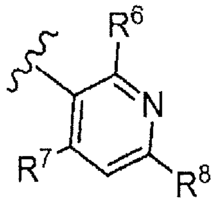 , 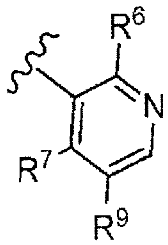 , 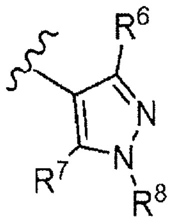 , 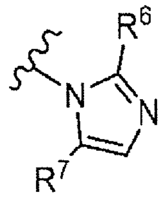 ,

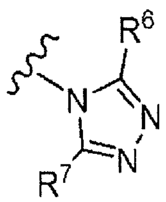 , 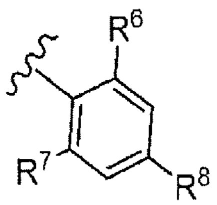 , 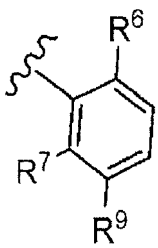 or 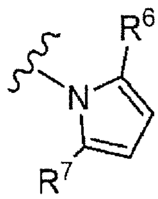

wherein

$R^6$ is substituted or unsubstituted alkyl or substituted or unsubstituted non-aromatic carbocyclyl;
$R^7$ is halogen, substituted or unsubstituted alkyl, substituted or unsubstituted alkenyl, substituted or unsubstituted alkynyl, or cyano;
$R^8$ is a hydrogen atom or substituted or unsubstituted alkyl; and
$R^9$ is a hydrogen atom, halogen, or substituted or unsubstituted alkyloxy.

(5) The compound according to the above item (4) or a pharmaceutically acceptable salt thereof, wherein $R^1$ is a group represented by Formula:

[Chemical formula 3]

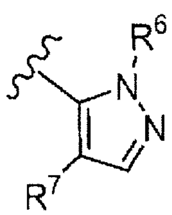 , 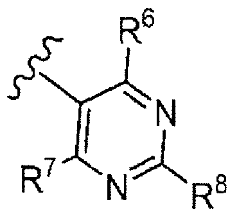 or 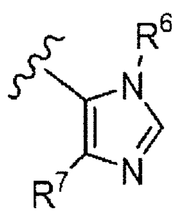

wherein $R^6$, $R^7$, and $R^8$ are the same as those in the above item (4), or a pharmaceutically acceptable salt thereof.
(6) The compound according to any one of the above items (1) to (5), wherein $R^4$ is a hydrogen atom, or a pharmaceutically acceptable salt thereof.
(7) The compound according to any one of the above items (1) to (6), wherein $R^2$ is a hydrogen atom, halogen, or substituted or unsubstituted alkyl, or a pharmaceutically acceptable salt thereof.
(8) The compound according to any one of the above items (1) to (6), wherein $A^1$ is CH, or a pharmaceutically acceptable salt thereof.

(9) The compound according to any one of the above items (1) to (8), wherein $R^3$ is a hydrogen atom, halogen, or substituted or unsubstituted alkyl, or a pharmaceutically acceptable salt thereof.

(10) The compound according to any one of the above items (1) to (8), wherein $A^2$ is $CR^3$; and $R^3$ is halogen or substituted or unsubstituted alkyl, or a pharmaceutically acceptable salt thereof.

(11) The compound according to any one of the above items (1) to (10), wherein $R^5$ is substituted or unsubstituted aromatic carbocyclyl or substituted or unsubstituted aromatic heterocyclyl, or a pharmaceutically acceptable salt thereof.

(12) The compound according to any one of the above items (1) to (10), wherein $R^5$ is substituted or unsubstituted phenyl or substituted or unsubstituted 6-membered aromatic heterocyclyl, or a pharmaceutically acceptable salt thereof.

(13) The compound according to the above items (1) to (10), wherein $R^5$ is a group represented by Formula:

[Chemical formula 4]

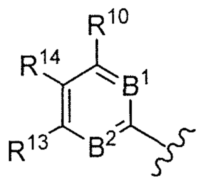

wherein

$R^{10}$ and $R^{13}$ are each independently a hydrogen atom or substituted or unsubstituted alkyl;
$R^{14}$ is a hydrogen atom, halogen, substituted or unsubstituted alkyl, substituted or unsubstituted alkyloxy, substituted or unsubstituted non-aromatic carbocyclyl, substituted or unsubstituted alkyloxycarbonyl, substituted or unsubstituted alkylcarbonyl, or cyano;
$B^1$ is $CR^{11}$ or N;
$B^2$ is $CR^{12}$ or N; and
$R^{11}$ and $R^{12}$ are each independently a hydrogen atom or halogen, or a pharmaceutically acceptable salt thereof.

(14) The compound of the above item (13), wherein $B^1$ is N, and $B^2$ is $CR^{12}$, or a pharmaceutically acceptable salt thereof.

(15) The compound according to the above item (13) or (14), wherein $R^{14}$ is a hydrogen atom, halogen, substituted or unsubstituted alkyl, substituted or unsubstituted alkyloxy, or substituted or unsubstituted non-aromatic carbocyclyl, or a pharmaceutically acceptable salt thereof.

(16) A pharmaceutical composition comprising the compound according to any one of the above items (1) to (15) or a pharmaceutically acceptable salt thereof.

(17) The pharmaceutical composition according to the above item (16), wherein the pharmaceutical composition is a serotonin 5-HT2A receptor antagonist and/or inverse agonist.

(18) The pharmaceutical composition according to the above item (16), wherein the pharmaceutical composition is a serotonin 5-HT2A receptor antagonist and/or inverse agonist and a 5-HT2C receptor antagonist and/or inverse agonist.

(19) A method for treating and/or preventing a disease associated with a serotonin 5-HT2A receptor, the method including administering the compound according to any one of the above items (1) to (15) or a pharmaceutically acceptable salt thereof.

(20) A method for treating and/or preventing a disease associated with a serotonin 5-HT2A receptor and a serotonin 5-HT2C receptor, the method including administering the compound according to any one of the above items (1) to (15) or a pharmaceutically acceptable salt thereof.

(21) The compound according to any one of the above items (1) to (15) or a pharmaceutically acceptable salt thereof, for use in treating and/or preventing a disease associated with a serotonin 5-HT2A receptor.

(22) The compound according to any one of the above items (1) to (15) or a pharmaceutically acceptable salt thereof, for use in treating and/or preventing a disease associated with a serotonin 5-HT2A receptor and a serotonin 5-HT2C receptor.

(23) Use of the compound according to any one of the above items (1) to (15) or a pharmaceutically acceptable salt thereof, for the manufacture of a therapeutic agent and/or prophylactic agent for a disease associated with a serotonin 5-HT2A receptor.

(24) Use of the compound according to any one of the above items (1) to (15) or a pharmaceutically acceptable salt

thereof, for the manufacture of a therapeutic agent and/or prophylactic agent for a disease associated with a serotonin 5-HT2A receptor and a serotonin 5-HT2C receptor.

(1') A compound represented by Formula (I):

[Chemical formula 5]

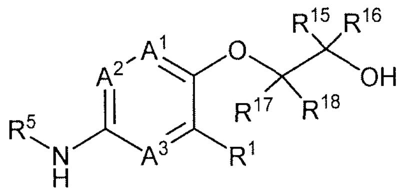

(I)

wherein

$R^1$ is substituted or unsubstituted 6-membered aromatic heterocyclyl or substituted or unsubstituted 5-membered aromatic heterocyclyl (provided that substituted or unsubstituted tetrazolyl and substituted or unsubstituted tetrazolonyl are excluded), substituted or unsubstituted 6-membered aromatic carbocyclyl, substituted or unsubstituted bicyclic 9-membered non-aromatic heterocyclyl, or substituted or unsubstituted bicyclic 10-membered non-aromatic heterocyclyl;
$A^1$ is $CR^2$ or N;
$A^2$ is $CR^3$ or N;
$A^3$ is $CR^4$ or N;
$R^2$, $R^3$, and $R^4$ are the following (i) or (ii);

(i) $R^2$, $R^3$, and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;
(ii) when $A^1$ is $CR^2$, $A^2$ is $CR^3$, $A^3$ is $CR^4$ or N, $R^2$ and $R^3$ are taken together with a carbon atom to which $R^2$ and $R^3$ are bonded to form a substituted or unsubstituted aromatic carbocycle, a substituted or unsubstituted non-aromatic carbocycle, a substituted or unsubstituted aromatic heterocycle, or a substituted or unsubstituted non-aromatic heterocycle, and $R^4$ each independently is hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;

$R^5$ is substituted or unsubstituted aromatic heterocyclyl, substituted or unsubstituted aromatic carbocyclyl, substituted or unsubstituted non-aromatic heterocyclyl, or substituted or unsubstituted non-aromatic carbocyclyl;
$R^{15}$ and $R^{16}$ are each independently a hydrogen atom or substituted or unsubstituted alkyl; or
$R^{15}$ and $R^{16}$ may be taken together with a carbon atom to which $R^{15}$ and $R^{16}$ are bonded to form a substituted or unsubstituted non-aromatic carbocycle;
$R^{17}$ and $R^{18}$ are each independently a hydrogen atom, halogen, or substituted or unsubstituted alkyl; or
$R^{17}$ and $R^{18}$ may be taken together with a carbon atom to which $R^{17}$ and $R^{18}$ are bonded to form a substituted or unsubstituted non-aromatic carbocycle, or a pharmaceutically acceptable salt thereof.

(2') The compound according to the above item (1'), wherein $R^{15}$ and $R^{16}$ are each a hydrogen atom, and $R^{17}$ and $R^{18}$ are each independently a hydrogen atom, halogen, or alkyl, or a pharmaceutically acceptable salt thereof.

(3') The compound according to the above item (1'), wherein $R^{15}$, $R^{16}$, $R^{17}$, and $R^{18}$ are each a hydrogen atom, or a pharmaceutically acceptable salt thereof.

(4') The compound according to any one of the above items (1') to (3'), wherein $R^1$ is a group represented by Formula:

[Chemical formula 6]

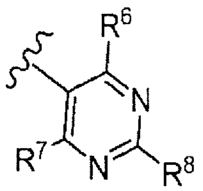 , 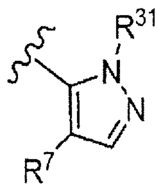 , 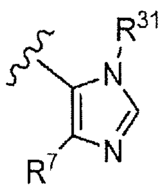 , 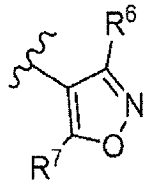 , 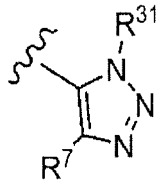 ,

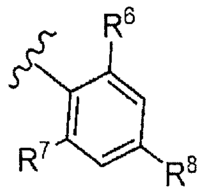 , 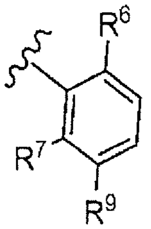 , 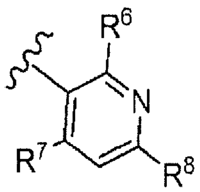 , 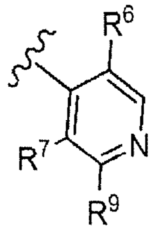 , 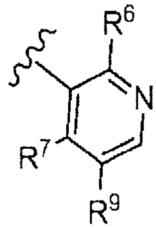 ,

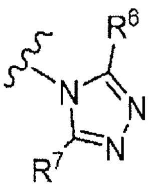 , 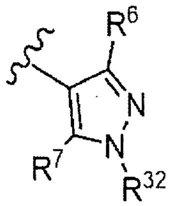 , 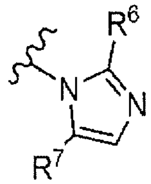 , 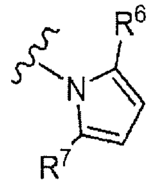 , 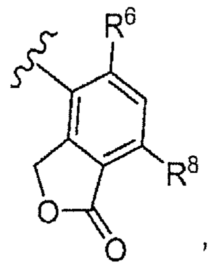 ,

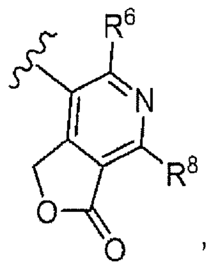 , 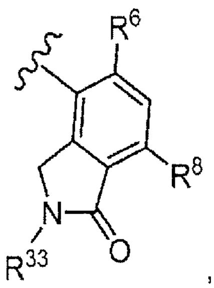 , 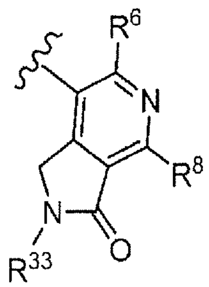 , 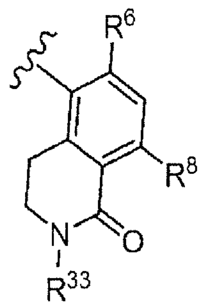

or 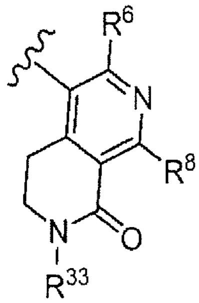

wherein

$R^6$ and $R^7$ are each independently a hydrogen atom, halogen, substituted or unsubstituted alkyl, substituted or unsubstituted alkyloxy, substituted or unsubstituted alkenyl, substituted or unsubstituted alkynyl, cyano, or substituted or unsubstituted non-aromatic carbocyclyl (provided that when $R^6$ is a hydrogen atom, $R^7$ is

halogen, substituted or unsubstituted alkyl, substituted or unsubstituted alkyloxy, substituted or unsubstituted alkenyl, substituted or unsubstituted alkynyl, cyano, or substituted or unsubstituted non-aromatic carbocyclyl);

$R^8$ and $R^9$ are each independently a hydrogen atom, halogen, substituted or unsubstituted alkyl, substituted or unsubstituted alkyloxy, substituted or unsubstituted alkyloxycarbonyl, substituted or unsubstituted carbamoyl, cyano, substituted or unsubstituted non-aromatic carbocyclyloxy, substituted or unsubstituted non-aromatic heterocyclyloxy, substituted or unsubstituted non-aromatic carbocyclyl, or substituted or unsubstituted non-aromatic heterocyclyl;

$R^{31}$ is substituted or unsubstituted alkyl, or substituted or unsubstituted non-aromatic carbocyclyl; and

$R^{32}$ and $R^{33}$ are each independently a hydrogen atom, substituted or unsubstituted alkyl, or substituted or unsubstituted non-aromatic carbocyclyl, or a pharmaceutically acceptable salt thereof.

(5') The compound according to any one of the above items (1') to (4'), wherein $R^1$ is a group represented by Formula:

[Chemical formula 7]

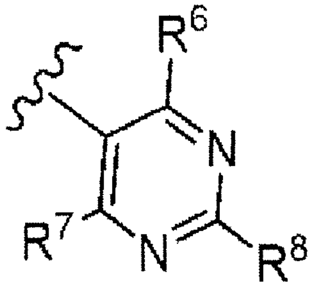
,
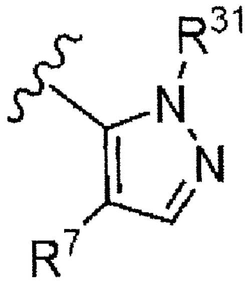
or
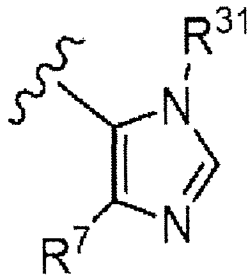

wherein

$R^6$ and $R^7$ are each independently halogen, substituted or unsubstituted alkyl, substituted or unsubstituted alkyloxy, substituted or unsubstituted alkenyl, substituted or unsubstituted alkynyl, or cyano;

$R^8$ is a hydrogen atom, substituted or unsubstituted alkyl, substituted or unsubstituted alkyloxy, substituted or unsubstituted non-aromatic carbocyclyloxy, substituted or unsubstituted non-aromatic heterocyclyloxy, substituted or unsubstituted non-aromatic carbocyclyl, or substituted or unsubstituted non-aromatic heterocyclyl; and

$R^{31}$ is substituted or unsubstituted alkyl or substituted or unsubstituted non-aromatic carbocyclyl, or a pharmaceutically acceptable salt thereof.

(6') The compound according to any one of the above items (1') to (5'), wherein $A^1$ is $CR^2$ or N;

$A^2$ is $CR^3$ or N;

$A^3$ is $CR^4$ or N;

$R^2$, $R^3$, and $R^4$ are the following (i-1):

(i-1) $R^2$ and $R^3$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy, and $R^4$ is a hydrogen atom, or a pharmaceutically acceptable salt thereof.

(7') The compound according to any one of the above items (1') to (6'), wherein $A^1$ is $CR^2$ or N;

$A^2$ is $CR^3$ or N;

$A^3$ is $CR^4$ or N;

$R^2$, $R^3$, and $R^4$ are following (i-2):

(i-2) $R^2$ is a hydrogen atom, halogen, or substituted or unsubstituted alkyl, and $R^3$ and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;
or a pharmaceutically acceptable salt thereof.

(8') The compound according to the above items (1') to (7'), wherein $A^1$ is $CR^2$ or N;

$A^2$ is $CR^3$ or N;
$A^3$ is $CR^4$ or N;
$R^2$, $R^3$, and $R^4$ are the following (i-3):

(i-3) $R^2$ is a hydrogen atom, $R^3$ and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;
or a pharmaceutically acceptable salt thereof.

(9') The compound according to any one of the above items (1') to (8'), wherein $A^1$ is $CR^2$ or N;

$A^2$ is $CR^3$ or N;
$A^3$ is $CR^4$ or N;
$R^2$, $R^3$, and $R^4$ are the following (i-4) :

(i-4) $R^3$ is a hydrogen atom, halogen, or substituted or unsubstituted alkyl, and $R^2$ and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;
or a pharmaceutically acceptable salt thereof.

(10') The compound according to any one of the above items (1') to (9'), wherein $A^1$ is $CR^2$ or N;

$A^2$ is $CR^3$ or N;
$A^3$ is $CR^4$ or N;
$R^2$, $R^3$, and $R^4$ are the following (i-5):

(i-5) $R^3$ is a hydrogen atom or halogen, $R^2$ and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;
or a pharmaceutically acceptable salt thereof.

(11') The compound according to any one of the above items (1') to (10'), wherein (i') $A^1$ is CH, $A^2$ is N, and $A^3$ is CH; or (ii') $A^1$ is CH, $A^2$ is $CR^3$, $R^3$ is a hydrogen atom, halogen, or substituted or unsubstituted alkyl, and $A^3$ is N, or a pharmaceutically acceptable salt thereof.
(12') The compound according to any one of the above items (1') to (11'), wherein $R^5$ is substituted or

unsubstituted aromatic heterocyclyl or substituted or unsubstituted aromatic carbocyclyl, or a pharmaceutically acceptable salt thereof.

(13') The compound according to any one of the above items (1') to (12'), wherein $R^5$ is substituted or unsubstituted 6-membered aromatic heterocyclyl or substituted or unsubstituted phenyl, or a pharmaceutically acceptable salt thereof.

(14') The compound according to any one of the above items (1') to (13'), wherein $R^5$ is a group represented by Formula:

[Chemical formula 8]

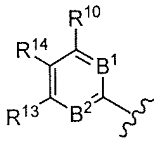

wherein

$R^{10}$ and $R^{13}$ are each independently a hydrogen atom, cyano, substituted or unsubstituted alkyloxy, or substituted or unsubstituted alkyl;
$R^{14}$ is a hydrogen atom, halogen, substituted or unsubstituted alkyl, substituted or unsubstituted alkyloxy, substituted or unsubstituted non-aromatic carbocyclyl, substituted or unsubstituted non-aromatic heterocyclyl, substituted or unsubstituted aromatic carbocyclyl, substituted or unsubstituted aromatic heterocyclyl, substituted or unsubstituted non-aromatic carbocyclyloxy, substituted or unsubstituted aromatic carbocyclyloxy, substituted or unsubstituted alkyloxycarbonyl, substituted or unsubstituted alkylcarbonyl, or cyano;
$B^1$ is $CR^{11}$ or N;
$B^2$ is $CR^{12}$ or N; and
$R^{11}$ and $R^{12}$ are each independently a hydrogen atom, halogen, methyloxy, methyl, or halomethyl, or a pharmaceutically acceptable salt thereof.

(15') The compound according to the above item (14'), wherein

(i") $B^1$ is N, and $B^2$ is $CR^{12}$; or
(ii") $B^1$ and $B^2$ are each N, or a pharmaceutically acceptable salt thereof.

(16') The compound of the above item (14') or (15'), wherein $R^{14}$ is a hydrogen atom, halogen, substituted or unsubstituted alkyl, substituted or unsubstituted alkyloxy, substituted or unsubstituted aromatic carbocyclyl, substituted or unsubstituted aromatic heterocyclyl, substituted or unsubstituted non-aromatic carbocyclyloxy, or substituted or unsubstituted non-aromatic carbocyclyl, or a pharmaceutically acceptable salt thereof.

(17') The compound according to the above item (1'), wherein the compound is selected from the group consisting of compounds I-006, I-062, I-063, I-073, 1-127, I-200, I-211, I-247, I-257, I-266, I-267, I-271, I-359, I-376, and I-378, or a pharmaceutically acceptable salt thereof.

(18') A pharmaceutical composition comprising the compound according to any one of the above items (1') to (17') or a pharmaceutically acceptable salt thereof.

(19') The pharmaceutical composition according to the above item (18'), wherein the pharmaceutical composition is a serotonin 5-HT2A receptor antagonist and/or inverse agonist.

(20') The pharmaceutical composition according to the above item (18'), wherein the pharmaceutical composition is a serotonin 5-HT2A receptor and 5-HT2C receptor antagonist and/or inverse agonist.

(21') A method for treating and/or preventing a disease associated with a serotonin 5-HT2A receptor, the method including administering the compound according to any one of the above items (1') to (17') or a pharmaceutically acceptable salt thereof.

(22') A method for treating and/or preventing a disease associated with serotonin 5-HT2A and serotonin 5-HT2C receptors, the method including administering the compound according to any one of the above items (1') to (17') or a pharmaceutically acceptable salt thereof.

(23') The compound according to any one of the above items (1') to (17') or a pharmaceutically acceptable salt thereof, for use in treating and/or preventing a disease associated with an antagonist and/or inverse agonist of a serotonin 5-HT2A receptor.

(24') The compound according to any one of the above items (1') to (17') or a pharmaceutically acceptable salt

thereof, for use in treating and/or preventing a disease associated with an antagonist and/or agonist of a serotonin 5-HT2A receptor and a serotonin 5-HT2C receptor.

(25') Use of the compound according to any one of the above items (1') to (17') or a pharmaceutically acceptable salt thereof, for the manufacture of a therapeutic agent and/or prophylactic agent for a disease associated with a serotonin 5-HT2A receptor.

(26') Use of the compound according to any one of the above items (1') to (17') or a pharmaceutically acceptable salt thereof, for the manufacture of a therapeutic agent and/or prophylactic agent for a disease associated with a serotonin 5-HT2A receptor and a serotonin 5-HT2C receptor.

EFFECT OF THE INVENTION

The compound according to the present invention has serotonin 5-HT2A receptor antagonism and/or inverse agonism and 5-HT2C receptor antagonism and/or inverse agonism, and is useful as a therapeutic agent and/or prophylactic agent for Parkinson's disease- and/or dementia-related hallucinations and delusions.

MODE FOR CARRYING OUT THE INVENTION [0013]

Hereinafter, the meaning of each term used in the present specification will be described. Unless particularly stated otherwise, each term is used in the same sense, either alone or in combination with other terms.

The term "consist of" means having only the constituent elements.

The term "comprise" means that elements are not limited to the constituent elements, and elements that are not described are not excluded.

The present invention will be described below with reference to embodiments. Throughout this specification, it should be understood that the representation of the singular also includes the concept of the plural unless stated otherwise. Thus, unless stated otherwise, singular articles (e.g., "a", "an", "the", and the like in English) can be understood to include the concepts of the plural forms.

Unless otherwise defined, it should be understood that the terms used in the present specification are used in a meaning normally used in the art. Accordingly, unless otherwise defined, all terminologies and scientific and technical terms used in the present specification have the same meanings as commonly understood by those having ordinary skill in the art in the field to which the present invention belongs. In case of conflict, this specification (including definitions) shall prevail.

The term "halogen" includes a fluorine atom, a chlorine atom, a bromine atom, and an iodine atom. Particularly, a fluorine atom and a chlorine atom are preferred.

As "halogen" in $R^7$, a chlorine atom and a bromine atom are preferred.

The term "alkyl" includes a C1 to C15, preferably a C1 to C10, more preferably a C1 to C6 and further preferably a C1 to C4 linear or branched hydrocarbon group. Examples include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl.

Preferred embodiments of "alkyl" include methyl, ethyl, n-propyl, isopropyl, n-butyl, isobutyl, sec-butyl, tert-butyl, and n-pentyl. More preferred embodiments include methyl, ethyl, n-propyl, isopropyl, and tert-butyl.

The term "C1-C3 alkyl" encompasses methyl, ethyl, n-propyl, and isopropyl.

The term "haloalkyl" means the alkyl substituted with at least one halogen. If the alkyl is substituted with two or more halogens, the halogens may be identical to or different from each other. Examples of the haloalkyl include, fluoromethyl, difluoromethyl, trifluoromethyl, chlorofluoromethyl, 1,1-difluoroethyl, 2,2-difluoroethyl, 2,2,2-trifluoroethyl, 2,2-difluoropropyl, 3,3,3-trifluoropropyl, 2,2,3,3-tetrafluoropropyl, 2,2,3,3,3-pentafluoropropyl, 4,4,4-trifluorobutyl, and 3,3,3-trifluoro-2-(trifluoromethyl)propyl. Preferred embodiments of "haloalkyl" include difluoromethyl, trifluoromethyl, 1,1-difluoroethyl, 2,2-difluoroethyl, 2,2,2-trifluoroethyl, 2,2-difluoropropyl, and 2,2,3,3,3-pentafluoropropyl. More preferred embodiments include difluoromethyl, trifluoromethyl, 1,1-difluoroethyl, 2,2-difluoroethyl, and 2,2,2-trifluoroethyl.

The term "alkyloxy" means a group in which the above "alkyl" is bound to an oxygen atom. Examples of the alkyloxy include methyloxy, ethyloxy, n-propyloxy, isopropyloxy, n-butyloxy, tert-butyloxy, isobutyloxy, sec-butyloxy, n-pentyloxy, isopentyloxy, n-hexyloxy, and the like.

Preferred embodiments of "alkyloxy" include methyloxy, ethyloxy, n-propyloxy, isopropyloxy, n-butyloxy, isobutyloxy, sec-butyloxy, and tert-butyloxy. More preferred embodiments include methyloxy, ethyloxy, n-propyloxy, and isopropyloxy.

The term "haloalkyloxy" means a group in which the above "haloalkyl" is bound to an oxygen atom. Examples of the haloalkyloxy include difluoromethyloxy, 2-monofluoroethyloxy, 3-monofluoropropyloxy, 2,2,3,3,3-pentafluoropropyloxy, trifluoromethyloxy, 2,2,2-trifluoroethyloxy, 2,2,2-trichloroethyloxy, 2,2,2-trifluoroethyloxy, 2,2-difluoroethyloxy, 3,3,3-trifluoropropyloxy, 2,2,3,3,3-pentafluoropropyloxy, 2,2,3,3,4,4,4-heptafluorobutyloxy, and the like.

Preferred embodiments of "haloalkyloxy" include difluoromethyloxy, trifluoromethyloxy, 2,2,2-trifluoroethyloxy, 2,2-difluoroethyloxy, and 3,3,3-trifluoropropyloxy. More preferred embodiments includes difluoromethyloxy, trifluoromethyloxy, and 2,2,2-trifluoroethyloxy.

The term "alkenyl" includes a C2 to C15, preferably a C2 to C10, more preferably a C2 to C6 and further preferably a C2 to C4 linear or branched hydrocarbon group having at least one double bond at any position. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, and pentadecenyl.

Preferred embodiments of "alkenyl" include vinyl, allyl, propenyl, isopropenyl, and butenyl. More preferred embodiments include vinyl and n-propenyl.

The term "alkynyl" includes a C2 to C10, preferably a C2 to C8, more preferably a C2 to C6 and further preferably a C2 to C4 linear or branched hydrocarbon group having at least one triple bond at any position. Furthermore, it may have double bond(s) at any position(s). Examples include ethynyl, propynyl, butynyl, pentynyl, hexynyl, heptynyl, octynyl, nonynyl, and decynyl.

Preferred embodiments of "alkynyl" include ethynyl, propynyl, butynyl, and pentynyl. More preferred embodiments include ethynyl and propynyl.

The term "Aromatic carbocyclyl" means a cyclic aromatic hydrocarbon group having a single ring or two or more rings. Examples include phenyl, naphthyl, anthryl, and phenanthryl. Preferred embodiments of the "aromatic carbocyclyl" include phenyl.

Examples of 6-membered aromatic carbocyclyl include phenyl.

The term "aromatic carbocycle" means a ring derived from the above "aromatic carbocyclyl".

Examples of "aromatic carbocycle formed by $R^2$ and $R^3$ together with a carbon atom to which $R^2$ and $R^3$ are bonded" include the following rings.

[Chemical formula 9]

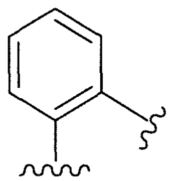

The term "non-aromatic carbocyclyl" means a cyclic saturated hydrocarbon group or a cyclic non-aromatic unsaturated hydrocarbon group, which is monocyclic or polycyclic having two or more rings. The "non-aromatic carbocyclyl" which is polycyclic having two or more rings includes a fused ring group wherein a non-aromatic carbocyclyl, which is monocyclic or polycyclic having two or more rings, is fused with a ring of the above "aromatic carbocyclyl".

In addition, examples of the "non-aromatic carbocyclyl" also include a group having a bridge or a group to form a spiro ring as follows:

[Chemical formula 10]

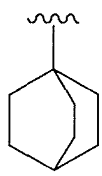
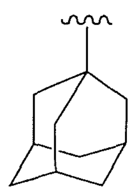
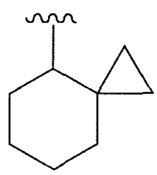

The non-aromatic carbocyclyl which is monocyclic is preferably C3 to C16, more preferably C3 to C12 and further preferably C4 to C8 carbocyclyl. Examples include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, and cyclohexadienyl.

The non-aromatic carbocyclyl which is polycyclic having two or more rings is preferably C8 to C20, more preferably C8 to C16 carbocyclyl. Examples include indanyl, indenyl, acenaphthyl, tetrahydronaphthyl, and fluorenyl.

The term "non-aromatic carbocycle" means a ring derived from the above "non-aromatic carbocyclyl".

Examples of "non-aromatic carbocycle formed by $R^2$ and $R^3$ together with a carbon atom to which $R^2$ and $R^3$ are bonded" include the following rings.

[Chemical formula 11]

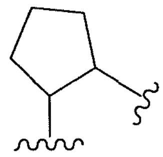 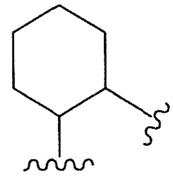

Examples of "non-aromatic carbocycle formed by $R^{15}$ and $R^{16}$ together with a carbon atom to which $R^{15}$ and $R^{16}$ are bonded" include the following rings.

[Chemical formula 12]

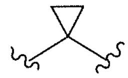 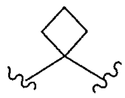

Examples of "non-aromatic carbocycle formed by $R^{17}$ and $R^{18}$ together with a carbon atom to which $R^{17}$ and $R^{18}$ are bonded" include the following rings.

[Chemical formula 13]

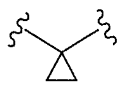 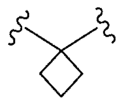

The term "aromatic heterocyclyl" means an aromatic cyclyl having a single ring or two or more rings, which has at least one identical or different heteroatom optionally selected from O, S, and N in the ring(s).

The aromatic heterocyclyl having two or more rings also includes an aromatic heterocyclyl having a single ring or two or more rings, to which a ring in the "aromatic carbocyclyl" is fused, and the bond may be held in any ring.

The aromatic heterocyclyl having a single ring is preferably a 5- to 8-membered ring, and more preferably a 5-membered or 6-membered ring. Examples of 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl, and thiadiazolyl. Examples of the 6-membered aromatic heterocyclyl include pyridyl, pyridazinyl, pyrimidinyl, pyrazinyl, triazinyl.

The bicyclic aromatic heterocyclyl is preferably an 8- to 10-membered ring, and more preferably a 9- or 10-membered ring.

Examples include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, and thiazolopyridyl.

Examples of 9-membered aromatic heterocyclyl include indolyl, isoindolyl, indazolyl, indolizinyl, purinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, oxazolopyridyl, and thiazolopyridyl.

Examples of 10-membered aromatic heterocyclyl include quinolinyl, isoquinolinyl, cinnolinyl, phthalazinyl, quinazolinyl, naphthyridinyl, quinoxalinyl, pteridinyl, and pyrazinopyridazinyl.

The aromatic heterocyclyl, which is polycyclic having three or more rings, is preferably a 13- to 15-membered ring. Examples of aromatic heterocyclyl, which is polycyclic having three or more rings, include carbazolyl, acridinyl, xanthenyl, phenothiazinyl, phenoxathiinyl, phenoxazinyl, and dibenzofuryl.

The term "aromatic heterocycle" means a ring derived from the above "aromatic heterocyclyl".

Examples of "aromatic heterocycle formed by $R^2$ and $R^3$ together with a carbon atom to which $R^2$ and $R^3$ are bonded" include the following rings.

[Chemical formula 14]

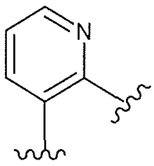 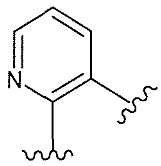 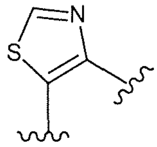 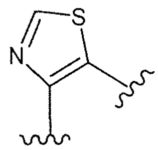 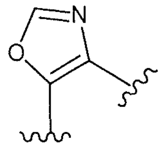 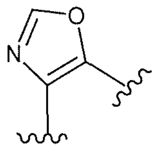

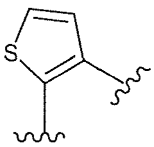 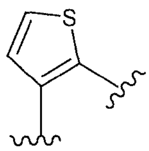 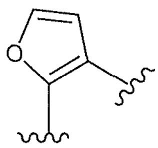 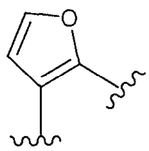 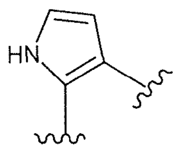 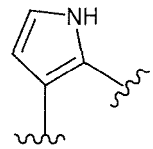

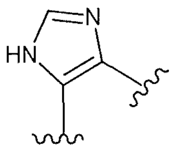 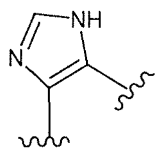 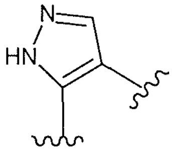 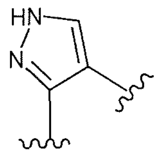 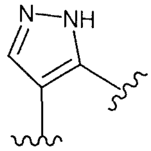 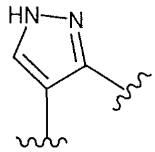

The term "non-aromatic heterocyclyl" means a non-aromatic cyclyl having a single ring or two or more rings, which has at least one identical or different heteroatom optionally selected from O, S, and N in the ring(s). The "non-aromatic heterocyclyl", which is polycyclic having two or more rings, includes a non-aromatic heterocyclyl, which is monocyclic or polycyclic having two or more rings, fused with a ring of the above "aromatic carbocyclyl", "non-aromatic carbocyclyl" and/or "aromatic heterocyclyl", and further includes a non-aromatic carbocyclyl, which is monocyclic or polycyclic having two or more rings, fused with a ring of the above "aromatic heterocyclyl", the bond may be held in any ring.

In addition, examples of the "non-aromatic heterocyclyl" also include a group having a bridge or a group to form a spiro ring as follows:

[Chemical formula 15]

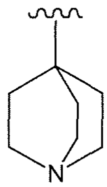 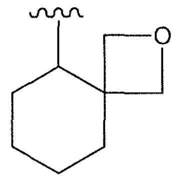

The non-aromatic heterocyclyl, which is monocyclic, is preferably a 3- to 8-membered and more preferably a 5- to 6-membered ring.

Examples of the 3-membered non-aromatic heterocyclyl include thiiranyl, oxiranyl and aziridinyl. Examples of the 4-membered non-aromatic heterocyclyl include oxetanyl and azetidinyl. Examples of the 5-membered non-aromatic heterocyclyl include oxathiolanyl, thiazolidinyl, pyrrolidinyl, pyrrolinyl, imidazolidinyl, imidazolinyl, pyrazolidinyl, pyrazolinyl, tetrahydrofuryl, dihydrothiazolyl, tetrahydroisothiazolyl, dioxolanyl, dioxolyl, and thiolanyl. Examples of the 6-membered non-aromatic heterocyclyl include dioxanyl, thianyl, piperidyl, piperazinyl, morpholinyl, morpholino, thiomorpholinyl, thiomorpholino, dihydropyridyl, tetrahydropyridyl, tetrahydropyranyl, dihydrooxazinyl, tetrahydropyridazinyl, hexahydropyrimidinyl, dioxazinyl, thiinyl, and thiazinyl. Examples of the 7-membered non-aromatic heterocyclyl include hexahydroazepinyl, tetrahydrodiazepinyl, and oxepanyl. Examples of the 8-membered non-aromatic heterocyclyl include azocane, thiocane and oxocane.

The non-aromatic heterocyclyl, which is polycyclic having two or more rings, is preferably an 8- to 20-membered and more preferably an 8- to 10- membered ring. Examples of non-aromatic heterocyclyl, which is polycyclic having two or more rings, include indolinyl, isoindolinyl, chromanyl, and isochromanyl.

The bicyclic 9-membered non-aromatic heterocyclyl includes the groups shown below.

[Chemical formula 16]

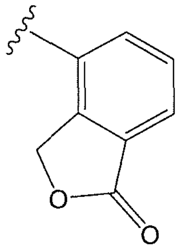 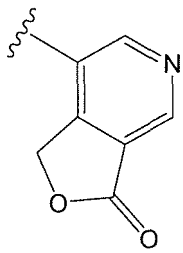 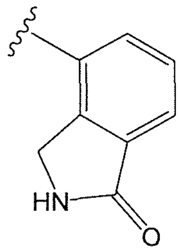 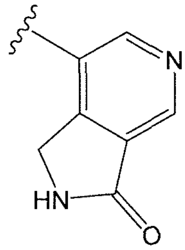

The bicyclic 10-membered non-aromatic heterocyclyl includes the groups shown below.

[Chemical formula 17]

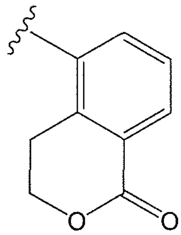 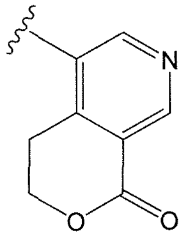 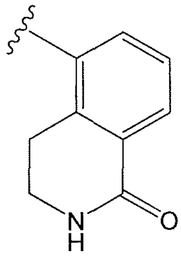 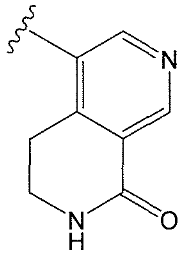

The term "non-aromatic heterocycle" means a ring derived from the above "non-aromatic heterocyclyl".

Examples of "non-aromatic heterocycle formed by $R^2$ and $R^3$ together with a carbon atom to which $R^2$ and $R^3$ are bonded" include the following rings.

[Chemical formula 18]

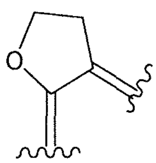 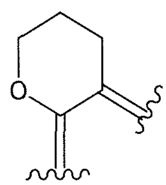

The term "trialkylsilyl" means a group in which the above three "alkyls" are bound to a silicon atom. The three alkyls may be the same or different. Examples include trimethylsilyl, triethylsilyl and tert-butyldimethylsilyl.

In the present description, the phrase "is optionally substituted with substituent group $\alpha$" means that "is optionally substituted with at least one group selected from substituent group $\alpha$". The same also applies to substituent groups $\beta$, $\gamma$, and $\gamma'$.

Substituents for "substituted alkyl", "substituted alkenyl", "substituted alkynyl", "substituted alkyloxy", "substituted alkenyloxy", "substituted alkynyloxy", "substituted alkylcarbonyloxy", "substituted alkenylcarbonyloxy", "substituted alkynylcarbonyloxy", "substituted alkylcarbonyl", "substituted alkenylcarbonyl", "substituted alkynylcarbonyl", "substituted alkyloxycarbonyl", "substituted alkenyloxycarbonyl", "substituted alkynyloxycarbonyl", "substituted alkylsulfanyl", "substituted alkenylsulfanyl", "substituted alkynylsulfanyl", "substituted alkylsulfinyl", "substituted alkenylsulfinyl", "substituted alkynylsulfinyl", "substituted alkylsulfonyl", "substituted alkenylsulfonyl", "substituted alkynylsulfonyl", and the like include the following substituent group A. A carbon atom at any position may be bonded to at least one group selected from the following substituent group A.

Substituent group A: halogen, hydroxy, carboxy, formyl, formyloxy, sulfanyl, sulfino, sulfo, thioformyl, thiocarboxy, dithiocarboxy, thiocarbamoyl, cyano, nitro, nitroso, azide, hydrazino, ureide, amidino, guanidino, pentafluorothio, trialkylsilyl,

alkyloxy optionally substituted with substituent group $\alpha$, alkenyloxy optionally substituted with substituent group $\alpha$, alkynyloxy optionally substituted with substituent group $\alpha$, alkylcarbonyloxy optionally substituted with substituent

group α, alkenylcarbonyloxy optionally substituted with substituent group α, alkynylcarbonyloxy optionally substituted with substituent group α, alkylcarbonyl optionally substituted with substituent group α, alkenylcarbonyl optionally substituted with substituent group α, alkynylcarbonyl optionally substituted with substituent group α, alkyloxycarbonyl optionally substituted with substituent group α,

alkenyloxycarbonyl optionally substituted with substituent group α, alkynyloxycarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α,

alkynylsulfinyl optionally substituted with substituent group α, alkylsulfonyl optionally substituted with substituent group α, alkenylsulfonyl optionally substituted with substituent group α, alkynylsulfonyl optionally substituted with substituent group α,

amino optionally substituted with substituent group β, imino optionally substituted with substituent group β, carbamoyl optionally substituted with substituent group β, sulfamoyl optionally substituted with substituent group β,

aromatic carbocyclyl optionally substituted with substituent group γ, non-aromatic carbocyclyl optionally substituted with substituent group γ', aromatic heterocyclyl optionally substituted with substituent group γ, non-aromatic heterocyclyl optionally substituted with substituent group γ', aromatic carbocyclyloxy optionally substituted with substituent group γ, non-aromatic carbocyclyloxy optionally substituted with substituent group γ', aromatic heterocyclyloxy optionally substituted with substituent group γ, non-aromatic heterocyclyloxy optionally substituted with substituent group γ', aromatic carbocyclylcarbonyloxy optionally substituted with substituent group γ, non-aromatic carbocyclylcarbonyloxy optionally substituted with substituent group γ', aromatic heterocyclylcarbonyloxy optionally substituted with substituent group γ, non-aromatic heterocyclylcarbonyloxy optionally substituted with substituent group γ', aromatic carbocyclylcarbonyl optionally substituted with substituent group γ, non-aromatic carbocyclylcarbonyl optionally substituted with substituent group γ', aromatic heterocyclylcarbonyl optionally substituted with substituent group γ, non-aromatic heterocyclylcarbonyl optionally substituted with substituent group γ', aromatic carbocyclyloxycarbonyl optionally substituted with substituent group γ, non-aromatic carbocyclyloxycarbonyl optionally substituted with substituent group γ', aromatic heterocyclyloxycarbonyl optionally substituted with substituent group γ, non-aromatic heterocyclyloxycarbonyl optionally substituted with substituent group γ', aromatic carbocyclylalkyloxy optionally substituted with substituent group γ, non-aromatic carbocyclylalkyloxy optionally substituted with substituent group γ', aromatic heterocyclylalkyloxy optionally substituted with substituent group γ, non-aromatic heterocyclylalkyloxy optionally substituted with substituent group γ', aromatic carbocyclylalkyloxycarbonyl optionally substituted with substituent group γ, non-aromatic carbocyclylalkyloxycarbonyl optionally substituted with substituent group γ', aromatic heterocyclylalkyloxycarbonyl optionally substituted with substituent group γ, non-aromatic heterocyclylalkyloxycarbonyl optionally substituted with substituent group γ', aromatic carbocyclylsulfanyl optionally substituted with substituent group γ, non-aromatic carbocyclylsulfanyl optionally substituted with substituent group γ', aromatic heterocyclylsulfanyl optionally substituted with substituent group γ, non-aromatic heterocyclylsulfanyl optionally substituted with substituent group γ', aromatic carbocyclylsulfinyl optionally substituted with substituent group γ, non-aromatic carbocyclylsulfinyl optionally substituted with substituent group γ', aromatic heterocyclylsulfinyl optionally substituted with substituent group γ, non-aromatic heterocyclylsulfinyl optionally substituted with substituent group γ', aromatic carbocyclylsulfonyl optionally substituted with substituent group γ, non-aromatic carbocyclylsulfonyl optionally substituted with substituent group γ', aromatic heterocyclylsulfonyl optionally substituted with substituent group γ, and non-aromatic heterocyclylsulfonyl optionally substituted with substituent group γ'.

Substituent group α: halogen, hydroxy, carboxy, alkyloxy, alkyloxy substituted with alkyloxy, haloalkyloxy, alkenyloxy, alkynyloxy, sulfanyl, and cyano.

Substituent group β: halogen, hydroxy, carboxy, cyano, alkyl optionally substituted with substituent group α, alkenyl optionally substituted with substituent group α, alkynyl optionally substituted with substituent group α, alkylcarbonyl optionally substituted with substituent group α, alkenylcarbonyl optionally substituted with substituent group α, alkynylcarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α, alkynylsulfinyl optionally substituted with substituent group α, alkylsulfonyl optionally substituted with substituent group α, alkenylsulfonyl optionally substituted with substituent group α, alkynylsulfonyl optionally substituted with substituent group α,

aromatic carbocyclyl optionally substituted with substituent group γ, non-aromatic carbocyclyl optionally substituted with substituent group γ', aromatic heterocyclyl optionally substituted with substituent group γ, non-aromatic heterocyclyl optionally substituted with substituent group γ', aromatic carbocyclylalkyl optionally substituted with substituent group γ, non-aromatic carbocyclylalkyl optionally substituted with substituent group γ', aromatic heterocyclylalkyl optionally substituted with substituent group γ, non-aromatic heterocyclylalkyl optionally substituted with substituent group γ',

aromatic carbocyclylcarbonyl optionally substituted with substituent group γ, non-aromatic carbocyclylcarbonyl optionally substituted with substituent group γ', aromatic heterocyclylcarbonyl optionally substituted with substituent group γ, non-aromatic heterocyclylcarbonyl optionally substituted with substituent group γ', aromatic carbocyclyloxycarbonyl optionally substituted with substituent group γ, non-aromatic carbocyclyloxycarbonyl optionally substituted with substituent group γ', aromatic heterocyclyloxycarbonyl optionally substituted with substituent group γ, non-aromatic heterocyclyloxycarbonyl optionally substituted with substituent group γ', aromatic carbocyclylsulfanyl optionally substituted with substituent group γ, non-aromatic carbocyclylsulfanyl optionally substituted with substituent group γ', aromatic heterocyclylsulfanyl optionally substituted with substituent group γ, non-aromatic heterocyclylsulfanyl optionally substituted with substituent group γ', aromatic carbocyclylsulfinyl optionally substituted with substituent group γ, non-aromatic carbocyclylsulfinyl optionally substituted with substituent group γ', aromatic heterocyclylsulfinyl optionally substituted with substituent group γ, non-aromatic heterocyclylsulfinyl optionally substituted with substituent group γ', aromatic carbocyclylsulfonyl optionally substituted with substituent group γ, non-aromatic carbocyclylsulfonyl optionally substituted with substituent group γ', aromatic heterocyclylsulfonyl optionally substituted with substituent group γ, and non-aromatic heterocyclylsulfonyl optionally substituted with substituent group γ'.

Substituent group γ: substituent group α, alkyl, alkyl substituted with alkyloxy, haloalkyl, hydroxyalkyl, alkenyl, alkynyl, alkylcarbonyl, haloalkylcarbonyl, alkenylcarbonyl, and alkynylcarbonyl.

Substituent group γ': substituent group γ and oxo.

The substituents on the rings of "aromatic carbocycle" and "aromatic heterocycle"of "substituted aromatic carbocyclyl", "substituted aromatic heterocyclyl", "substituted aromatic carbocycle formed by $R^2$ and $R^3$ together with a carbon atom to which $R^2$ and $R^3$ are bonded", "substituted aromatic heterocycle formed by $R^2$ and $R^3$ together with a carbon atom to which $R^2$ and $R^3$ are bonded", "substituted aromatic carbocyclyloxy", "substituted aromatic heterocyclyloxy", "substituted aromatic carbocyclylcarbonyloxy", "substituted aromatic heterocyclylcarbonyloxy", "substituted aromatic carbocyclylcarbonyl", "substituted aromatic heterocyclylcarbonyl", "substituted aromatic carbocyclyloxycarbonyl", "substituted aromatic heterocyclyloxycarbonyl", "substituted aromatic carbocyclylsulfanyl", "substituted aromatic heterocyclylsulfanyl", "substituted aromatic carbocyclylsulfinyl", "substituted aromatic heterocyclylsulfinyl", "substituted aromatic carbocyclylsulfonyl", and "substituted aromatic heterocyclylsulfonyl" include the following substituent group B. An atom at any position on the ring may be bonded to at least one group selected from the following substituent group B.

Substituent Group B: halogen, hydroxy, carboxy, formyl, formyloxy, sulfanyl, sulfino, sulfo, thioformyl, thiocarboxy, dithiocarboxy, thiocarbamoyl, cyano, nitro, nitroso, azide, hydrazino, ureide, amidino, guanidino, pentafluorothio, trialkylsilyl,

alkyl optionally substituted with substituent group α, alkenyl optionally substituted with substituent group α, alkynyl optionally substituted with substituent group α, alkyloxy optionally substituted with substituent group α, alkenyloxy optionally substituted with substituent group α, alkynyloxy optionally substituted with substituent group α,

alkylcarbonyloxy optionally substituted with substituent group α, alkenylcarbonyloxy optionally substituted with substituent group α, alkynylcarbonyloxy optionally substituted with substituent group α, alkylcarbonyl optionally substituted with substituent group α, alkenylcarbonyl optionally substituted with substituent group α, alkynylcarbonyl optionally substituted with substituent group α, alkyloxycarbonyl optionally substituted with substituent group α, alkenyloxycarbonyl optionally substituted with substituent group α, alkynyloxycarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α,

alkynylsulfinyl optionally substituted with substituent group α, alkylsulfonyl optionally substituted with substituent group α, alkenylsulfonyl optionally substituted with substituent group α, alkynylsulfonyl optionally substituted with substituent group α,

amino optionally substituted with substituent group β, imino optionally substituted with substituent group β, carbamoyl optionally substituted with substituent group β, sulfamoyl optionally substituted with substituent group β,

aromatic carbocyclyl optionally substituted with substituent group γ, non-aromatic carbocyclyl optionally substituted with substituent group γ', aromatic heterocyclyl optionally substituted with substituent group γ, non-aromatic heterocyclyl optionally substituted with substituent group γ', aromatic carbocyclyloxy optionally substituted with substituent group γ, non-aromatic carbocyclyloxy optionally substituted with substituent group γ', aromatic heterocyclyloxy optionally substituted with substituent group γ, non-aromatic heterocyclyloxy optionally substituted with the substituent group γ', aromatic carbocyclylcarbonyloxy optionally substituted with substituent group γ, non-aromatic carbocyclylcarbonyloxy optionally substituted with substituent group γ', aromatic heterocyclylcarbonyloxy optionally substituted with substituent group γ, and non-aromatic heterocyclylcarbonyloxy optionally substituted with substituent group γ', aromatic carbocyclylcarbonyl optionally substituted with substituent group γ, non-aromatic carbocyclylcarbonyl optionally substituted with substituent group γ', aromatic heterocyclylcarbonyl optionally substituted with substituent group γ, non-aromatic heterocyclylcarbonyl optionally substituted with substituent group γ', aromatic

carbocyclyloxycarbonyl optionally substituted with substituent group γ, non-aromatic carbocyclyloxycarbonyl optionally substituted with substituent group γ', aromatic heterocyclyloxycarbonyl optionally substituted with substituent group γ, non-aromatic heterocyclyloxycarbonyl optionally substituted with the substituent group γ', aromatic carbocyclyl alkyl optionally substituted with substituent group γ, non-aromatic carbocyclyl alkyl optionally substituted with substituent group γ', aromatic heterocyclyl alkyl optionally substituted with substituent group γ, non-aromatic heterocyclyl alkyl optionally substituted with substituent group γ', aromatic carbocyclyl alkyloxy optionally substituted with substituent group γ, non-aromatic carbocyclyl alkyloxy optionally substituted with substituent group γ', aromatic heterocyclyl alkyloxy optionally substituted with substituent group γ, non-aromatic heterocyclyl alkyloxy optionally substituted with substituent group γ', aromatic carbocyclyloxy alkyl optionally substituted with substituent group γ, non-aromatic carbocyclyloxy alkyl optionally substituted with substituent group γ', aromatic heterocyclyloxy alkyl optionally substituted with substituent group γ, non-aromatic heterocyclyloxy alkyl optionally substituted with the substituent group γ', aromatic carbocyclylalkyloxycarbonyl optionally substituted with substituent group γ, non-aromatic carbocyclylalkyloxycarbonyl optionally substituted with substituent group γ', aromatic heterocyclylalkyloxycarbonyl optionally substituted with substituent group γ, non-aromatic heterocyclylalkyloxycarbonyl optionally substituted with substituent group γ', aromatic carbocyclylalkyloxyalkyl optionally substituted with substituent group γ, non-aromatic carbocyclylalkyloxyalkyl optionally substituted with substituent group γ', aromatic heterocyclylalkyloxyalkyl optionally substituted with substituent group γ, non-aromatic heterocyclylalkyloxyalkyl optionally substituted with substituent group γ', aromatic carbocyclylsulfanyl optionally substituted with substituent group γ, non-aromatic carbocyclylsulfanyl optionally substituted with substituent group γ', aromatic heterocyclylsulfanyl optionally substituted with substituent group γ, non-aromatic heterocyclylsulfanyl optionally substituted with substituent group γ', aromatic carbocyclylsulfinyl optionally substituted with substituent group γ, non-aromatic carbocyclylsulfinyl optionally substituted with substituent group γ', aromatic heterocyclylsulfinyl optionally substituted with substituent group γ, non-aromatic heterocyclylsulfinyl optionally substituted with substituent group γ', aromatic carbocyclylsulfonyl optionally substituted with substituent group γ, non-aromatic carbocyclylsulfonyl optionally substituted with substituent group γ', aromatic heterocyclylsulfonyl optionally substituted with substituent group γ, and non-aromatic heterocyclylsulfonyl optionally substituted with substituent group γ'.

The substituents on the rings of "Non-aromatic carbocycles" and "non-aromatic heterocycles" of "substituted non-aromatic carbocyclyl", "substituted non-aromatic heterocyclyl", "substituted non-aromatic carbocycle formed by $R^2$ and $R^3$ together with a carbon atom to which $R^2$ and $R^3$ are bonded", "substituted non-aromatic heterocycle formed by $R^2$ and $R^3$ together with a carbon atom to which $R^2$ and $R^3$ are bonded", "substituted aromatic carbocycle formed by $R^2$ and $R^3$ together with a carbon atom to which $R^2$ and $R^3$ are bonded", "substituted aromatic heterocycle formed by $R^2$ and $R^3$ together with a carbon atom to which $R^2$ and $R^3$ are bonded", "substituted non-aromatic carbocycle formed by $R^{15}$ and $R^{16}$ together with a carbon atom to which $R^{15}$ and $R^{16}$ are bonded", "substituted non-aromatic carbocycle formed by $R^{17}$ and $R^{18}$ together with a carbon atom to which $R^{17}$ and $R^{18}$ are bonded", "substituted non-aromatic carbocyclyloxy", "substituted non-aromatic heterocyclyloxy", "substituted non-aromatic carbocyclylcarbonyloxy", "substituted non-aromatic heterocyclylcarbonyloxy", "substituted non-aromatic carbocyclylcarbonyl", "substituted non-aromatic heterocyclylcarbonyl", "substituted non-aromatic carbocyclyloxycarbonyl", "substituted non-aromatic heterocyclyloxycarbonyl", "substituted non-aromatic carbocyclylsulfanyl", "substituted non-aromatic heterocyclylsulfanyl", "substituted non-aromatic carbocyclylsulfinyl", "substituted non-aromatic heterocyclylsulfinyl", "substituted non-aromatic carbocyclylsulfonyl", and "substituted non-aromatic heterocyclylsulfonyl" include the following substituent group C. An atom at any position on the ring may be bonded to at least one group selected from the following substituent group C.

Substituent Group C: substituent group B and oxo.

When the "non-aromatic carbocycle" and the "non-aromatic heterocycle" are substituted with "oxo", this means a ring in which two hydrogen atoms on a carbon atom are substituted as follows.

[Chemical formula 19]

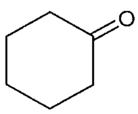 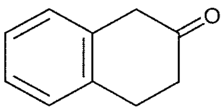 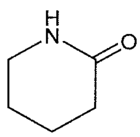 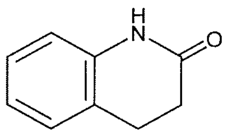

The substituents for "substituted amino", "substituted imino", "substituted carbamoyl", and "substituted sulfamoyl" include the following substituent group D. These may be optionally substituted with one or two group(s) selected from substituent group D.

Substituent Group D: halogen, hydroxy, carboxy, cyano, alkyl optionally substituted with substituent group α,

alkenyl optionally substituted with substituent group α, alkynyl optionally substituted with substituent group α, alkylcarbonyl optionally substituted with substituent group α,

alkenylcarbonyl optionally substituted with substituent group α, alkynylcarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α, alkynylsulfinyl optionally substituted with substituent group α, alkylsulfonyl optionally substituted with substituent group α,
alkenylsulfonyl optionally substituted with substituent group α, alkynylsulfonyl optionally substituted with substituent group α,
amino optionally substituted with substituent group β, imino optionally substituted with substituent group β, carbamoyl optionally substituted with substituent group β, sulfamoyl optionally substituted with substituent group β,
aromatic carbocyclyl optionally substituted with substituent group γ, non-aromatic carbocyclyl optionally substituted with substituent group γ', aromatic heterocyclyl optionally substituted with substituent group γ, non-aromatic heterocyclyl optionally substituted with substituent group γ', aromatic carbocyclylalkyl optionally substituted with substituent group γ, non-aromatic carbocyclylalkyl optionally substituted with substituent group γ', aromatic heterocyclylalkyl optionally substituted with substituent group γ, non-aromatic heterocyclylalkyl optionally substituted with substituent group γ', aromatic carbocyclylcarbonyl optionally substituted with substituent group γ, non-aromatic carbocyclylcarbonyl optionally substituted with substituent group γ', aromatic heterocyclylcarbonyl optionally substituted with substituent group γ, non-aromatic heterocyclylcarbonyl optionally substituted with substituent group γ', aromatic carbocyclyloxycarbonyl optionally substituted with substituent group γ, non-aromatic carbocyclyloxycarbonyl optionally substituted with substituent group γ', aromatic heterocyclyloxycarbonyl optionally substituted with substituent group γ, non-aromatic heterocyclyloxycarbonyl optionally substituted with substituent group γ', aromatic carbocyclylsulfanyl optionally substituted with substituent group γ, non-aromatic carbocyclylsulfanyl optionally substituted with substituent group γ', aromatic heterocyclylsulfanyl optionally substituted with substituent group γ, non-aromatic heterocyclylsulfanyl optionally substituted with substituent group γ', aromatic carbocyclylsulfinyl optionally substituted with substituent group γ, non-aromatic carbocyclylsulfinyl optionally substituted with substituent group γ', aromatic heterocyclylsulfinyl optionally substituted with substituent group γ, non-aromatic heterocyclylsulfinyl optionally substituted with substituent group γ', aromatic carbocyclylsulfonyl optionally substituted with substituent group γ, non-aromatic carbocyclylsulfonyl optionally substituted with substituent group γ', aromatic heterocyclylsulfonyl optionally substituted with substituent group γ, and non-aromatic heterocyclylsulfonyl optionally substituted with substituent group γ'.

Examples of the substituents on the rings of "substituted 6-membered aromatic heterocyclyl", "substituted 5-membered aromatic heterocyclyl", "substituted 6-membered aromatic carbocyclyl", "substituted bicyclic 9-membered non-aromatic heterocyclyl", and "substituted bicyclic 10-membered non-aromatic heterocyclyl" in $R^1$ include alkyl, haloalkyl, alkyl substituted with alkyloxy, alkyl substituted with haloalkyloxy, alkyl substituted with alkyloxy substituted with alkyloxy, hydroxyalkyl, carbamoyl substituted with alkyl, alkyl substituted with non-aromatic heterocyclyloxy, alkyl substituted with non-aromatic carbocyclyloxy, alkyl substituted with aromatic heterocyclyl, alkyl substituted with aromatic heterocyclyl substituted with alkyl, alkyl substituted with aromatic carbocyclyl, alkyl substituted with aromatic carbocyclyl substituted with alkyl, alkyloxy, haloalkyloxy, alkyloxy substituted with alkyloxy, haloalkyloxy substituted with alkyloxy, alkyloxy substituted with non-aromatic heterocyclyl, alkyloxy substituted with non-aromatic carbocyclyl, non-aromatic heterocyclyloxy, non-aromatic carbocyclyloxy, non-aromatic heterocyclyl, non-aromatic carbocyclyl, halogen, cyano, alkenyl, and alkynyl. An atom at any position on the ring is optionally substituted with at least one group selected from these.
Examples of the substituents of "substituted alkyl", "substituted alkyloxy", "substituted alkenyl", and "substituted alkynyl" in $R^6$ and $R^7$ include halogen.
Examples of the substituents on the rings of "substituted non-aromatic carbocyclyl" in $R^6$ and $R^7$ include halogen.
Examples of the substituents of "substituted alkyl", "substituted alkyloxy", and "substituted alkyloxycarbonyl" in $R^8$ and $R^9$ include halogen, alkyloxy, alkyloxy substituted with alkyloxy, haloalkyloxy, hydroxy, non-aromatic heterocyclyloxy, non-aromatic carbocyclyloxy, aromatic heterocyclyl, aromatic heterocyclyl substituted with alkyl, aromatic carbocyclyl, aromatic carbocyclyl substituted with alkyl, non-aromatic heterocyclyl, and non-aromatic carbocyclyl. A carbon atom at any position is optionally substituted with at least one group selected from these.
Examples of the substituents of "substituted carbamoyl" in $R^8$ and $R^9$ include alkyl and non-aromatic carbocyclyl. A carbon atom at any position is optionally substituted with at least one group selected from these.
Examples of the substituents on the rings of "substituted non-aromatic carbocyclyloxy", "substituted non-aromatic heterocyclyloxy", "substituted non-aromatic carbocyclyl", and "substituted non-aromatic heterocyclyl" in $R^8$ and $R^9$ include alkyl, haloalkyl, alkyloxy, haloalkyloxy, and halogen. An atom at any position on the ring is optionally

substituted with at least one group selected from these.

Examples of the substituent of "substituted alkyl" in $R^{31}$ include halogen.

Examples of the substituent on the ring of "substituted non-aromatic carbocyclyl" in $R^{31}$ include halogen.

Examples of the substituents of "substituted alkyl" in $R^{32}$ and $R^{33}$ include halogen and hydroxy. A carbon atom at any position is optionally substituted with at least one group selected from these.

Examples of the substituents on the rings of "substituted non-aromatic carbocyclyl" in $R^{32}$ and $R^{33}$ include alkyl, haloalkyl, alkyloxy, haloalkyloxy, and halogen. An atom at any position on the ring is optionally substituted with at least one group selected from these.

Examples of the substituents of "substituted alkyl" and "substituted alkyloxy" in $R^2$, $R^3$, and $R^4$ include halogen.

Examples of the substituents of "substituted carbamoyl" in $R^2$, $R^3$, and $R^4$ include alkyl.

Examples of the substituents on the rings of "substituted aromatic carbocyclyl", "substituted non-aromatic carbocyclyl", "substituted aromatic heterocyclyl", "substituted non-aromatic heterocyclyl", "substituted aromatic carbocyclyloxy", "substituted non-aromatic carbocyclyloxy", "substituted aromatic heterocyclyloxy", and "substituted non-aromatic heterocyclyloxy" in $R^2$, $R^3$, and $R^4$ include halogen and alkyl. An atom at any position on the ring is optionally substituted with at least one group selected from these.

Examples of the substituents on the rings of "substituted aromatic carbocycle", "substituted non-aromatic carbocycle", "substituted aromatic heterocycle", and "substituted non-aromatic heterocycle" formed by $R^2$ and $R^3$ together with a carbon atom to which $R^2$ and $R^3$ are bondedinclude halogen.

Examples of the substituents on the rings of "substituted aromatic heterocyclyl", "substituted 6-membered aromatic heterocyclyl", "substituted aromatic carbocyclyl", "substituted phenyl", "substituted non-aromatic heterocyclyl", and "substituted non-aromatic carbocyclyl" in $R^5$ include alkyl, haloalkyl, alkyloxy, haloalkyloxy, alkyloxy substituted with aromatic carbocyclyl, alkyloxy substituted with non-aromatic carbocyclyl, aromatic carbocyclyloxy, aromatic carbocyclyloxy substituted with halogen, non-aromatic carbocyclyloxy, non-aromatic carbocyclyloxy substituted with halogen, aromatic carbocyclyl, aromatic carbocyclyl substituted with alkyl substituted with alkyloxy, aromatic carbocyclyl substituted with halogen, aromatic carbocyclyl substituted with alkyl, aromatic carbocyclyl substituted with haloalkyl, aromatic heterocyclyl substituted with alkyloxy, aromatic carbocyclyl substituted with haloalkyloxy, aromatic carbocyclyl substituted with cyano, aromatic carbocyclyl substituted with cyano and halogen, aromatic carbocyclyl substituted with non-aromatic carbocyclyl, aromatic carbocycle substituted with aromatic carbocyclyl, non-aromatic carbocyclyl, non-aromatic carbocyclyl substituted with halogen, non-aromatic carbocyclyl substituted with alkyl, non-aromatic carbocyclyl substituted with haloalkyl, non-aromatic carbocyclyl substituted with haloalkyloxy, aromatic heterocyclyl, aromatic heterocyclyl substituted with alkyl substituted with alkyloxy, aromatic heterocyclyl substituted with halogen, aromatic heterocyclyl substituted with alkyl, aromatic heterocyclyl substituted with haloalkyl, aromatic heterocyclyl substituted with alkyloxy, aromatic heterocyclyl substituted with haloalkyloxy, aromatic heterocyclyl substituted with cyano, aromatic heterocyclyl substituted with cyano and halogen, aromatic heterocyclyl substituted with non-aromatic carbocyclyl, aromatic heterocyclyl substituted with aromatic heterocyclyl, halogen, cyano, alkyloxycarbonyl, and alkyl carbamoyl. An atom at any position on the ring is optionally substituted with at least one group selected from these.

Examples of the substituents of "substituted alkyloxy" and "substituted alkyl" in $R^{10}$ and $R^{13}$ include halogen.

Examples of the substituents of "substituted alkyl", "substituted alkyloxy", "substituted alkyloxycarbonyl", and "substituted alkylcarbonyl" in $R^{14}$ include halogen, non-aromatic carbocyclyl, and aromatic carbocyclyl. A carbon atom at any position is optionally substituted with at least one group selected from these.

Examples of the substituents on the rings of "substituted non-aromatic carbocyclyl", "substituted non-aromatic heterocyclyl", "substituted aromatic carbocyclyl", "substituted aromatic heterocyclyl", "substituted non-aromatic carbocyclyloxy", and "substituted aromatic carbocyclyloxy" in $R^{14}$ include alkyl, halogen, alkyloxy, haloalkyl, haloalkyloxy, and cyano. An atom at any position on the ring is optionally substituted with at least one group selected from these.

Examples of the substituents of "substituted alkyl" in $R^{15}$ and $R^{16}$ include halogen.

Examples of the substituent on the ring of "substituted non-aromatic carbocycle" formed by $R^{15}$ and $R^{16}$ together with a carbon atom to which $R^{15}$ and $R^{16}$ are bonded include halogen.

Examples of the substituents of "substituted alkyl" in $R^{17}$ and $R^{18}$ include halogen.

Examples of the substituent on the ring of "substituted non-aromatic carbocycle" formed by $R^{17}$ and $R^{18}$ together with a carbon atom to which $R^{17}$ and $R^{18}$ are bonded include halogen.

Preferred embodiments of $R^1$, $R^6$, $R^7$, $R^8$, $R^9$, $R^{31}$, $R^{32}$, $R^{33}$, $A^1$, $A^2$, $A^3$, $R^2$, $R^3$, $R^4$, $R^5$, $B^1$, $B^2$, $R^{10}$, $R^{11}$, $R^{12}$, $R^{13}$, $R^{14}$, $R^{15}$, $R^{16}$, $R^{17}$, and $R^{18}$ in the compound represented by Formula (I) are as follows. Regarding the compound represented by Formula (I), embodiments of all the combinations of specific examples shown below are mentioned as examples.

$R^1$ may be substituted or unsubstituted 6-membered aromatic heterocyclyl or substituted or unsubstituted 5-membered aromatic heterocyclyl (provided that substituted or unsubstituted tetrazolyl and substituted or unsubstituted tetrazolonyl are excluded), substituted or unsubstituted 6-membered aromatic carbocyclyl, substituted or unsubstituted bicyclic 9-membered non-aromatic aromatic heterocyclyl, or substituted or unsubstituted bicyclic 10-membered non-aromatic heterocyclyl (hereinafter, referred to as A-1).

$R^1$ may be substituted or unsubstituted 6-membered aromatic carbocyclyl, substituted or unsubstituted 6-membered aromatic heterocyclyl, or substituted or unsubstituted 5-membered aromatic heterocyclyl (provided that substituted or unsubstituted tetrazolyl and substituted or unsubstituted tetrazolonyl are excluded) (hereinafter, referred to as A-2).

$R^1$ may be a group represented by Formula:

[Chemical formula 20]

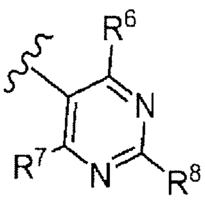 , 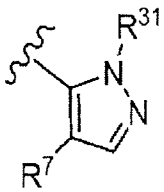 , 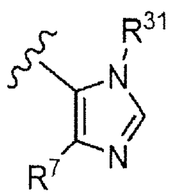 , 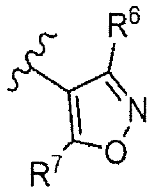 , 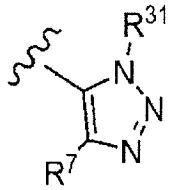 ,

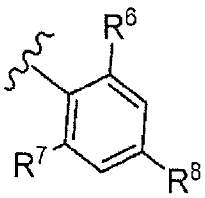 , 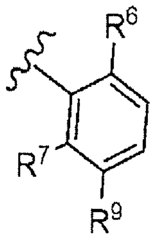 , 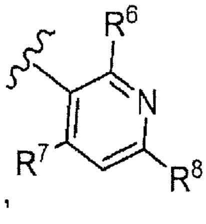 , 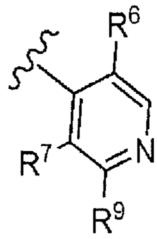 , 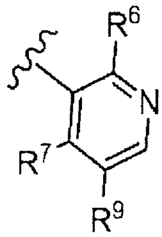 ,

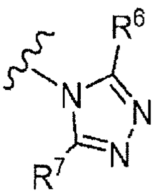 , 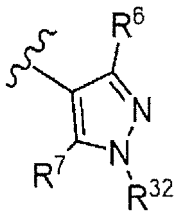 , 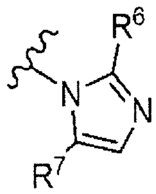 , 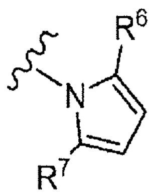 , 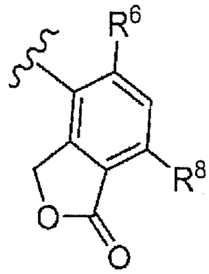 ,

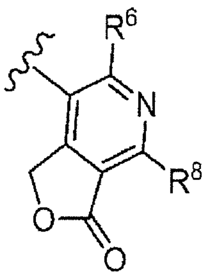 , 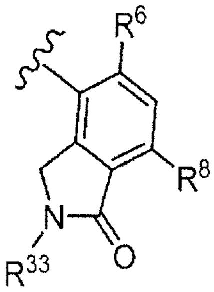 , 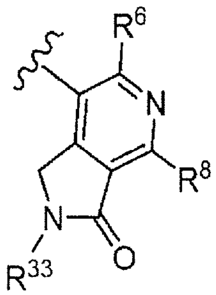 , 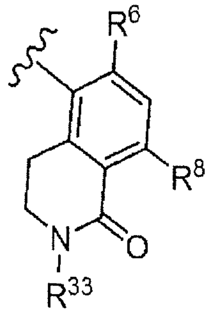

or 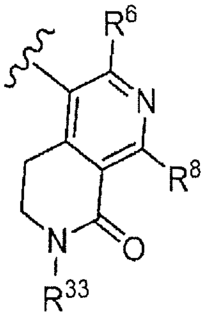

(hereinafter, referred to as A-3).

$R^1$ may be a group represented by Formula:

[Chemical formula 21]

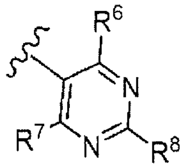 , 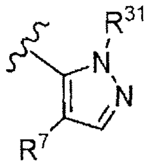 or 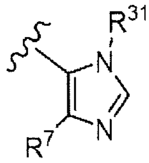

(hereinafter, referred to as A-4).

$R^1$ may be a group represented by Formula:

[Chemical formula 22]

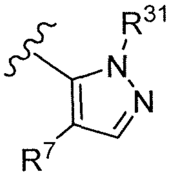

(hereinafter, referred to as A-5).

$R^1$ may be a group represented by Formula:

[Chemical formula 23]

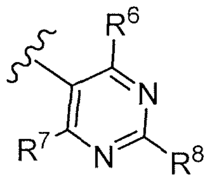

(hereinafter, referred to as A-6).

$R^1$ may be a group represented by Formula:

[Chemical formula 24]

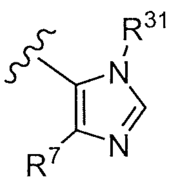

(hereinafter, referred to as A-7).

$R^6$ may be a hydrogen atom, halogen, substituted or unsubstituted alkyl, substituted or unsubstituted alkyloxy, substituted or unsubstituted alkenyl, substituted or unsubstituted alkynyl, cyano, or substituted or unsubstituted non-aromatic carbocyclyl (hereinafter, referred to as B-1).

$R^6$ may be halogen, substituted or unsubstituted alkyl, substituted or unsubstituted alkyloxy, substituted or unsubstituted alkenyl, substituted or unsubstituted alkynyl, or cyano (hereinafter, referred to as B-2).

$R^6$ may be substituted or unsubstituted alkyl or substituted or unsubstituted alkyloxy (hereinafter, referred to as B-3).

$R^6$ may be alkyl or alkyloxy (hereinafter, referred to as B-4). $R^6$ may be methyl or methyloxy (hereinafter, referred

to as B-5).

$R^6$ may be methyl (hereinafter, referred to as B-6).

$R^7$ may be a hydrogen atom, halogen, substituted or unsubstituted alkyl, substituted or unsubstituted alkyloxy, substituted or unsubstituted alkenyl, substituted or unsubstituted alkynyl, cyano, or substituted or unsubstituted non-aromatic carbocyclyl (provided that when $R^6$ is a hydrogen atom, $R^7$ is halogen, substituted or unsubstituted alkyl, substituted or unsubstituted alkyloxy, substituted or unsubstituted alkenyl, substituted or unsubstituted alkynyl, or cyano) (hereinafter, referred to as C-1).

$R^7$ may be halogen, substituted or unsubstituted alkyl, substituted or unsubstituted alkenyl, substituted or unsubstituted alkynyl, or cyano (hereinafter, referred to as C-2).

$R^7$ may be halogen, substituted or unsubstituted alkyl, or cyano (hereinafter, referred to as C-3).

$R^7$ may be halogen, alkyl, haloalkyl, or cyano (hereinafter, referred to as C-4).

$R^7$ may be halogen, alkyl, or cyano (hereinafter, referred to as C-5).

$R^7$ may be halogen, methyl, ethyl, isopropyl, or cyano (hereinafter, referred to as C-6).

$R^7$ may be halogen, methyl, or cyano (hereinafter, referred to as C-7).

$R^7$ may be halogen or alkyl (hereinafter, referred to as C-8). $R^7$ may be halogen or methyl (hereinafter, referred to as C-9). $R^7$ may be alkyl (hereinafter, referred to as C-10).

$R^7$ may be methyl (hereinafter, referred to as C-11).

$R^7$ may be halogen (hereinafter, referred to as C-12).

$R^8$ may be a hydrogen atom, halogen, substituted or unsubstituted alkyl, substituted or unsubstituted alkyloxy, substituted or unsubstituted alkyloxycarbonyl, substituted or unsubstituted carbamoyl, cyano, substituted or unsubstituted non-aromatic carbocyclyloxy, substituted or unsubstituted non-aromatic heterocyclyloxy, substituted or unsubstituted non-aromatic carbocyclyl, or substituted or unsubstituted non-aromatic heterocyclyl (hereinafter, referred to as D-1).

$R^8$ may be a hydrogen atom, substituted or unsubstituted alkyl, substituted or unsubstituted alkyloxy, substituted or unsubstituted non-aromatic carbocyclyloxy, substituted or unsubstituted non-aromatic heterocyclyloxy, substituted or unsubstituted non-aromatic carbocyclyl, or substituted or unsubstituted non-aromatic heterocyclyl (hereinafter, referred to as D-2).

$R^8$ may be a hydrogen atom or substituted or unsubstituted alkyl (hereinafter, referred to as D-3).

$R^8$ may be substituted or unsubstituted alkyl (hereinafter, referred to as D-4).

$R^8$ may be alkyl, alkyl substituted with alkyloxy, or alkyl substituted with non-aromatic heterocyclyloxy (hereinafter referred to as D-5).

$R^8$ may be methyl, methyl substituted with alkyloxy, or methyl substituted with non-aromatic heterocyclyloxy (hereinafter referred to as D-6).

$R^9$ may be a hydrogen atom, halogen, substituted or unsubstituted alkyl, substituted or unsubstituted alkyloxy, substituted or unsubstituted alkyloxycarbonyl, substituted or unsubstituted carbamoyl, cyano, substituted or unsubstituted non-aromatic carbocyclyloxy, substituted or unsubstituted non-aromatic heterocyclyloxy, substituted or unsubstituted non-aromatic carbocyclyl, or substituted or unsubstituted non-aromatic heterocyclyl (hereinafter, referred to as E-1).

$R^9$ may be a hydrogen atom, halogen, or substituted or unsubstituted alkyloxy (hereinafter, referred to as E-2).

$R^9$ may be a hydrogen atom, halogen, or alkyloxy (hereinafter, referred to as E-3).

$R^9$ may be a hydrogen atom or halogen (hereinafter, referred to as E-4).

$R^{31}$ may be substituted or unsubstituted alkyl or substituted or unsubstituted non-aromatic carbocyclyl (hereinafter, referred to as E'-1).

$R^{31}$ may be alkyl or non-aromatic carbocyclyl (hereinafter, referred to as E'-2).

$R^{31}$ may be methyl, ethyl, propyl, isopropyl, cyclopropyl, or cyclobutyl (hereinafter, referred to as E'-3).

$R^{31}$ may be methyl, ethyl, or cyclopropyl (hereinafter referred to as E'-4).

$R^{31}$ may be alkyl (hereinafter, referred to as E'-5).

$R^{31}$ may be methyl (hereinafter, referred to as E'-6).

$R^{32}$ may be a hydrogen atom, substituted or unsubstituted alkyl, or substituted or unsubstituted non-aromatic carbocyclyl (hereinafter, referred to as E"-1).

$R^{32}$ may be a hydrogen atom or substituted or unsubstituted alkyl (hereinafter, referred to as E"-2).

$R^{32}$ may be a hydrogen atom or alkyl (hereinafter, referred to as E"-3).

$R^{32}$ may be a hydrogen atom (hereinafter, referred to as E''-4). $R^{32}$ may be alkyl (hereinafter, referred to as E"-5).

$R^{33}$ may be a hydrogen atom, substituted or unsubstituted alkyl, or substituted or unsubstituted non-aromatic carbocyclyl (hereinafter, referred to as E‴-1).

$R^{33}$ may be a hydrogen atom or substituted or unsubstituted alkyl (hereinafter, referred to as E‴-2).

$R^{33}$ may be a hydrogen atom or alkyl (hereinafter, referred to as E‴-3).

$R^{33}$ may be a hydrogen atom (hereinafter, referred to as E‴-4).

$R^{33}$ may be alkyl (hereinafter, referred to as E‴-5).

$A^1$ may be $CR^2$ or N (hereinafter, referred to as F-1).

$A^1$ may be $CR^2$ (hereinafter, referred to as F-2).

$A^1$ may be N (hereinafter, referred to as F-3).

$R^2$ may be a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy (hereinafter, referred to as G-1).

$R^2$ may be a hydrogen atom, halogen, or substituted or unsubstituted alkyl (hereinafter, referred to as G-2).

$R^2$ may be a hydrogen atom, halogen, or alkyl (hereinafter, referred to as G-3).

$R^2$ may be a hydrogen atom or halogen (hereinafter, referred to as G-4).

$R^2$ may be a hydrogen atom (hereinafter, referred to as G-5).

$A^2$ may be $CR^3$ or N (hereinafter, referred to as H-1).

$A^2$ may be $CR^3$ (hereinafter, referred to as H-2).

$A^2$ may be N (hereinafter, referred to as H-3).

$R^3$ may be a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy (hereinafter, referred to as J-1).

$R^3$ may be a hydrogen atom, halogen, or substituted or unsubstituted alkyl (hereinafter, referred to as J-2).

$R^3$ may be halogen or substituted or unsubstituted alkyl (hereinafter, referred to as J-3).

$R^3$ may be halogen or alkyl (hereinafter, referred to as J-4). $R^3$ may be a hydrogen atom or halogen (hereinafter, referred to as J-5).

$R^3$ may be a hydrogen atom (hereinafter, referred to as J-6). $R^3$ may be halogen (hereinafter, referred to as J-7).

$R^2$ and $R^3$ may be taken together with a carbon atom to which $R^2$ and $R^3$ are bonded to form a substituted or unsubstituted aromatic carbocycle, a substituted or unsubstituted non-aromatic carbocycle, a substituted or unsubstituted aromatic heterocycle, or a substituted or unsubstituted non-aromatic heterocycle (hereinafter, referred to as J-8).

$R^2$ and $R^3$ may be taken together with a carbon atom to which $R^2$ and $R^3$ are bonded to form an aromatic carbocycle, non-aromatic carbocycle, aromatic heterocycle, or non-aromatic heterocycle (hereinafter, referred to as J-9).

$A^3$ may be $CR^4$ or N (hereinafter, referred to as K-1).

$A^3$ may be $CR^4$ (hereinafter, referred to as K-2).

$A^3$ may be N (hereinafter, referred to as K-3).

$R^4$ may be a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy (hereinafter, referred to as L-1).

$R^4$ may be a hydrogen atom, halogen, cyano, substituted or unsubstituted alkyl, or substituted or unsubstituted alkyloxy (hereinafter, referred to as L-2).

$R^4$ may be a hydrogen atom, halogen, cyano, alkyl, or alkyloxy (hereinafter, referred to as L-3).

$R^4$ may be a hydrogen atom, halogen, or cyano (hereinafter, referred to as L-4).

$R^4$ may be a hydrogen atom (hereinafter, referred to as L-5).

$R^5$ may be substituted or unsubstituted aromatic heterocyclyl, substituted or unsubstituted non-aromatic heterocyclyl, substituted or unsubstituted aromatic carbocyclyl, or substituted or unsubstituted non-aromatic carbocyclyl (hereinafter, referred to as M-1).

$R^5$ may be substituted or unsubstituted aromatic heterocyclyl or substituted or unsubstituted aromatic carbocyclyl (hereinafter, referred to as M-2).

$R^5$ may be substituted or unsubstituted 6-membered aromatic heterocyclyl or substituted or unsubstituted phenyl (hereinafter, referred to as M-3).

$R^5$ is a group represented by Formula:

[Chemical formula 25]

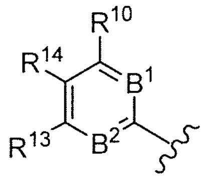

(hereinafter, referred to as M-4).

$R^{10}$ may be a hydrogen atom, cyano, substituted or unsubstituted alkyloxy, or substituted or unsubstituted alkyl (hereinafter, referred to as N-1').

$R^{10}$ may be a hydrogen atom or substituted or unsubstituted alkyl (hereinafter, referred to as N-1).

$R^{10}$ may be a hydrogen atom, alkyl, or haloalkyl (hereinafter, referred to as N-2).

$R^{10}$ may be a hydrogen atom or haloalkyl (hereinafter, referred to as N-3).

$R^{10}$ may be a hydrogen atom (hereinafter, referred to as N-4).

$R^{13}$ may be a hydrogen atom, cyano, substituted or unsubstituted alkyloxy, or substituted or unsubstituted alkyl (hereinafter, referred to as O-1').

$R^{13}$ may be a hydrogen atom or substituted or unsubstituted alkyl (hereinafter, referred to as O-1).

$R^{13}$ may be a hydrogen atom, alkyl, or haloalkyl (hereinafter, referred to as O-2).

$R^{13}$ may be a hydrogen atom or haloalkyl (hereinafter, referred to as O-3).

$R^{13}$ may be a hydrogen atom (hereinafter, referred to as O-4).

$R^{14}$ may be a hydrogen atom, halogen, substituted or unsubstituted alkyl, substituted or unsubstituted alkyloxy, substituted or unsubstituted non-aromatic carbocyclyl, substituted or unsubstituted non-aromatic heterocyclyl, substituted or unsubstituted aromatic carbocyclyl, substituted or unsubstituted aromatic heterocyclyl, substituted or unsubstituted non-aromatic carbocyclyloxy, substituted or unsubstituted aromatic carbocyclyloxy, substituted or unsubstituted alkyloxycarbonyl, substituted or unsubstituted alkylcarbonyl, or cyano (hereinafter, referred to as P-1').

$R^{14}$ may be a hydrogen atom, halogen, substituted or unsubstituted alkyl, substituted or unsubstituted alkyloxy, substituted or unsubstituted non-aromatic carbocyclyl, substituted or unsubstituted alkyloxycarbonyl, substituted or unsubstituted alkylcarbonyl, or cyano (hereinafter, referred to as P-1).

$R^{14}$ may be a hydrogen atom, halogen, substituted or unsubstituted alkyl, substituted or unsubstituted alkyloxy, substituted or unsubstituted aromatic carbocyclyl, substituted or unsubstituted aromatic heterocyclyl, substituted or unsubstituted non-aromatic carbocyclyloxy, or substituted or unsubstituted non-aromatic carbocyclyl (hereinafter, referred to as P-2').

$R^{14}$ may be halogen, substituted or unsubstituted alkyl, or substituted or unsubstituted alkyloxy, substituted or unsubstituted non-aromatic carbocyclyl, or cyano (hereinafter, referred to as P-2).

$R^{14}$ may be halogen, substituted or unsubstituted alkyl, substituted or unsubstituted alkyloxy, or substituted or unsubstituted non-aromatic carbocyclyl (hereinafter, referred to as P-3).

$R^{14}$ may be halogen, alkyl, haloalkyl, alkyloxy, haloalkyloxy, or non-aromatic carbocyclyl (hereinafter referred to as P-4). $R^{14}$ may be halogen, haloalkyl, haloalkyloxy, or non-aromatic carbocyclyl (hereinafter, referred to as P-5).

$R^{14}$ may be halogen or haloalkyl (hereinafter, referred to as P-6).

$B^1$ may be $CR^{11}$ or N (hereinafter, referred to as Q-1).

$B^1$ may be $CR^{11}$ (hereinafter, referred to as Q-2).

$B^1$ may be N (hereinafter, referred to as Q-3).

$R^{11}$ may be a hydrogen atom, halogen, methyloxy, methyl, or halomethyl (hereinafter, referred to as R-1').

$R^{11}$ may be a hydrogen atom or halogen (hereinafter, referred to as R-1).

$R^{11}$ may be halogen (hereinafter, referred to as R-2).

$R^{11}$ may be a hydrogen atom (hereinafter, referred to as R-3).

$B^2$ may be $CR^{12}$ or N (hereinafter, referred to as S-1).

$B^2$ may be $CR^{12}$ (hereinafter, referred to as S-2).

$B^2$ may be N (hereinafter, referred to as S-3).

$R^{12}$ may be a hydrogen atom, halogen, methyloxy, methyl, or halomethyl (hereinafter, referred to as T-1').

$R^{12}$ may be a hydrogen atom or halogen (hereinafter, referred to as T-1).

$R^{12}$ may be halogen (hereinafter, referred to as T-2).

$R^{12}$ may be a hydrogen atom (hereinafter, referred to as T-3).

$R^{15}$ may be a hydrogen atom or substituted or unsubstituted alkyl (hereinafter, referred to as U-1).

$R^{15}$ may be a hydrogen atom or alkyl (hereinafter, referred to as U-2).

$R^{15}$ may be a hydrogen atom (hereinafter, referred to as U-3).

$R^{16}$ may be a hydrogen atom or substituted or unsubstituted alkyl (hereinafter, referred to as V-1).
$R^{16}$ may be a hydrogen atom or alkyl (hereinafter, referred to as V-2).
$R^{16}$ may be a hydrogen atom (hereinafter, referred to as V-3). $R^{15}$ and $R^{16}$ may be taken together with a carbon atom to which $R^{15}$ and $R^{16}$ are bonded to form a substituted or unsubstituted non-aromatic carbocycle (hereinafter, referred to as V-4).
$R^{15}$ and $R^{16}$ may be taken together with a carbon atom to which $R^{15}$ and $R^{16}$ are bonded to form a non-aromatic carbocycle (hereinafter, referred to as V-5).
$R^{17}$ may be a hydrogen atom, halogen, or substituted or unsubstituted alkyl (hereinafter, referred to as W-1).
$R^{17}$ may be a hydrogen atom, halogen, or alkyl (hereinafter, referred to as W-2).
$R^{17}$ may be a hydrogen atom (hereinafter, referred to as W-3).
$R^{18}$ may be a hydrogen atom, halogen, or substituted or unsubstituted alkyl (hereinafter, referred to as X-1).
$R^{18}$ may be a hydrogen atom, halogen, or alkyl (hereinafter, referred to as X-2).
$R^{18}$ may be a hydrogen atom (hereinafter, referred to as X-3). $R^{17}$ and $R^{18}$ may be taken together with a carbon atom to which $R^{17}$ and $R^{18}$ are bonded to form a substituted or unsubstituted non-aromatic carbocycle (hereinafter, referred to as X-4). $R^{17}$ and $R^{18}$ may be taken together with a carbon atom to which $R^{17}$ and $R^{18}$ are bonded to form a non-aromatic carbocycle (hereinafter, referred to as X-5).
One embodiment includes the following aspects.

(i) A compound represented by Formula (I-1):

[Chemical formula 26]

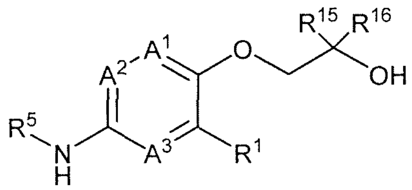

( I-1 )

wherein
$R^1$ is a group represented by the formula:

[Chemical formula 27]

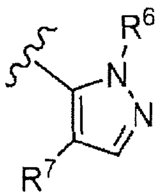, 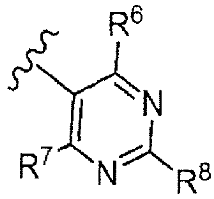, 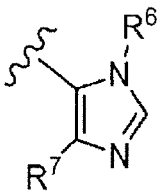, 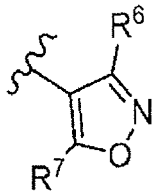, 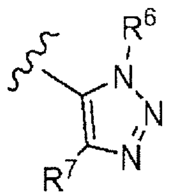,

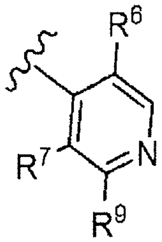, 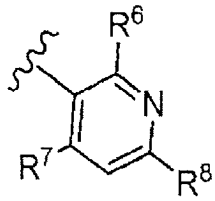, 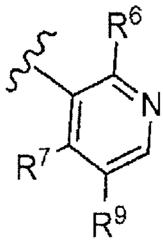, 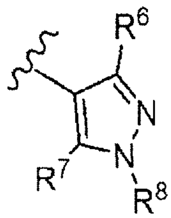, 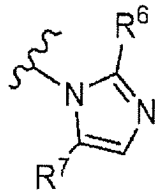,

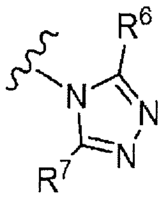, 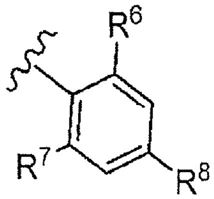, 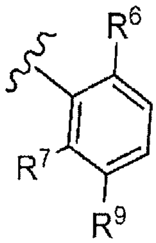 or 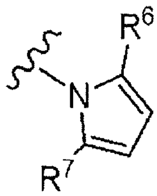

wherein

$R^6$ is substituted or unsubstituted alkyl or substituted or unsubstituted non-aromatic carbocyclyl;
$R^7$ is halogen, substituted or unsubstituted alkyl, substituted or unsubstituted alkenyl, substituted or unsubstituted alkynyl, or cyano;
$R^8$ is a hydrogen atom or substituted or unsubstituted alkyl;
$R^9$ is a hydrogen atom, halogen, or substituted or unsubstituted alkyloxy;
$A^1$ is $CR^2$ or N;
$R^2$ is a hydrogen atom, halogen, or substituted or unsubstituted alkyl;
$A^2$ is $CR^3$ or N;
$R^3$ is a hydrogen atom, halogen, or substituted or unsubstituted alkyl;
$A^3$ is $CR^4$ or N;
$R^4$ is a hydrogen atom;
$R^5$ is substituted or unsubstituted aromatic carbocyclyl or substituted or unsubstituted aromatic heterocyclyl; and
$R^{15}$ and $R^{16}$ are each a hydrogen atom,
or a pharmaceutically acceptable salt thereof.

(ii) A compound represented by Formula (I-1):

[Chemical formula 28]

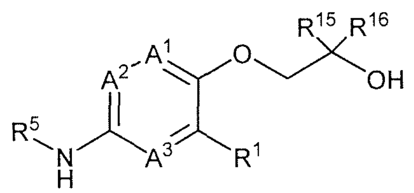

( I-1 )

wherein
$R^1$ is a group represented by formula:

[Chemical formula 29]

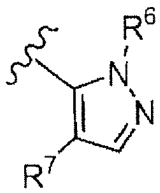 , 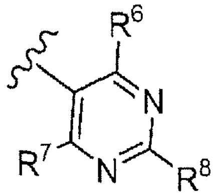 or 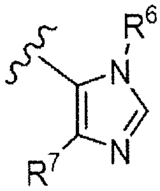

wherein

$R^6$ is substituted or unsubstituted alkyl or substituted or unsubstituted non-aromatic carbocyclyl;
$R^7$ is halogen, substituted or unsubstituted alkyl, substituted or unsubstituted alkenyl, substituted or unsubstituted alkynyl, or cyano;
$R^8$ is a hydrogen atom or substituted or unsubstituted alkyl;
$A^1$ is $CR^2$ or N;
$R^2$ is a hydrogen atom, halogen, or substituted or unsubstituted alkyl;
$A^2$ is $CR^3$ or N;
$R^3$ is a hydrogen atom, halogen, or substituted or unsubstituted alkyl;
$A^3$ is $CR^4$ or N;
$R^4$ is a hydrogen atom;
$R^5$ is a group represented by formula:

[Chemical formula 30]

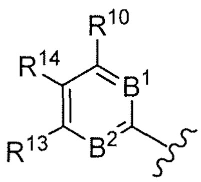

wherein

$R^{10}$ and $R^{13}$ are each independently a hydrogen atom or substituted or unsubstituted alkyl;
$R^{14}$ is a hydrogen atom, halogen, substituted or unsubstituted alkyl, substituted or unsubstituted alkyloxy, substituted or unsubstituted non-aromatic carbocyclyl, substituted or unsubstituted alkyloxycarbonyl, substituted or unsubstituted alkylcarbonyl, or cyano;
$B^1$ is $CR^{11}$ or N;
$B^2$ is $CR^{12}$ or N;
$R^{11}$ and $R^{12}$ are each independently a hydrogen atom or halogen; and
$R^{15}$ and $R^{16}$ are each a hydrogen atom,
or a pharmaceutically acceptable salt thereof.

(iii) A compound represented by Formula (I-1):

[Chemical formula 31]

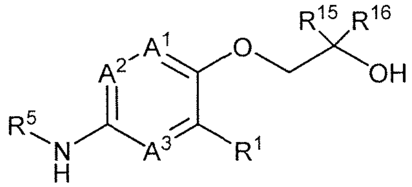

( I-1 )

wherein
$R^1$ is a group represented by Formula:

[Chemical formula 32]

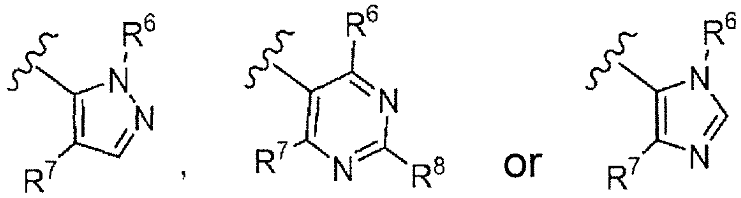

, or

wherein

$R^6$ is substituted or unsubstituted alkyl or substituted or unsubstituted non-aromatic carbocyclyl;
$R^7$ is halogen, substituted or unsubstituted alkyl, substituted or unsubstituted alkenyl, substituted or unsubstituted alkynyl, or cyano;
$R^8$ is a hydrogen atom or substituted or unsubstituted alkyl;
$A^1$ is $CR^2$;
$R^2$ is a hydrogen atom;
$A^2$ is $CR^3$;
$R^3$ is halogen or substituted or unsubstituted alkyl;
$A^3$ is $CR^4$ or N;
$R^4$ is a hydrogen atom;
$R^5$ is a group represented by Formula:

[Chemical formula 33]

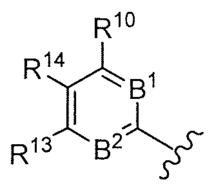

wherein

$R^{10}$ and $R^{13}$ are each independently a hydrogen atom or substituted or unsubstituted alkyl;
$R^{14}$ is a hydrogen atom, halogen, substituted or unsubstituted alkyl, substituted or unsubstituted alkyloxy, or substituted or unsubstituted non-aromatic carbocyclyl;
$B^1$ is N;
$B^2$ is $CR^{12}$;
$R^{12}$ is a hydrogen atom or halogen; and
$R^{15}$ and $R^{16}$ are each a hydrogen atom,
or a pharmaceutically acceptable salt thereof.

(iv) A compound represented by Formula (I-1):

[Chemical formula 34]

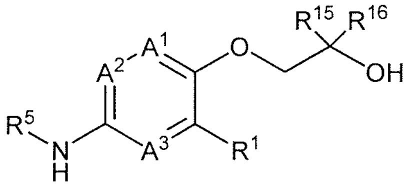

( I-1 )

wherein

$R^1$ is a group represented by Formula:

[Chemical formula 35]

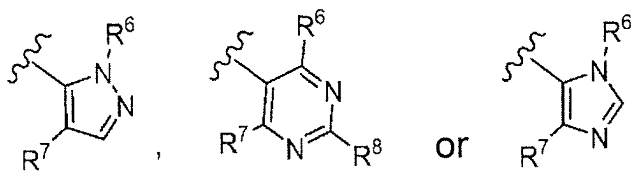

wherein

$R^6$ is alkyl or non-aromatic carbocyclyl;
$R^7$ is halogen, alkyl, haloalkyl, alkenyl, alkynyl or cyano;
$R^8$ is a hydrogen atom or alkyl;
$A^1$ is $CR^2$;
$R^2$ is a hydrogen atom;
$A^2$ is $CR^3$;
$R^3$ is halogen or alkyl;
$A^3$ is $CR^4$ or N;
$R^4$ is a hydrogen atom;
$R^5$ is a group represented by Formula:

[Chemical formula 36]

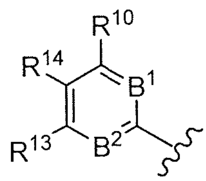

wherein

$R^{10}$ and $R^{13}$ are each independently a hydrogen atom, alkyl, or haloalkyl;
$R^{14}$ is halogen, alkyl, haloalkyl, alkyloxy, haloalkyloxy, or non-aromatic carbocyclyl;
$B^1$ is N;
$B^2$ is $CR^{12}$;
$R^{12}$ is a hydrogen atom or halogen; and
$R^{15}$ and $R^{16}$ are each a hydrogen atom,
or a pharmaceutically acceptable salt thereof.

(v) A compound represented by Formula (I):

[Chemical formula 37]

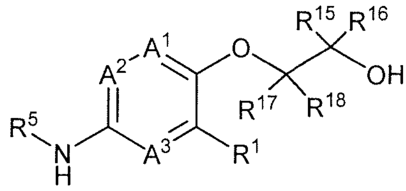

(I)

wherein
$R^1$ is a group represented by Formula:

[Chemical formula 38]

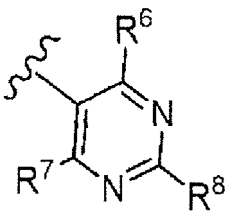

,

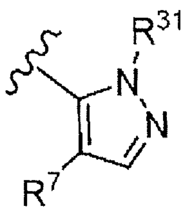

or

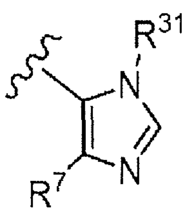

wherein

$R^6$ is substituted or unsubstituted alkyl;
$R^7$ is halogen or substituted or unsubstituted alkyl;
$R^8$ is substituted or unsubstituted alkyl;
$R^{31}$ is a substituted or unsubstituted alkyl;
a combination of ($A^1$, $A^2$, $A^3$) is (CH, N, CH) or (CH, $CR^3$, N);
$R^3$ is a hydrogen atom or halogen;
$R^5$ is a group represented by Formula:

[Chemical formula 39]

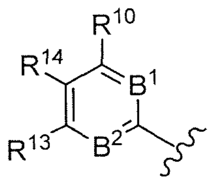

wherein

$R^{10}$ and $R^{13}$ are each independently a hydrogen atom, substituted or unsubstituted alkyloxy, or substituted or unsubstituted alkyl;
$R^{14}$ is a hydrogen atom, halogen, substituted or unsubstituted alkyl, substituted or unsubstituted alkyloxy, substituted or unsubstituted aromatic carbocyclyl, substituted or unsubstituted aromatic heterocyclyl, substituted or unsubstituted non-aromatic carbocyclyloxy, or substituted or unsubstituted non-aromatic carbocyclyl;
$B^1$ is $CR^{11}$ or N;
$R^{11}$ is a hydrogen atom or halogen;
$B^2$ is N;
$R^{15}$, $R^{16}$, $R^{17}$, and $R^{18}$ are each a hydrogen atom, or a pharmaceutically acceptable salt thereof.

(vi) A compound represented by Formula (I):

[Chemical formula 40]

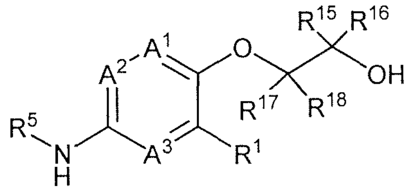

(I)

wherein
$R^1$ is a group represented by Formula:

[Chemical formula 41]

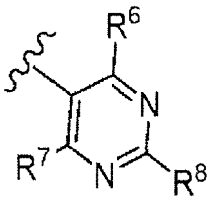

,

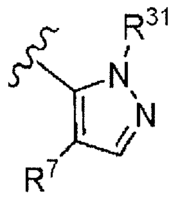

or

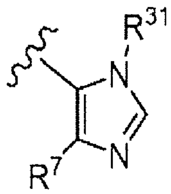

wherein

$R^6$ is substituted or unsubstituted alkyl;
$R^7$ is halogen or substituted or unsubstituted alkyl;
$R^8$ is substituted or unsubstituted alkyl;
$R^{31}$ is substituted or unsubstituted alkyl;
$A^1$ is CH;
$A^2$ is $CR^3$;
$A^3$ is N;
$R^3$ is a hydrogen atom or halogen;
$R^5$ is a group represented by Formula:

[Chemical formula 42]

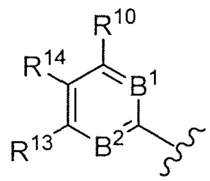

wherein

$R^{10}$ and $R^{13}$ are each independently a hydrogen atom, substituted or unsubstituted alkyloxy, or substituted or unsubstituted alkyl;
$R^{14}$ is a hydrogen atom, halogen, substituted or unsubstituted alkyl, substituted or unsubstituted alkyloxy, substituted or unsubstituted aromatic carbocyclyl, substituted or unsubstituted aromatic heterocyclyl, substituted or unsubstituted non-aromatic carbocyclyloxy, or substituted or unsubstituted non-aromatic carbocyclyl;
$B^1$ is CH;
$B^2$ is N;
$R^{15}$, $R^{16}$, $R^{17}$, and $R^{18}$ are each a hydrogen atom,
or a pharmaceutically acceptable salt thereof.

(vii) A compound represented by Formula (I):

[Chemical formula 43]

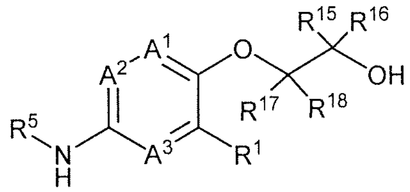

(I)

wherein

$R^1$ is a group represented by Formula:

[Chemical formula 44]

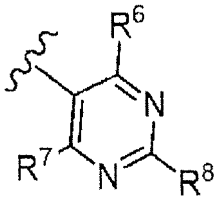

,

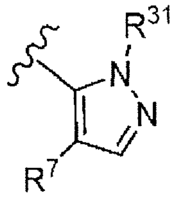

or

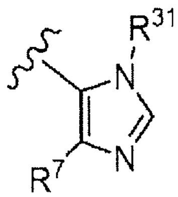

wherein

$R^6$ is alkyl;
$R^7$ is halogen or alkyl;
$R^8$ is alkyl substituted with alkyloxy, alkyl substituted with non-aromatic heterocyclyl, or unsubstituted alkyl;
$R^{31}$ is alkyl;
$A^1$ is CH;
$A^2$ is $CR^3$;
$A^3$ is N;
$R^3$ is a hydrogen atom or halogen;
$R^5$ is a group represented by Formula:

[Chemical formula 45]

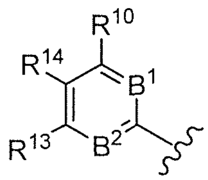

wherein

$R^{10}$ and $R^{13}$ are each independently a hydrogen atom, alkyloxy, or alkyl;
$R^{14}$ is a hydrogen atom, halogen, haloalkyl, alkyl, haloalkyloxy, alkyloxy, aromatic carbocyclyl substituted with at least one group selected from the substituent group Z, unsubstituted aromatic carbocyclyl, aromatic heterocyclyl substituted with at least one group selected from the substituent group Z, unsubstituted aromatic heterocyclyl, non-aromatic carbocyclyloxy substituted with halogen, unsubstituted non-aromatic carbocyclyloxy, non-aromatic carbocyclyl substituted with halogen, or unsubstituted non-aromatic carbocyclyl;
Substituent Group Z: halogen, haloalkyl, alkyl substituted with alkyloxy, alkyl, haloalkyloxy, alkyloxy, and cyano $B^1$ is CH;
$B^2$ is N;

$R^{15}$, $R^{16}$, $R^{17}$, and $R^{18}$ are each a hydrogen atom,
or a pharmaceutically acceptable salt thereof.

(viii) A compound represented by Formula (I):

[Chemical formula 46]

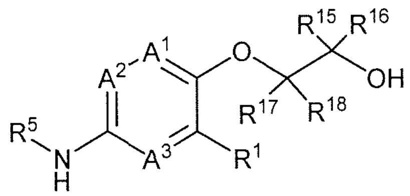

(I)

wherein
$R^1$ is a group represented by Formula:

[Chemical formula 47]

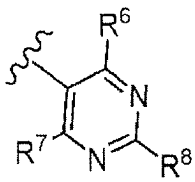

,

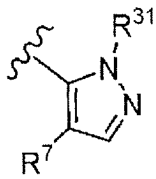

or

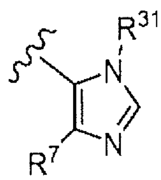

wherein

$R^6$ is substituted or unsubstituted alkyl,
$R^7$ is halogen or substituted or unsubstituted alkyl;
$R^8$ is substituted or unsubstituted alkyl;
$R^{31}$ is a substituted or unsubstituted alkyl;
$A^1$ is CH;
$A^2$ is N;
$A^3$ is CH;
$R^3$ is a hydrogen atom or halogen;
$R^5$ is a group represented by Formula:

[Chemical formula 48]

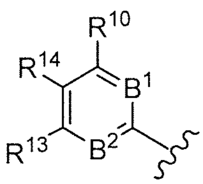

wherein

$R^{10}$ and $R^{13}$ are each independently a hydrogen atom, substituted or unsubstituted alkyloxy, or substituted or unsubstituted alkyl;
$R^{14}$ is a hydrogen atom, halogen, substituted or unsubstituted alkyl, substituted or unsubstituted alkyloxy, substituted or unsubstituted aromatic carbocyclyl, substituted or unsubstituted aromatic heterocyclyl, substituted or unsubstituted non-aromatic carbocyclyloxy, or substituted or unsubstituted non-aromatic carbocyclyl;
$B^1$ is $CR^{11}$ or N;

$R^{11}$ is a hydrogen atom or halogen;
$B^2$ is N;
$R^{15}$, $R^{16}$, $R^{17}$, and $R^{18}$ are each a hydrogen atom,
or a pharmaceutically acceptable salt thereof.

(ix) A compound represented by Formula (I):

[Chemical formula 49]

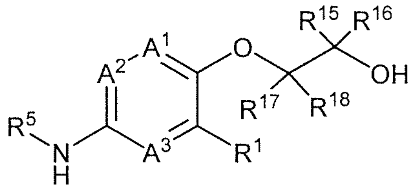

(I)

wherein
$R^1$ is a group represented by Formula:

[Chemical formula 50]

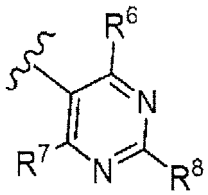
,
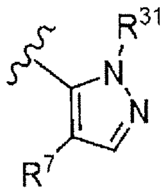
or
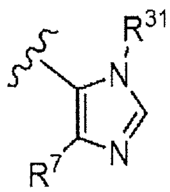

wherein

$R^6$ is alkyl;
$R^7$ is halogen or alkyl;
$R^8$ is alkyl substituted with alkyloxy, alkyl substituted with non-aromatic heterocyclyl, or unsubstituted alkyl;
$R^{31}$ is alkyl;
$A^1$ is CH;
$A^2$ is N;
$A^3$ is CH;
$R^3$ is a hydrogen atom or halogen;
$R^5$ is a group represented by Formula:

[Chemical formula 51]

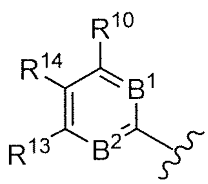

wherein

$R^{10}$ and $R^{13}$ are each independently a hydrogen atom, alkyloxy, or alkyl;
$R^{14}$ is a hydrogen atom, halogen, haloalkyl, alkyl, haloalkyloxy, alkyloxy, aromatic carbocyclyl substituted with at least one group selected from the substituent group Z, unsubstituted aromatic carbocyclyl, aromatic heterocyclyl substituted with at least one group selected from the substituent group Z, unsubstituted aromatic

heterocyclyl, non-aromatic carbocyclyloxy substituted with halogen, unsubstituted non-aromatic carbocyclyloxy, non-aromatic carbocyclyl substituted with halogen, or unsubstituted non-aromatic carbocyclyl;
Substituent Group Z: halogen, haloalkyl, alkyl substituted with alkyloxy, alkyl, haloalkyloxy, alkyloxy, and cyano $B^1$ is $CR^{11}$ or N;
$R^{11}$ is a hydrogen atom or halogen;
$B^2$ is N;
$R^{15}$, $R^{16}$, $R^{17}$, and $R^{18}$ are each a hydrogen atom,
or a pharmaceutically acceptable salt thereof.

The compound represented by Formula (I) or (I-1) is not limited to specific isomers, but includes all possible isomers (e.g., keto-enol isomer, imine-enamine isomers, diastereoisomers, optical isomers, rotational isomers, etc.), racemates or mixtures thereof.

One or more hydrogen, carbon and/or other atoms of the compound represented by Formula (I) or (I-1) is optionally substituted with isotopes of hydrogen, carbon, and/or other atoms. Examples of such isotopes include hydrogen, carbon, nitrogen, oxygen, phosphorus, sulfur, fluorine, iodine, and chlorine such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$ and $^{36}Cl$ respectively. The compound represented by Formula (I) or (I-1) also encompass compounds substituted with such isotopes. The compounds substituted with the isotopes are also useful as pharmaceuticals. The compound represented by Formula (I) or (I-1) encompasses all radiolabeled forms of the compound represented by Formula (I) or (I-1) substituted with radioactive isotopes contained in the isotopes. Furthermore, a "radiolabeling method" for producing the "radiolabeled forms" is also included in the present invention, and the "radiolabeled forms" are useful as tools for metabolic pharmacokinetics studies, studies on binding assay, and/or diagnostics.

Radioactive labels of the compound represented by Formula (I) or (I-1) are prepared by methods well known in the art. For example, a tritium-labeled compound of Formula (I) or (I-1) can be prepared by introducing tritium into certain compound of Formula (I) or (I-1) by a catalytic dehalogenation reaction using tritium. The method involves the reaction of tritium gas with an appropriately halogen-substituted precursor of the compound represented by Formula (I) or (I-1) in the presence or absence of a base, in the presence of a suitable catalyst, e.g. Pd/C. Other suitable methods for preparing a tritium-labeled compound are described in "Isotopes in the Physical and Biomedical Sciences, Vol.1, Labeled Compounds (Part A), Chapter 6 (1987)". $^{14}C$-labeled compounds can be prepared by using a raw material having $^{14}C$ carbon.

Examples of the pharmaceutically acceptable salts of the compound represented by Formula (I) or (I-1) include salts of the compound represented by Formula (I) or (I-1) with alkaline metal (e.g., lithium, sodium, or potassium), alkaline earth metal (e.g., calcium or barium), magnesium, transition metal (e.g., zinc or iron), ammonia, organic bases (e.g., trimethylamine, triethylamine, dicyclohexylamine, ethanolamine, diethanolamine, triethanolamine, meglumine, ethylenediamine, pyridine, picoline, or quinoline), or amino acids, or salts with inorganic acids (e.g., hydrochloric acid, sulfuric acid, nitric acid, carbonic acid, hydrobromic acid, phosphoric acid, or hydroiodic acid) or organic acids (e.g., formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, ethanesulfonic acid, or trifluoroacetic acid). These salts can be formed according to methods that are conventionally carried out.

The compound represented by Formula (I) or (I-1) of the present invention or a pharmaceutically acceptable salt thereof may form a solvate (e.g., a hydrate), a co-crystal and/or a crystal polymorphism, and the present invention encompasses such various solvates, co-crystals, and crystal polymorphisms. A "solvate" may have the compound represented by Formula (I) or (I-1) coordinated with any number of solvent molecules (e.g., water molecules, etc.). When the compound represented by Formula (I) or (I-1) or a pharmaceutically acceptable salt thereof is allowed to stand in the atmosphere, the compound may absorb water, resulting in attachment of adsorbed water or formation of a hydrate. Furthermore, a crystale polymorph may be formed by recrystallizing the compound represented by Formula (I) or (I-1) or pharmaceutically acceptable salts thereof. The term "co-crystal" means that the compound represented by Formula (I) or (I-1) or a salt thereof and a counter molecule are present in the same crystal lattice, and may contain any number of counter molecules.

The compound represented by Formula (I) or (I-1) of the present invention, or a pharmaceutically acceptable salt thereof, may form a prodrug, and the present invention also encompasses such various prodrugs. Prodrugs are derivatives of the compounds of the present invention having chemically or metabolically degradable groups, which are compounds that will be the compounds of the present invention that are pharmaceutically active by solvolysis or under physiological conditions in vivo. The prodrugs encompass compounds that are enzymatically oxidized, reduced, hydrolyzed or the like under physiological conditions in vivo and converted to the compound represented by Formula (I) or (I-1), compounds that are hydrolyzed by gastric acid or the like and converted to the compound represented by Formula (I) or (I-1), and the like. Methods for selecting and preparing suitable prodrug derivatives are described in, for example, "Design of Prodrugs, Elsevier, Amsterdam, 1985". A prodrug may have activity per se.

When the compound represented by Formula (I) or (I-1) or pharmaceutically acceptable salt thereof has hydroxy

group(s), examples of the prodrug include acyloxy derivatives and sulfonyloxy derivatives that are prepared by, for example, reacting compounds having hydroxy group(s) with suitable acyl halide, suitable acid anhydride, suitable sulfonyl chloride, suitable sulfonyl anhydride and mixed anhydride, or with a condensing agent. Examples include $CH_3COO$-, $C_2H_5COO$-, tert-BuCOO-, $C_{15}H_{31}COO$-, PhCOO-, (m-NaOOCPh)COO-, $NaOOCCH_2CH_2COO$-, $CH_3CH(NH_2)COO$-, $CH_2N(CH_3)_2COO$-, $CH_3SO_3$-, $CH_3CH_2SO_3$-, $CF_3SO_3$-, $CH_2FSO_3$-, $CF_3CH_2SO_3$-, p-$CH_3O$-$PhSO_3$-, $PhSO_3$-, and p-$CH_3PhSO_3$-.

Since the compound according to the present invention has serotonin 5-HT2A receptor antagonism and/or inverse agonism and serotonin 5-HT2C receptor antagonism and/or inverse agonism, the compound is useful as a therapeutic agent and/or prophylactic agent for a disease associated with a serotonin 5-HT2A receptor and/or serotonin 5-HT2C receptor. Diseases associated with serotonin 5-HT2A receptor and/or serotonin 5-HT2C receptor include serotonin-mediated diseases such as Parkinson's disease-related hallucinations and delusions, dementia-related hallucinations and delusions, schizophrenia-related hallucinations and delusions, depression-related hallucinations and delusions, neurodegenerative diseases-related hallucinations and delusions, depression, schizophrenia, autism, dependence, dyskinesia, sleep disorder, obstructive sleep apnea syndrome, Parkinson's disease-related irritability, dementia-related irritability, schizophrenia-related irritability, sexual dysfunction and the like. Preferable examples include Parkinson's disease-related hallucinations and delusions, dementia-related hallucinations and delusions, schizophrenia-related hallucinations and delusions, depression-related hallucinations and delusions, sleep disorder, obstructive sleep apnea syndrome, Parkinson's disease-related irritability, dementia-related irritability, and schizophrenia-related irritability. More preferable examples include Parkinson's disease-related hallucinations and delusions, and dementia-related hallucinations and delusions, and obstructive sleep apnea syndrome.

(Method for producing compound of present invention)

The compound represented by Formula (I) or (I-1) according to the present invention can be produced by, for example, the general synthetic method described below. Regarding extraction, purification, and the like, the treatments carried out in ordinary experiments of organic chemistry may be carried out.

The compounds of the present invention can be synthesized with reference to methods known in the art.

General Synthetic Method 1

(Method A)

[Chemical formula 52]

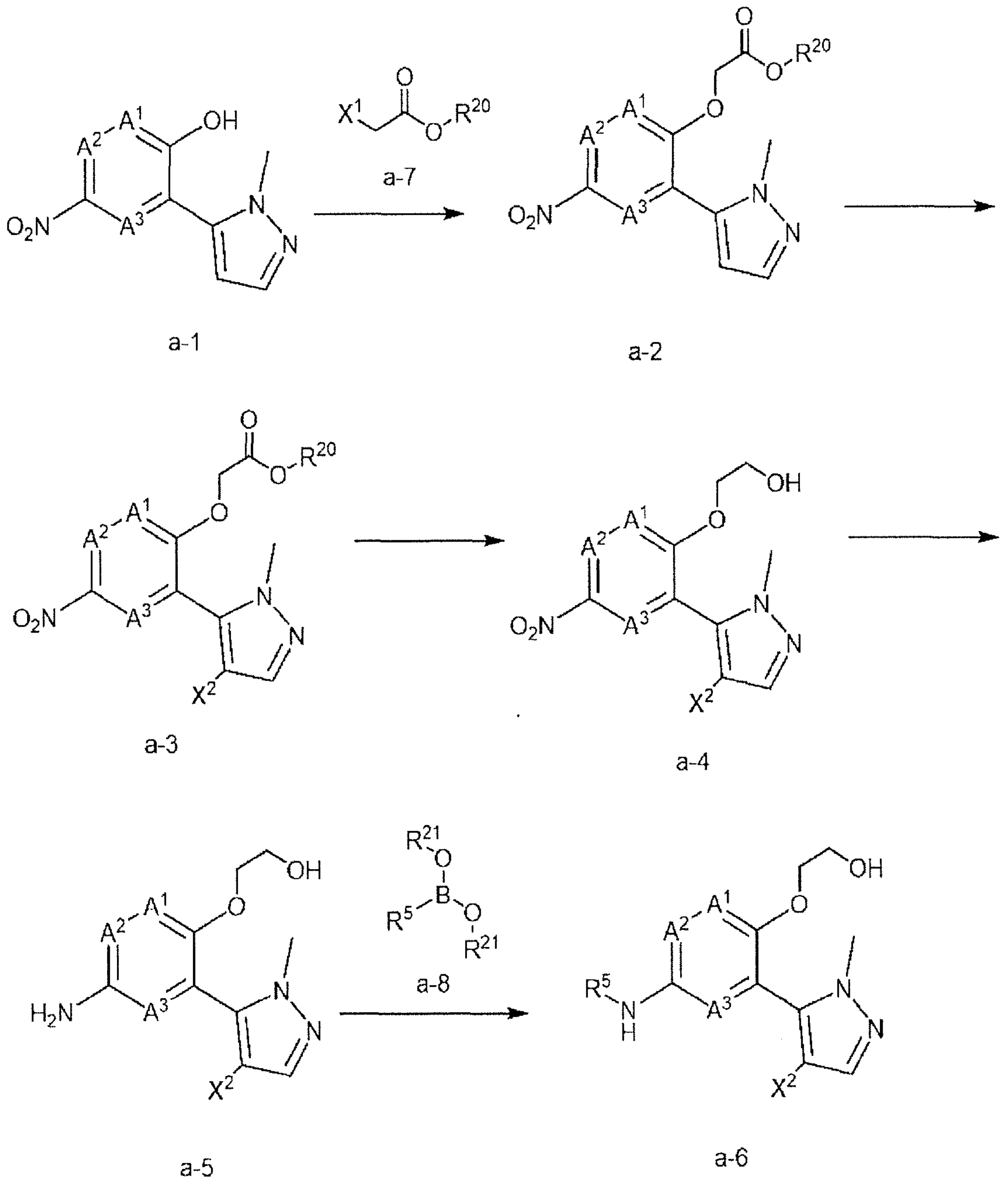

a-7

a-1

a-2

a-3

a-4

a-8

a-5

a-6

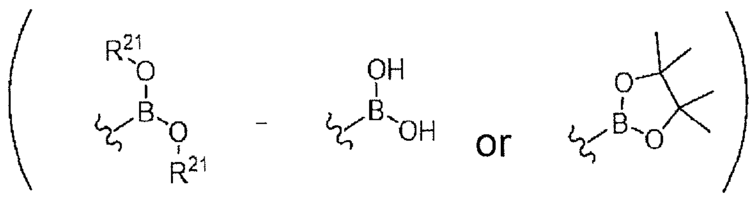

or

wherein $R^{20}$ is a hydrogen atom or a C1-C3 alkyl, $X^1$ is a leaving group such as a chlorine atom or a bromine atom, $X^2$ is halogen, and the other symbols have the same meanings as in the above item (1).

Step 1

Compound (a-2) can be obtained by reacting Compound (a-7) with Compound (a-1) in the presence of a base.

The reaction temperature is 0°C to 150°C, preferably 0°C to 50°C.

The reaction time is 0.5 hours to 12 hours, preferably 1 hour to 6 hours.

Examples of the base include sodium hydroxide, sodium carbonate, sodium bicarbonate, potassium carbonate, calcium carbonate, cesium carbonate, pyridine, triethylamine, and DMAP, and the base can be used in an amount of 1 to 5 molar equivalents relative to Compound (a-1).
Examples of the reaction solvent include dichloromethane, acetonitrile, tetrahydrofuran, dioxane, DMF, DMA, and DMSO, and each solvent can be used alone or mixed with the others.

Step 2

Compound (a-3) can be obtained by reacting Compound (a-2) with a halogenating agent.
Examples of the halogenating agent include N-bromosuccinimide, N-chlorosuccinimide, N-iodosuccinimide, Selectfluor (registered trademark), silver(II) fluoride, and the halogenating agent can be used in an amount of 1 to 5 molar equivalents relative to Compound (a-2).
The reaction temperature is -20°C to 50°C, preferably 0°C to 20°C.
The reaction time is 0.5 hours to 24 hours, preferably 0.5 hours to 6 hours.
Examples of the reaction solvent include acetonitrile, tetrahydrofuran, toluene, dichloromethane, DMF, and each solvent can be used alone or mixed with the others.

Step 3

Compound (a-4) can be obtained by reacting a reducing agent with Compound (a-3).
Examples of the reducing agent include sodium borohydride, lithium borohydride, and lithium aluminium hydride, and the reducing agent can be used in an amount of 1 to 10 molar equivalents relative to Compound (a-3).
The reaction temperature is 0°C to the reflux temperature, preferably 20°C to the reflux temperature.
The reaction time is 0.2 hours to 48 hours, preferably 1 hour to 24 hours.
Examples of the reaction solvent include methanol, ethanol, propanol, isopropanol, butanol, tetrahydrofuran, diethyl ether, dichloromethane, and water, and each solvent can be used alone or mixed with the others.

Step 4

Compound (a-5) can be obtained by reacting Compound (a-4) with ammonium chloride and iron.
The reaction temperature is 50°C to the reflux temperature. The reaction time is 0.2 hours to 12 hours, preferably 1 hour to 4 hours.
Examples of the reaction solvent include methanol, ethanol, propanol, isopropanol, butanol, tetrahydrofuran, and water, and each solvent can be used alone or mixed with the others.

Step 5

Compound (a-6) can be obtained by reacting Compound (a-5) with Compound (a-8) in the presence of a metal catalyst and a base. Examples of the metal catalyst include copper acetate, and the metal catalyst can be used in an amount of 0.01 to 1 molar equivalents relative to Compound (a-5).
Examples of the base include pyridine and triethylamine, and the base can be used in an amount of 1 to 10 molar equivalents relative to Compound (a-5).
Compound (a-8) can be used in an amount of 1 to 10 molar equivalents relative to Compound (a-5).
The reaction time is 4 to 72 hours, preferably 12 hours to 24 hours.
Examples of the reaction solvent include dichloromethane, tetrahydrofuran, toluene, DMF, and dioxane, and each solvent can be used alone or mixed with the others.

General Synthetic Method 2

(Method B)

[Chemical formula 53]

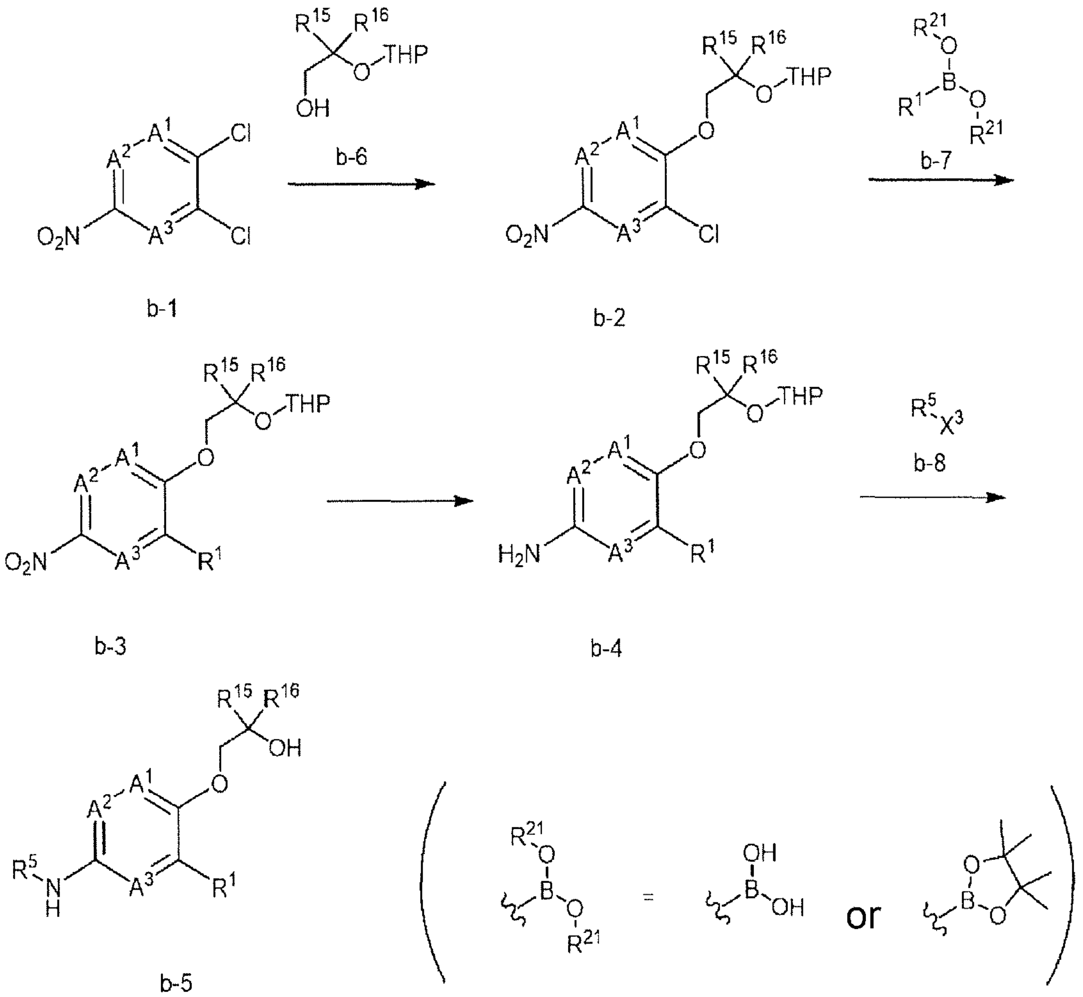


b-1 b-2 b-3 b-4 b-5

or

wherein $X^3$ is a leaving group such as a chlorine atom or a fluorine atom, and the other symbols have the same meanings as in the above (1).

Step 1

Compound (b-2) can be obtained by reacting Compound (b-1) with Compound (b-6) in the presence of NaH.
The reaction temperature is 0°C to 100°C, preferably 0°C to 50°C.
The reaction time is 0.5 hours to 12 hours, preferably 0.5 hours to 2 hours.
Examples of the reaction solvent include dichloromethane, acetonitrile, tetrahydrofuran, dioxane, DMF, DMA, and DMSO, and each solvent can be used alone or mixed with the others.

Step 2

Compound (b-3) can be obtained by reacting Compound (b-2) with Compound (b-7) in the presence of a metal catalyst and a base. Examples of the metal catalyst include palladium acetate, bis(dibenzylideneacetone)palladium, tetrakis(triphenylphosphine)palladium, bis(triphenylphosphine)palladium(II) dichloride, bis(tri-tert-butylphosphine)palladium, and XPhos Pd G3, and the metal catalyst can be used in an amount of 0.001 to 0.5 molar equivalents relative to Compound (b-2).
Examples of the base include lithium hydroxide, sodium hydroxide, potassium hydroxide, potassium tert-butoxide, sodium tert-butoxide, sodium carbonate, potassium carbonate, sodium bicarbonate, sodium phosphate, sodium hydrogen phosphate, potassium phosphate, and potassium hydrogen phosphate, and the base can be used in an amount of 1 to 10 molar equivalents relative to Compound (b-2).
Compound (b-7) can be used in an amount of 1 to 10 molar equivalents relative to Compound (b-2).
The reaction temperature is 20°C to the reflux temperature of the solvent, or in some cases, the temperature under microwave irradiation.

The reaction time is 0.1 to 48 hours, preferably 0.5 hours to 12 hours.
Examples of the reaction solvent include tetrahydrofuran, toluene, DMF, dioxane, and water, and each solvent can be used alone or mixed with the others.

Step 3

Compound (b-4) can be obtained by using Compound (b-3) as a raw material and using the same method as in Step 4 of Method A described above.

Step 4

Compound (b-5) can be obtained by reacting Compound (b-4) with Compound (b-8) in the presence of an acid.
The reaction temperature is 30°C to 150°C, preferably 100°C to 130°C.
The reaction time is 0.5 hours to 24 hours, preferably 1 hour to 18 hours.
Examples of the acid include hydrochloric acid, sulfuric acid, TFA, formic acid, trifluoroborane, p-TsOH, and PPTS, and the acid can be used in an amount of 0.1 molar equivalents or more, preferably 0.1 to 1 molar equivalents relative to Compound (b-4).
Examples of the reaction solvent include methanol, ethanol, 2-propanol, t-butyl alcohol, water, acetone, acetonitrile, tetrahydrofuran, and dioxane, and each solvent can be used alone or mixed with the others.

Step 4'

Compound (b-5) can be obtained by reacting Compound (b-4) with Compound (b-8) in the presence of a base and then reacting with an acid.

Reaction with Compound (b-8):

The reaction temperature is 0°C to 30°C
The reaction time is 10 minutes to 6 hours, preferably 10 minutes to 1 hour.
Examples of the base include NaH and potassium tert-butoxide, and the base can be used in an amount of 0.1 molar equivalents or more, preferably 1 to 5 molar equivalents relative to Compound (b-4).
Examples of the reaction solvent include DMF, acetone, acetonitrile, tetrahydrofuran, and dioxane, and each solvent can be used alone or mixed with the others.

Reaction with acid:

The reaction temperature is 30°C to 150°C, preferably 100°C to 130°C.
The reaction time is 0.5 hours to 24 hours, preferably 1 hour to 18 hours.
Examples of the acid include hydrochloric acid, sulfuric acid, TFA, formic acid, trifluoroborane, p-TsOH, and PPTS, and the acid can be used in an amount of 0.1 molar equivalents or more, preferably 0.1 to 1 molar equivalents relative to Compound (b-4).
Examples of the reaction solvent include methanol, ethanol, 2-propanol, t-butyl alcohol, water, acetone, acetonitrile, tetrahydrofuran, and dioxane, and each solvent can be used alone or mixed with the others.

General Synthetic Method 3

(Method C)

[Chemical formula 54]

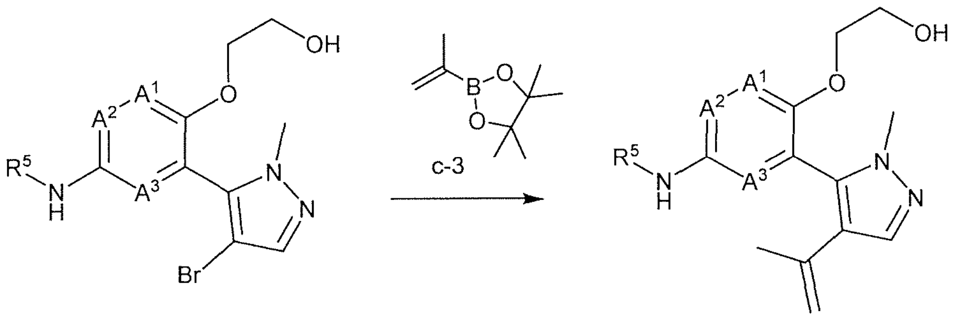


c-1 c-2

wherein each symbol has the same meanings as the above (1).

Step 1

Compound (c-2) can be obtained by reacting Compound (c-1) with Compound (c-3) in the presence of a metal catalyst and a base. Examples of the metal catalyst include palladium acetate, bis(dibenzylideneacetone)palladium, tetrakis(triphenylphosphine)palladium, bis(triphenylphosphine)palladium(II) dichloride, bis(tri-tert-butylphosphine) palladium, and XPhos Pd G3, and $PdCl_2$(dtbpf), and the metal catalyst can be used in an amount of 0.001 to 0.5 molar equivalents relative to Compound (c-1).
Examples of the base include lithium hydroxide, sodium hydroxide, potassium hydroxide, potassium tert-butoxide, sodium tert-butoxide, sodium carbonate, potassium carbonate, sodium bicarbonate, sodium phosphate, sodium hydrogen phosphate, potassium phosphate, and potassium hydrogen phosphate, and the base can be used in an amount of 1 to 10 molar equivalents relative to Compound (c-1).
Compound (c-3) can be used in an amount of 1 to 10 molar equivalents relative to Compound (c-1).
The reaction temperature is 20°C to the reflux temperature of the solvent, or in some cases, the temperature under microwave irradiation.
The reaction time is 0.1 to 48 hours, preferably 0.5 hours to 12 hours.
Examples of the reaction solvent include tetrahydrofuran, toluene, DMF, dioxane, and water, and each solvent can be used alone or mixed with the others.

General Synthetic Method 4

(Method D)

[Chemical formula 55]

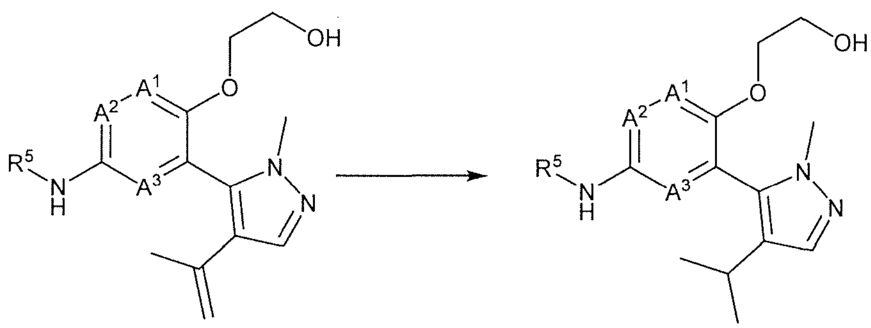

c-2 d-1

wherein each symbol has the same meanings as the above (1).

Step 1

Compound (d-1) can be obtained by reacting Compound (c-2) with hydrogen gas in the presence of a metal catalyst.
Examples of the metal catalyst include palladium-carbon, platinum oxide, rhodium-aluminum oxide, and chlorotris(triphenylphosphine)rhodium(I), and the metal catalyst can be used at 0.01 wt% to 100 wt% relative to Compound (c-2).
The hydrogen pressure can be 1 to 50 atm. As the hydrogen source, cyclohexene, 1,4-cyclohexadiene, formic acid, ammonium formate, or the like can also be used instead of hydrogen gas. The reaction temperature is 0°C to the reflux temperature of the solvent, preferably 20°C to 40°C.
The reaction time is 0.5 to 72 hours, preferably 1 to 12 hours.
Examples of the reaction solvent include methanol, ethanol, propanol, isopropanol, butanol, tetrahydrofuran, diethyl ether, toluene, ethyl acetate, acetic acid, and water, and each solvent can be used alone or mixed with the others.

General Synthetic Method 5

(Method E)

[Chemical formula 56]

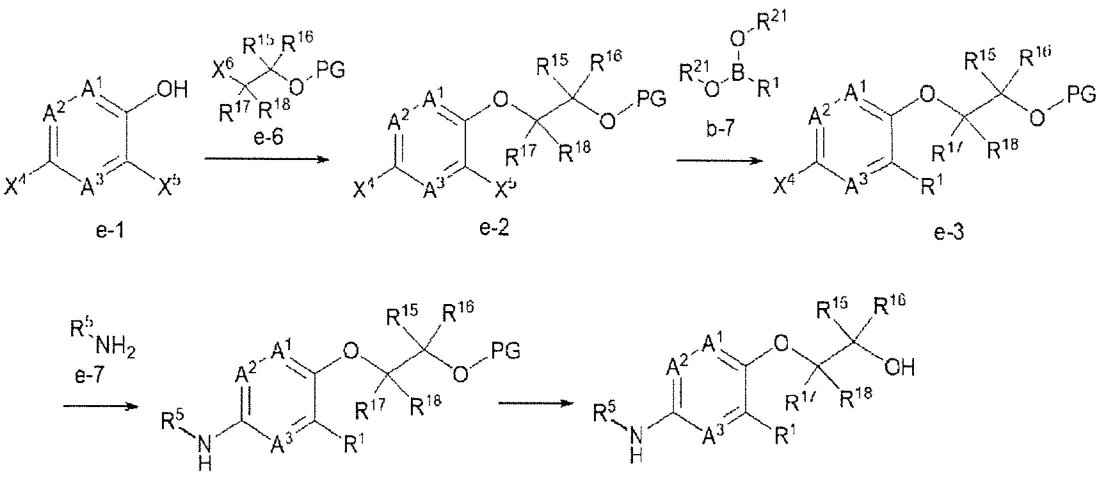


e-1 e-2 e-3 e-4 e-5

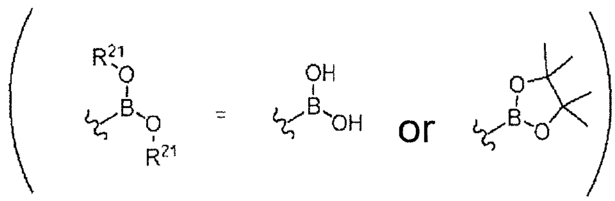

wherein $X^4$, $X^5$, and $X^6$ are each independently a leaving group such as a chlorine atom, a bromine atom, or an iodine atom, PG is a protecting group such as TBS, and other symbols have the same meanings as in the above (1).

Step 1

Compound (e-2) can be obtained by reacting Compound (e-1) with Compound (e-6) in the presence of a base.
The reaction temperature is 0°C to the reflux temperature of the solvent, preferably 60°C to 80°C.
The reaction time is 0.5 hours to 12 hours, preferably 1 hour to 6 hours.
Examples of the base include sodium hydroxide, sodium carbonate, sodium bicarbonate, potassium carbonate, calcium carbonate, cesium carbonate, pyridine, triethylamine, and DMAP, and the base can be used in an amount of 1 to 5

molar equivalents relative to Compound (e-1).

Examples of the reaction solvent include dichloromethane, acetonitrile, tetrahydrofuran, dioxane, DMF, DMA, and DMSO, and each solvent can be used alone or mixed with the others.

Step 2

Compound (e-3) can be obtained by reacting Compound (e-2) with Compound (b-7) in the presence of a metal catalyst and a base. Examples of the metal catalyst include palladium acetate, [1,1'-bis(di-tert-butylphosphino)ferrocene] palladium(II) dichloride, bis(dibenzylideneacetone)palladium, tetrakis(triphenylphosphine)palladium, bis(triphenylphosphine)palladium(II) dichloride, bis(tri-tert-butylphosphine) palladium, and XPhos Pd G3, and the metal catalyst can be used in an amount of 0.001 to 0.5 molar equivalents relative to Compound (e-2).

Examples of the base include lithium hydroxide, sodium hydroxide, potassium hydroxide, potassium tert-butoxide, sodium tert-butoxide, sodium carbonate, potassium carbonate, sodium bicarbonate, sodium phosphate, sodium hydrogen phosphate, potassium phosphate, and potassium hydrogen phosphate, and the base can be used in an amount of 1 to 5 molar equivalents relative to Compound (e-2).

Compound (b-7) can be used in an amount of 1 to 5 molar equivalents relative to Compound (e-2).

The reaction temperature is 20°C to the reflux temperature of the solvent, or in some cases, the temperature under microwave irradiation.

The reaction time is 0.1 to 48 hours, preferably 0.5 hours to 12 hours.

Examples of the reaction solvent include tetrahydrofuran, toluene, DMF, dioxane, and water, and each solvent can be used alone or mixed with the others.

Step 3

Compound (e-4) can be obtained by reacting Compound (e-3) with Compound (e-7) in the presence of a metal catalyst, a ligand, and a base.

Examples of the metal catalyst include combinations of (dibenzylideneacetone)palladium, palladium acetate, palladium chloride, and the like and Xantphos, BINAP, X-Phos, BrettPhos, triphenylphosphine, 1,1'-bis(diphenylphosphinoferrocene), and the like, and the metal catalyst can be used in an amount of 0.001 to 0.5 molar equivalents relative to Compound (e-3). Alternatively, examples of the metal catalyst include tetrakis(triphenylphosphine)palladium, 1,1'-bis(diphenylphosphinoferrocene)palladium(II) dichloride, $PdCl_2$ (dtbpf), bis(triphenylphosphine)palladium(II) dichloride, bis(tri-tert-butylphosphine)palladium, Xantphos Pd G3, XPhos Pd G3, and Brettphos Pd G3, and the metal catalyst can be used in an amount of 0.001 to 0.5 molar equivalents relative to Compound (e-3).

Examples of the base include lithium hydroxide, sodium hydroxide, potassium hydroxide, potassium tert-butoxide, sodium tert-butoxide, sodium carbonate, potassium carbonate, cesium carbonate, sodium bicarbonate, sodium phosphate, sodium hydrogen phosphate, potassium phosphate, and potassium hydrogen phosphate, and the base can be used in an amount of 1 to 5 molar equivalents relative to Compound (e-3).

Compound (e-7) can be used in an amount of 1 to 5 molar equivalents relative to Compound (e-3).

The reaction temperature is 50°C to the reflux temperature of the solvent, or in some cases, the temperature under microwave irradiation.

The reaction time is 1 to 48 hours, preferably 1 hour to 12 hours.

Examples of the reaction solvent include tetrahydrofuran, toluene, and dioxane, and each solvent can be used alone or mixed with the others.

Step 4

Compound (e-5) can be obtained by reacting Compound (e-4) with a deprotecting agent.

Examples of the deprotecting agent include tetrabutylammonium fluoride, hydrogen fluoride pyridine, trifluoroacetic acid, and hydrochloric acid, and the deprotecting agent can be used in an amount of 0.2 to 10 molar equivalents relative to Compound (e-4).

The reaction temperature is 0°C to 60°C, preferably 20°C to 60°C.

The reaction time is 0.5 hours to 24 hours, preferably 0.5 hours to 2 hours.

Examples of the reaction solvent include tetrahydrofuran, dichloromethane, dichloroethane, and methanol, and each solvent can be used alone or mixed with the others.

General Synthetic Method 6

(Method F)

[Chemical formula 57]

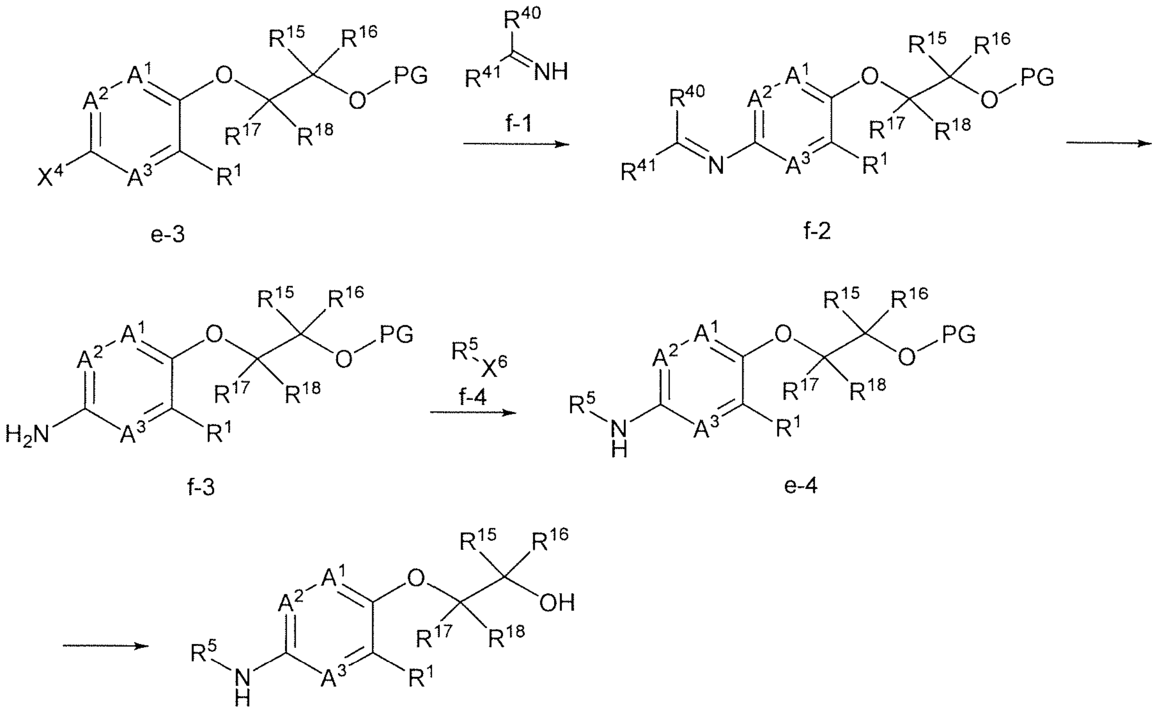


e-3 f-2 f-3 e-4 e-5

wherein $R^{40}$ and $R^{41}$ are each independently substituted or unsubstituted aromatic heterocyclyl or substituted or unsubstituted aromatic carbocyclyl, $X^4$ and $X^6$ are each independently a leaving group such as a chlorine atom, a bromine atom, or an iodine atom, PG is a protecting group such as TBS, and the other symbols have the same meanings as in the above (1).

Step 1

Compound (f-2) can be obtained by reacting Compound (e-3) with Compound (f-1) in the presence of a metal catalyst and a base. Examples of the metal catalyst include combinations of bis(dibenzylideneacetone)palladium, palladium acetate, palladium chloride and the like and Xantphos, BINAP, X-Phos, BrettPhos, triphenylphosphine, 1,1'-bis(diphenylphosphinoferrocene), and the like, and the metal catalyst can be used in an amount of 0.001 to 0.5 molar equivalents relative to Compound (e-3). Alternatively, examples of the metal catalyst include Xantphos Pd G3, XPhos Pd G3, Brettphos Pd G3, 1,1'-bis(diphenylphosphinoferrocene)palladium(II) dichloride, tetrakis(triphenylphosphine)palladium, $PdCl_2$ (dtbpf), bis(triphenylphosphine)palladium(II) dichloride, and bis(tri-tert-butylphosphine) palladium, and the metal catalyst can be used in an amount of 0.001 to 0.5 molar equivalents relative to Compound (e-3).

Examples of the base include cesium carbonate, sodium carbonate, potassium carbonate, sodium bicarbonate, sodium phosphate, sodium hydrogen phosphate, potassium phosphate, potassium hydrogen phosphate, potassium acetate, sodium acetate, sodium tert-butoxide, and potassium tert-butoxide, and the base can be used in an amount of 1 to 10 molar equivalents relative to Compound (e-3).

Compound (f-1) can be used in an amount of 1 to 10 molar equivalents relative to Compound (e-3).

The reaction temperature is 20°C to the reflux temperature of the solvent, or in some cases, the temperature under microwave irradiation.

The reaction time is 0.1 to 72 hours, preferably 0.5 hours to 48 hours.

Examples of the reaction solvent include dioxane, toluene, and tetrahydrofuran, and each solvent can be used alone or mixed with the others.

Step 2

Compound (f-3) can be obtained by reacting Compound (f-2) with hydroxylamine hydrochloride and a base.
Hydroxylamine hydrochloride can be used in an amount of 1 to 5 molar equivalents relative to Compound (f-2).
Examples of the base include triethylamine, pyridine, sodium acetate, and potassium acetate, and the base can be used in an amount of 1 to 10 molar equivalents relative to Compound (f-2).
The reaction temperature is 20°C to the reflux temperature of the solvent.
The reaction time is 0.1 hour to 10 hours, preferably 0.1 hours to 5 hours.
Examples of the reaction solvent include ethanol, methanol, and 2-propanol, and each solvent can be used alone or mixed with the others.

Step 3

Compound (e-4) can be obtained by reacting Compound (f-3) with Compound (f-4) in the presence of a metal catalyst and a base. Examples of the metal catalyst include combinations of (dibenzylideneacetone)palladium, palladium acetate, palladium chloride, and the like and Xantphos, BINAP, X-Phos, BrettPhos, triphenylphosphine, 1,1'-bis(diphenylphosphinoferrocene), and the like, and the metal catalyst can be used in an amount of 0.001 to 0.5 molar equivalents relative to Compound (f-3). Alternatively, examples of the metal catalyst include tetrakis(triphenylphosphine)palladium, 1,1'-bis(diphenylphosphinoferrocene)palladium(II) dichloride, $PdCl_2$ (dtbpf), bis(triphenylphosphine)palladium(II) dichloride, bis(tri-tert-butylphosphine)palladium, Xantphos Pd G3, XPhos Pd G3, and Brettphos Pd G3, and the metal catalyst can be used in an amount of 0.001 to 0.5 molar equivalents relative to Compound (f-3).
Examples of the base include cesium carbonate, sodium carbonate, potassium carbonate, sodium bicarbonate, sodium phosphate, sodium hydrogen phosphate, potassium phosphate, potassium hydrogen phosphate, potassium acetate, sodium acetate, lithium hydroxide, sodium hydroxide, potassium hydroxide, sodium tert-butoxide, and potassium tert-butoxide, and the base can be used in an amount of 1 to 5 molar equivalents relative to Compound (f-3).
Compound (f-4) can be used in an amount of 1 to 5 molar equivalents relative to Compound (f-3).
The reaction temperature is 50°C to the reflux temperature of the solvent, or in some cases, the temperature under microwave irradiation.
The reaction time is 0.5 to 48 hours, preferably 0.5 to 12 hours.
Examples of the reaction solvent include toluene, dioxane, and tetrahydrofuran, and each solvent can be used alone or mixed with the others.

Step 5

Compound (e-5) can be obtained by reacting Compound (e-4) with a deprotecting agent.
Examples of the deprotecting agent include tetrabutylammonium fluoride, hydrogen fluoride pyridine, trifluoroacetic acid, and p-toluenesulfonic acid, and the deprotecting agent can be used in an amount of 0.2 to 10 molar equivalents relative to Compound (e-4).
The reaction temperature is 0°C to 60°C, preferably 20°C to 60°C.
The reaction time is 0.5 hours to 24 hours, preferably 0.5 hours to 2 hours.
Examples of the reaction solvent include tetrahydrofuran, dichloromethane, dichloroethane, and methanol, and each solvent can be used alone or mixed with the others.

General Synthetic Method 7

(Method G)

[Chemical formula 58]

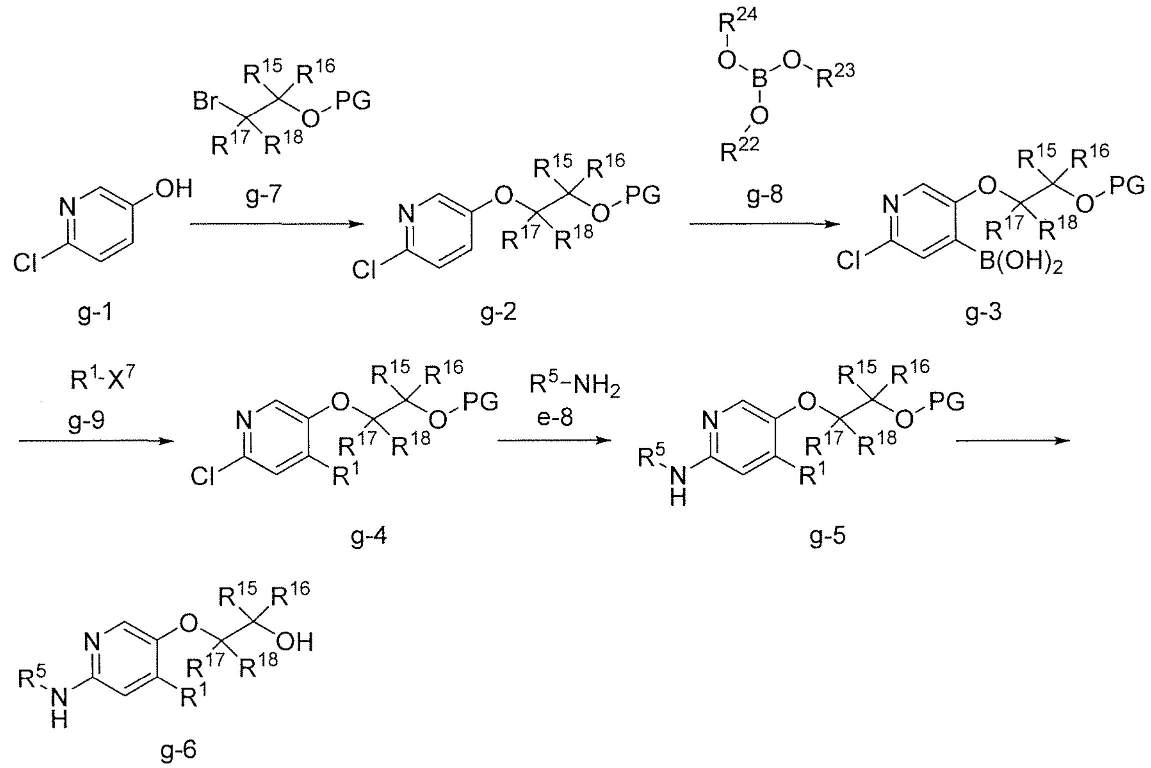


g-1, g-2, g-3, g-4, g-5, g-6

wherein $R^{22}$, $R^{23}$, and $R^{24}$ are each independently C1-C3 alkyl, $X^7$ is iodine, bromine, or the like, PG is a protecting group such as TBS, and other symbols have the same meanings as in the above (1).

Step 1

Compound (g-2) can be obtained by reacting Compound (g-1) with Compound (g-7) in the presence of a base.
The reaction temperature is 0°C to the reflux temperature of the solvent, preferably 60°C to 80°C.
The reaction time is 0.5 hours to 12 hours, preferably 1 hour to 6 hours.
Examples of the base include sodium hydroxide, sodium carbonate, sodium bicarbonate, potassium carbonate, calcium carbonate, cesium carbonate, pyridine, triethylamine, and DMAP, and the base can be used in an amount of 1 to 5 molar equivalents relative to Compound (g-1).
Examples of the reaction solvent include dichloromethane, acetonitrile, tetrahydrofuran, dioxane, DMF, DMA, and DMSO, and each solvent can be used alone or mixed with the others.

Step 2

Compound (g-3) can be obtained by reacting a base with Compound (g-2) and then reacting with Compound (g-8), and adding an acid.
The reaction temperature is -78°C to 20°C.
The reaction time is 0.1 hours to 10 hours, preferably 0.1 hours to 5 hours.
Examples of the reaction solvent include diethyl ether, tetrahydrofuran, and toluene, and each solvent can be used alone or mixed with the others.
Examples of base include n-butyllithium, sec-butyllithium, tert-butyllithium, lithium diisopropylamine, lithium bis(trimethylsilyl)amide, and potassium bis(trimethylsilyl)amide, and the base can be used in an amount 1 to 2 molar equivalents relative to Compound (g-2).

Step 3

Compound (g-4) can be obtained by reacting Compound (g-3) with Compound (g-9) in the presence of a metal catalyst and a base. The reaction temperature is 20°C to the reflux temperature of the solvent, or in some cases, the

temperature under microwave irradiation.

The reaction time is 0.1 to 72 hours, preferably 0.5 hours to 48 hours.

Examples of the reaction solvent include dioxane, toluene, and tetrahydrofuran, and each solvent can be used alone or mixed with the others.

Examples of the metal catalyst include combinations of bis(dibenzylideneacetone)palladium, palladium acetate, palladium chloride, and the like and Xantphos, BINAP, X-Phos, BrettPhos, triphenylphosphine, 1,1'-bis(diphenylphosphinoferrocene), and the like, and the metal catalyst can be used in an amount of 0.001 to 0.5 molar equivalents relative to Compound (g-3). Alternatively, examples of the metal catalyst include Xantphos Pd G3, XPhos Pd G3, Brettphos Pd G3, 1,1'-bis(diphenylphosphinoferrocene)palladium(II) dichloride, tetrakis(triphenylphosphine)palladium, $PdCl_2$ (dtbpf), bis(triphenylphosphine)palladium(II) dichloride, and bis(tri-tert-butylphosphine) palladium, and the metal catalyst can be used in an amount of 0.001 to 0.5 molar equivalents relative to Compound (g-3).

Examples of the base include cesium carbonate, sodium carbonate, potassium carbonate, sodium bicarbonate, sodium phosphate, sodium hydrogen phosphate, potassium phosphate, potassium hydrogen phosphate, potassium acetate, sodium acetate, sodium tert-butoxide, and potassium tert-butoxide, and the base can be used in an amount of 1 to 10 molar equivalents relative to Compound (g-3).

Compound (g-9) can be used in an amount of 1 to 10 molar equivalents relative to Compound (g-3).

The reaction temperature is 20°C to the reflux temperature of the solvent, or in some cases, the temperature under microwave irradiation.

The reaction time is 0.1 to 72 hours, preferably 0.5 hours to 48 hours.

Examples of the reaction solvent include dioxane, toluene, and tetrahydrofuran, and each solvent can be used alone or mixed with the others.

Step 4

By the method described in Step 3 of the method E, Compound (g-5) can be obtained from Compound (g-4).

Step 5

By the method described in Step 4 of the method E, Compound (g-6) can be obtained from Compound (g-5).

Since the compound according to the present invention has serotonin 5-HT2A receptor antagonism and/or inverse agonism and serotonin 5-HT2C receptor antagonism and/or inverse agonism, the compound is useful as a therapeutic agent and/or prophylactic agent for Parkinson's disease- and/or dementia-related hallucinations and delusions.

Furthermore, the compound according to the present invention has utility as a medicine, and preferably, the compound has any or a plurality of the following excellent features.

a) Inhibitory activity against CYP enzymes (for example, CYP1A2, CYP2C9, CYP2C19, CYP2D6, and CYP3A4) is weak.
b) Satisfactory pharmacokinetics such as high bioavailability and adequate clearance are exhibited.
c) Metabolic stability is high.
d) Irreversible inhibitory activity is not exhibited against CYP enzymes (for example, CYP3A4) within the concentration range of the measurement conditions described in the present specification.
e) Mutagenicity is not exhibited.
f) The cardiovascular risk is low.
g) High solubility is exhibited.
h) High binding ability for a serotonin 5-HT2A receptor is exhibited.
i) High binding ability for a serotonin 5-HT2C receptor is exhibited.
j) Brain distribution ability is high.
k) P-gp substrate property is low.
l) BCRP (breast cancer resistance protein) substrate property is low.

The pharmaceutical composition of the present invention can be administered by either an oral method or a parenteral method. Examples of a parenteral administration method include percutaneous, subcutaneous, intravenous, intra-arterial, intramuscular, intraperitoneal, transmucosal, inhalation, transnasal, ocular instillation, ear instillation, and intravaginal administration.

In the case of oral administration, the pharmaceutical composition may be prepared into any dosage form that is commonly used, such as a solid preparation for internal use (for example, a tablet, a powder preparation, a granular preparation, a capsule, a pill, or a film preparation), or a liquid preparation for internal use (for example, a suspension, an

emulsion, an elixir, a syrup, a limonade, a spirit preparation, an aromatic water preparation, an extraction, a decoction, or a tincture) and administered. The tablet may be a sugar-coated tablet, a film-coated tablet, an enteric-coated tablet, a sustained release tablet, a troche, a sublingual tablet, a buccal tablet, a chewable tablet, or an orally disintegrating tablet; the powder preparation and granular preparation may be dry syrups; and the capsule may be a soft capsule, a microcapsule, or a sustained release capsule.

In the case of parenteral administration, the pharmaceutical composition can be suitably administered in any dosage form that is commonly used, such as an injectable preparation, an infusion, or a preparation for external use (for example, an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder preparation for external use, or a suppository). The injectable preparation may be an O/W, W/O, O/W/O, or W/O/W type emulsion, or the like.

A pharmaceutical composition can be obtained by mixing an effective amount of the compound of the present invention with various pharmaceutical additives appropriate for the dosage form, such as an excipient, a binder, a disintegrating agent, and a lubricating agent, as necessary. Furthermore, the pharmaceutical composition can be formulated as a pharmaceutical composition for children, the elderly, the critically ill or for surgical use by appropriately modifying the effective amount of the compound of the present invention, the dosage form, and/or various pharmaceutical additives. For example, a pharmaceutical composition for children can be administered to neonates (less than 4 weeks after birth), infants (4 weeks to less than 1 year after birth), toddlers (1 to less than 7 years of age), children (7 to less than 15 years of age), or patients aged 15 to 18 years. For example, a pharmaceutical composition for an elderly may be administered to a patient 65 years of age or older.

It is desirable to set the amount of administration of the pharmaceutical composition of the present invention, after considering the age and body weight of the patient, the type and degree of the disease, the route of administration, and the like; however, in the case of oral administration, the amount of administration is usually 0.05 to 100 mg/kg/day and is preferably in the range of 0.1 to 10 mg/kg/day. In the case of parenteral administration, the amount of administration may vary greatly depending on the route of administration; however, the amount of administration is usually 0.005 to 10 mg/kg/day and is preferably in the range of 0.01 to 1 mg/kg/day. This may be administered once a day or several times a day.

The compound according to the present invention can be used in combination with another therapeutic agent for Parkinson's disease, Alzheimer's disease, psychosis or depression (hereinafter, referred to as concomitant drug), for the purpose of enhancing the action of the compound, reducing the amount of administration of the compound, or the like. At this time, the timing of administration for the compound of the present invention and the concomitant drug is not limited, and these may be administered simultaneously to the target of administration or may be administered with a time difference. Furthermore, the compound of the present invention and the concomitant drug may be administered as two or more kinds of preparations each including active ingredients, or may be administered as a single preparation including those active ingredients.

The amount of administration of the concomitant drug can be appropriately selected based on the clinically used dosage. Furthermore, the blending ratio of the compound of the present invention and the concomitant drug can be appropriately selected according to the target of administration, the route of administration, the target disease, symptoms, combination, and the like. For example, when the target of administration is a human being, 0.01 to 100 parts by weight of the concomitant drug may be used with respect to 1 part by weight of the compound of the present invention.

Examples of the therapeutic agent for Parkinson's disease include levodopa preparations.

Examples of the therapeutic agent for Alzheimer's disease include donepezil.

Examples of the therapeutic agent for psychosis include quetiapine.

Examples of the therapeutic agent for depression include escitalopram.

## Examples

Hereinafter, the present invention will be described in more detail by way of Examples, Reference Examples, and Test Examples; however, the present invention is not intended to be limited by these.

Furthermore, abbreviations used in the present specification denote the following meanings.

BINAP: (f)-2,2'-bis(diphenylphosphino)-1,1'-binaphthyl
BrettPhos: 2-(dicyclohexylphosphino)-3,6-dimethoxy-2',4',6'-triisopropyl-1,1'-biphenyl
BrettPhos Pd G3: [(2-dicyclohexylphosphino-3,6-dimethoxy-2',4',6'-triisopropyl-1,1'-biphenyl)-2-(2'-amino-1,1'-biphenyl)]palladium (II) methanesulfonate
$CDCl_3$: Deuterated chloroform
DMSO-$d_6$: Deuterated dimethyl sulfoxide
DMF: N,N-dimethylformamide
DMSO: Dimethyl sulfoxide

DMA: N,N-dimethylacetamide
DMAP: 4-(dimethylamino)pyridine
HCl: Hydrogen chloride
IPE: Diisopropylether
NaH: Sodium hydride
$PdCl_2$(dtbpf): [1,1'-bis(di-tert-butylphosphino)ferrocene]dichloro palladium (II)
p-TsOH: p-Toluenesulfonic acid
PPTS: Pyridinium p-toluenesulfonate
TBS: tert-Butyldimethylsilyl
TFA: Trifluoroacetic acid
THF: Tetrahydrofuran
THP: 2-Tetrahydropyranyl
TMS: Trimethylsilyl
Tr: Trityl
X-Phos: 2-dicyclohexylphosphino-2',4',6'-triisopropylbiphenyl
XPhos Pd G3: (2-dicyclohexylphosphino-2',4',6'-triisopropyl-1,1'-biphenyl)[2-(2'-amino-1,1'-biphenyl)]palladium (II) methanesulfonate
Xantphos: 4,5-Bis(diphenylphosphino)-9,9-dimethylxanthene
Xantphos Pd G3: [(4,5-Bis(diphenylphosphino)-9,9-dimethylxanthene)-2-(2'-amino-1,1'-biphenyl)]palladium(II) methanesulfonate
HPLC High performance liquid chromatography
nM: nmol/L
pM: µmol/L

(Method for identifying compound)

The NMR analysis obtained in each Example was performed at 400 MHz, and measurement was made using DMSO-$d_6$ or $CDCl_3$. Furthermore, when NMR data are shown, there are occasions in which all the measured peaks are not described.

The term "RT" in the specification indicates retention time in an LC/MS: liquid chromatography/mass spectrometry, and the retention time was measured under the following conditions. The unit of "RT" is minute.

(Measurement condition 1)

Column: Shim-pack XR-ODS (2.2 µm, i.d. 3.0 × 50 mm) (Shimadzu)
Flow rate: 1.6 mL/min
UV detection wavelength: 254 nm
Mobile phase: [A] was 0.1% formic acid-containing aqueous solution, and [B] was 0.1% formic acid-containing acetonitrile solution.
Gradient: A linear gradient of 10% to 100% solvent [B] was carried out for 3 minutes, and then 100% solvent [B] was maintained for 0.5 minutes.

(Measurement condition 2)

Column: ACQUITY UPLC (registered trademark) BEH C18 (1.7 µm i.d. 2.1 × 50 mm) (Waters)
Flow rate: 0.8 mL/min
UV detection wavelength: 254 nm
Mobile phase: [A] was 0.1% formic acid-containing aqueous solution, and [B] was 0.1% formic acid-containing acetonitrile solution.
Gradient: A linear gradient of 5% to 100% solvent [B] was carried out for 3.5 minutes, and then 100% solvent [B] was maintained for 0.5 minutes.

(Measurement condition 3)

Column: ACQUITY UPLC (registered trademark) BEH C18 (1.7 μm i.d. 2.1 × 50 mm) (Waters)
Flow rate: 0.8 mL/min
UV detection wavelength: 254 nm
Mobile phase: [A] was 10 mM ammonium carbonate-containing aqueous solution, and [B] was acetonitrile.
Gradient: A linear gradient of 5% to 100% solvent [B] was carried out for 3.5 minutes, and then 100% solvent [B] was maintained for 0.5 minutes.

(Measurement condition 4)

Column: Shim-pack Scepter (1.9 um i.d. 2.1 × 50 mm) (Shimadzu)
Flow rate: 0.8 mL/min
UV detection wavelength: 254 nm
Mobile phase: [A] was 0.1% formic acid-containing aqueous solution, and [B] was 0.1% formic acid-containing acetonitrile solution.
Gradient: A linear gradient of 5% to 100% solvent [B] was carried out for 3.5 minutes, and then 100% solvent [B] was maintained for 0.5 minutes.

In the specification, the term MS (m/z) indicates a value measured by mass spectrometry. Unless otherwise noted, MS (m/z) indicates a value of $[M+H]^+$.
Also in the specification, the "Compound No.", indicates a compound number, the "Structure" indicates a chemical structure, and the "LC/MS method" indicates measurement conditions of the LC/MS.

Example 1: Synthesis of Compound I-002

[Chemical formula 59]

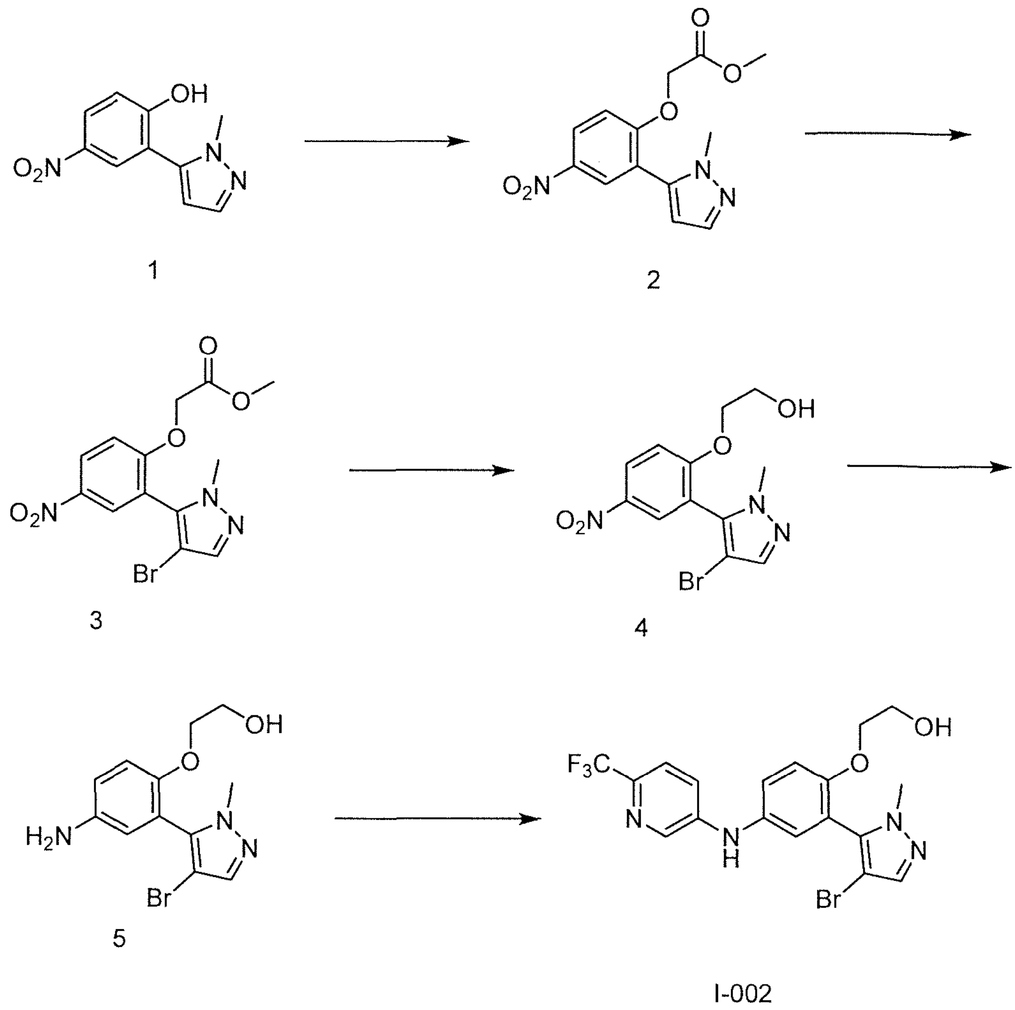

1 2 3 4 5 I-002

Step 1: Synthesis of Compound 2

Under a nitrogen atmosphere, Compound 1 (synthetic method described in WO 2005/01225) (5.00 g, 22.8 mmol) was dissolved in dimethylformamide (50 mL), and then, potassium carbonate (3.47 g, 25.1 mmol), methyl bromoacetate (2.31 mL, 25.1 mmol) were added and stirred at room temperature for 2 hours and 30 minutes. Ethyl acetate (120 mL), water (120 mL), 2 mol/L hydrochloric acid were added and extracted with ethyl acetate. The organic layer was washed with 1 mol/L hydrochloric acid, saturated sodium bicarbonate solution, water, and brine, and then, dried over anhydrous sodium sulfate. The solvent was removed under reduced pressure and the resulting residue was purified by silica gel column chromatography (hexane-ethyl acetate) to give Compound 2 (4.90 g, yield: 74%).
$^1$H-NMR ($CDCl_3$) δ: 3.82 (s, 3H), 3.82 (s, 3H), 4.78 (s, 2H), 6.35 (d, J = 2.0 Hz, 1H), 6.90 (d, J = 9.0 Hz, 1H), 7.57 (d, J = 2.0 Hz, 1H), 8.23 (d, J = 2.8 Hz, 1H), 8.30 (dd, J = 9.0, 2.8 Hz, 1H)

Step 2: Synthesis of Compound 3

Under a nitrogen atmosphere, Compound 2 (4.19 g, 14.4 mmol) was dissolved in dimethylformamide (42 mL), and N-bromosuccinimide (2.56 g, 14.4 mmol) was added under ice-cooling and stirred at room temperature for 1 hour. Under ice-cooling, N-bromosuccinimide (512 mg, 2.88 mmol) was added and stirred at room temperature for 1 hour and 45 minutes. Ethyl acetate (100 mL), sodium thiosulfate (2 g), and water (50 mL) were added and extracted with ethyl acetate. The organic layer was washed with 1 mol/L hydrochloric acid, saturated sodium bicarbonate solution, water, and brine, and then, dried over anhydrous sodium sulfate. The solvent was removed under reduced pressure and the resulting solid was washed with hexanes (20 mL)-ethyl acetate (10 mL) and then filtered off to give Compound 3 (5.23 g, yield: 98%).
$^1$H-NMR ($CDCl_3$) δ: 3.81 (s, 3H), 3.82 (s, 3H), 4.79 (s, 2H), 6.93 (d, J = 9.3 Hz, 1H), 7.57 (s, 1H), 8.25 (d, J = 2.8 Hz,

1H), 8.36 (dd, J = 9.2, 2.9 Hz, 1H).

Step 3: Synthesis of Compound 4

Under a nitrogen atmosphere, Compound 3 (5.08 g, 13.7 mmol) was dissolved in methanol (36 mL)-dichloromethane (72 mL), and sodium borohydride (1.04 g, 27.4 mmol) was added under ice-cooling and stirred at room temperature for 1 hour. Sodium borohydride (1.04 g, 27.4 mmol) was added under ice-cooling and stirred at room temperature for 1 hour. Sodium borohydride (260 mg, 6.86 mmol) was added under ice-cooling and stirred at room temperature for 1 hour. 1 mol/L hydrochloric acid (70 mL) was added under ice-cooling and extracted with ethyl acetate (200 mL). The organic layer was washed with saturated sodium bicarbonate solution, water, and brine, and then dried over anhydrous sodium sulfate. The solvent was removed under reduced pressure, and the resulting residue was purified by silica gel column chromatography (chloroform-methanol) to give Compound 4 (2.66 g, yield: 57%). $^1$H-NMR ($CDCl_3$) $\delta$: 1.96 (t, J = 6.1 Hz, 1H), 3.77 (s, 3H), 3.92-3.96 (m, 2H), 4.19-4.29 (m, 2H), 7.14 (d, J = 9.0 Hz, 1H), 7.57 (s, 1H), 8.21 (d, J = 2.8 Hz, 1H), 8.39 (dd, J = 9.2, 2.9 Hz, 1H).

Step 4: Synthesis of Compound 5

Compound 4 (2.66 g, 7.77 mmol), iron (1.30 g, 23.3 mmol), and ammonium chloride (4.16 g, 78 mmol) were suspended in ethanol (53 mL)-water (13 mL) and stirred at 70°C for 1 hour. Iron (217 mg, 3.89 mmol) was added and stirred at 70°C for 50 minutes. After cooling, ethyl acetate (70 mL) and water (50 mL) were added, an insoluble was filtered through celite (registered trademark) and the mother liquor was extracted with ethyl acetate. The organic layer was washed with water and brine, and then dried over anhydrous sodium sulfate. The solvent was removed under reduced pressure and the resulting residue was purified by silica gel column chromatography (chloroform-methanol) to give Compound 5 (1.89 g, yield: 78%). $^1$H-NMR ($CDCl_3$) $\delta$: 1.85 (br s, 1H), 3.58 (br s, 2H), 3.73 (s, 3H), 3.73-3.79 (m, 2H), 3.90-4.02 (m, 2H), 6.60 (d, J = 3.0 Hz, 1H), 6.78 (dd, J = 8.7, 2.9 Hz, 1H), 6.89 (d, J = 8.8 Hz, 1H), 7.52 (s, 1H).

Step 5: Synthesis of Compound I-002

Compound 5 (30.0 mg, 0.096 mmol) was dissolved in dichloromethane (2.7 mL) and (6-trifluoromethylpyridin-3-yl)boronic acid (36.7 mg, 0.192 mmol), copper acetate (17.5 mg, 0.096 mmol), and triethylamine (0.027 mL, 0.192 mmol) were added and stirred at room temperature for 20 hours and 40 minutes. 6-trifluoromethylpyridin-3-yl)boronic acid (36.7 mg, 0.192 mmol), copper acetate (17.5 mg, 0.096 mmol), and triethylamine (0.027 mL, 0.192 mmol) were added and stirred at room temperature for 23 hours and 45 minutes. The solvent was removed under reduced pressure and the resulting residue was purified by silica gel column chromatography (amino silica gel, chloroform-methanol) to give Compound I-002 (24.4 mg, yield: 56%).

$^1$H-NMR ($CDCl_3$) $\delta$: 1.96 (br s, 1H), 3.77 (s, 3H), 3.87 (br s, 2H), 4.06-4.14 (m, 2H), 5.98 (s, 1H), 7.07 (d, J = 8.8 Hz, 1H), 7.13 (d, J = 2.8 Hz, 1H), 7.28 (dd, J = 9.0, 2.8 Hz, 1H), 7.32 (dd, J = 8.6, 2.4 Hz, 1H), 7.50 (d, J = 8.8 Hz, 1H), 7.53 (s, 1H), 8.31 (d, J = 2.3 Hz, 1H).

Example 2: Synthesis of Compound I-006

[Chemical formula 60]

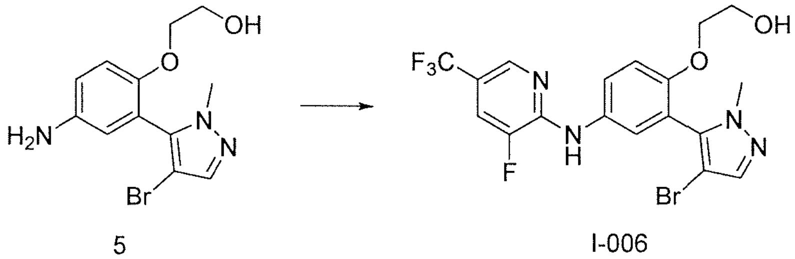

Step 1: Synthesis of Compound I-006

Under a nitrogen atmosphere, Compound 5 (255 mg, 0.817 mmol) was dissolved in 2-propanol (2.5 mL) and 2,3-difluoro-5-trifluoromethylpyridine (122 μL, 0.981 mmol) and p-toluenesulfonic acid monohydrate (78 mg, 0.409 mmol) were added and stirred at 100°C for 18 hours. After cooling, saturated sodium bicarbonate solution (0.5 mL) and water (0.5 mL) were added and stirred for 5 minutes. The precipitated solid was filtered off and dried to give Compound I-006 (252 mg, yield: 65%).

1H-NMR (CDCl3) δ: 8.26 (1H, s), 7.76 (1H, dd, J = 9.0, 2.8 Hz), 7.56 (1H, d, J = 2.8 Hz), 7.55 (1H, s), 7.47 (1H, dd J = 10.8, 1.9 Hz), 7.07 (1H, d, J = 9.0 Hz), 6.81 (1H, s), 4.10 (2H, ddd, J = 19.7, 9.9, 4.1 Hz), 3.87-3.85 (2H, br m) 3.79 (3H, s), 1.87 (1H, t, J = 6.3 Hz).

Example 3: Synthesis of Compound I-062

[Chemical formula 61]

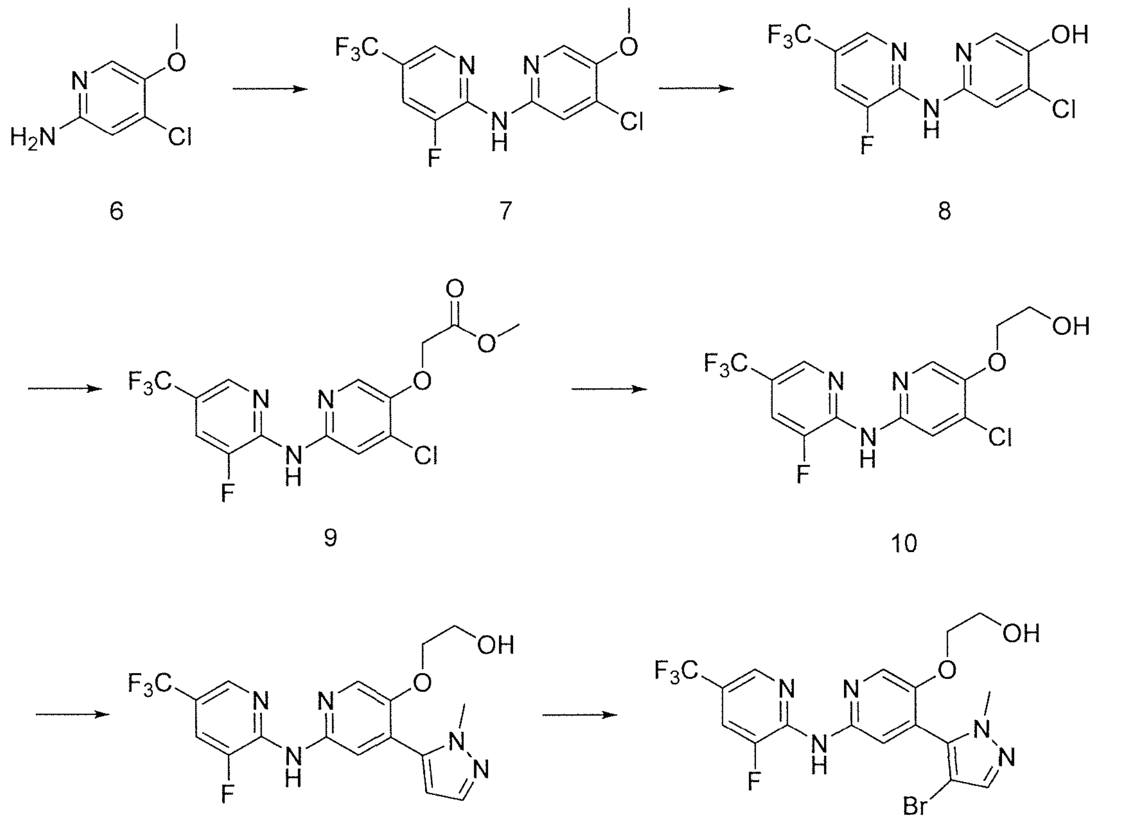

6 7 8

9 10

11 I-062

Step 1: Synthesis of Compound 7

Under a nitrogen atmosphere, Compound 6 (1.00 g, 6.32 mmol) and 2,3-difluoro-5-trifluoromethylpyridine (1.16 g, 6.32 mmol) were dissolved in dimethylformamide (10 mL), and a solution of potassium tert-butoxide (3.47 g, 25.1 mmol)-tetrahydrofuran (5 mL) was added under ice-cooling and stirred for 10 minutes under ice-cooling. Saturated aqueous ammonium chloride (15 mL) and water (5 mL) were added and stirred for 5 minutes.

The precipitated solid was filtered off and dried to give Compound 7 (2.03 g, yield: 63%).

1H-NMR (CDCl3) δ: 8.64 (1H, s), 8.37 (1H, s), 7.96 (1H, s), 7.59 (1H, s), 7.51 (1H, d, J = 10.5 Hz), 3.98 (3H, s).

Step 2: Synthesis of Compound 8

Under a nitrogen atmosphere, Compound 7 (1.29 g, 4.00 mmol) was dissolved in dichloromethane (13 mL) and a 1 mol/L boron tribromide-dichloromethane solution (28 mL, 28.0 mmol) was added under ice-cooling and stirred at room temperature for 15 hours. 1 mol/L aqueous sodium hydroxide solution (60 mL) was added under ice-cooling and stirred for

5 minutes. The precipitated solid was filtered off and dried to give Compound 8 (1.22 g, yield: 99%).

1H-NMR (CDCl3) δ: 8.64 (1H, s), 8.37 (1H, d, J = 1.0 Hz), 8.09 (1H, s), 7.63 (1H, s), 7.52 (1H, dd, J = 10.5, 1.0 Hz), 5.35 (1H, br s).

Step 3: Synthesis of Compound 9

Under a nitrogen atmosphere, Compound 8 (1.02 g, 3.30 mmol) was dissolved in dimethylformamide (50 mL), and potassium carbonate (0.48 g, 3.47 mmol) and methyl bromoacetate (0.31 mL, 3.34 mmol) were added and stirred at room temperature for 1 hour. Water (15 mL) was added under ice-cooling and stirred for 5 minutes. The precipitated solid was filtered off and dried to give Compound 9 (1.11 g, yield: 89%).

1H-NMR (CDCl3) δ: 8.68 (1H, s), 8.38 (1H, s), 7.98 (1H, s), 7.63 (1H, s), 7.53 (1H, d, J = 9.9 Hz), 4.74 (2H, s), 3.83 (3H, s).

Step 4: Synthesis of Compound 10

Under a nitrogen atmosphere, Compound 9 (1.11 g, 2.93 mmol) was dissolved in methanol (11 mL)-tetrahydrofuran (11 mL), sodium borohydride (0.55 g, 14.6 mmol) was added under ice-cooling and stirred at room temperature for 15 hours. Under ice-cooling, saturated aqueous ammonium chloride (10 mL) and water (10 mL) were added and stirred for 5 minutes. The precipitated solid was filtered off and dried to give Compound 10 (0.953 g, yield: 93%).

1H-NMR (CDCl3) δ: 8.66 (1H, s), 8.38 (1H, s), 8.01 (1H, s), 7.62 (1H, s), 7.52 (1H, d, J = 10.4 Hz), 4.23-4.21 (2H, br m), 4.03-4.01 (2H, br m), 2.17 (1H, br s).

Step 5: Synthesis of Compound 11

Compound 10 (379 mg, 1.08 mmol) was dissolved in 1,4-dioxane (3.8 mL)-water (0.80 mL), and 1-methyl-5-(4,4,5,5-tetramethyl-1,3,2-dioxaborolan-2-yl)-1H-pyrazole (449 mg, 2.16 mmol), [1,1-bis(di-tert-butylphosphino)ferrocene]dichloropalladium (70.3 mg, 0.11 mmol), and potassium carbonate (298 mg, 2.16 mmol) were added and stirred at 100°C for 4 hours. Ethyl acetate (10 mL) and water (5 mL) were added and extracted with ethyl acetate. The organic layer was washed with brine (5 mL) and then dried over anhydrous sodium sulfate. The solvent was removed under reduced pressure and the resulting residue was purified by silica gel column chromatography (hexane-ethyl acetate) to give Compound 11 (222 mg, yield: 52%).

1H-NMR (CDCl3) δ: 8.52 (1H, s), 8.31 (1H, s), 8.11 (1H, s), 7.67 (1H, s), 7.59 (1H, d, J = 1.9 Hz), 7.52 (1H, d, J = 10.5 Hz), 6.46 (1H, d, J = 1.9 Hz), 4.15-4.14 (2H, br m), 3.89-3.87 (5H, br m), 1.77 (1H, t, J = 6.1 Hz).

Step 6: Synthesis of Compound I-062

Compound 11 (77.2 mg, 0.194 mmol) was dissolved in dimethylformamide (0.77 mL), and N-bromosuccinimide (38.0 mg, 0.214 mmol) was added under ice-cooling and stirred at room temperature for 2 hours. 10% sodium thiosulfate (0.4 mL) and saturated sodium bicarbonate solution (0.4 mL) were added and stirred for 5 minutes. The precipitated solid was filtered off and dried to give Compound I-062 (77.5 mg, yield: 84%). 1H-NMR (CDCl3) δ: 8.51 (1H, s), 8.32 (1H, s), 8.16 (1H, s), 7.74 (1H, d, J = 1.8 Hz), 7.58 (1H, s), 7.52 (1H, dd, J = 10.5, 1.8 Hz), 4.18-4.16 (2H, br m), 3.89 (2H, dd, J = 4.5, 2.3 Hz), 3.84 (3H, s), 2.03 (1H, br s).

Example 4: Synthesis of Compound I-063

[Chemical formula 62]

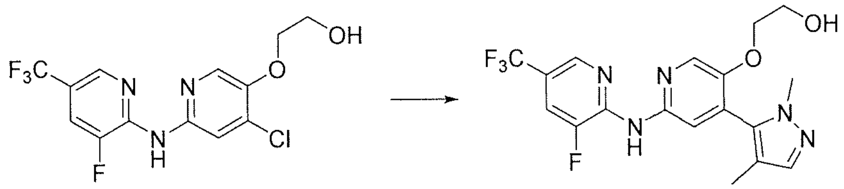

10 I-063

Step 1: Synthesis of Compound I-063

Compound 10 (1.02 g, 2.89 mmol) was dissolved in 1,4-dioxane (10 mL)-water (2 mL), and 1,4-dimethyl-5-(4,4,5,5-tetramethyl-1,3,2-dioxaborolan-2-yl)-1H-pyrazole (1.28 g, 5.78 mmol), [1,1-bis(di-tert-butylphosphino)ferrocene]dichloropalladium(188 mg, 0.29 mmol), and potassium carbonate (799 mg, 5.78 mmol) were added and stirred at 100°C for 4 hours. Ethyl acetate (20 mL) and water (10 mL) were added and extracted with ethyl acetate. The organic layer was washed with brine (10 mL) and then dried over anhydrous sodium sulfate. The solvent was removed under reduced pressure and the resulting residue was purified by silica gel column chromatography (hexane-ethyl acetate, ethyl acetate-methanol) to give Compound I-063 (740 mg, yield: 62%). 1H-NMR (CDCl3) δ: 8.43 (1H, s), 8.30 (1H, s), 8.14 (1H, s), 7.68 (1H, s), 7.52 (1H, d, J = 8.7 Hz), 7.43 (1H, s), 4.12-4.09 (2H, br m), 3.86-3.84 (2H, m), 3.78 (3H, s), 2.04 (3H, s), 1.77 (1H, t, J = 6.3 Hz).

Example 5: Synthesis of Compound I-073

[Chemical formula 63]

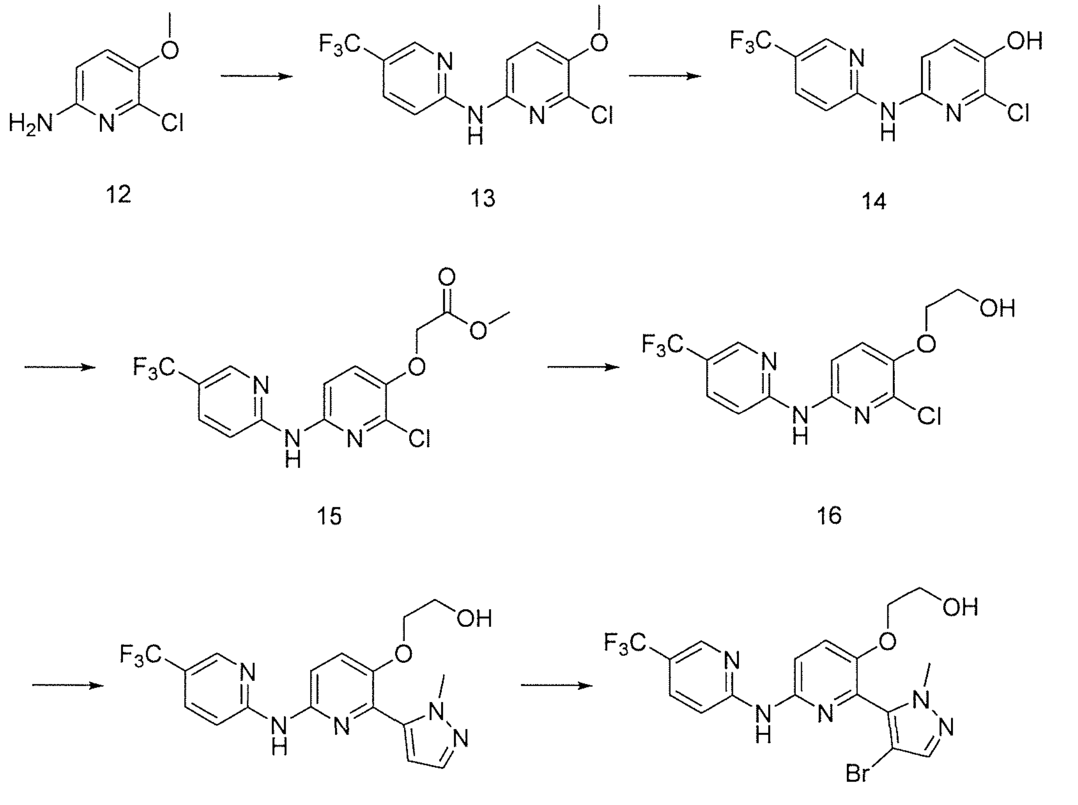

12 13 14

15 16

17 I-073

Step 1: Synthesis of Compound 13

Under a nitrogen atmosphere, Compound 12 (503 mg, 3.17 mmol) and 2-fluoro-5-trifluoromethylpyridine (374 μL, 3.17 mmol) were dissolved in dimethylformamide (5 mL), and a solution of potassium tert-butoxide (748 mg, 25.1 mmol)-tetrahydrofuran (2.5 mL) was added under ice-cooling and stirred for 10 minutes under ice-cooling. Saturated aqueous ammonium chloride (15 mL) and water (5 mL) were added and stirred for 5 minutes. The precipitated solid was filtered off and dried to give Compound 13 (638 g, yield: 66%).

1H-NMR (CDCl3) δ: 8.49 (1H, s), 7.79-7.74 (2H, m), 7.43 (1H, s), 7.30 (2H, d, J = 8.8 Hz), 3.91 (3H, s).

Step 2: Synthesis of Compound 14

Under a nitrogen atmosphere, Compound 13 (617 mg, 2.00 mmol) was dissolved in dichloromethane (6 mL) and a 1 mol/L boron tribromide-dichloromethane solution (16.2 mL, 16.2 mmol) was added under ice-cooling and stirred at room temperature for 15 hours. 1 mol/L aqueous sodium hydroxide solution (20 mL) was added under ice-cooling and stirred for 5 minutes. The precipitated solid was filtered off and dried to give Compound 14 (512 mg, yield: 86%).
1H-NMR (CDCl3) δ: 8.49 (1H, s), 7.79 (1H, dd, J = 8.8, 2.4 Hz), 7.58 (1H, d, J = 8.7 Hz), 7.44 (1H, d, J = 8.8 Hz), 7.38-7.35 (2H, m), 5.24 (1H, br s).

Step 3: Synthesis of Compound 15

Under a nitrogen atmosphere, Compound 14 (375 mg, 1.29 mmol) was dissolved in dimethylformamide (3.7 mL), and potassium carbonate (197 mg, 1.42 mmol) and methyl bromoacetate (141 μL, 1.31 mmol) were added and stirred at room temperature for 1 hour. Water (15 mL) was added under ice-cooling and stirred for 5 minutes. The precipitated solid was filtered off and dried to give Compound 15 (436 mg, yield: 93%).
1H-NMR (CDCl3) δ: 8.50 (1H, s), 7.79 (1H, dd, J = 8.8, 2.4 Hz), 7.71 (1H, d, J = 8.8 Hz), 7.40 (1H, br s), 7.35-7.33 (2H, m), 4.70 (2H, s), 3.82 (3H, s).

Step 4: Synthesis of Compound 16

Under a nitrogen atmosphere, Compound 15 (436 mg, 1.20 mmol) was dissolved in methanol (4.3 mL)-tetrahydrofuran (4.3 mL), and sodium borohydride (228 mg, 6.02 mmol) was added under ice-cooling and stirred at room temperature for 15 hours. Under ice-cooling, saturated aqueous ammonium chloride solution (5 mL) and water (5 mL) were added and stirred for 5 minutes. Ethyl acetate (10 mL) was added and extracted with ethyl acetate. The organic layer was washed with water (10 mL) and brine (10 mL), and then dried over anhydrous sodium sulfate. The solvent was removed under reduced pressure and the resulting residue was purified by silica gel column chromatography (hexane-ethyl acetate) to give Compound 16 (414 mg, yield: 100%).
1H-NMR (CDCl3) δ: 8.50 (1H, s), 7.78 (1H, dd, J = 8.8, 2.3 Hz), 7.74 (1H, d, J = 8.8 Hz), 7.42 (1H, s), 7.35-7.33 (2H, m), 4.16-4.15 (2H, m), 4.01-3.99 (2H, m), 2.20 (1H, t, J = 6.4 Hz).

Step 5: Synthesis of Compound 17

Compound 16 (99.6 mg, 0.297 mmol) was dissolved in 1,4-dioxane (1 mL)-water (0.20 ml), and 1-methyl-5-(4,4,5,5-tetramethyl-1,3,2-dioxaborolan-2-yl)-1H-pyrazole (124 mg, 0.60 mmol), [1,1-bis(di-tert-butylphosphino)ferrocene]dichloropalladium (19.5 mg, 0.03 mmol), and potassium carbonate (124 mg, 0.60 mmol) were added and stirred at 100°C for 4 hours. Ethyl acetate (10 mL) and water (5 mL) were added and extracted with ethyl acetate. The organic layer was washed with brine (5 mL) and then dried over anhydrous sodium sulfate. The solvent was removed under reduced pressure, and the resulting residue was purified by silica gel column chromatography (hexane-ethyl acetate, ethyl acetate-methanol) to give Compound 17 (62 mg, yield: 55%).
1H-NMR (CDCl3) δ: 8.51 (1H, s), 7.77-7.73 (2H, m), 7.56 (2H, d, J = 1.9 Hz), 7.43-7.40 (2H, m), 6.68 (1H, d, J = 1.9 Hz), 4.13-4.11 (2H, m), 4.04 (3H, s), 3.94-3.92 (2H, m), 2.07-2.06 (1H, br m).

Step 6: Synthesis of Compound I-073

Compound 17 (56.4 mg, 0.149 mmol) was dissolved in dimethylformamide (564 μL), and N-bromosuccinimide (29.1 mg, 0.164 mmol) was added under ice-cooling and stirred at room temperature for 1 hour. 10% sodium thiosulfate (0.2 mL), saturated sodium bicarbonate solution (0.2 mL), and ethyl acetate (5 mL) were added and stirred for 5 minutes, and then extracted with ethyl acetate. The organic layer was washed with water (5 mL) and brine (5 mL), and then dried over anhydrous sodium sulfate. The solvent was removed under reduced pressure, and the resulting residue was purified by silica gel column chromatography (hexane-ethyl acetate, ethyl acetate-methanol) to give Compound I-073 (9.8 mg, yield: 14%). 1H-NMR (CDCl3) δ: 8.50 (1H, s), 7.81 (1H, d, J = 9.2 Hz), 7.77 (1H, dd, J = 8.8, 2.4 Hz), 7.69 (1H, br s), 7.55 (1H, s), 7.48-7.45 (2H, m), 4.12-4.11 (2H, m), 3.91-3.89 (2H, m), 3.84 (3H, s), 2.14 (1H, t, J = 6.3 Hz).

Example 6: Synthesis of Compound I-082

[Chemical formula 64]

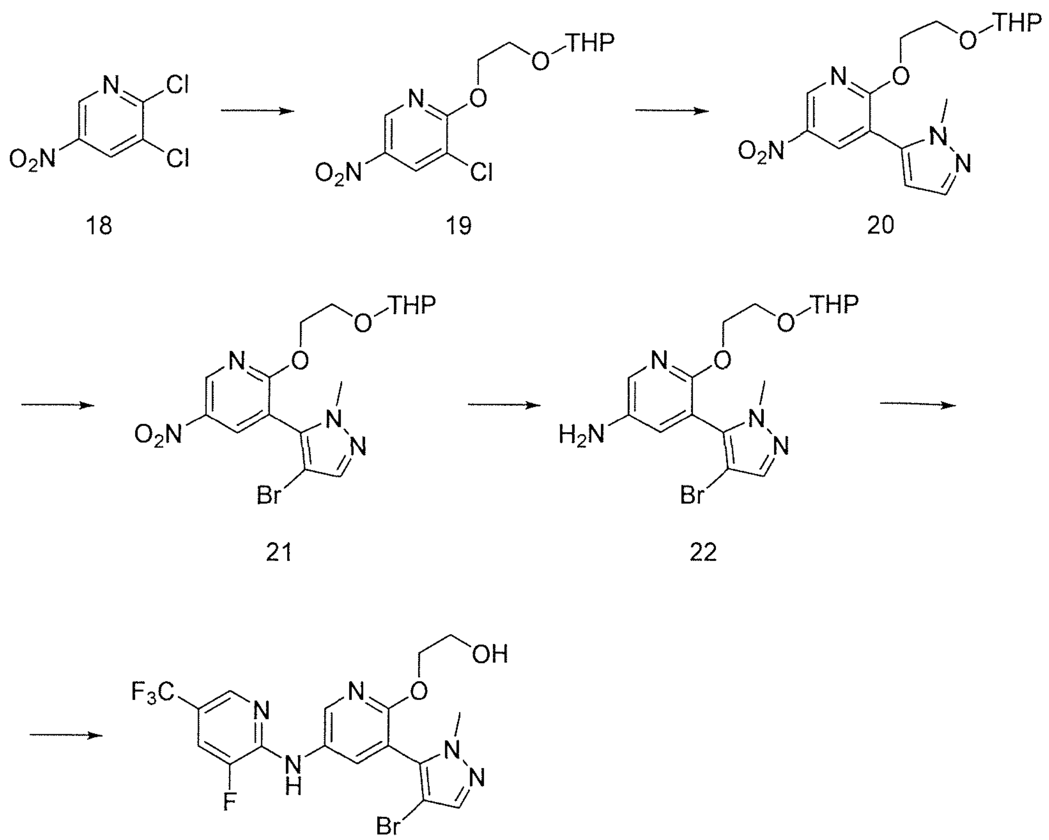

18 19 20

21 22

I-082

Step 1: Synthesis of Compound 19

Under ice-cooling, tetrahydropyranyl ethylene glycol (3.87 mL, 28.5 mmol) was added dropwise to a solution of sodium hydride (1.14 g, 28.5 mmol)-THF (50 mL) and stirred for 10 minutes. Under ice-cooling, a solution of Compound 18 (5.0 g, 25.9 mmol)-THF (25 mL) was added dropwise and stirred at room temperature for 1 hour. Saturated aqueous ammonium chloride solution (20 mL) and water (10 mL) were added and stirred for 5 minutes. Ethyl acetate (10 mL) was added and extracted with ethyl acetate. The organic layer was washed with water (20 mL) and brine (20 mL), and then dried over anhydrous sodium sulfate. The solvent was removed under reduced pressure and the resulting residue was purified by silica gel column chromatography (hexane-ethyl acetate) to give Compound 19 (6.62 g, yield: 84%).

1H-NMR (CDCl3) δ: 8.96 (1H, d, J = 2.5 Hz), 8.45 (1H, d, J = 2.5 Hz), 4.73-4.70 (3H, m), 4.13-4.10 (1H, m), 3.90-3.87 (2H, m), 3.55-3.52 (1H, m), 1.83-1.71 (2H, m), 1.63-1.62 (2H, br m), 1.54-1.52 (2H, br m).

Step 2: Synthesis of Compound 20

Under a nitrogen atmosphere, Compound 19 (3.13 g, 10.3 mmol) was dissolved in dioxane (31 mL)-water (6 mL), and 1-methyl-5-(4,4,5,5-tetramethyl-1,3,2-dioxaborolan-2-yl)-1H-pyrazole (4.30 g, 20.7 mmol), XPhos Pd G3 (438 mg, 0.52 mmol), and potassium carbonate (4.29 g, 31.0 mmol) were added and stirred at 100°C for 2 hours. After cooling, water (10 mL) and ethyl acetate (20 mL) were added, filtered through celite (registered trademark), and extracted with ethyl acetate. The organic layer was washed with water (20 mL) and brine (20 mL), and then dried over anhydrous sodium sulfate. The solvent was removed under reduced pressure, and the resulting residue was purified by silica gel column chromatography (hexane-ethyl acetate) to give Compound 20 (3.6 g, yield: 90%).

1H-NMR (CDCl3) δ: 9.12 (1H, d, J = 2.8 Hz), 8.38 (1H, d, J = 2.8 Hz), 7.55 (1H, d, J = 1.8 Hz), 6.38 (1H, d, J = 1.8 Hz), 4.69-4.67 (2H, m), 4.61 (1H, t, J = 3.4 Hz), 4.08-4.02 (1H, m), 3.84 (3H, s), 3.76-3.74 (2H, m), 3.47-3.44 (1H, br m), 1.73-1.69 (3H, m), 1.53-1.51 (3H, br m).

Step 3: Synthesis of Compound 21

Under a nitrogen atmosphere, Compound 20 (2.02 g, 5.79 mmol) was dissolved in dimethylformamide (20 mL), and N-bromosuccinimide (1.13 g, 6.37 mmol) was added under ice-cooling, and stirred at room temperature for 1 hour. 10% sodium thiosulfate (20 mL), saturated sodium bicarbonate solution (10 mL), and ethyl acetate (20 mL) were added and stirred for 5 minutes, and then extracted with ethyl acetate. The organic layer was washed with water (10 mL) and brine (10 mL), and then dried over anhydrous sodium sulfate. The solvent was removed under reduced pressure to give Compound 21 (2.52 g).

Step 4: Synthesis of Compound 22

Under a nitrogen atmosphere, Compound 21 was dissolved in ethanol (50 mL) and water (13 mL), and iron (1.32 g, 23.6 mmol) and ammonium chloride (3.15 g, 59.0 mmol) were added and stirred at 70°C for 1 hour. After cooling, water (20 mL) was added and filtered through celite (registered trademark) and extracted with ethyl acetate (30 mL). The organic layer was washed with water (20 mL) and brine (20 mL), and then dried over anhydrous sodium sulfate. The solvent was removed under reduced pressure, and the resulting residue was purified by silica gel column chromatography (hexane-ethyl acetate, ethyl acetate-methanol) to give Compound 22 (1.9 g, yield: 80%). 1H-NMR (CDCl3) δ: 7.76 (1H, d, J = 2.9 Hz), 7.50 (1H, s), 7.06 (1H, d, J = 2.9 Hz), 4.57 (1H, t, J = 3.3 Hz), 4.50-4.47 (1H, br m), 4.41-4.38 (1H, br m), 3.98-3.95 (1H, br m), 3.78 (3H, s), 3.72-3.69 (2H, br m), 3.44-3.42 (1H, br m), 1.76-1.68 (3H, br m), 1.52-1.47 (5H, br m).

Step 5: Synthesis of Compound I-082

Under a nitrogen atmosphere, Compound 22 (436 mg, 1.20 mmol) was dissolved in 2-propanol (381 μL), and 2,3-difluoro-5-trifluoromethylpyridine (13.3 μL, 0.11 mmol) and p-toluenesulfonic acid monohydrate (25.4 mg, 0.13 mmol) were added and stirred at 100°C for 18 hours. After cooling, saturated sodium bicarbonate solution (0.5 mL) and water (0.5 mL) were added and stirred for 5 minutes. The precipitated solid was filtered off and dried to give Compound I-082 (9.2 mg, yield: 22%).

1H-NMR (CDCl3) δ: 8.49 (1H, d, J = 2.8 Hz), 8.26 (1H, s), 8.11 (1H, d, J = 2.8 Hz), 7.56 (1H, s), 7.51 (1H, dd, J = 10.8, 1.9 Hz), 6.89 (1H, d, J = 3.3 Hz), 4.54-4.52 (2H, m), 3.94-3.92 (2H, m), 3.82 (3H, s), 3.18 (1H, t, J = 5.5 Hz).

Example 7: Synthesis of Compound I-064

[Chemical formula 65]

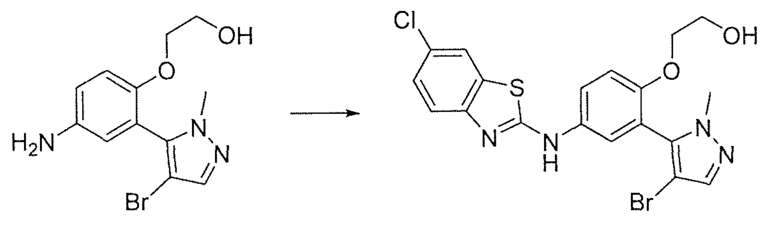

5 I-064

Step 1: Synthesis of Compound I-064

Compound 5 (25 mg, 0.080 mmol) was dissolved in 2-propanol (0.25 mL), and 2,6-dichlorobenzthiazole (18 mg, 0.088 mmol) and p-toluenesulfonic acid monohydrate (7.6 mg, 0.040 mmol) were added and stirred at 80°C for 1.5 hours. The resultant was further stirred at 100°C for 2.5 hours. After cooling, water (0.25 mL) was added and stirred for 5 minutes. The precipitated solid was washed with 2-propanol and hexane, then filtered off and dried to give Compound I-064 (23 mg, yield: 61%) .

1H-NMR (DMSO-D6) δ: 3.63-3.69 (2H, m), 3.71 (3H, s), 4.01-4.07 (2H, m), 4.86 (1H, br s), 7.25 (1H, d, J = 9.1 Hz), 7.32 (1H, dd, J = 8.6, 2.2 Hz), 7.53 (1H, d, J = 8.6 Hz), 7.63 (1H, d, J = 2.8 Hz), 7.64 (1H, s), 7.89 (1H, dd, J = 9.1, 2.8 Hz), 7.93 (1H, d, J = 2.2 Hz), 10.56 (1H, s).

Example 8: Synthesis of Compound I-031

[Chemical formula 66]

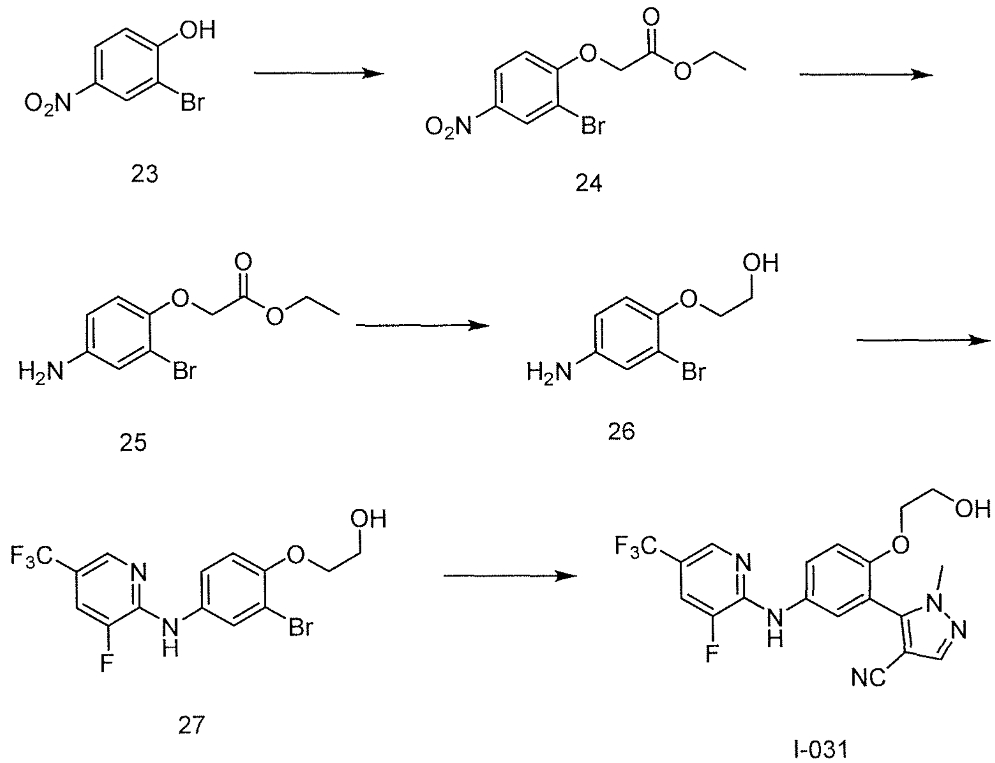

23 24 25 26 27 I-031

Step 1: Synthesis of Compound 24

Compound 24 (8.9 g, yield: 100%) was synthesized in the same manner as in Step 1 of Example 1 by using Compound 23 and ethyl bromoacetate.

$^1$H-NMR ($CDCl_3$) δ: 1.31 (3H, t, J = 7.2 Hz), 4.29 (2H, q, J = 7.2 Hz), 4.82 (2H, s), 6.83 (1H, d, J = 9.2 Hz), 8.19 (1H, dd, J = 9.2, 2.8 Hz), 8.50 (1H, d, J = 2.8 Hz).

Step 2: Synthesis of Compound 25

Compound 25 (8.9 g, yield: 99%) was synthesized in the same manner as in Step 4 of Example 1 by using Compound 24.

$^1$H-NMR ($CDCl_3$) δ: 1.29 (3H, t, J = 7.2 Hz), 3.52 (2H, brs), 4.26 (2H, q, J = 7.2 Hz), 4.58 (2H, s), 6.56 (1H, dd, J = 8.7, 2.8 Hz), 6.77 (1H, d, J = 8.7 Hz), 6.92 (1H, d, J = 2.8 Hz).

Step 3: Synthesis of Compound 26

Compound 26 (7.5 g, yield: 99%) was synthesized in the same manner as in Step 3 of Example 1 by using Compound 25.

$^1$H-NMR ($CDCl_3$) 2.27 (1H, brs), 3.51 (2H, brs), 3.92 (2H, t, J = 4.3 Hz), 4.06 (2H, dd, J = 4.8, 4.3 Hz), 6.59 (1H, dd, J = 8.7, 2.8 Hz), 6.80 (1H, d, J = 8.7 Hz), 6.91 (1H, d, J = 2.8 Hz).

Step 4: Synthesis of Compound 27

Compound 27 (3.5 g, yield: 92%) was synthesized in the same manner as in Step 1 of Example 2 by using Compound 26.

$^1$H-NMR ($CDCl_3$) 2.22 (1H, t, J = 6.5 Hz), 3.97-4.01 (2H, m), 4.15 (2H, t, J = 4.5 Hz), 6.72 (1H, brs), 6.95 (1H, d, J =

8.9 Hz), 7.46 (1H, dd, J = 10.9, 1.9 Hz), 7.52 (1H, dd, J = 8.8, 2.7 Hz), 7.94 (1H, d, J = 2.7 Hz), 8.28 (1H, s).

Step 5: Synthesis of Compound I-031

Under a nitrogen atmosphere, Compound 27 (20 mg, 0.051 mmol) was dissolved in dioxane (400 μL), and 1-methyl-5-(4,4,5,5-tetramethyl-1,3,2-dioxaborolan-2-yl)-1H-pyrazole-4-carbonitrile (24 mg, 0.101 mmol), 2 mol/L aqueous sodium carbonate solution (76 μL, 0.152 mmol), and $PdCl_2$ (dtbpf) (3.3 mg, 5.1 μmol) were added and stirred at 80°C for 5 hours. After cooling, water was added and extracted with chloroform. The organic layer was dried over anhydrous magnesium sulfate. The solvent was removed under reduced pressure, and the resulting residue was purified by silica gel column chromatography (chloroform-methanol) to give Compound I-031 (11.4 mg, yield: 53%).
$^1$H-NMR ($CDCl_3$) δ: 2.18 (1H, t, J = 6.3 Hz), 3.89 (3H, s), 3.94 (2H, dd, J = 9.8, 5.1 Hz), 4.17 (2H, t, J = 4.3 Hz), 6.86 (1H, s), 7.09 (1H, d, J = 9.0 Hz), 7.48 (1H, dd, J = 10.7, 1.8 Hz), 7.68 (1H, dd, J = 8.9, 2.8 Hz), 7.75 (1H, d, J = 2.8 Hz), 7.87 (1H, s), 8.25 (1H, s).

Example 9: Synthesis of Compound I-058

[Chemical formula 67]

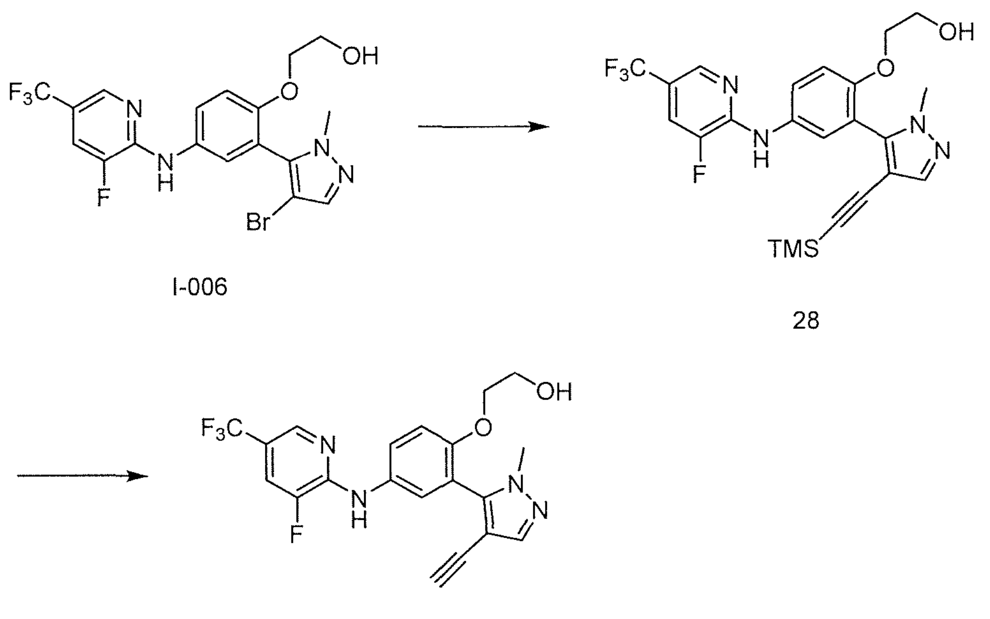

I-006

28

I-058

Step 1: Synthesis of Compound 28

Under a nitrogen atmosphere, Compound I-006 (20 mg, 0.042 mmol) was dissolved in dioxane (300 μL), and trimethyl((4,4,5,5-tetramethyl-1,3,2-dioxaborolan-2-yl)ethynyl)silane (18.9 mg, 0.084 mmol), 2 mol/L aqueous sodium carbonate solution (63 μL, 0.126 mmol), and $PdCl_2$(dtbpf) (2.7 mg, 4.2 μmol) were added and stirred at 80°C for 3.5 hours. After cooling, water was added and extracted with chloroform. The organic layer was dried over anhydrous magnesium sulfate. The solvent was removed under reduced pressure, and the resulting residue was purified by silica gel column chromatography (hexane-ethyl acetate) to give Compound 28 (8.1 mg, yield: 39%).
$^1$H-NMR ($CDCl_3$) δ: 0.11 (9H, s), 2.25 (1H, t, J = 6.3 Hz), 3.80 (3H, s), 3.79-3.91 (2H, m), 4.11 (2H, t, J = 4.9 Hz), 6.78 (1H, s), 7.04 (1H, d, J = 8.9 Hz), 7.46 (1H, d, J = 10.9 Hz), 7.63 (1H, d, J = 2.8 Hz), 7.66 (1H, s), 7.73 (1H, dd, J = 8.9, 2.8 Hz), 8.26 (1H, s).

Step 2: Synthesis of Compound I-058

Compound 28 (8.1 mg, 0.042 mmol) was dissolved in tetrahydrofuran (100 μL), and 1 mol/L tetrabutylammonium fluoride tetrahydrofuran solution (32 μL, 0.021 mmol) was added and stirred at room temperature for 1 hour. The solvent

was removed under reduced pressure, and the resulting residue was purified by silica gel column chromatography (hexane-ethyl acetate) to give Compound I-058 (4.1 mg, yield: 60%).

$^1$H-NMR ($CDCl_3$) δ: 2.13 (1H, t, J = 6.2 Hz), 2.98 (1H, s), 3.79 (3H, s), 3.85-3.90 (2H, m), 4.11 (2H, t, J = 4.3 Hz), 6.81 (1H, s), 7.06 (1H, d, J = 8.9 Hz), 7.47 (1H, d, J = 10.7 Hz), 7.61 (1H, d, J = 1.6 Hz), 7.69 (1H, s), 7.74 (1H, t, J = 4.5 Hz), 8.25 (1H, s).

Example 10: Synthesis of Compound I-054

[Chemical formula 68]

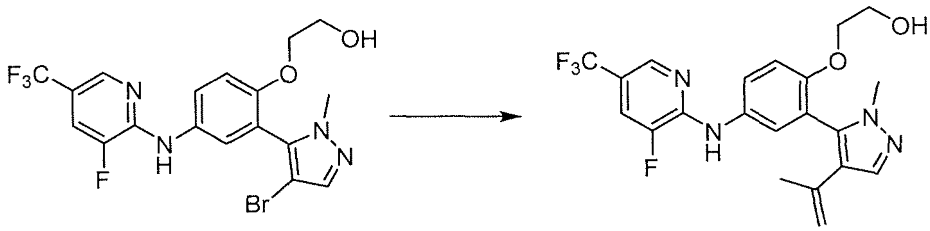

I-006 I-054

Step 1: Synthesis of Compound I-054

Under a nitrogen atmosphere, Compound I-006 (30 mg, 0.063 mmol) was dissolved in dioxane (600 μL), and 4,4,5,5-tetramethyl-2-(prop-1-en-2-yl)-1,3,2-dioxaborolane (21 mg, 0.126 mmol), 2 mol/L aqueous sodium carbonate solution (95 μL, 0.189 mmol), and $PdCl_2$(dtbpf) (4.1 mg, 6.3 μmol) were added and stirred at 80°C for 6.5 hours. After cooling, water was added and extracted with chloroform. The organic layer was dried over anhydrous magnesium sulfate. The solvent was removed under reduced pressure, and the resulting residue was purified by silica gel column chromatography (hexane-ethyl acetate) to give Compound I-054 (18.1 mg, yield: 66%).

$^1$H-NMR ($CDCl_3$) δ: 1.90 (3H, s), 1.92 (1H, t, J = 6.0 Hz), 3.70 (3H, s), 3.76-3.81 (2H, m), 4.02-4.09 (2H, m), 4.76 (1H, t, J = 1.6 Hz), 4.84 (1H, s), 6.80 (1H, s), 7.03 (1H, d, J = 8.9 Hz), 7.46 (1H, dd, J = 10.8, 2.0 Hz), 7.53 (1H, d, J = 2.8 Hz), 7.59 (1H, s), 7.75 (1H, dd, J = 8.9, 2.8 Hz), 8.25 (1H, s).

Example 11: Synthesis of Compound I-056

[Chemical formula 69]

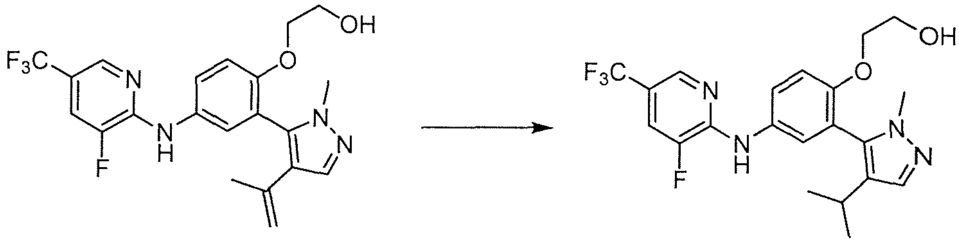

I-054 I-056

Step 1: Synthesis of Compound I-056

Compound I-054 (10 mg, 0.023 mmol) was dissolved in tetrahydrofuran (1 mL), and 5% palladium carbon (50% wet) (5 mg, 2.3 μmol) was added and stirred at room temperature under a hydrogen atmosphere for 5 hours. After filtration through celite (registered trademark), the solvent was removed under reduced pressure, and the resulting residue was

purified by silica gel column chromatography (chloroform-methanol) to give Compound I-056 (9.8 mg, yield: 97%).

$^1$H-NMR ($CDCl_3$) δ: 1.09 (3H, d, J = 6.9 Hz), 1.19 (3H, d, J = 6.9 Hz), 1.81 (1H, t, J = 6.4 Hz), 2.68-2.75 (1H, m), 3.68 (3H, s), 3.76-3.82 (2H, m), 3.98-4.11 (2H, m), 6.82 (1H, s), 7.05 (1H, d, J = 8.8 Hz), 7.44 (1H, s), 7.46 (1H, dd, J = 10.8, 1.6 Hz), 7.55 (1H, d, J = 2.8 Hz), 7.68 (1H, dd, J = 8.8, 2.8 Hz), 8.23 (1H, s).

Example 12: Synthesis of Compound I-065

[Chemical formula 70]

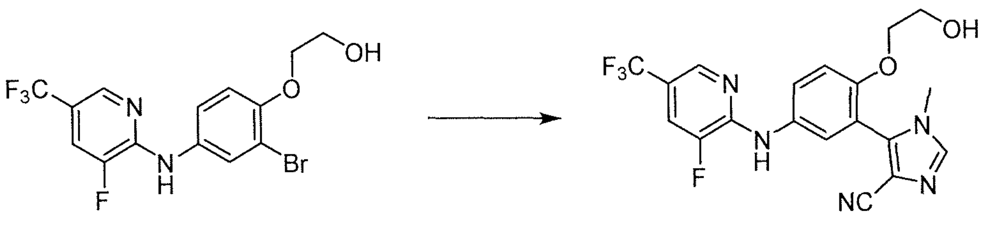

27 I-065

Step 1: Synthesis of Compound I-065

Under a nitrogen atmosphere, Compound 27 (50 mg, 0.127 mmol) was dissolved in dioxane (1.5 mL), and bis(pinacolate)diboron (96 mg, 0.380 mmol), potassium acetate (62 mg, 0.633 mmol), and $PdCl_2$(dppf) (46.3 mg, 63 μmol) were added and stirred at 100°C overnight. After cooling, 5-bromo-1-methyl-1H-imidazole-4-carbonitrile (70.6 mg, 0.380 mmol) and 2 mol/L aqueous sodium carbonate solution (380 μL, 0.759 mmol) were added and stirred at 100°C for 7 hours. After cooling, water was added and extracted with chloroform. The organic layer was dried over anhydrous magnesium sulfate. The solvent was removed under reduced pressure, and the resulting residue was purified by prep-HPLC (acetonitrile-water) to give Compound I-065 (8.9 mg, yield: 17%).

$^1$H-NMR (DMSO-D6) δ: 3.60 (3H, s), 3.65-3.70 (2H, m), 4.06 (2H, t, J = 4.9 Hz), 4.86 (1H, t, J = 5.3 Hz), 7.23 (1H, d, J = 9.0 Hz), 7.79 (1H, d, J = 2.7 Hz), 7.92 (1H, dd, J = 9.0, 2.7 Hz), 7.97 (1H, s), 7.98 (1H, dd, J = 11.6, 2.0 Hz), 8.30 (1H, s), 9.52 (1H, s).

Example 13: Synthesis of Compound I-108

[Chemical formula 71]

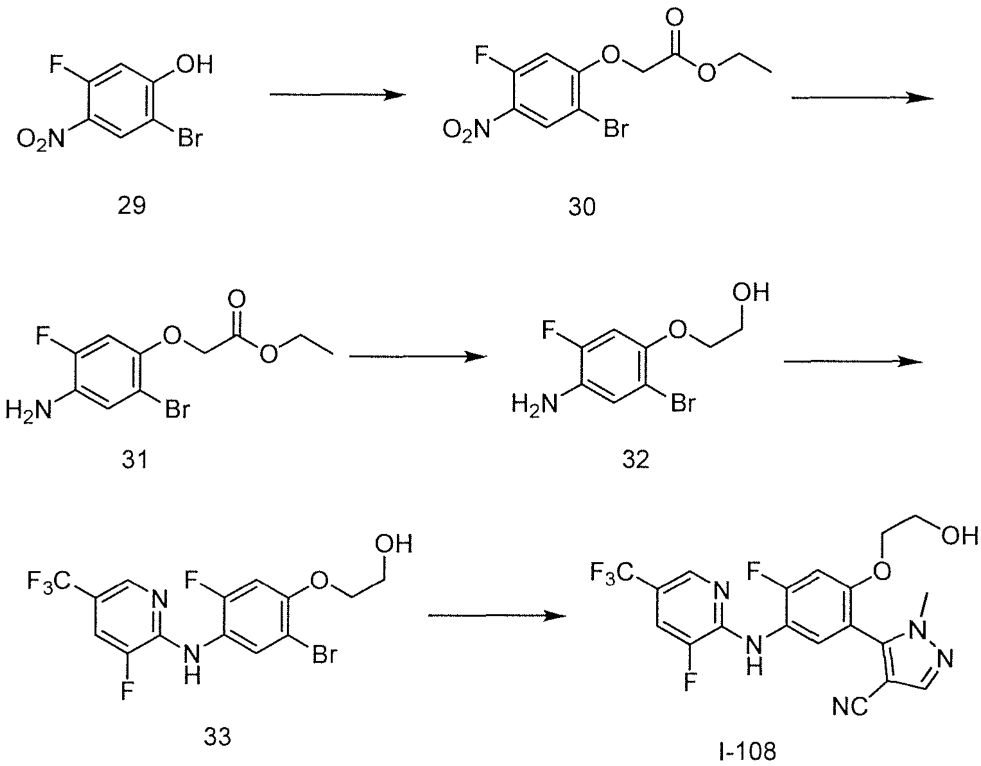

29

30

31

32

33

I-108

Step 1: Synthesis of Compound 30

Compound 30 (2.6 g, yield: 96%) was synthesized in the same manner as in Step 1 of Example 1 by using Compound 29 and ethyl bromoacetate.

$^1$H-NMR ($CDCl_3$) $\delta$: 1.32 (3H, t, J = 7.2 Hz), 4.31 (2H, q, J = 7.2 Hz), 4.79 (2H, s), 6.64 (1H, d, J = 11.9 Hz), 8.39 (1H, d, J = 8.0 Hz).

Step 2: Synthesis of Compound 31

Compound 31 (2.2 g, yield: 95%) was synthesized in the same manner as in Step 4 of Example 1 by using Compound 30.

$^1$H-NMR ($CDCl_3$) $\delta$: 1.30 (3H, t, J = 7.2 Hz), 3.55 (2H, brs), 4.27 (2H, q, J = 7.2 Hz), 4.58 (2H, s), 6.68 (1H, d, J = 11.8 Hz), 7.00 (1H, d, J = 9.2 Hz).

Step 3: Synthesis of Compound 32

Compound 32 (1.9 g, yield: 100%) was synthesized in the same manner as in Step 3 of Example 1 by using Compound 31.

$^1$H-NMR ($CDCl_3$) $\delta$: 2.21 (1H, t, J = 6.3 Hz), 3.53 (2H, brs), 3.91-3.97 (2H, m), 4.04 (2H, t, J = 4.5 Hz), 6.71 (1H, d, J = 11.9 Hz), 7.00 (1H, d, J = 9.3 Hz).

Step 4: Synthesis of Compound 33

Compound 33 (153 mg, yield: 82%) was synthesized in the same manner as in Step 1 of Example 2 by using Compound 32.

$^1$H-NMR ($CDCl_3$) $\delta$: 2.18 (1H, t, J = 6.5 Hz), 3.98-4.02 (2H, m), 4.12 (2H, t, J = 4.4 Hz), 6.80 (1H, s), 6.83 (1H, s), 7.50 (1H, dd, J = 10.7, 1.9 Hz), 8.32 (1H, s), 8.66 (1H, d, J = 8.7 Hz).

Step 5: Synthesis of Compound I-108

Compound I-108 (38 mg, yield: 72%) was synthesized in the same manner as in Step 5 of Example 8 by using Compound 33.

$^{1}$H-NMR ($CDCl_3$) δ: 2.30 (1H, t, J = 6.3 Hz), 3.90 (3H, s), 3.94-3.98 (2H, m), 4.16 (2H, t, J = 4.3 Hz), 6.95 (1H, d, J = 12.4 Hz), 6.95 (1H, brs), 7.52 (1H, dd, J = 10.5, 1.9 Hz), 7.87 (1H, s), 8.27 (1H, d, J = 1.9 Hz), 8.48 (1H, d, J = 8.8 Hz).

Example 14: Synthesis of Compound I-109

[Chemical formula 72]

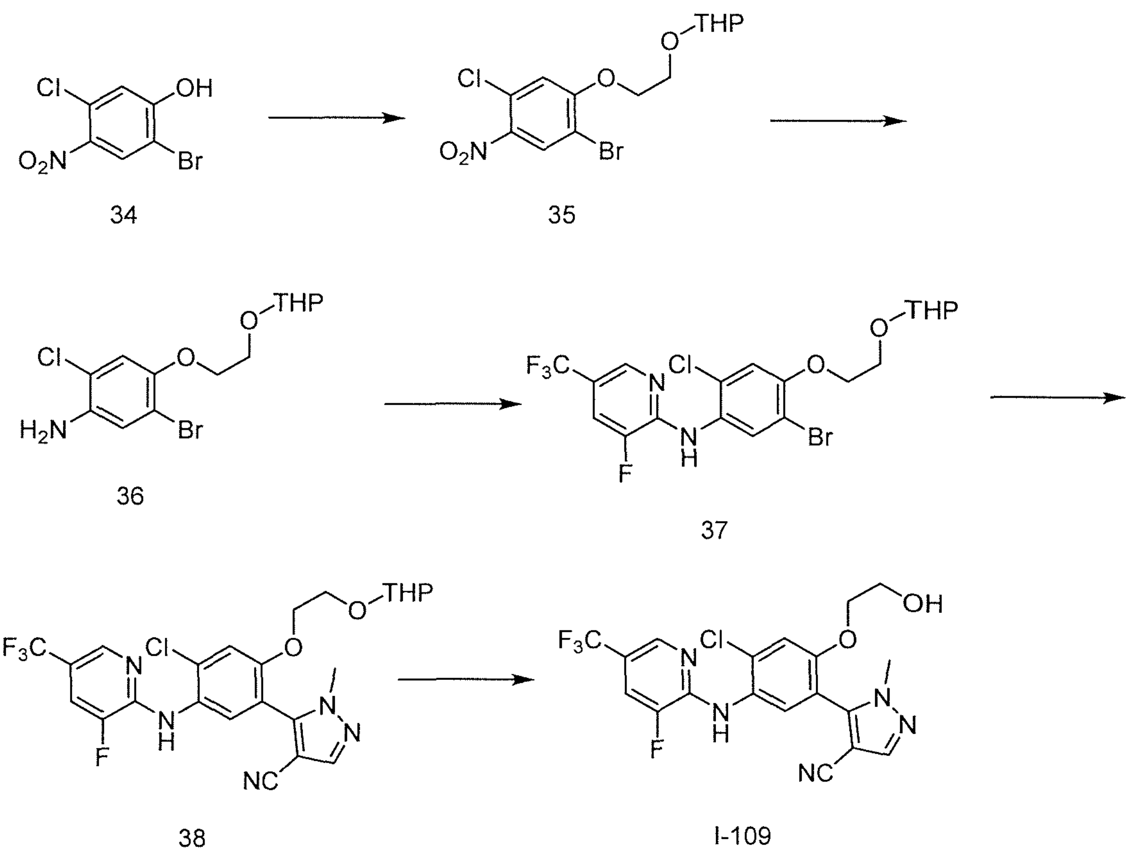

34 35 36 37 38 I-109

Step 1: Synthesis of Compound 35

Under a nitrogen atmosphere, THF (5 mL) was added to NaH (86 mg, 2.167 mmol), and 2-((tetrahydro-2H-pyran-2-yl)oxy)ethan-1-ol (293 μL, 2.162 mmol) was added at 0°C and stirred at the same temperature for 10 minutes. Compound 34 (500 mg, 1.965 mmol) was added and stirred at room temperature for 1 hour. Saturated aqueous ammonium chloride solution was added and extracted with chloroform. The organic layer was washed with water and brine, and dried over anhydrous magnesium sulfate. The solvent was removed under reduced pressure, and the resulting residue was purified by silica gel column chromatography (hexane-ethyl acetate) to give Compound 35 (596 mg, 80%).

$^{1}$H-NMR ($CDCl_3$) δ: 1.48-1.66 (4H, m), 1.70-1.86 (2H, m), 3.54-3.57 (1H, m), 3.86-3.93 (2H, m), 4.10-4.16 (1H, m), 4.31-4.34 (2H, m), 4.75 (1H, t, J = 3.3 Hz), 7.11 (1H, s), 8.27 (1H, s).

Step 2: Synthesis of Compound 36

Compound 36 (525 mg, yield: 80%) was synthesized in the same manner as in Step 4 of Example 1 by using Compound 35.

$^{1}$H-NMR ($CDCl_3$) δ: 1.45-1.68 (4H, m), 1.70-1.80 (1H, m), 1.80-1.90 (1H, m), 3.50-3.57 (1H, m), 3.78-3.85 (3H,

m), 3.89-3.96 (1H, m), 4.01-4.08 (1H, m), 4.12 (2H, t, J = 5.1 Hz), 4.76 (1H, t, J = 3.5 Hz), 6.95 (1H, s), 6.99 (1H, s).

Step 3: Synthesis of Compound 37

Compound 37 (140 mg, yield: 48%) was synthesized in the same manner as in Step 1 of Example 5 by using Compound 36.

$^1$H-NMR ($CDCl_3$) δ: 2.18 (1H, t, J = 6.5 Hz), 3.98-4.02 (2H, m), 4.12 (2H, t, J = 4.4 Hz), 6.82 (1H, d, J = 12.2 Hz), 6.82 (1H, s), 7.50 (1H, dd, J = 10.7, 1.9 Hz), 8.32 (1H, s), 8.66 (1H, d, J = 8.7 Hz).

Step 4: Synthesis of Compound 38

Compound 38 (15.2 mg, yield: 32%) was synthesized in the same manner as in Step 5 of Example 8 by using Compound 37.

$^1$H-NMR (CDCl3) δ: 1.47-1.60 (4H, m), 1.65-1.80 (2H, m), 3.44-3.50 (1H, m), 3.69-3.77 (2H, m), 3.86 (3H, s), 3.99-4.04 (1H, m), 4.19-4.23 (2H, m), 4.59 (1H, t, J = 3.5 Hz), 7.21 (1H, s), 7.52 (1H, dd, J = 10.5, 1.9 Hz), 7.84 (1H, s), 8.29 (1H, s), 8.58 (1H, s).

Step 5: Synthesis of Compound I-109

Compound 38 (14.5 mg, 0.027 mmol) was dissolved in methanol (1 mL), and p-toluenesulfonic acid monohydrate (1.0 mg, 5.4 μmol) was added and stirred at 40°C overnight. Saturated sodium bicarbonate solution was added, extracted with chloroform, and the organic layer was dried over anhydrous magnesium sulfate. The solvent was removed under reduced pressure, and the resulting residue was purified by silica gel column chromatography (chloroform-methanol) to give Compound I-109 (10.7 mg, yield: 87%).

$^1$H-NMR (CDCl3) δ: 2.31 (1H, t, J = 6.3 Hz), 3.92 (3H, s), 3.94-3.98 (2H, m), 4.17 (2H, t, J = 4.3 Hz), 7.18 (1H, s), 7.29 (1H, dd, J = 13.6, 2.2 Hz), 7.53 (1H, dd, J = 11.2, 2.2 Hz), 7.87 (1H, s), 8.27 (1H, s), 8.60 (1H, s).

Reference Example 1: Synthesis of Compound 40

[Chemical formula 73]

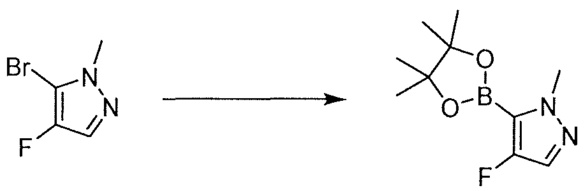

39

40

Step 1: Synthesis of Compound 40

Under a nitrogen atmosphere, Compound 39 (500 mg, 2.79 mmol) was dissolved in dioxane (10 mL), and bis(pinacolate)diboron (2.13 g, 8.38 mmol), potassium acetate (1.37 g, 0.633 mmol), and $PdCl_2$(dppf) (204 mg, 0.279 mmol) were added and stirred at 100°C for 2 hours. After cooling, the resultant was filtered through celite (registered trademark) to remove an insoluble. The solvent was removed under reduced pressure and the resulting residue was purified by column chromatography (hexane-ethyl acetate) to give Compound 40 (294 mg, yield: 47%) .

$^1$H-NMR (CDCl3) δ: 1.35 (12H, s), 3.98 (3H, s), 7.29 (1H, d, J = 4.4 Hz).

Example 15: Synthesis of Compound I-127

[Chemical formula 74]

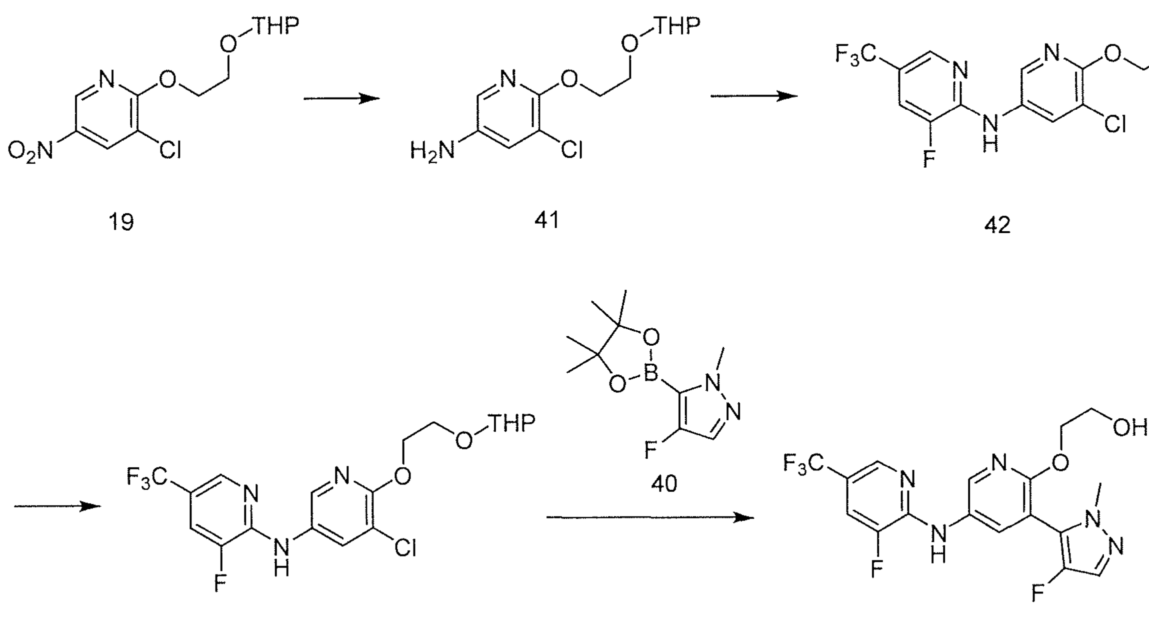

19 41 42

43 I-127

Step 1: Synthesis of Compound 41

Compound 41 (324 mg, yield: 60%) was synthesized in the same manner as in Step 4 of Example 1 by using Compound 19 obtained in Step 1 of Example 6.

$^1$H-NMR (CDCl3) δ: 1.48-1.66 (4H, m), 1.68-1.77 (1H, m), 1.78-1.89 (1H, m), 3.39 (2H, brs), 3.48-3.55 (1H, m), 3.79-3.86 (1H, m), 3.89-3.96 (1H, m), 4.01-4.07 (1H, m), 4.45-4.48 (2H, m), 4.75 (1H, t, J = 3.4 Hz), 7.11 (1H, d, J = 2.6 Hz), 7.53 (1H, d, J = 2.6 Hz).

Step 2: Synthesis of Compound 42

Compound 42 (133 mg, yield: 32%) was synthesized in the same manner as in Step 1 of Example 2 by using Compound 41.

$^1$H-NMR (CDCl3) δ: 2.95 (1H, t, J = 6.0 Hz), 3.98-4.02 (2H, m), 4.54 (2H, t, J = 4.5 Hz), 6.70 (1H, s), 7.50 (1H, dd, J = 10.9, 1.8 Hz), 8.21 (1H, d, J = 2.4 Hz), 8.24 (1H, d, J = 2.4 Hz), 8.28 (1H, s).

Step 3: Synthesis of Compound 43

Compound 42 (125 mg, 0.355 mmol) was dissolved in THF (2.5 mL), and 3,4-dihydro-2H-pyran (49 μL, 0.533 mmol) and p-toluenesulfonic acid monohydrate (3.33 mg, 0.018 mmol) were added and stirred at room temperature for 19 hours. Further 3,4-dihydro-2H-pyran (49 μL, 0.533 mmol) was added and stirred at room temperature for 27 hours. Saturated sodium bicarbonate solution was added and extracted with ethyl acetate. The organic layer was dried over anhydrous magnesium sulfate. The solvent was removed under reduced pressure, and the resulting residue was purified by silica gel column chromatography (hexane-ethyl acetate) to give Compound 43 (155 mg, yield: 100%).

$^1$H-NMR (CDCl3) δ: 1.48-1.68 (4H, m), 1.70-1.79 (1H, m), 1.80-1.90 (1H, m), 3.50-3.57 (1H, m), 3.84-3.89 (1H, m), 3.91-3.97 (1H, m), 4.06-4.13 (1H, m), 4.55-4.59 (2H, m), 4.77 (1H, t, J = 3.5 Hz), 6.67 (1H, s), 7.49 (1H, dd, J = 10.7, 1.8 Hz), 8.18 (1H, d, J = 2.4 Hz), 8.21 (1H, d, J = 2.4 Hz), 8.27 (1H, s).

Step 4: Synthesis of Compound I-127

Under a nitrogen atmosphere, Compound 43 (50 mg, 0.115 mmol) was dissolved in dioxane (0.5 mL), and Compound 40 (51.9 mg, 0.229 mmol), 2 mol/L aqueous potassium carbonate solution (172 μL, 0.344 mmol), and XPhos Pd G3 (4.88 mg, 5.74 mmol) were added and stirred at 100°C for 3.3 hours. After cooling, water was added and extracted with chloroform. The organic layer was dried over anhydrous magnesium sulfate. The solvent was removed under reduced pressure, and the resulting residue was purified by silica gel column chromatography (hexane-ethyl acetate). The resulting

residue was dissolved in MeOH (1 mL), p-toluenesulfonic acid monohydrate (3.96 mg, 0.021 mmol) was added and stirred at 40°C for 16 hours. Saturated sodium bicarbonate solution was added and extracted with chloroform, and the organic layer was dried over anhydrous magnesium sulfate. The solvent was removed under reduced pressure, and the resulting residue was purified by silica gel column chromatography (chloroform-methanol) to give Compound I-127 (10.4 mg, yield: 22%).

$^1$H-NMR (CDCl3) δ: 1.91 (3H, s), 3.02 (1H, t, J = 5.8 Hz), 3.81 (3H, s), 3.92-3.96 (2H, m), 4.53 (2H, t, J = 4.3 Hz), 6.78 (1H, s), 7.42 (1H, d, J = 4.4 Hz), 7.51 (1H, d, J = 10.4 Hz), 8.11 (1H, d, J = 2.6 Hz), 8.25 (1H, s), 8.44 (1H, d, J = 2.6 Hz).

Reference Example 2: Synthesis of Compound 45

[Chemical formula 75]

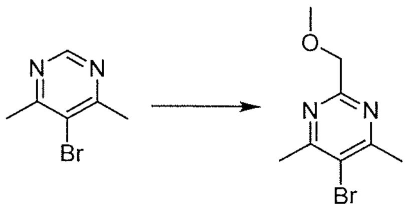

44 45

Step 1: Synthesis of Compound 45

Compound 44 (34.0 g, 182 mmol) was dissolved in dichloromethane (340 mL) and water (340 mL), and methoxyacetic acid (32.7 g, 364 mmol), silver nitrate (3.09 g, 18.2 mmol), and ammonium peroxodisulfate (83.0 g, 364 mmol) were added at 0°C and stirred at room temperature for 1 hour. Water (100 mL) and potassium carbonate (75.0 g, 545 mmol) were added and stirred at room temperature for 30 minutes. Brine (100 mL) was added followed by filtration over celite (registered trademark) and extraction with chloroform (300 mL). The organic layer was washed with brine, and then dried over anhydrous magnesium sulfate. The solvent was removed under reduced pressure, and the resulting residue was purified by silica gel column chromatography (hexane-ethyl acetate) to give Compound 45 (34.6 g, yield: 82%).

$^1$H-NMR (CDCl3) δ: 2.66 (s, 6H), 3.53 (s, 3H), 4.58 (s, 2H).

Reference Example 3: Synthesis of Compound 47

[Chemical formula 76]

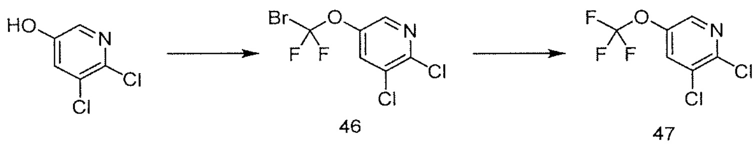

46 47

Step 1: Synthesis of Compound 46

5,6-Dichloro-3-pyridin-ol (100 mg, 0.61 mmol) was dissolved in DMF (1 mL) and cooled to 0°C. To the reaction solution, sodium hydride (60%, dispersed in paraffin liquid) (80 mg, 1.83 mmol) was added and further stirred at 0°C for 30 minutes. Further, dibromodifluoromethane (384 mg, 1.83 mmol) was added, and heated and stirred at 60°C for 1 hour during sealing. After cooling, the resultant was quenched with saturated aqueous ammonium chloride solution and extracted with ethyl acetate. The organic layer was washed with brine and dried over anhydrous sodium sulfate. The solvent was removed under reduced pressure, and the resulting residue was purified by silica gel column chromatography (hexane-ethyl acetate) to give Compound 46 (125.6 mg, yield: 70%).

1H-NMR (CDCl3) δ: 7.74 (d, J = 2.8 Hz, 1H), 8.30 (d, J = 2.8 Hz, 1H).

Step 2: Synthesis of Compound 47

Compound 46 (116 mg, 0.395 mmol) was dissolved in dichloromethane (1.2 mL) and cooled to -78°C. Silver tetrafluoroborate (154 mg, 0.791 mmol) was added to the reaction solution and warmed to room temperature. After stirring for 1 hour, the resultant was allowed to stand overnight. The reaction solution was filtered over celite (registered trademark), and an insoluble was removed. The solvent of the filtrate was removed under reduced pressure, and the resulting residue was purified by silica gel column chromatography (hexane-ethyl acetate) to give Compound 47 (68 mg, yield: 74%).

1H-NMR (CDCl3) δ: 7.71-7.72 (m, 1H), 8.29 (d, J = 1.6 Hz, 1H).

Example 16: Synthesis of Compound I-200

[Chemical formula 77]

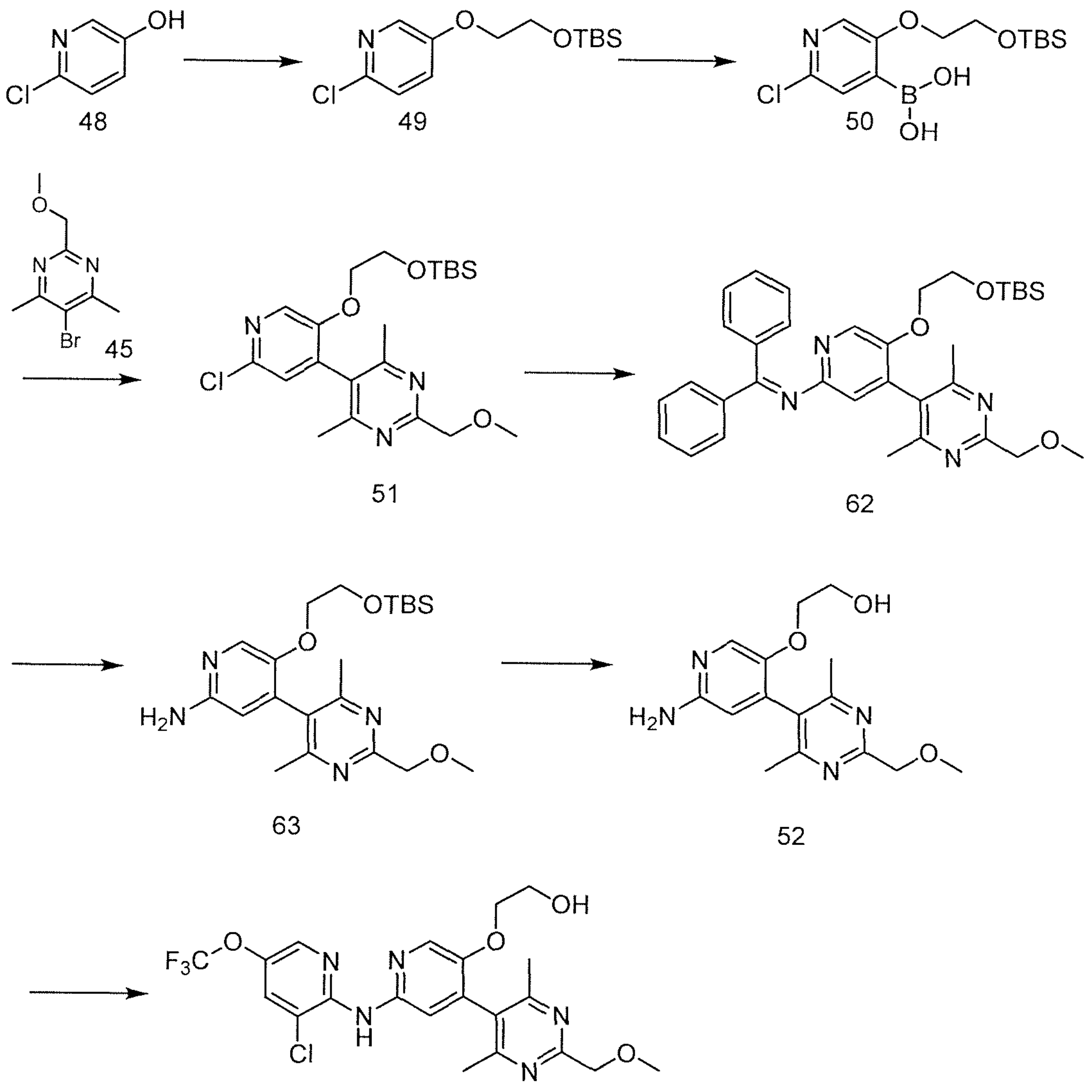

48 49 50 45 51 62 63 52

I-200

Step 1: Synthesis of Compound 49

Compound 48 (25.0 g, 193 mmol) was dissolved in N,N-dimethylformamide (125 mL), and cesium carbonate (82.0 g, 251 mmol) and (2-bromoethoxy)-tert-butyldimethylsilane (50.8 g, 212 mmol) were added and stirred at 80°C for 1 hour. After cooling, water was added and extracted with ethyl acetate (200 mL). The organic layer was washed with water

and brine, and then dried over anhydrous magnesium sulfate. The solvent was removed under reduced pressure, and the resulting residue was purified by silica gel column chromatography (hexane-ethyl acetate) to give Compound 49 (54.1 g, yield: 97%).

$^1$H-NMR ($CDCl_3$) δ: 0.09 (s, 6H), 0.90 (s, 9H), 3.96-3.98 (m, 2H), 4.07-4.09 (m, 2H), 7.21-7.22 (m, 2H), 8.06-8.07 (m, 1H).

Step 2: Synthesis of Compound 50

Compound 49 (54.0 g, 188 mmol) and triisopropyl borate (70.6 g, 375 mmol) were dissolved in THF (540 mL), and 2 mol/L of lithium diisopropylamine (tetrahydrofuran/heptane/ethylbenzene solution, 122 mL, 244 mmol) was added at -78°C and stirred at -78°C for 30 minutes. After stirring at room temperature for 1 hour, saturated aqueous ammonium chloride solution (200 mL) was added and stirred at room temperature for 1 hour and extracted with ethyl acetate (300 mL). The organic layer was washed with brine, and then dried over anhydrous magnesium sulfate. The solvent was removed under reduced pressure, diisopropylether was added, and a solid was precipitated and filtered off to give Compound 50 (35.6 g, yield: 57%).

$^1$H-NMR ($CDCl_3$) δ: 0.11 (s, 6H), 0.91 (s, 9H), 3.98-4.00 (m, 2H), 4.21-4.23 (m, 2H), 5.94 (br s, 2H), 7.68 (s, 1H), 8.08 (s, 1H).

Step 3: Synthesis of Compound 51

1,4-Dioxane (195 mL)-water (39.0 mL) was added to Compound 45 (19.5 g, 84.0 mmol), Compound 50 (30.8 g, 93.0 mmol), [1,1-bis(di-tert-butylphosphino)ferrocene]dichloropalladium (5.50 g, 8.44 mmol), and potassium carbonate (23.3 g, 169 mmol), and stirred at 80°C for 1 hour. After cooling, water was added and extracted with ethyl acetate (200 mL). The organic layer was washed with brine, and then dried over anhydrous magnesium sulfate. The solvent was removed under reduced pressure, and the resulting residue was purified by silica gel column chromatography (hexane-ethyl acetate) to give Compound 51 (27.3 g, yield: 74%).

$^1$H-NMR ($CDCl_3$) δ: -0.10 (s, 6H), 0.80 (s, 9H), 2.28 (s, 6H), 3.58 (s, 3H), 3.80 (t, J = 4.6 Hz, 2H), 4.11 (t, J = 4.8 Hz, 2H), 4.67 (s, 2H), 7.07 (s, 1H), 8.19 (s, 1H).

Step 4: Synthesis of Compound 62

Under a nitrogen atmosphere, Compound 51 (10.00 g, 22.8 mmol), xantphos (2.64 g, 4.57 mmol), and cesium carbonate (29.8 g, 91 mmol) were suspended in dioxane (150 mL), and diphenylmethanimine (4.60 ml, 27.4 mmol) and tris(dibenzylideneacetone)dipalladium (2.09 g, 2.28 mmol) were added, stirred at 100°C for 8 hours and 25 minutes, and allowed to stand at room temperature for 14 hours and 40 minutes. Xantphos (2.64 g, 4.57 mmol) and tris(dibenzylideneacetone)dipalladium (2.09 g, 2.28 mmol) were added and further stirred at 100°C for 8 hours and 45 minutes. An insoluble was filtered through celite (registered trademark) and washed with chloroform. The filtrate was concentrated under reduced pressure, and the resulting residue was purified by silica gel column chromatography (hexane-ethyl acetate) to give Compound 62 (10.34 g, yield: 78%).

$^1$H-NMR (DMSO-d6) δ: -0.21 (s, 6H), 0.73 (s, 9H), 1.91 (s, 6H), 3.37 (s, 3H), 3.70-3.71 (m, 2H), 4.04-4.05 (m, 2H), 4.45 (s, 2H), 6.43 (s, 1H), 7.10-7.17 (m, 2H), 7.30-7.36 (m, 3H), 7.44-7.60 (m, 3H), 7.68-7.70 (m, 2H), 8.20 (s, 1H).

Step 5: Synthesis of Compound 63

Under a nitrogen atmosphere, Compound 62 (10.30 g, 17.7 mmol) was suspended in ethanol (154 mL), and triethylamine (5.14 ml, 37.1 mmol) and hydroxylamine hydrochloride (2.46 g, 35.3 mmol) were added and stirred at 100°C for 1 hour and 10 minutes. After cooling, ethyl acetate (300 mL) and water (200 mL) were added and extracted with ethyl acetate. The organic layer was washed with water and brine, and then dried over anhydrous sodium sulfate. The solvent was removed under reduced pressure, and the resulting residue was purified by silica gel column chromatography (chloroform-methanol) to give Compound 63 (6.58 g, yield: 89%).

$^1$H-NMR (CDCl3) δ: -0.14 (s, 6H), 0.80 (s, 9H), 2.30 (s, 6H), 3.57 (s, 3H), 3.74 (t, J = 4.8 Hz, 2H), 3.95 (t, J = 4.8 Hz, 2H), 4.27 (s, 2H), 4.65 (s, 2H), 6.28 (s, 1H), 7.89 (s, 1H).

Step 6: Synthesis of Compound 52

Compound 63 (600 mg, 1.43 mmol) was dissolved in methanol (6 mL), and trifluoroacetic acid (817 mg, 7.17 mmol) was added, and stirred at 60°C for 2 hours. After cooling, the solvent was removed under reduced pressure. The resulting residue was purified by amino silica gel column chromatography (ethyl acetate-methanol) and solidified from a

mixture of ethyl acetate-hexane to give Compound 52 (382 mg, yield: 88%). $^1$H-NMR ($CDCl_3$) δ: 1.55-1.60 (m, 1H), 2.31 (s, 6H), 3.59 (s, 3H), 3.72-3.76 (m, 2H), 3.99 (t, J = 4.4 Hz, 2H), 4.31 (brs, 2H), 4.67 (s, 2H), 6.30 (s, 1H), 7.90 (s, 1H).

Step 7: Synthesis of Compound I-200

Compound 47 (103 mg, 0.44 mmol), bis(dibenzylideneacetone)palladium (25.5 mg, 0.044 mmol), BINAP (55.2 mg, 0.089 mmol), and cesium carbonate (289 mg, 0.89 mmol) were added to Compound 52 (135 mg, 0.44 mmol), and toluene (3.4 mL) was added and heated and stirred at 100°C for 4 hours. After cooling, the reaction solution was filtered through celite (registered trademark) and washed with ethyl acetate. The solvent of the filtrate was removed under reduced pressure, and the resulting residue was purified by silica gel column chromatography (chloroform-methanol) to give Compound I-200 (43.3 mg, yield: 20%).

$^1$H-NMR ($CDCl_3$) δ: 1.59 (t, J = 6.4 Hz, 1H), 2.35 (s, 6H), 3.61 (s, 3H), 3.79-3.83 (m, 2H), 4.12 (t, J = 4.8 Hz, 2H), 4.70 (s, 2H), 7.58 (d, J = 1.6 Hz, 1H), 7.84 (s, 1H), 8.07 (d, J = 1.6 Hz, 1H), 8.11 (s, 1H), 8.24 (s, 1H).

Reference Example 4: Synthesis of Compound 56

[Chemical formula 78]

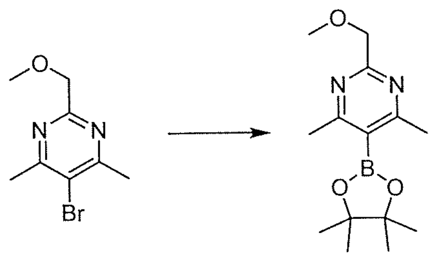

45 56

Step 1: Synthesis of Compound 56

Dioxane (300 mL) was added to Compound 45 (30 g, 130 mmol), bis(pinacolate)diboron (65.9 g, 260 mmol), [1,1-bis(diphenylphosphino)ferrocene]dichloropalladium (9.5 g, 13 mmol), and potassium acetate (31.9 g, 325 mmol) and stirred at 100°C for 8 hours. After cooling, the resultant was filtered through celite (registered trademark), an insoluble was removed, and the solvent was removed under reduced pressure. Hexane was added to the resulting residue, the precipitated insoluble was removed again by filtration, and the solvent was removed under reduced pressure. The resulting residue was roughly purified by silica gel column chromatography (hexane-ethyl acetate). The resulting crude residue was purified by silica gel column chromatography (chloroform-methanol) to give Compound 56 (26.1 g, yield: 72%).

1H-NMR (CDCl3) δ: 1.40 (s, 12H), 2.61 (s, 6H), 3.52 (s, 3H), 4.61 (s, 2H).

Example 17: Synthesis of Compound 1-247

[Chemical formula 79]

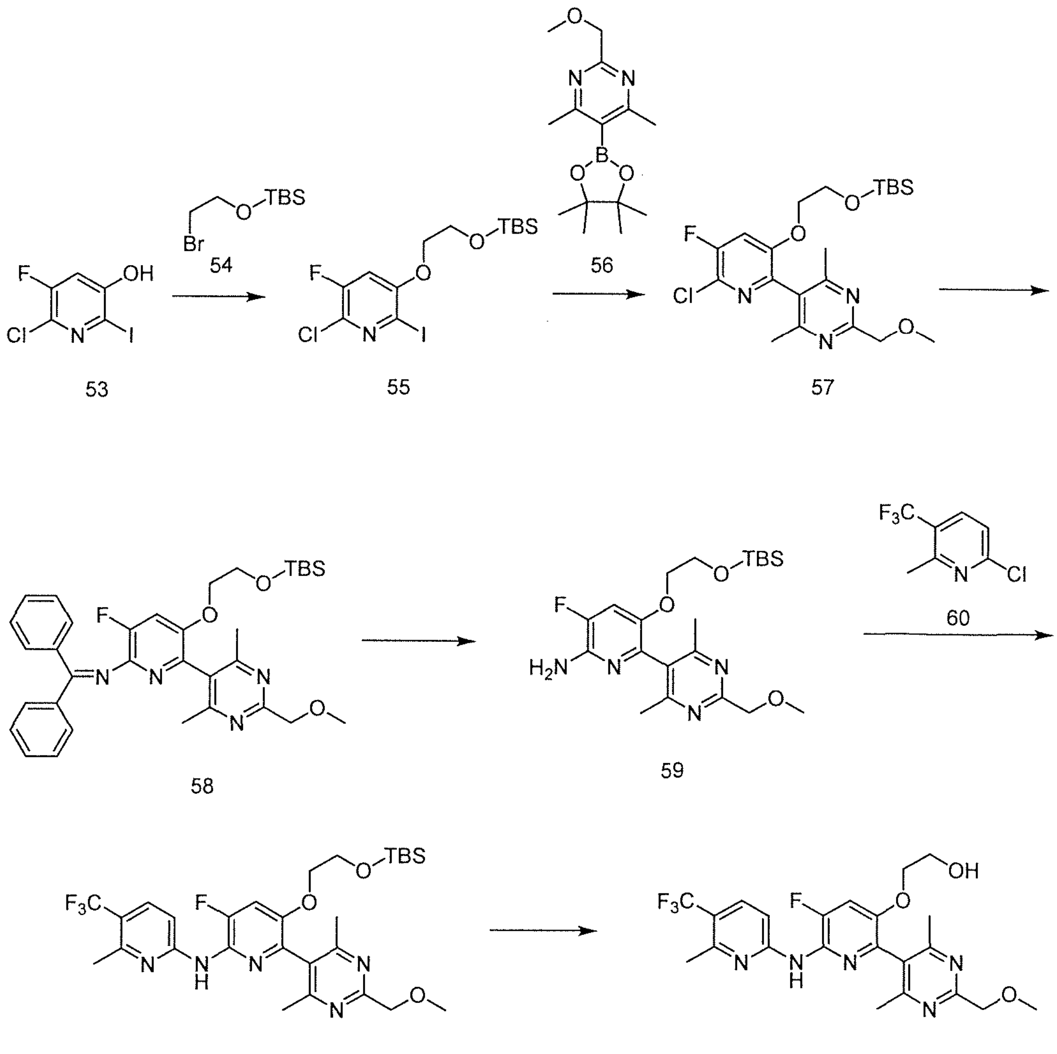

53 54 55 56 57

58 59 60

61 I-247

Step 1: Synthesis of Compound 55

Compound 53 (10 g, 36.6 mmol), potassium carbonate (7.58 g, 54.9 mmol), sodium iodide (5.48 g, 36.6 mmol), and Compound 54 (10.5 g, 43.9 mmol) were suspended in N,N-dimethylformamide (100 mL), and stirred at 60°C for 2 hours and stirred at 70°C for 3 hours. After cooling, water and ethyl acetate were added and extracted with ethyl acetate. The organic layer was washed with water and brine, and then dried over anhydrous sodium sulfate. The solvent was removed under reduced pressure, and the resulting residue was purified by silica gel column chromatography (hexane-ethyl acetate) to give Compound 55 (15.35 g, yield: 97%).

$^1$H-NMR (CDCl3) δ: 0.11 (s, 6H), 0.90 (s, 9H), 4.02 (t, J = 4.8 Hz, 2H), 4.15 (t, J = 4.8 Hz, 2H), 7.04 (d, J = 9.2 Hz, 1H).

Step 2: Synthesis of Compound 57

Compound 55 (12.7 g, 29.4 mmol), Compound 56 (12.27 g, 44.1 mmol), $PdCl_2$ (dtbpf) (1.92 g, 2.94 mmol), and potassium carbonate (8.13 g, 58.8 mmol) were suspended in dioxane (127 mL) and water (25.4 mL), and under a nitrogen atmosphere, stirred at 100°C for 6 hours. Ethyl acetate and water were added and extracted with ethyl acetate. After washing with brine, the resultant was dried over anhydrous magnesium sulfate. The solvent was removed under reduced pressure, and the resulting residue was purified by silica gel column chromatography (hexane-ethyl acetate) to give Compound 57 (8.92 g, yield: 67%).

$^1$H-NMR (CDCl3) δ: -0.07 (s, 6H), 0.81 (s, 9H), 2.28 (s, 6H), 3.55 (s, 3H), 3.83 (t, J = 4.6 Hz, 2H), 4.07 (t, J = 4.6 Hz,

2H), 4.67 (s, 2H), 7.34 (d, J = 9.2 Hz, 1H).

Step 3: Synthesis of Compound 58

Under a nitrogen atmosphere, Compound 57 (500 mg, 1.10 mmol) was dissolved in dioxane (10 mL), and diphenylmethanimine (0.22 ml, 1.32 mmol), xantphos (63.4 mg, 0.110 mmol), cesium carbonate (1.07 g, 3.29 mmol), and palladium acetate (49.2 mg, 0.219 mmol) were added, stirred at 100°C for 5 hours and 20 minutes, and allowed to stand at room temperature for 12 hours and 30 minutes. Palladium acetate (49.2 mg, 0.219 mmol) and xantphos (63.4 mg, 0.110 mmol) were added and further stirred at 100°C for 8 hours and 40 minutes. An insoluble was filtered through celite (registered trademark) and washed with chloroform. The filtrate was concentrated under reduced pressure, and the resulting residue was purified by silica gel column chromatography (hexane-ethyl acetate) to give Compound 58 (386 mg, yield: 59%).

$^1$H-NMR (CDCl3) δ: -0.11 (s, 6H), 0.80 (s, 9H), 2.04 (s, 6H), 3.52 (s, 3H), 3.75 (t, J = 4.7 Hz, 2H), 3.94 (t, J = 4.8 Hz, 2H), 4.62 (s, 2H), 7.13 (d, J = 10.8 Hz, 1H), 7.19-7.23 (m, 2H), 7.27-7.32 (m, 3H), 7.37-7.43 (m, 2H), 7.46-7.53 (m, 1H), 7.77-7.82 (m, 2H).

Step 4: Synthesis of Compound 59

Under a nitrogen atmosphere, Compound 58 (385 mg, 0.641 mmol) was dissolved in ethanol (3.9 mL), and hydroxylamine hydrochloride (89 mg, 1.28 mmol) and triethylamine (0.187 ml, 1.35 mmol) were added and stirred at 100°C for 2 hours. After cooling, chloroform and water were added and extracted with chloroform. The organic layer was dried over anhydrous magnesium sulfate. The solvent was removed under reduced pressure, and the resulting residue was purified by silica gel column chromatography (hexane-ethyl acetate) to give Compound 59 (141 mg, yield: 51%).

$^1$H-NMR (CDCl3) δ: -0.70 (s, 6H), 0.82 (s, 9H), 2.29 (s, 6H), 3.54 (s, 3H), 3.75 (t, J = 4.8 Hz, 2H), 3.90 (t, J = 4.8 Hz, 2H), 4.37 (br s, 2H), 4.66 (s, 2H), 7.22 (d, J = 11.2 Hz, 1H).

Step 5: Synthesis of Compound 61

Under a nitrogen atmosphere, Compound 59 (40 mg, 0.092 mmol) and Compound 60 (26.9 mg, 0.137 mmol) were suspended in toluene (1 mL), and BINAP (11.4 mg, 0.018 mmol), cesium carbonate (90 mg, 0.275 mmol), and tris(dibenzylideneacetone)dipalladium (8.4 mg, 0.0092 mmol) were added and stirred at 100°C for 7 hours and 45 minutes. An insoluble was filtered through celite (registered trademark) and washed with chloroform. The filtrate was concentrated under reduced pressure, and the resulting residue was purified by silica gel column chromatography (hexane-ethyl acetate) to give Compound 61 (41.7 mg, yield: 76%).

$^1$H-NMR (CDCl3) δ: -0.62 (s, 6H), 0.82 (s, 9H), 2.32 (s, 6H), 2.58 (s, 3H), 3.60 (s, 3H), 3.80 (t, J = 4.6 Hz, 2H), 4.00 (t, J = 4.6 Hz, 2H), 4.69 (s, 2H), 7.34 (d, J = 11.5 Hz, 1H), 7.52 (br s, 1H), 7.73 (d, J = 9.0 Hz, 1H), 8.02 (d, J = 8.8 Hz, 1H).

Step 6: Synthesis of Compound I-247

Under a nitrogen atmosphere, Compound 61 (39.1 mg, 0.066 mmol) was dissolved in tetrahydrofuran (0.8 mL), and 1 mol/L tetrabutylammonium fluoride tetrahydrofuran solution (0.079 mL, 0.079 mmol) was added under ice-cooling, and stirred at room temperature for 50 minutes. Water and ethyl acetate were added and extracted with ethyl acetate. The organic layer was washed with water and brine, and then dried over anhydrous sodium sulfate. The solvent was removed under reduced pressure, and the resulting residue was purified by silica gel column chromatography (chloroform-methanol) to give Compound I-247 (25.0 mg, yield: 79%).

$^1$H-NMR (CDCl3) δ: 1.70 (t, J = 6.0 Hz, 1H), 2.32 (s, 6H), 2.58 (s, 3H), 3.61 (s, 3H), 3.78-3.84 (m, 2H), 4.03 (t, J = 4.5 Hz, 2H), 4.70 (s, 2H), 7.29 (d, J = 11.6 Hz, 1H), 7.55 (br s, 1H), 7.74 (d, J = 8.8 Hz, 1H), 8.03 (d, J = 8.8 Hz, 1H).

Example 18: Synthesis of Compound I-257

[Chemical formula 80]

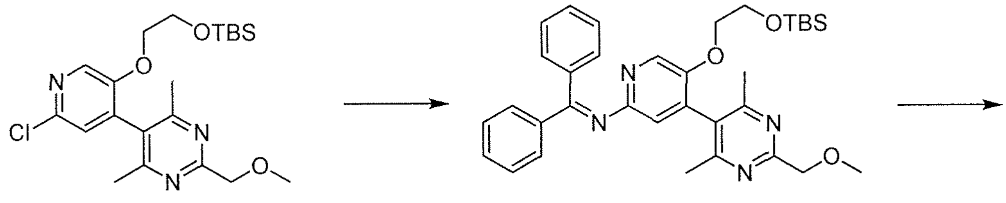

51 62

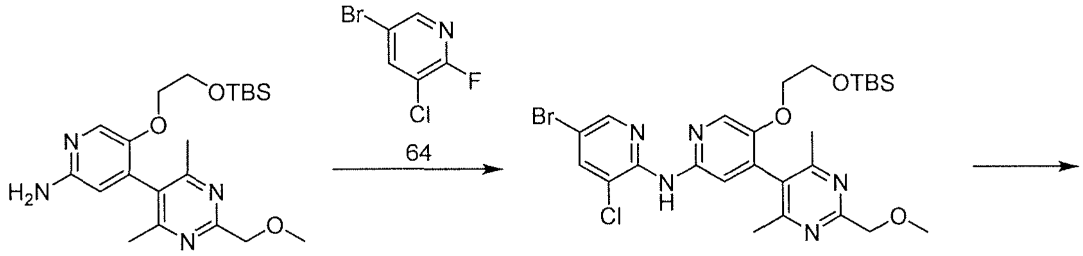


63 65

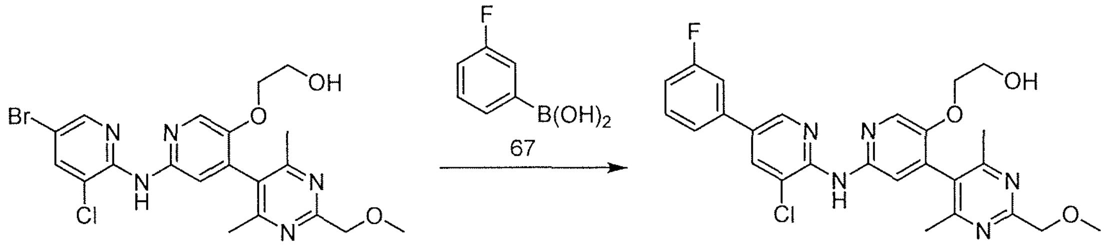


66 I-257

Step 1: Synthesis of Compound 62

Under a nitrogen atmosphere, Compound 51 (10.00 g, 22.8 mmol), xantphos (2.64 g, 4.57 mmol), and cesium carbonate (29.8 g, 91 mmol) were suspended in dioxane (150 mL), and diphenylmethanimine (4.60 ml, 27.4 mmol) and tris(dibenzylideneacetone)dipalladium (2.09 g, 2.28 mmol) were added, stirred at 100°C for 8 hours and 25 minutes, and allowed to stand at room temperature for 14 hours and 40 minutes. Xantphos (2.64 g, 4.57 mmol) and tris(dibenzylideneacetone)dipalladium (2.09 g, 2.28 mmol) were added and further stirred at 100°C for 8 hours and 45 minutes. An insoluble was filtered through celite (registered trademark) and washed with chloroform. The filtrate was concentrated under reduced pressure, and the resulting residue was purified by silica gel column chromatography (hexane-ethyl acetate) to give Compound 62 (10.34 g, yield: 78%).

$^1$H-NMR (DMSO-d6) δ: -0.21 (s, 6H), 0.73 (s, 9H), 1.91 (s, 6H), 3.37 (s, 3H), 3.70-3.71 (m, 2H), 4.04-4.05 (m, 2H), 4.45 (s, 2H), 6.43 (s, 1H), 7.10-7.17 (m, 2H), 7.30-7.36 (m, 3H), 7.44-7.60 (m, 3H), 7.68-7.70 (m, 2H), 8.20 (s, 1H).

Step 2: Synthesis of Compound 63

Under a nitrogen atmosphere, Compound 62 (10.30 g, 17.7 mmol) was suspended in ethanol (154 mL), and triethylamine (5.14 ml, 37.1 mmol) and hydroxylamine hydrochloride (2.46 g, 35.3 mmol) were added and stirred at 100°C for 1 hour and 10 minutes. After cooling, ethyl acetate (300 mL) and water (200 mL) were added and extracted with ethyl acetate. The organic layer was washed with water and brine, and then dried over anhydrous sodium sulfate. The solvent was removed under reduced pressure, and the resulting residue was purified by silica gel column chromatography (chloroform-methanol) to give Compound 63 (6.58 g, yield: 89%).

$^1$H-NMR (CDCl3) δ: -0.14 (s, 6H), 0.80 (s, 9H), 2.30 (s, 6H), 3.57 (s, 3H), 3.74 (t, J = 4.8 Hz, 2H), 3.95 (t, J = 4.8 Hz, 2H), 4.27 (s, 2H), 4.65 (s, 2H), 6.28 (s, 1H), 7.89 (s, 1H).

Step 3: Synthesis of Compound 65

Under a nitrogen atmosphere, Compound 63 (1.00 g, 2.39 mmol) and Compound 64 (603 mg, 2.87 mmol) were dissolved in N,N-dimethylformamide (20 mL), and under ice-cooling, a solution of potassium tert-butoxide (670 mg, 5.97 mmol) in tetrahydrofuran (12 mL) was added, and stirred for 40 minutes under ice cooling. Water (50 mL) and ethyl acetate (30 mL) were added and extracted with ethyl acetate. The organic layer was washed with water and brine, and then dried over anhydrous sodium sulfate. The solvent was removed under reduced pressure, and the resulting residue was purified by silica gel column chromatography (hexane-ethyl acetate) to give Compound 65 (858 mg, yield: 59%).

$^1$H-NMR (CDCl3) δ: -0.09 (s, 6H), 0.81 (s, 9H), 2.33 (s, 6H), 3.60 (s, 3H), 3.80 (t, J = 4.8H z, 2H), 4.07 (t, J = 4.8 Hz, 2H), 4.68 (s, 2H), 7.74 (d, J = 2.0 Hz, 1H), 7.76 (s, 1H), 8.09 (s, 1H), 8.12 (d, J = 2.0 Hz, 1H), 8.17 (s, 1H).

Step 4: Synthesis of Compound 66

Under a nitrogen atmosphere, Compound 65 (858 mg, 1.41 mmol) was dissolved in tetrahydrofuran (8.6 mL), and 1 mol/L tetrabutylammonium fluoride tetrahydrofuran solution (2.1 mL, 2.1 mmol) was added and stirred at room temperature for 1 hour. Saturated aqueous ammonium chloride solution and ethyl acetate were added and extracted with ethyl acetate. The organic layer was washed with brine, and then dried over anhydrous sodium sulfate. The solvent was removed under reduced pressure, and the resulting residue was solidified with diisopropylether to give Compound 66 (617 mg, yield: 89%).

$^1$H-NMR (CDCl3) δ: 1.63 (t, J = 6.3 Hz, 1H), 2.35 (s, 6H), 3.61 (s, 3H), 3.77-3.83 (m, 2H), 4.11 (t, J = 4.5 Hz, 2H), 4.70 (s, 2H), 7.75 (d, J = 2.0 Hz, 1H), 7.78 (s, 1H), 8.10 (s, 1H), 8.13 (d, J = 2.0 Hz, 1H), 8.21 (s, 1H).

Step 5: Synthesis of Compound I-257

Compound 67 (11 mg, 0.076 mmol), a solution of Compound 66 (25 mg, 0.051 mmol) in dioxane (0.5 mL), $PdCl_2$ (dtbpf) (3.3 mg, 0.0051 mmol), and a solution of potassium carbonate (21 mg, 0.152 mmol) in $H_2O$ (0.10 mL) were mixed, and under a nitrogen atmosphere, stirred at 100°C for 2 hours. Chloroform and water were added and extracted with chloroform. An insoluble was filtered through amino silica gel, the filtrate was concentrated under reduced pressure, and the resulting residue was dissolved in dimethyl sulfoxide. The resultant was purified by reverse phase liquid column chromatography (aqueous ammonium carbonate solution, acetonitrile) to give Compound I-257 (4.2 mg, yield: 16%).

$^1$H-NMR (CDCl3) δ: 1.67 (t, J = 5.5 Hz, 1H), 2.37 (s, 6H), 3.61 (s, 3H), 3.78-3.85 (m, 2H), 4.12 (t, J = 4.5 Hz, 2H), 4.70 (s, 2H), 7.05 (dt, J = 8.4, 5.9 Hz, 1H), 7.18-7.28 (m, 1H), 7.29 (d, J = 7.8 Hz, 1H), 7.37-7.44 (m, 1H), 7.84 (d, J = 2.2 Hz, 1H), 7.89 (s, 1H), 8.12 (s, 1H), 8.33 (d, J = 2.0 Hz, 1H), 8.37 (s, 1H).

Reference Example 5: Synthesis of Compound 69

[Chemical formula 81]

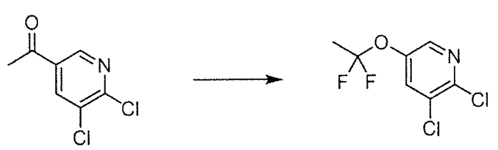

68 69

Step 1: Synthesis of Compound 69

Under a nitrogen atmosphere, xenon fluoride (535 mg, 3.16 mmol) was suspended in dichloromethane (6 mL), and Compound 68 (300 mg, 1.58 mmol) and 70% hydrogen fluoride pyridine (1.32 mL, 10.3 mmol) were added and stirred at room temperature for 25 hours and 55 minutes. The reaction solution was poured into a solution of sodium bicarbonate (5.31 g, 63.2 mmol) in H2O (30 mL) and extracted with ethyl acetate. The organic layer was washed with water and brine, and then dried over anhydrous sodium sulfate. The solvent was removed under reduced pressure, and the resulting residue was purified by silica gel column chromatography (hexane-ethyl acetate) to give Compound 69 (146 mg, yield: 40%).

$^1$H-NMR (CDCl3) δ: 1.98 (t, J = 13.5 Hz, 3H), 7.67 (d, J = 1.8 Hz, 1H), 8.17-8.23 (m, 1H).

Example 19: Synthesis of Compound I-266

[Chemical formula 82]

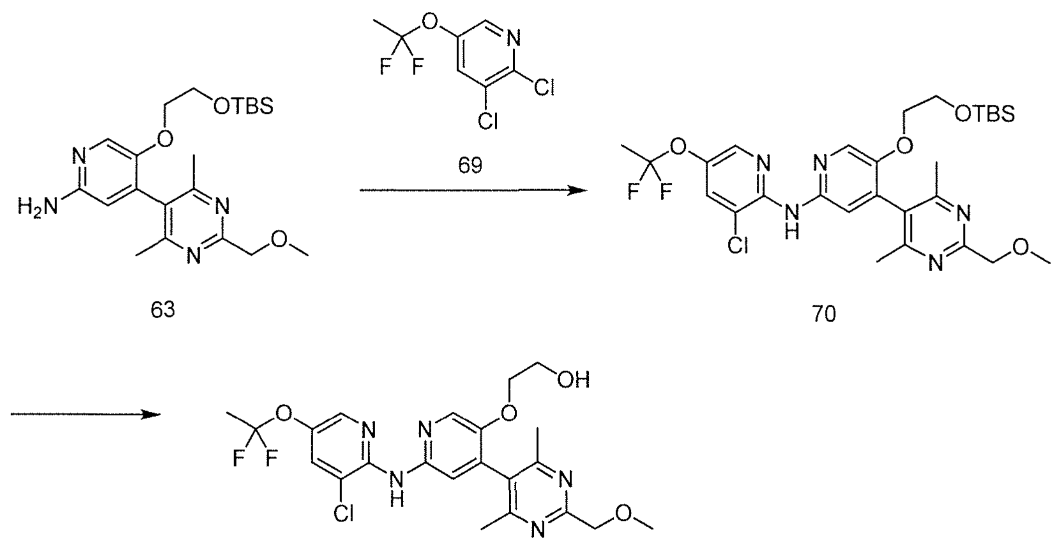

63 69 70

I-266

Step 1: Synthesis of Compound 70

Under a nitrogen atmosphere, Compound 63 (40 mg, 0.096 mmol) and Compound 69 (32.7 mg, 0.143 mmol) were suspended in toluene (0.8 mL), and BINAP (11.9 mg, 0.019 mmol), cesium carbonate (93 mg, 0.287 mmol), and tris(dibenzylideneacetone)dipalladium (8.8 mg, 0.0096 mmol) were added and stirred at 100°C for 3 hours and 30 minutes. An insoluble was filtered through celite (registered trademark) and washed with chloroform. The filtrate was concentrated under reduced pressure, and the resulting residue was purified by silica gel column chromatography (hexane-ethyl acetate) to give Compound 70 (45.9 mg, yield: 79%).

$^1$H-NMR (CDCl3) δ: -0.08 (s, 6H), 0.82 (s, 9H), 1.92 (t, J = 13.3 Hz, 3H), 2.34 (s, 6H), 3.59 (s, 3H), 3.80 (t, J = 4.8 Hz, 2H), 4.06 (t, J = 4.8 Hz, 2H), 4.68 (s, 2H), 7.53 (d, J = 2.0 Hz, 1H), 7.74 (s, 1H), 7.97-7.98 (m, 1H), 8.08 (s, 1H), 8.22 (s, 1H).

Step 2: Synthesis of Compound I-266

Under a nitrogen atmosphere, Compound 70 (43.6 mg, 0.071 mmol) was dissolved in tetrahydrofuran (0.9 mL), and under ice-cooling, 1 mol/L tetrabutylammonium fluoride tetrahydrofuran solution (0.086 mL) was added, and stirred at room temperature for 50 minutes. Water and ethyl acetate were added and extracted with ethyl acetate. The organic layer was washed with water and brine, and then dried over anhydrous sodium sulfate. The solvent was removed under reduced pressure, and the resulting residue was purified by silica gel column chromatography (chloroform-methanol) to give Compound I-266 (18.5 mg, yield: 52%).

$^1$H-NMR (CDCl3) δ: 1.66 (t, J = 6.1 Hz, 1H), 1.93 (t, J = 13.3 Hz, 3H), 2.35 (s, 6H), 3.61 (s, 3H), 3.78-3.83 (m, 2H), 4.10 (t, J = 4.4 Hz, 2H), 4.69 (s, 2H), 7.54 (d, J = 1.8 Hz, 1H), 7.77 (s, 1H), 7.97-8.00 (m, 1H), 8.09 (s, 1H), 8.25 (s, 1H).

Example 20: Synthesis of Compound I-271

[Chemical formula 83]

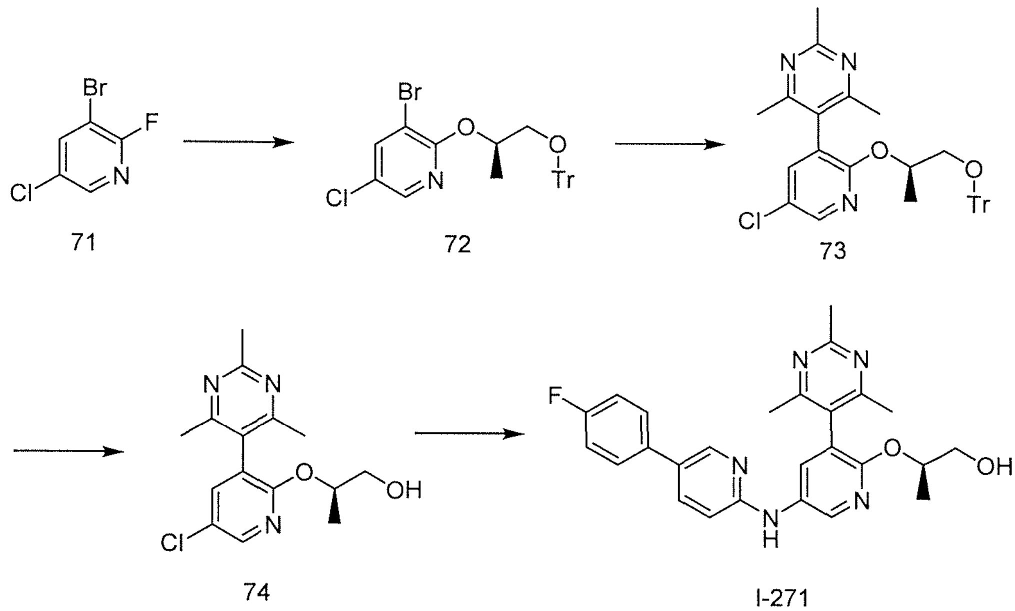

71 72 73

74 I-271

Step 1: Synthesis of Compound 72

Compound 71 (3.00 g, 14.3 mmol) was suspended in N,N-dimethylacetamide (30 mL), and potassium carbonate (2.96 g, 21.4 mmol) and (R)-1-(trityloxy)propan-2-ol (5.45 g, 17.1 mmol) were added and stirred at 150°C for 8 hours. After cooling, water was added and extracted with ethyl acetate (100 mL). The organic layer was washed with water and brine, and then dried over anhydrous magnesium sulfate. The solvent was removed under reduced pressure, and the resulting residue was purified by silica gel column chromatography (hexane-ethyl acetate). Methanol was added, and the solid was precipitated and then filtered off to give Compound 72 (6.42 g, yield: 89%).

$^1$H-NMR ($CDCl_3$) δ: 1.35 (d, J = 6.5 Hz, 3H), 3.16 (dd, J = 9.7, 3.6 Hz, 1H), 3.34 (dd, J = 9.8, 6.3 Hz, 1H), 5.43-5.50 (m, 1H), 7.20-7.29 (m, 9H), 7.44 (d, J = 7.5 Hz, 6H), 7.82 (d, J = 2.3 Hz, 1H), 8.02 (d, J = 2.5 Hz, 1H).

Step 2: Synthesis of Compound 73

1,4-Dioxane (30 mL)-water (6 mL) was added to Compound 72 (3.00 g, 5.90 mmol), 2,4,6-trimethyl-5-(4,4,5,5-tetramethyl-1,3,2-dioxaborolan-2-yl)pyrimidine (2.19 g, 8.84 mmol), [1,1-bis(di-tert-butylphosphino)ferrocene]dichloropalladium (384 mg, 0.590 mmol), and potassium carbonate (2.44 g, 17.7 mmol) and stirred at 80°C for 4 hours. After cooling, the resultant was filtered through celite (registered trademark), and the solvent was removed under reduced pressure. Water was added to the resulting residue, and extracted with acetic acid (50 mL). The organic layer was washed with brine, and then dried over anhydrous magnesium sulfate. The solvent was removed under reduced pressure, and the resulting residue was purified by silica gel column chromatography (hexane-ethyl acetate) to give Compound 73 (2.21 g, yield: 68%).

$^1$H-NMR ($CDCl_3$) δ: 1.21 (d, J = 6.5 Hz, 3H), 2.16 (s, 3H), 2.24 (s, 3H), 2.68 (s, 3H), 3.05 (dd, J = 9.5, 4.0 Hz, 1H), 3.11 (dd, J = 9.7, 6.7 Hz, 1H), 5.49-5.52 (m, 1H), 7.18-7.30 (m, 15H), 7.38 (d, J = 2.8 Hz, 1H), 8.18 (d, J = 2.5 Hz, 1H).

Step 3: Synthesis of Compound 74

Compound 73 (1.10 g, 2.00 mmol) was dissolved in dichloromethane (11 mL)-methanol (11 mL), and p-toluenesulfonic acid monohydrate (190 mg, 1.00 mmol) was added and stirred at room temperature for 24 hours. Saturated sodium bicarbonate solution was added and extracted with chloroform (20 mL). The organic layer was washed with brine, and then dried over anhydrous magnesium sulfate. The solvent was removed under reduced pressure, and the resulting residue was purified by silica gel column chromatography (chloroform-methanol) to give Compound 74 (572 mg, yield: 93%).

$^1$H-NMR ($CDCl_3$) δ: 1.22 (d, J = 6.5 Hz, 3H), 2.22 (s, 3H), 2.24 (s, 3H), 2.57 (t, J = 5.9 Hz, 1H), 2.71 (s, 3H), 3.57-3.72 (m, 2H), 5.22-5.27 (m, 1H), 7.40 (d, J = 2.5 Hz, 1H), 8.16 (d, J = 2.5 Hz, 1H).

Step 4: Synthesis of Compound I-271

Toluene (500 μL) was added to Compound 74 (50 mg, 0.162 mmol), 5-(4-fluorophenyl)pyridin-2-amine (33.6 mg, 0.179 mmol), cesium carbonate (106 mg, 0.325 mmol), X-Phos (15.5 mg, 0.032 mmol), and tris(dibenzylideneacetone) dipalladium (14.9 mg, 0.016 mmol), and stirred at 110°C for 9.5 hours. After cooling, X-Phos (15.5 mg, 0.032 mmol) and tris(dibenzylideneacetone)dipalladium (14.9 mg, 0.016 mmol) were added, and further stirred at 110°C for 26 hours. After cooling, the reaction mixture was filtered through celite (registered trademark), and the solvent was removed under reduced pressure. The resulting residue was purified by diol silica gel column chromatography (hexane-ethyl acetate). Diisopropylether-hexane was added, a solid was precipitated, and then filtered off to give Compound I-271 (2.0 mg, yield: 3%).

$^1$H-NMR ($CDCl_3$) δ: 1.22 (d, J = 6.5 Hz, 3H), 2.28 (s, 3H), 2.31 (s, 3H), 2.73 (s, 3H), 3.49-3.52 (m, 1H), 3.62-3.73 (m, 2H), 5.16-5.21 (m, 1H), 6.36 (br s, 1H), 6.73 (d, J = 9.0 Hz, 1H), 7.11-7.15 (m, 2H), 7.45-7.48 (m, 2H), 7.68-7.72 (m, 2H), 8.27 (d, J = 2.8 Hz, 1H), 8.37 (d, J = 2.0 Hz, 1H).

Example 21: Synthesis of Compound I-276

[Chemical formula 84]

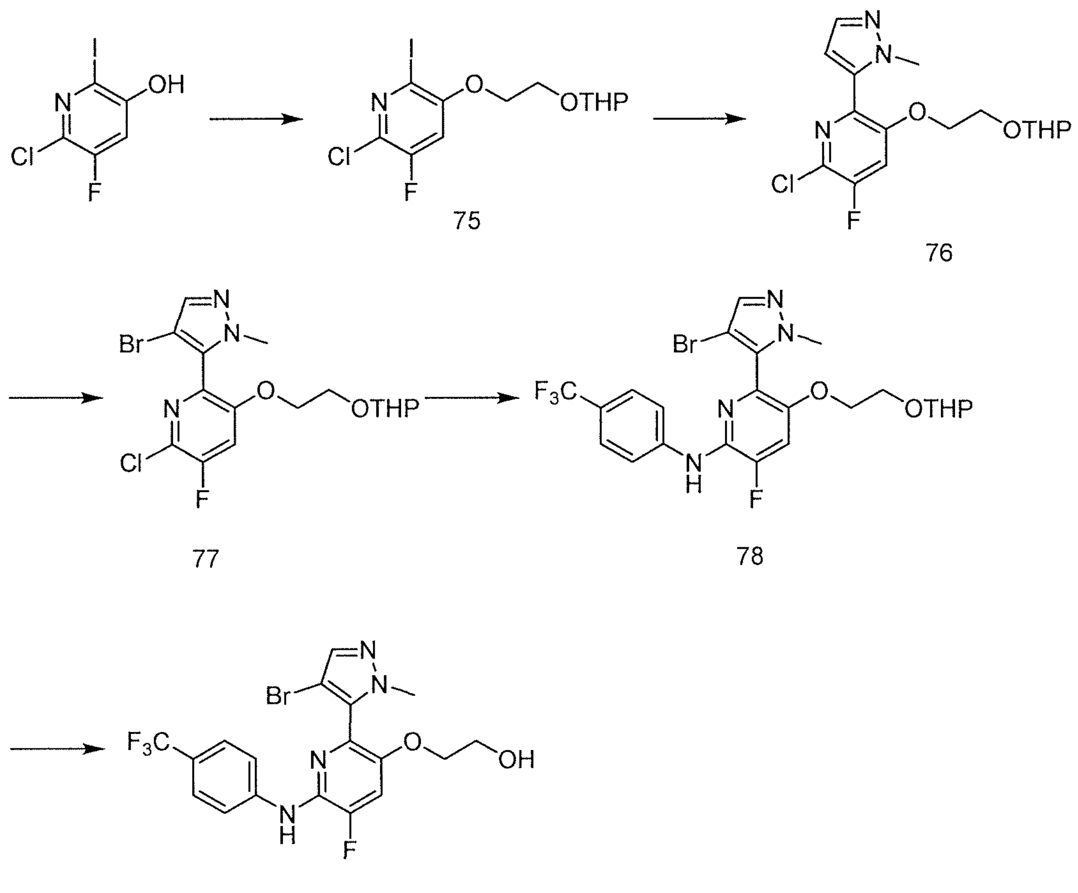

75

76

77

78

I-276

Step 1: Synthesis of Compound 75

6-Chloro-5-fluoro-2-iodo-pyridin-3-ol (300 mg, 1.10 mmol) was dissolved in DMF (3 mL), and potassium carbonate (227 mg, 1.65 mmol) and 2-(2-bromoethoxy)tetrahydro-2H-pyran (199 μL, 1.32 mmol) were added and stirred at 75°C for 4 hours. After cooling, water (30 mL) was added and extracted with ethyl acetate (20 mL). The organic layer was washed with water (30 mL) and brine (20 mL), and then dried over anhydrous sodium sulfate. The solvent was removed under reduced pressure to give Compound 75 (456 mg) as a crude product. The obtained Compound 75 was used in the

next step without further purification.

Step 2: Synthesis of Compound 76

Under a nitrogen atmosphere, the crude product of Compound 75 (424 mg) was dissolved in 1,4-dioxane (8.5 mL)-water (1.7 mL), and 1-methyl-5-(4,4,5,5-tetramethyl-1,3,2-dioxaborolan-2-yl)-1H-pyrazole (242 mg, 1.16 mmol), XPhosPdG3 (89 mg, 0.11 mmol), and potassium carbonate (175 mg, 1.27 mmol) were added, and stirred at 100°C for 1 hour. After cooling, the resultant was extracted with water (30 mL) and chloroform (30 mL). The organic layer was washed with brine (30 mL) and then dried over anhydrous sodium sulfate. The solvent was removed under reduced pressure, and the resulting residue was purified by silica gel column chromatography (hexane-ethyl acetate) to give Compound 76 (226 mg, yield: 60%).

1H-NMR (CDCl3) δ: 1.56-1.85 (m, 6H), 3.47-3.55 (m, 1H), 3.77-3.85 (m, 2H), 4.07-4.15 (m, 1H), 4.10 (s, 3H), 4.22-4.26 (m, 2H), 4.66-4.68 (m, 1H), 6.83 (d, J = 1.6 Hz, 1H), 7.25-7.28 (m, 1H), 7.50 (d, J = 1.6 Hz, 1H).

Step 3: Synthesis of Compound 77

Under a nitrogen atmosphere, Compound 76 (194 mg, 0.546 mmol) was dissolved in N,N-dimethylformamide (2 mL), and N-bromosuccinimide (117 mg, 0.655 mmol) was added and stirred at room temperature for 6 hours. Saturated sodium bicarbonate solution (20 mL) was added and extracted with ethyl acetate (10 mL). The organic layer was washed with water (20 mL) and brine (10 mL), and then dried over anhydrous sodium sulfate. The solvent was removed under reduced pressure, and the resulting residue was purified by silica gel column chromatography (hexane-ethyl acetate) to give Compound 77 (208 mg, yield: 88%).

1H-NMR (CDCl3) δ: 1.45-1.78 (m, 6H), 3.42-3.49 (m, 1H), 3.68-3.79 (m, 2H), 3.81 (s, 3H), 4.01-4.07 (m, 1H), 4.20-4.25 (m, 2H), 4.57-4.59 (m, 1H), 7.37 (d, J = 9.2 Hz, 1H), 7.50 (s, 1H).

Step 4: Synthesis of Compound 78

Under a nitrogen atmosphere, Compound 77 (22 mg, 0.051 mmol) was dissolved in toluene (0.5 mL), and 4-(trifluoromethyl)aniline (10 mg, 0.064 mmol), $Pd_2(dba)_3$ (4.7 mg, 5.1 μmol), BINAP (6.3 mg, 10.2 μmol), and cesium carbonate (33 mg, 0.102 mmol) were added, and stirred at 100°C for 3 hours. After cooling, water (10 mL) was added and extracted with chloroform (10 mL). The solvent was removed under reduced pressure, and the resulting residue was purified by thin layer chromatography (hexane-ethyl acetate) to give Compound 78 (19.6 mg, yield: 69%).

1H-NMR (CDCl3) δ: 1.56-1.78 (m, 6H), 3.43-3.50 (m, 1H), 3.68-3.78 (m, 2H), 3.82 (s, 3H), 3.97-4.03 (m, 1H), 4.13-4.17 (m, 2H), 4.57-4.60 (m, 1H), 6.71 (s, 1H), 7.35 (d, J = 11.7 Hz, 1H), 7.53 (s, 1H), 7.54 (d, J = 9.0 Hz, 2H), 7.76 (d, J = 9.0 Hz, 2H).

Step 5: Synthesis of I-276

Compound 78 (19.6 mg, 0.035 mmol) was dissolved in methanol (500 μL), and p-toluenesulfonic acid monohydrate (1.4 mg, 7.0 μmol) was added and stirred at 50°C for 1 hour. After cooling, water (1 mL) was added and stirred for 5 minutes. The precipitated solid was filtered off and dried to give I-276 (14.3 mg, yield: 86%).

1H-NMR (CDCl3) δ: 2.00 (t, J = 5.9 Hz, 1H), 3.84 (s, 3H), 3.88-3.93 (m, 2H), 4.08 (t, J = 4.2 Hz, 2H), 6.73 (s, 1H), 7.29 (d, J = 11.4 Hz, 1H), 7.55 (d, J = 8.5 Hz, 2H), 7.56 (s, 1H), 7.74 (d, J = 8.5 Hz, 2H).

Example 22: Synthesis of Compound I-329

[Chemical formula 85]

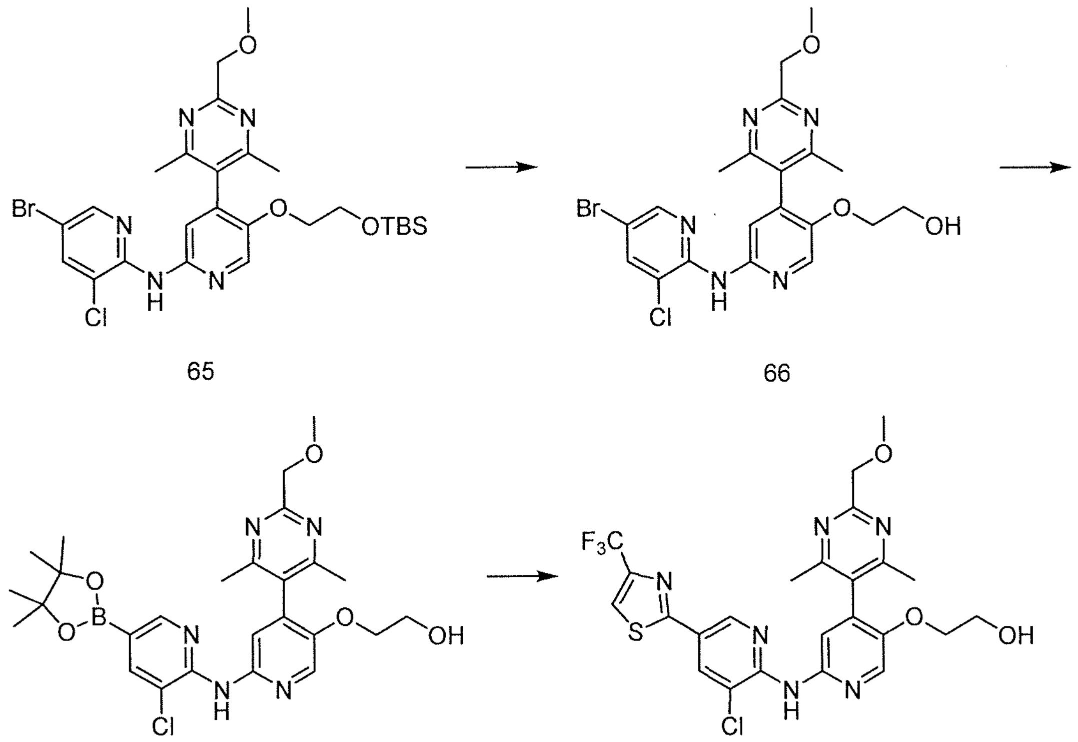

65

66

80

I-329

Step 1: Synthesis of Compound 66

Compound 65 (858 mg, 1.41 mmol) was dissolved in THF (8.6 mL), and 1 mol/L tetrabutylammonium fluoride-THF solution (2.11 mL, 2.11 mmol) was added and stirred at room temperature for 1 hour. Saturated aqueous ammonium chloride solution (30 mL) was added and extracted with ethyl acetate (30 mL). The organic layer was washed with brine (30 mL) and then dried over anhydrous sodium sulfate. The solvent was removed under reduced pressure, and IPE (5 mL) was added to the resulting residue. The precipitated solid was washed with IPE (6 mL), then filtered off, and dried to give Compound 66 (617 mg, yield: 89%).

$^1$H-NMR (CDCl3) δ: 1.63 (t, J = 6.3 Hz, 1H), 2.35 (s, 6H), 3.61 (s, 3H), 3.77-3.83 (m, 2H), 4.11 (t, J = 4.5 Hz, 2H), 4.70 (s, 2H), 7.75 (d, J = 2.0 Hz, 1H), 7.78 (s, 1H), 8.10 (s, 1H), 8.13 (d, J = 2.0 Hz, 1H), 8.21 (s, 1H).

Step 2: Synthesis of Compound 80

Under a nitrogen atmosphere, Compound 66 (100 mg, 0.202 mmol) was dissolved in 1,4-dioxane (1 mL), and bis(pinacolate)diboron (61.6 mg, 0.243 mmol), $PdCl_2$(dppf) (16.5 mg, 0.020 mmol), and potassium acetate (39.7 mg, 0.404 mmol) were added, and stirred at 80°C for 2 hours. After cooling, bis(pinacolate)diboron (26 mg, 0.102 mmol) was added and stirred at 80°C for 1.5 hours. After cooling, bis(pinacolate)diboron (30 mg, 0.118 mmol) was added and further stirred at 80°C for 1 hour. After cooling, water (10 mL) was added and extracted with ethyl acetate (10 mL). The organic layer was dried over anhydrous sodium sulfate, and the solvent was removed under reduced pressure. The resulting residue was purified by silica gel column chromatography (chloroform-methanol) to give Compound 80 (62 mg, yield: 57%). 1H-NMR (CDCl3) δ: 1.32 (s, 12H), 1.61-1.68 (m, 1H), 2.36 (s, 6H), 3.60 (s, 3H), 3.77-3.83 (m, 2H), 4.10 (t, J = 4.5 Hz, 2H), 4.70 (s, 2H), 7.92 (s, 1H), 7.94 (s, 1H), 8.10 (s, 1H), 8.37 (s, 1H), 8.43 (s, 1H).

Step 3: Synthesis of Compound I-329

Compound 80 (16.5 mg, 0.030 mmol) was dissolved in 1,4-dioxane (0.33 mL)-water (0.07 ml), and 2-bromo-4-(trifluoromethyl)thiazole (10.6 mg, 0.046 mmol), [1,1-bis(di-tert-butylphosphino)ferrocene]dichloropalladium (2.0 mg, 3.0 μmol), and potassium carbonate (12.6 mg, 0.091 mmol) were added, and stirred at 100°C for 4 hours. Chloroform (10 mL) and water (5 mL) were added and extracted with chloroform. The organic layer was dried over anhydrous sodium sulfate, and the solvent was removed under reduced pressure. The resulting residue was purified by thin layer chromatography (chloroform-methanol) and reverse phase liquid chromatography (acetonitrile-water) to give

Compound I-329 (6.6 mg, yield: 38%).

1H-NMR (CDCl3) δ: 1.63 (t, J = 6.3 Hz, 1H), 2.37 (s, 6H), 3.61 (s, 3H), 3.79-3.84 (m, 2H), 4.13 (t, J = 4.4 Hz, 2H), 4.71 (s, 2H), 7.71 (s, 1H), 8.04 (s, 1H), 8.13 (s, 1H), 8.31 (d, J = 2.1 Hz, 1H), 8.37 (s, 1H), 8.64 (d, J = 2.1 Hz, 1H).

Reference Example 6: Synthesis of Compound 82

[Chemical formula 86]

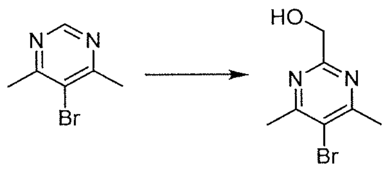

44 82

Step 1: Synthesis of Compound 82

Compound 44 (5.00 g, 26.7 mmol) was dissolved in dichloromethane (50 mL) and water (50 mL), and glycolic acid (4.07 g, 53.5 mmol), silver nitrate (454 mg, 2.67 mmol), and ammonium peroxodisulfate (12.2 g, 53.5 mmol) were added at 0°C, and stirred at room temperature for 1 hour. 2 mol/L aqueous potassium carbonate solution and brine were added, then filtered through celite (registered trademark) and extracted twice with chloroform (100 mL). The organic layer was dried over anhydrous magnesium sulfate. The solvent was removed under reduced pressure, and the resulting residue was purified by silica gel column chromatography (hexane-ethyl acetate) to give Compound 82 (1.69 g, yield: 29%).

1H-NMR (CDCl3) δ: 2.65 (6H, s), 3.59 (1H, br s), 4.71 (2H, d, J = 3.0 Hz).

Example 23: Synthesis of Compound I-376

[Chemical formula 87]

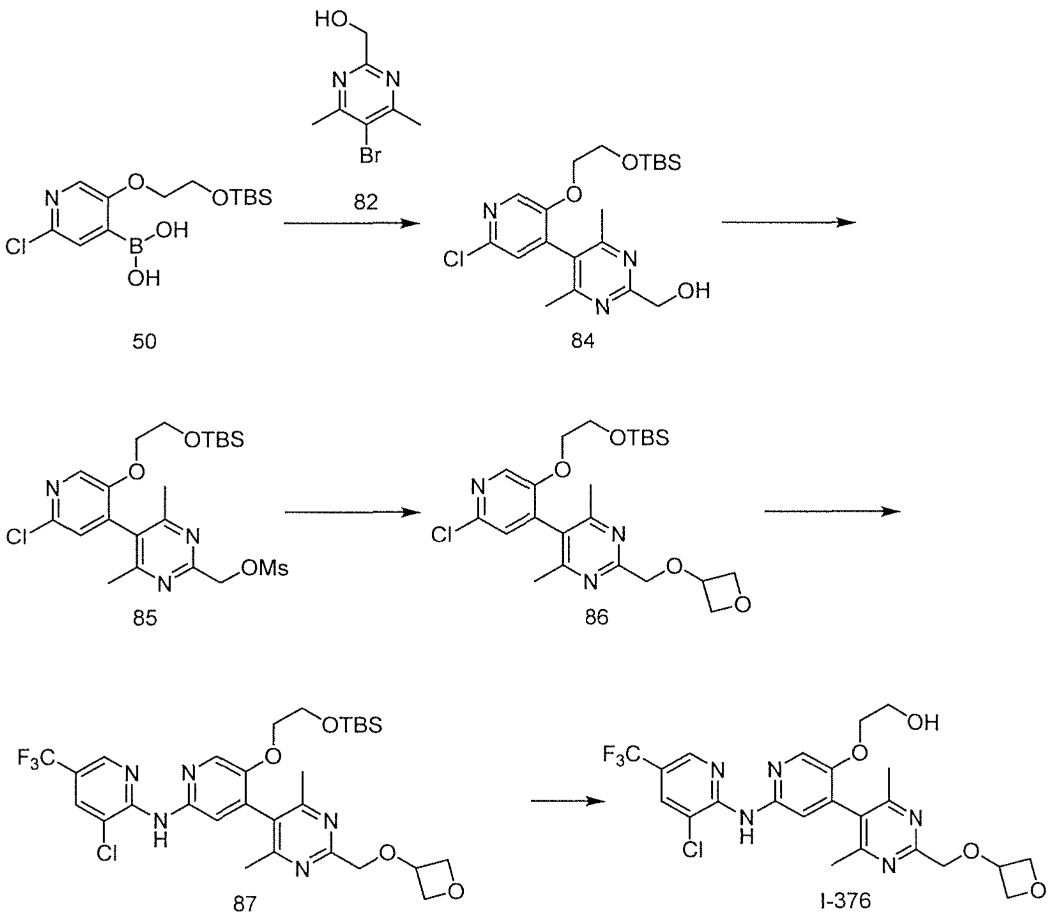

82

50

84

85

86

87

I-376

Step 1: Synthesis of Compound 84

1,4-Dioxane (17 mL)-water (3.4 mL) was added to Compound 50 (2.84 g, 8.56 mmol), Compound 82 (1.69 g, 7.79 mmol), [1,1-bis(di-tert-butylphosphino)ferrocene]dichloropalladium (507 mg, 0.779 mmol), and potassium carbonate (2.15 g, 15.6 mmol), and stirred at 80°C for 1 hour. After cooling, water was added and extracted with ethyl acetate (50 mL). The organic layer was washed with brine, and then dried over anhydrous magnesium sulfate. The solvent was removed under reduced pressure, and the resulting residue was purified by silica gel column chromatography (hexane-ethyl acetate) to give Compound 84 (1.36 g, yield: 41%).

1H-NMR (CDCl3) δ: -0.12 (6H, s), 0.79 (9H, s), 2.28 (6H, s), 3.81 (2H, t, J = 4.6 Hz), 3.84 (1H, br s), 4.12 (2H, t, J = 4.6 Hz), 4.79 (2H, s), 7.09 (1H, s), 8.19 (1H, s).

Step 2: Synthesis of Compound 85

Compound 84 (546 mg, 1.29 mmol) was dissolved in dichloromethane (5.46 mL), and triethylamine (268 μL, 1.93 mmol) and methanesulfonyl chloride (120 μL, 1.55 mmol) were added at 0°C, and stirred at room temperature for 15 minutes. The reaction solution was cooled to 0°C, saturated aqueous ammonium chloride solution was added, and extracted with dichloromethane (5 mL). The organic layer was washed with water and brine, and then dried over anhydrous magnesium sulfate. The solvent was removed under reduced pressure, and the resulting residue was purified by silica gel column chromatography (hexane-ethyl acetate) to give Compound 85 (638 mg, yield: 99%).

1H-NMR (CDCl3) δ: -0.11 (6H, s), 0.79 (9H, s), 2.27 (6H, s), 3.28 (3H, s), 3.81 (2H, t, J = 4.8 Hz), 4.12 (2H, t, J = 4.8 Hz), 5.39 (2H, s), 7.07 (1H, s) 8.20 (1H, s).

Step 3: Synthesis of Compound 86

Compound 85 (60.0 mg, 0.119 mmol) was dissolved in THF (600 μL), and sodium hydride (60%, dispersed in

paraffin liquid) (7.65 mg, 0.191 mmol) and 3-oxetanol (11.4 μL, 0.179 mmol) were added at 0°C and stirred at room temperature for 15 minutes. Water was added and extracted with dichloromethane (4 mL). The organic layer was washed with water and brine, and then dried over anhydrous magnesium sulfate. The solvent was removed under reduced pressure to give Compound 86 (50 mg) as a crude product. The obtained Compound 86 was used in the next step without further purification.

Step 4: Synthesis of Compound 87

Toluene (500 μL) was added to the crude product of Compound 86 (50 mg), 3-chloro-5-(trifluoromethyl)pyridin-2-amine (30.7 mg, 0.156 mmol), cesium carbonate (67.9 mg, 0.208 mmol), BINAP (13.0 mg, 0.021 mmol), and tris(dibenzylideneacetone)dipalladium (9.54 mg, 0.010 mmol), and stirred at 100°C for 15 hours. After cooling, the reaction solution was roughly purified by silica gel column chromatography (hexane-ethyl acetate, ethyl acetate-methanol) to give Compound 87 as a crude product. The resulting Compound 87 (43.8 mg) was used in the next step without further purification.

Step 5: Synthesis of Compound I-376

The crude product of Compound 87 (43.8 mg) was dissolved in THF (438 μL), and 1 mol/L tetrabutylammonium fluoride tetrahydrofuran solution (137 μL, 0.137 mmol) was added and stirred at room temperature for 1 hour. The solvent was removed under reduced pressure, and the resulting residue was purified by silica gel column chromatography (hexane-ethyl acetate, ethyl acetate-methanol) to give Compound I-376 (28.0 mg, yield: 78%).
1H-NMR (CDCl3) δ: 1.63 (1H, br s), 2.34 (6H, s), 3.82 (2H, dd, J = 9.7, 5.4 Hz), 4.14 (2H, t, J = 4.5 Hz), 4.69 (2H, s), 4.81-4.84 (5H, m) 7.83 (1H, d, J = 2.1 Hz), 8.03 (1H, s), 8.13 (1H, s), 8.32 (1H, s), 8.36 (1H, s).
The following compounds were synthesized according to the above general synthesis method and the method described in Examples. The structure and physical properties (LC/MS data) are shown in the following tables. Note that Compounds I-224 and Compounds I-225 in the table are chiral compounds whose absolute configuration is not determined, and the enantiomer of Compound 1-224 is Compound I-225. Furthermore, Compounds I-194, 1-198, I-214, I-216, and I-382 in the table are racemates.

[Table 1]

| Compound No. | Structure | MS(m/z) | RT | LCMS method |
|---|---|---|---|---|
| I-001 | 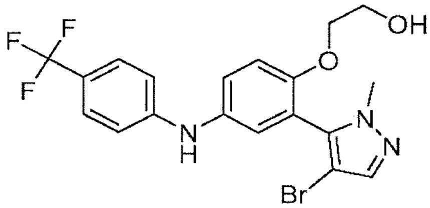 | 456 | 2.11 | 1 |
| I-002 | 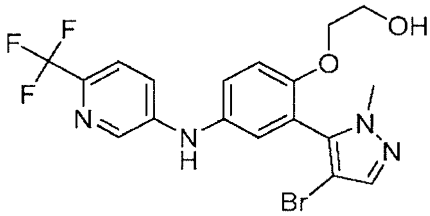 | 457 | 1.81 | 1 |
| I-003 | 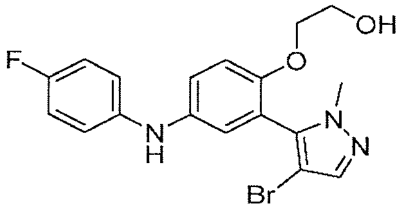 | 406 | 1.88 | 1 |

(continued)

| Compound No. | Structure | MS(m/z) | RT | LCMS method |
|---|---|---|---|---|
| I-004 | 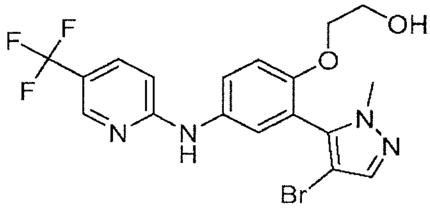 | 457 | 1.94 | 1 |
| I-005 | 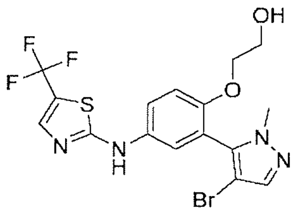 | 463 | 1.91 | 1 |
| I-006 | 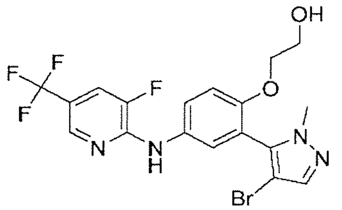 | 475 | 2.17 | 2 |
| I-007 | 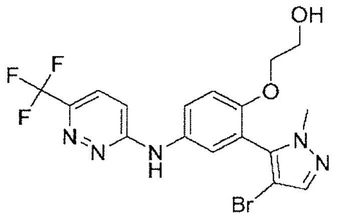 | 458 | 1.83 | 2 |

[Table 2]

| Compound No. | Structure | MS(m/z) | RT | LCMS method |
|---|---|---|---|---|
| I-008 | 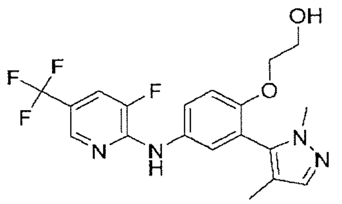 | 411 | 1.92 | 1 |
| I-009 | 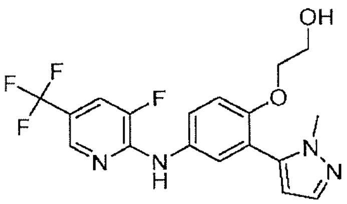 | 397 | 1.85 | 1 |
| I-010 | 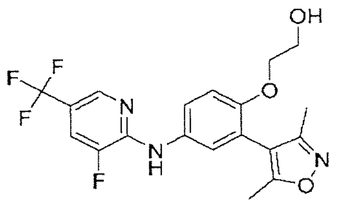 | 412 | 2.15 | 1 |

(continued)

| Compound No. | Structure | MS(m/z) | RT | LCMS method |
|---|---|---|---|---|
| I-011 | 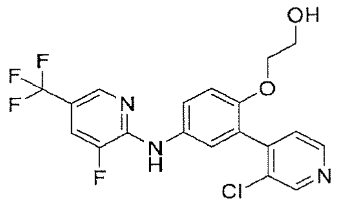 | 428 | 2.03 | 2 |
| I-012 | 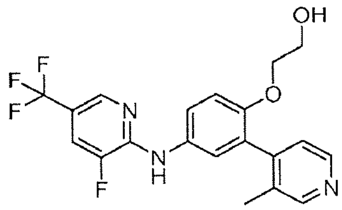 | 408 | 1.77 | 2 |
| I-013 | 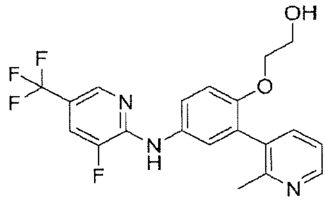 | 408 | 1.72 | 2 |
| I-014 | 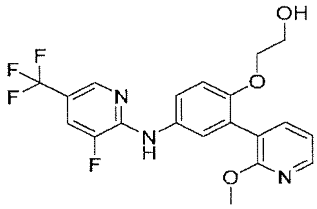 | 424 | 2.14 | 2 |

[Table 3]

| Compound No. | Structure | MS(m/z) | RT | LCMS method |
|---|---|---|---|---|
| I-015 | 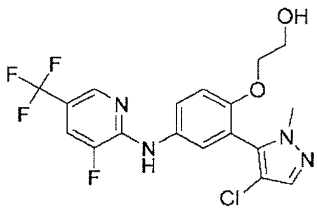 | 431 | 2.14 | 2 |
| I-016 | 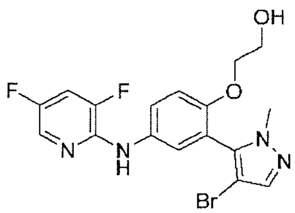 | 425 | 1.79 | 1 |
| I-017 | 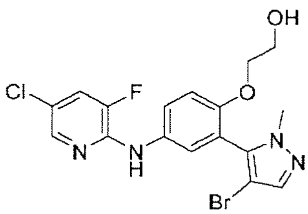 | 441 | 1.97 | 1 |

(continued)

| Compound No. | Structure | MS(m/z) | RT | LCMS method |
|---|---|---|---|---|
| I-018 | 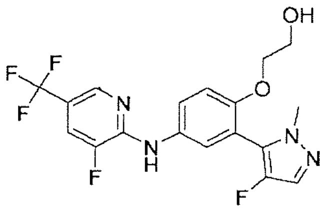 | 415 | 2.06 | 2 |
| I-019 | 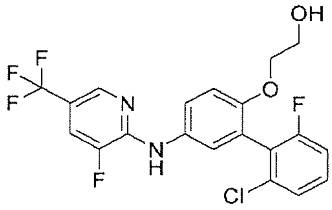 | 445 | 2.47 | 2 |
| I-020 | 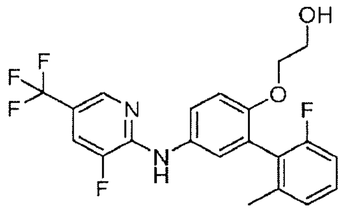 | 425 | 2.48 | 2 |
| I-021 | 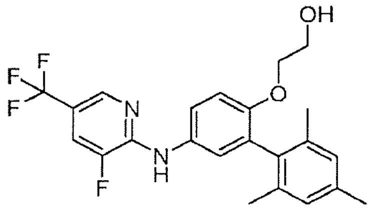 | 435 | 2.72 | 2 |

[Table 4]

| Compound No. | Structure | MS(m/z) | RT | LCMS method |
|---|---|---|---|---|
| I-022 | 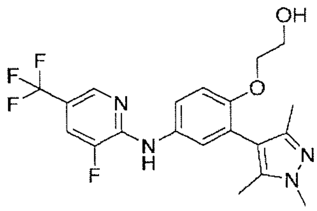 | 425 | 1.95 | 2 |
| I-023 | 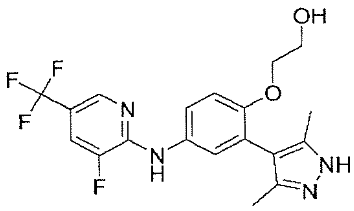 | 411 | 1.89 | 2 |
| I-024 | 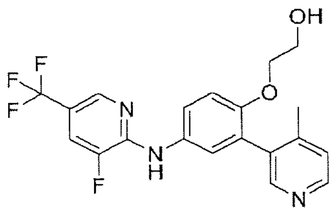 | 408 | 1.82 | 2 |

(continued)

| Compound No. | Structure | MS(m/z) | RT | LCMS method |
|---|---|---|---|---|
| I-025 | 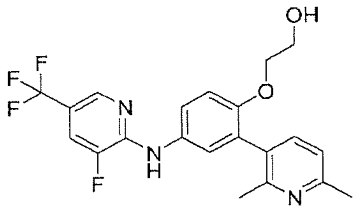 | 422 | 1.78 | 2 |
| I-026 | 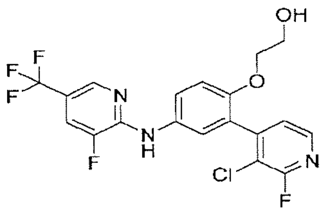 | 446 | 2.32 | 2 |
| I-027 | 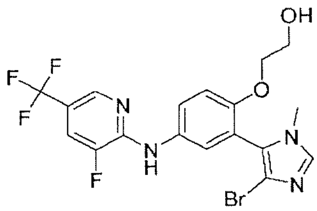 | 475 | 1.85 | 1 |
| I-028 | 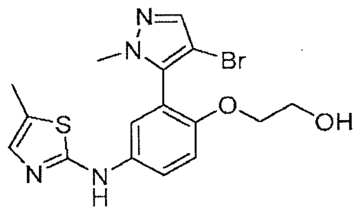 | 409 | 1.19 | 1 |

[Table 5]

| Compound No. | Structure | MS(m/z) | RT | LCMS method |
|---|---|---|---|---|
| I-029 | 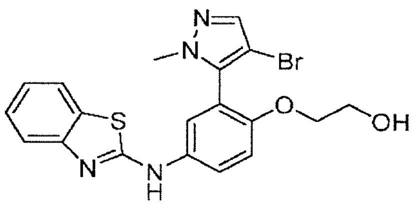 | 445 | 1.92 | 2 |
| I-030 | 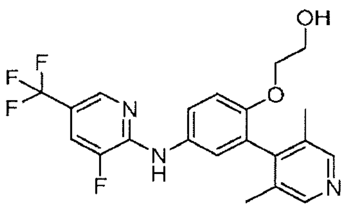 | 422 | 1.87 | 2 |
| I-031 | 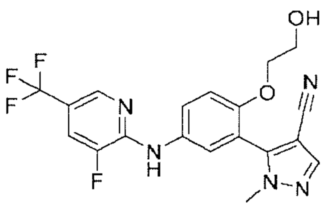 | 422 | 1.96 | 2 |

(continued)

| Compound No. | Structure | MS(m/z) | RT | LCMS method |
|---|---|---|---|---|
| I-032 | 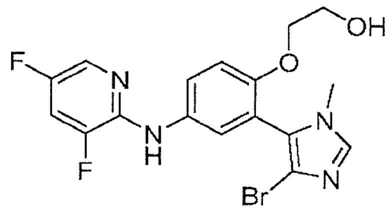 | 425 | 1.54 | 1 |
| I-033 | 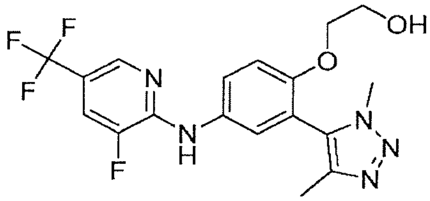 | 412 | 1.75 | 1 |
| I-034 | 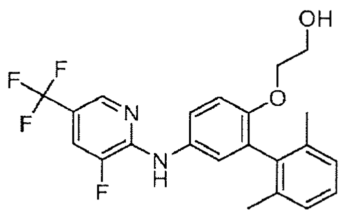 | 421 | 2.55 | 2 |
| I-035 | 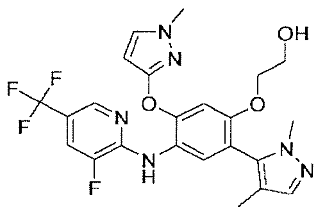 | 507 | 2 | 2 |

[Table 6]

| Compound No. | Structure | MS(m/z) | RT | LCMS method |
|---|---|---|---|---|
| I-036 | 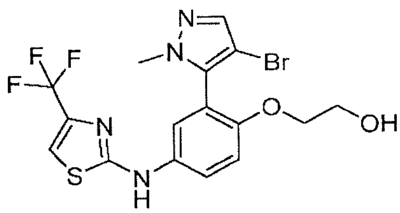 | 463 | 2.09 | 3 |
| I-037 | 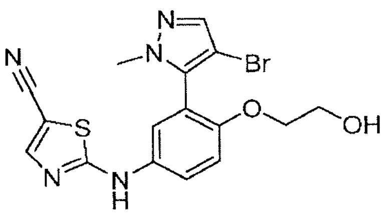 | 420 | 1.75 | 3 |
| I-038 | 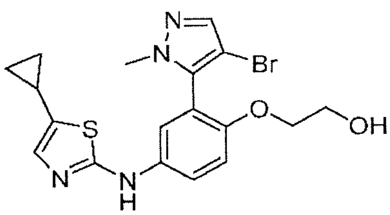 | 435 | 2.02 | 3 |

(continued)

| Compound No. | Structure | MS(m/z) | RT | LCMS method |
|---|---|---|---|---|
| I-039 | 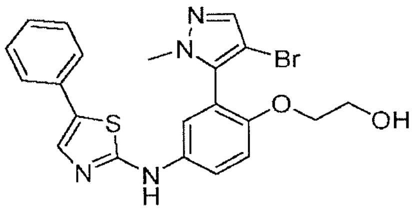 | 471 | 2.24 | 3 |
| I-040 | 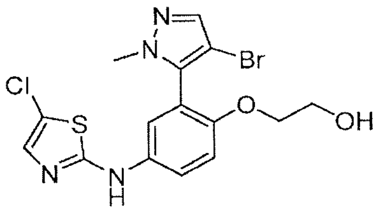 | 429 | 2.04 | 3 |
| I-041 | 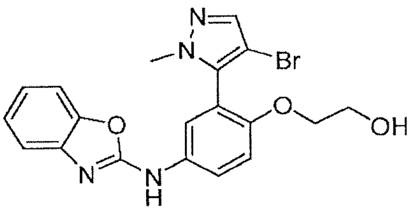 | 429 | 1.96 | 3 |
| I-042 | 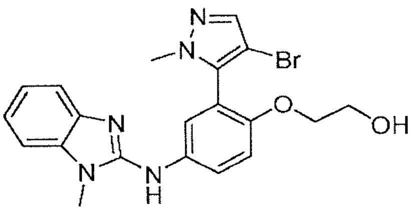 | 442 | 1.84 | 3 |

[Table 7]

| Compound No. | Structure | MS(m/z) | RT | LCMS method |
|---|---|---|---|---|
| I-043 | 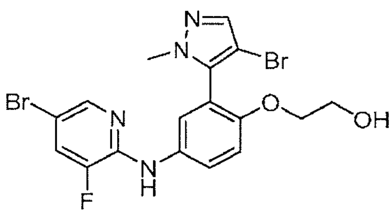 | 485 | 2.27 | 3 |
| I-044 | 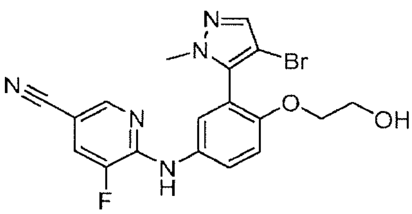 | 432 | 1.92 | 3 |
| I-045 | 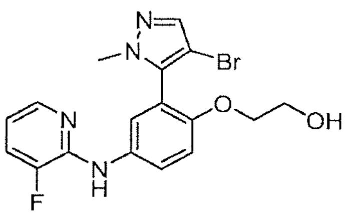 | 407 | 1.91 | 3 |

(continued)

| Compound No. | Structure | MS(m/z) | RT | LCMS method |
|---|---|---|---|---|
| I-046 | 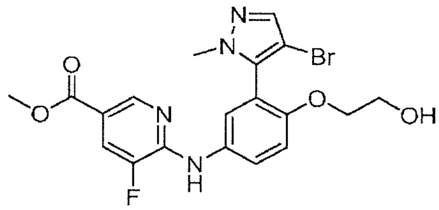 | 465 | 1.97 | 3 |
| I-047 | 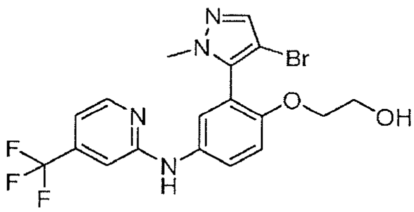 | 457 | 2.18 | 3 |
| I-048 | 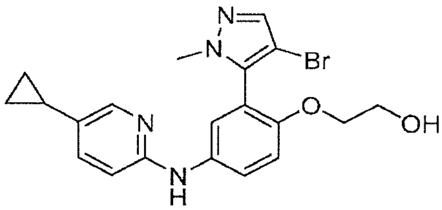 | 429 | 2.1 | 3 |
| I-049 | 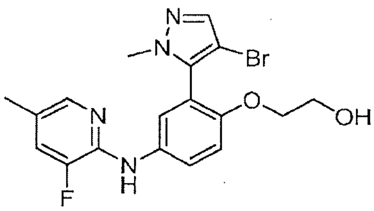 | 421 | 2.08 | 3 |

[Table 8]

| Compound No. | Structure | MS(m/z) | RT | LCMS method |
|---|---|---|---|---|
| I-050 | 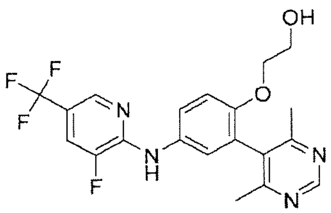 | 423 | 1.83 | 2 |
| I-051 | 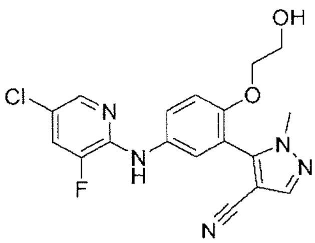 | 388 | 1.89 | 2 |

(continued)

| Compound No. | Structure | MS(m/z) | RT | LCMS method |
|---|---|---|---|---|
| I-052 | 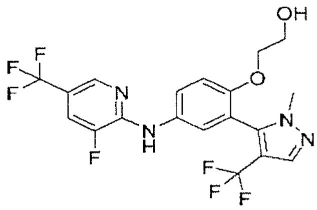 | 465 | 2.28 | 2 |
| I-053 | 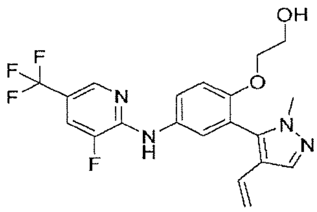 | 423 | 2.18 | 2 |
| I-054 | 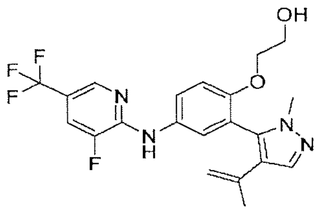 | 437 | 2.23 | 2 |
| I-055 | 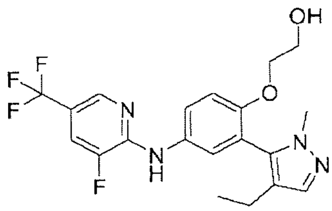 | 425 | 2.19 | 2 |
| I-056 | 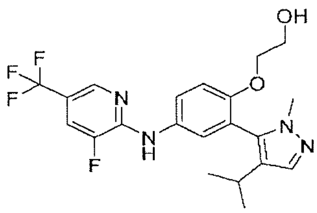 | 439 | 2.3 | 2 |

[Table 9]

| Compound No. | Structure | MS(m/z) | RT | LCMS method |
|---|---|---|---|---|
| I-057 | 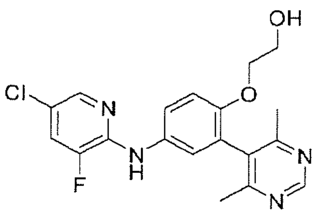 | 389 | 1.8 | 2 |
| I-058 | 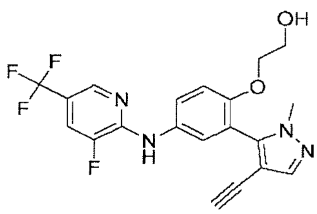 | 421 | 2.1 | 2 |

(continued)

| Compound No. | Structure | MS(m/z) | RT | LCMS method |
|---|---|---|---|---|
| I-059 | 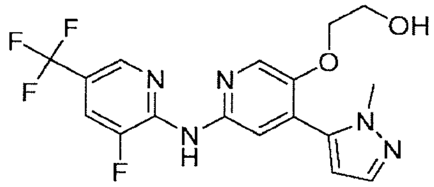 | 398 | 1.57 | 2 |
| I-060 | 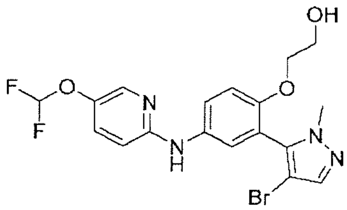 | 455 | 1.81 | 2 |
| I-061 | 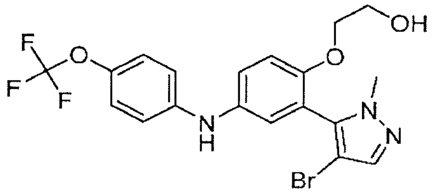 | 472 | 2.39 | 2 |
| I-062 | 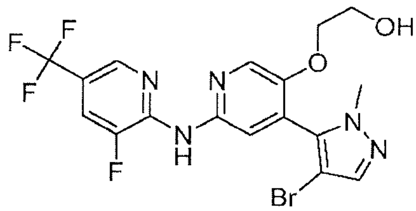 | 476 | 1.92 | 1 |
| I-063 | 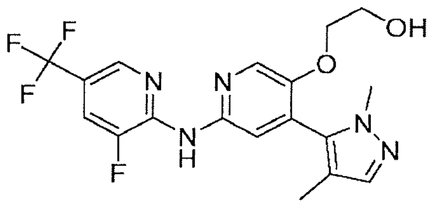 | 412 | 1.69 | 1 |

[Table 10]

| Compound No. | Structure | MS(m/z) | RT | LCMS method |
|---|---|---|---|---|
| I-064 | 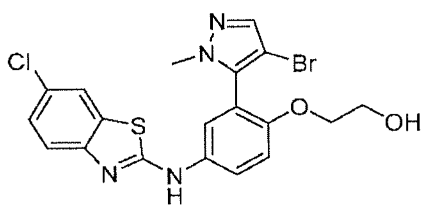 | 479 | 2.1 | 1 |
| I-065 | 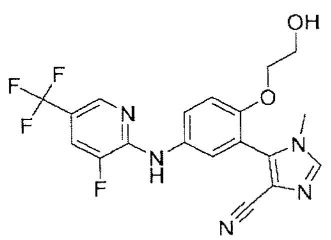 | 422 | 1.98 | 2 |

(continued)

| Compound No. | Structure | MS(m/z) | RT | LCMS method |
|---|---|---|---|---|
| I-066 | 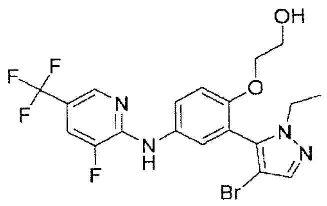 | 489 | 2.4 | 2 |
| I-067 | 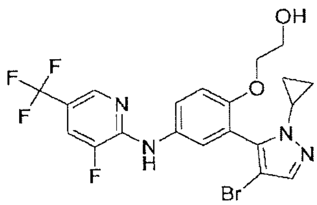 | 501 | 2.45 | 2 |
| I-068 | 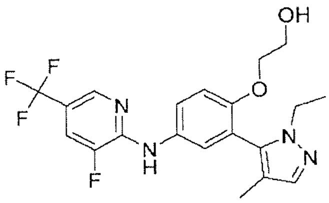 | 425 | 2.24 | 2 |
| I-069 | 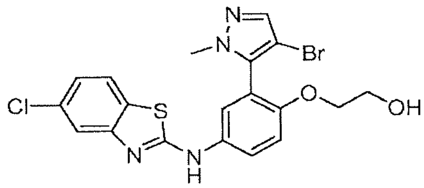 | 479 | 2.09 | 1 |
| I-070 | 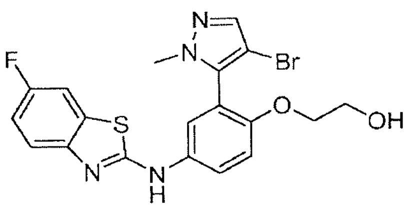 | 463 | 1.9 | 1 |

[Table 11]

| Compound No. | Structure | MS(m/z) | RT | LCMS method |
|---|---|---|---|---|
| I-071 | 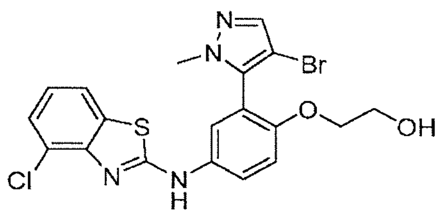 | 479 | 2.02 | 1 |
| I-072 | 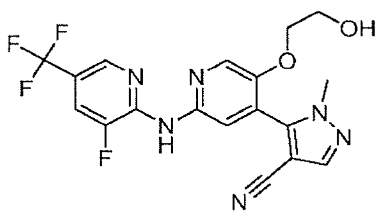 | 423 | 1.75 | 1 |

(continued)

| Compound No. | Structure | MS(m/z) | RT | LCMS method |
|---|---|---|---|---|
| I-073 | 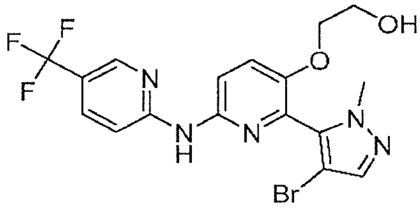 | 458 | 1.73 | 1 |
| I-074 | 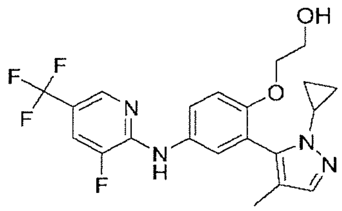 | 437 | 2.11 | 2 |
| I-075 | 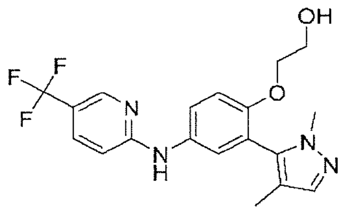 | 393 | 1.83 | 2 |
| I-076 | 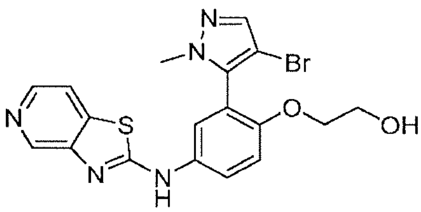 | 446 | 0.88 | 1 |
| I-077 | 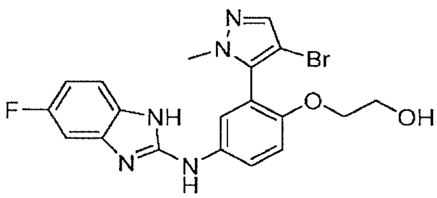 | 446 | 1.02 | 1 |

[Table 12]

| Compound No. | Structure | MS(m/z) | RT | LCMS method |
|---|---|---|---|---|
| I-078 | 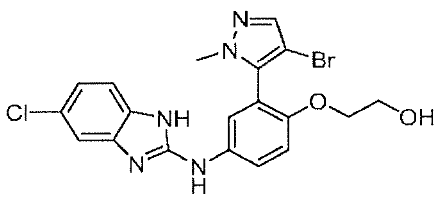 | 462 | 1.14 | 1 |
| I-079 | 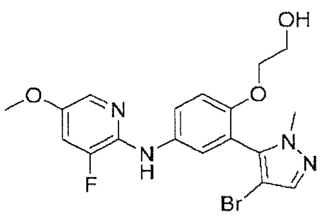 | 437 | 1.76 | 2 |

(continued)

| Compound No. | Structure | MS(m/z) | RT | LCMS method |
|---|---|---|---|---|
| I-080 | 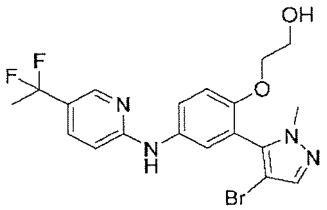 | 453 | 1.64 | 2 |
| I-081 | 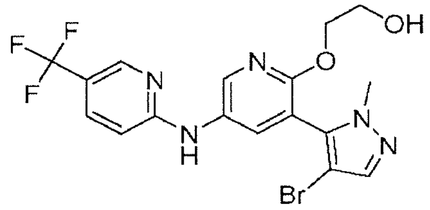 | 458 | 1.91 | 1 |
| I-082 | 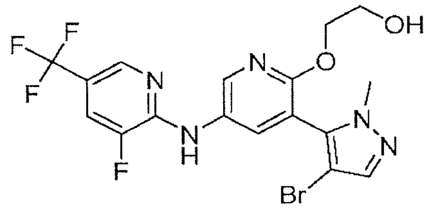 | 476 | 2.01 | 1 |
| I-083 | 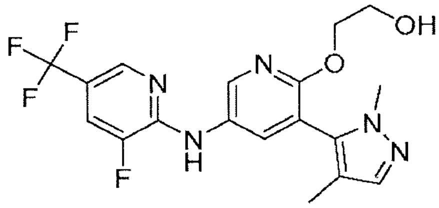 | 412 | 1.86 | 1 |
| I-084 | 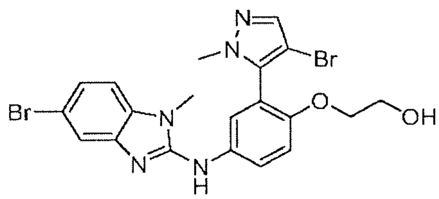 | 520 | 1.19 | 1 |

[Table 13]

| Compound No. | Structure | MS(m/z) | RT | LCMS method |
|---|---|---|---|---|
| I-085 | 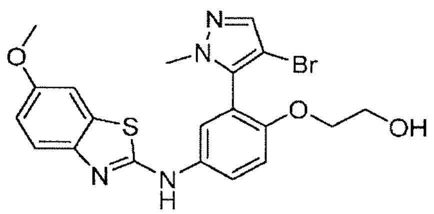 | 475 | 1.78 | 1 |
| I-086 | 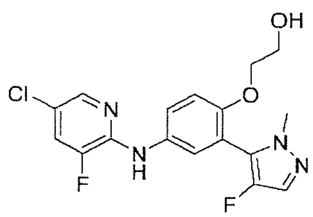 | 381 | 1.92 | 1 |

(continued)

| Compound No. | Structure | MS(m/z) | RT | LCMS method |
|---|---|---|---|---|
| I-087 | 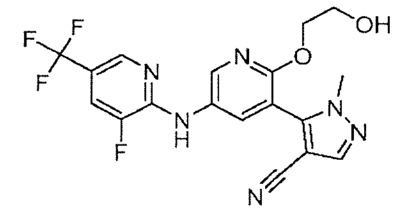 | 423 | 1.83 | 1 |
| I-088 | 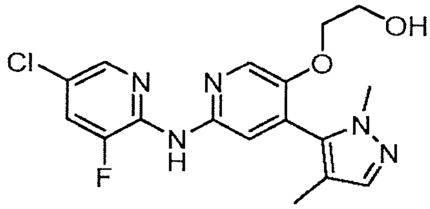 | 378 | 1.39 | 1 |
| I-089 | 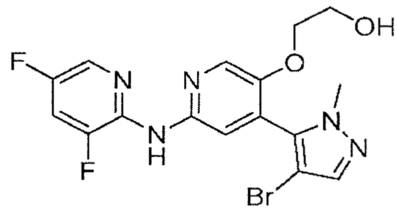 | 426 | 1.4 | 1 |
| I-090 | 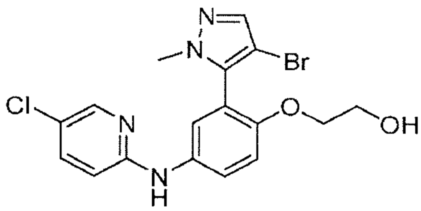 | 423 | 2.02 | 2 |
| I-091 | 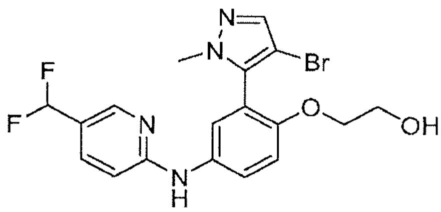 | 439 | 1.72 | 2 |

[Table 14]

| Compound No. | Structure | MS(m/z) | RT | LCMS method |
|---|---|---|---|---|
| I-092 | 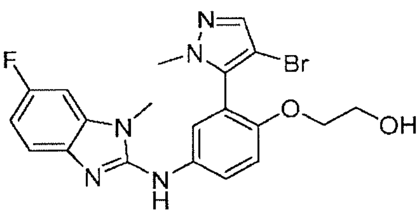 | 460 | 1.63 | 2 |
| I-093 | 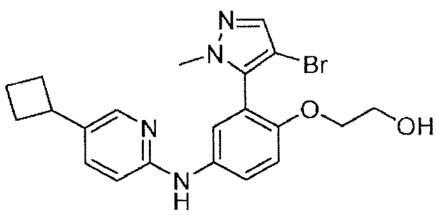 | 443 | 1.52 | 2 |

(continued)

| Compound No. | Structure | MS(m/z) | RT | LCMS method |
|---|---|---|---|---|
| I-094 | 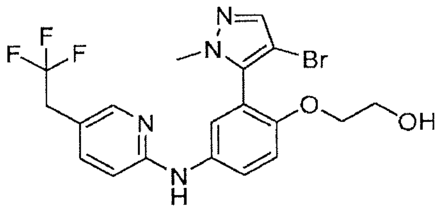 | 471 | 1.57 | 2 |
| I-095 | 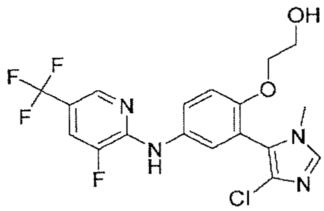 | 431 | 1.87 | 2 |
| I-096 | 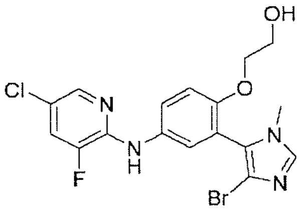 | 441 | 1.74 | 2 |
| I-097 | 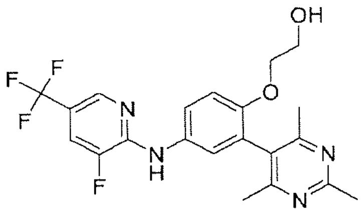 | 437 | 1.72 | 2 |
| I-098 | 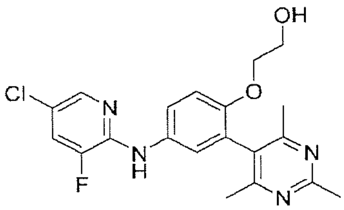 | 403 | 1.61 | 2 |

[Table 15]

| Compound No. | Structure | MS(m/z) | RT | LCMS method |
|---|---|---|---|---|
| I-099 | 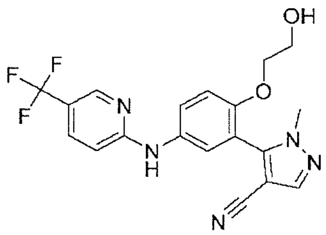 | 404 | 1.83 | 2 |

(continued)

| Compound No. | Structure | MS(m/z) | RT | LCMS method |
|---|---|---|---|---|
| I-100 | 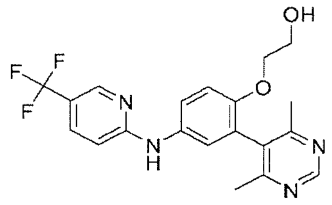 | 405 | 1.66 | 2 |
| I-101 | 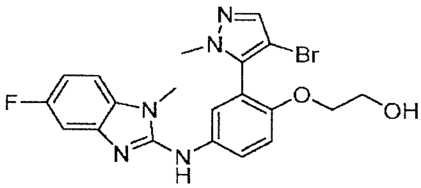 | 460 | 1.04 | 1 |
| I-102 | 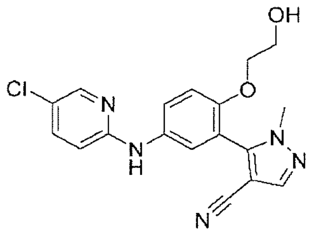 | 370 | 1.66 | 2 |
| I-103 | 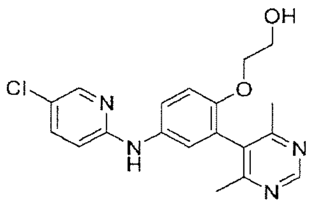 | 371 | 1.45 | 2 |
| I-104 | 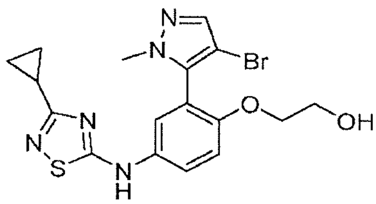 | 436 | 1.62 | 1 |
| I-105 | 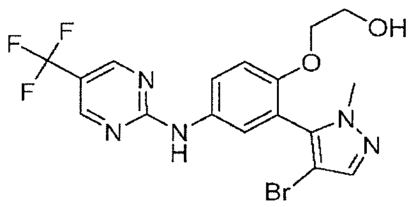 | 458 | 1.83 | 1 |

[Table 16]

| Compound No. | Structure | MS(m/z) | RT | LCMS method |
|---|---|---|---|---|
| I-106 | 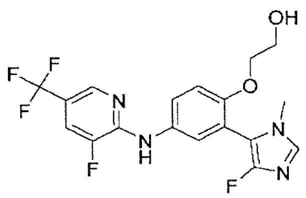 | 415 | 1.88 | 2 |

(continued)

| Compound No. | Structure | MS(m/z) | RT | LCMS method |
|---|---|---|---|---|
| I-107 | 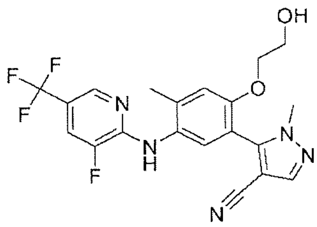 | 436 | 1.96 | 2 |
| I-108 | 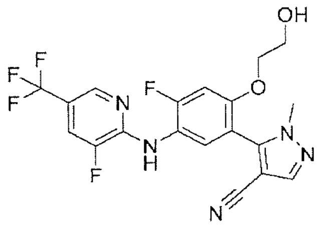 | 440 | 1.94 | 2 |
| I-109 | 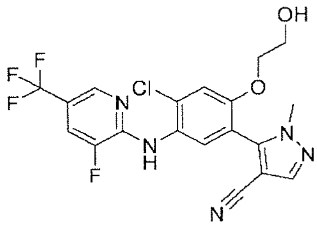 | 456 | 2.06 | 2 |
| 1-110 | 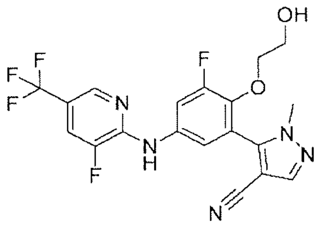 | 440 | 2.04 | 2 |
| I-111 | 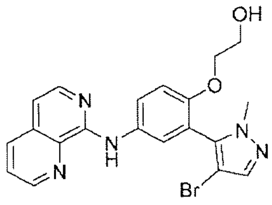 | 440 | 1.15 | 2 |
| I-112 | 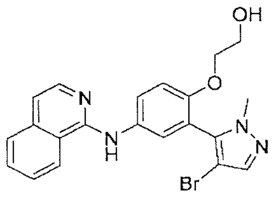 | 439 | 1.23 | 2 |

[Table 17]

| Compound No. | Structure | MS(m/z) | RT | LCMS method |
|---|---|---|---|---|
| I-113 | 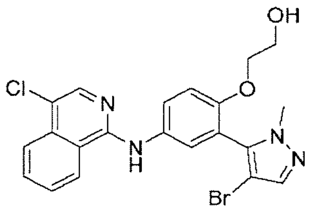 | 473 | 1.86 | 2 |
| I-114 | 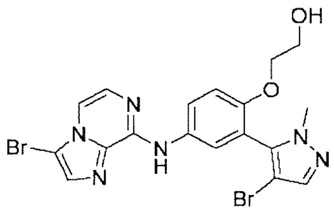 | 507 | 1.76 | 2 |
| I-115 | 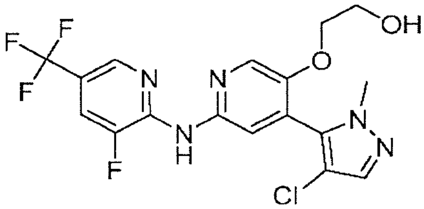 | 432 | 1.9 | 1 |
| I-116 | 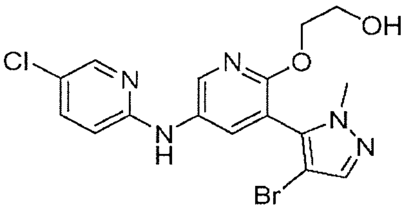 | 424 | 2.08 | 1 |
| I-117 | 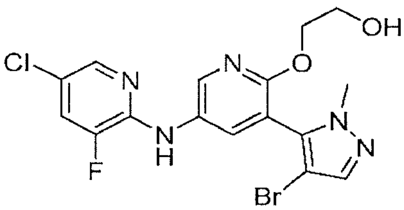 | 442 | 2.17 | 1 |
| I-118 | 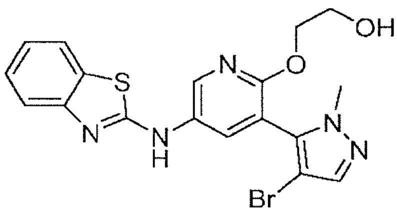 | 446 | 2.09 | 1 |
| I-119 | 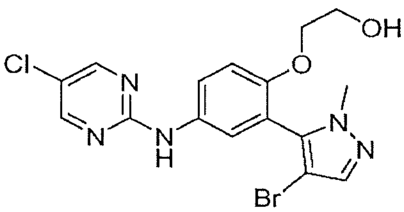 | 424 | 1.72 | 1 |

[Table 18]

| Compound No. | Structure | MS(m/z) | RT | LCMS method |
|---|---|---|---|---|
| I-120 | 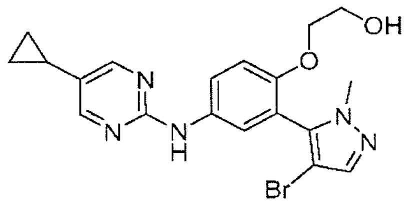 | 430 | 1.71 | 1 |
| I-121 | 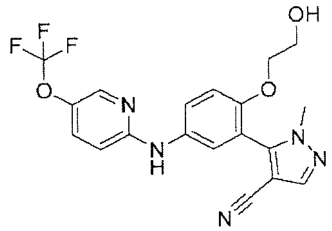 | 420 | 1.88 | 2 |
| I-122 | 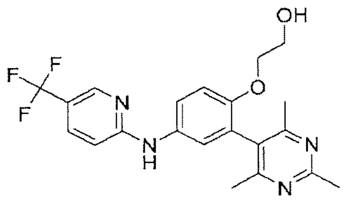 | 419 | 1.58 | 2 |
| I-123 | 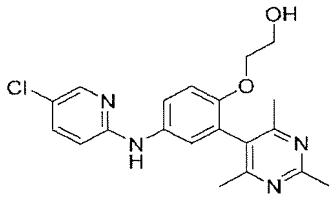 | 385 | 1.39 | 2 |
| I-124 | 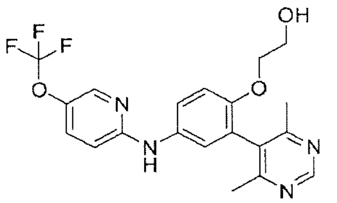 | 421 | 1.71 | 2 |
| I-125 | 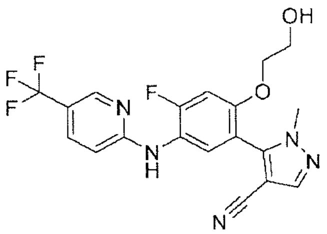 | 422 | 1.84 | 2 |
| I-126 | 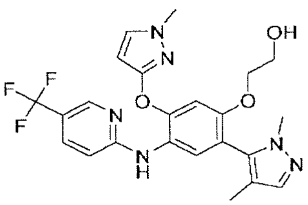 | 489 | 1.82 | 2 |

[Table 19]

| Compound No. | Structure | MS(m/z) | RT | LCMS method |
|---|---|---|---|---|
| I-127 | 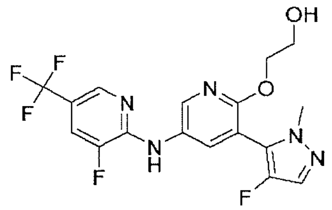 | 416 | 1.93 | 2 |
| I-128 | 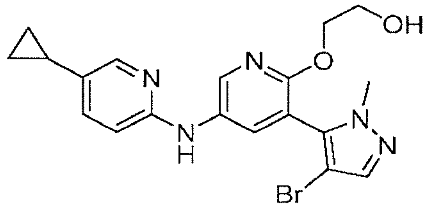 | 430 | 1.2 | 1 |
| I-129 | 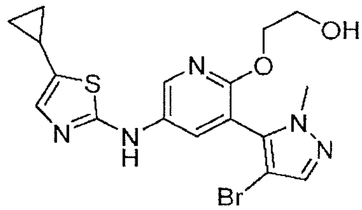 | 436 | 1.64 | 1 |
| I-130 | 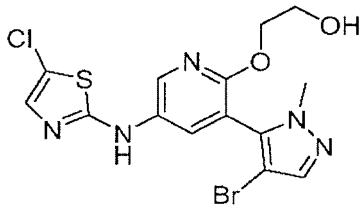 | 430 | 1.8 | 1 |
| I-131 | 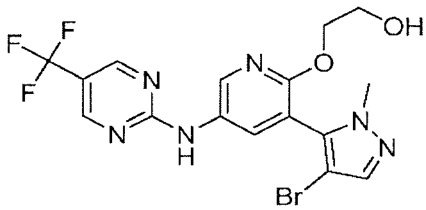 | 459 | 1.8 | 1 |
| I-132 | 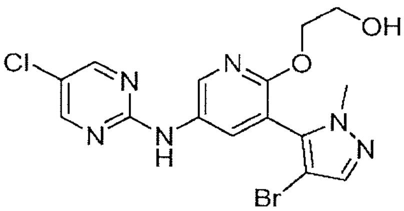 | 425 | 1.67 | 1 |
| I-133 | 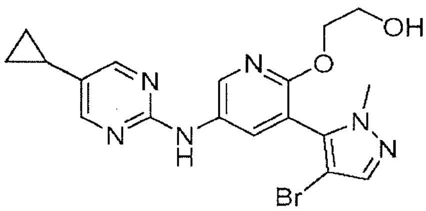 | 431 | 1.69 | 1 |

[Table 20]

| Compound No. | Structure | MS(m/z) | RT | LCMS method |
|---|---|---|---|---|
| I-134 | 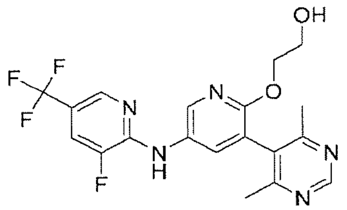 | 424 | 1.71 | 2 |
| 1-135 | 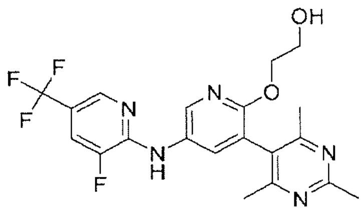 | 438 | 1.66 | 2 |
| I-136 | 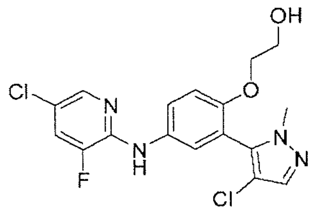 | 397 | 2.01 | 2 |
| I-137 | 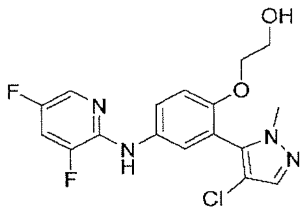 | 381 | 1.9 | 2 |
| I-138 | 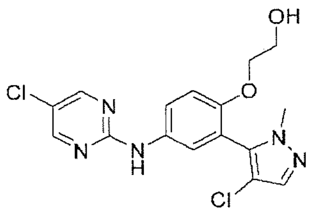 | 380 | 1.79 | 2 |
| I-139 | 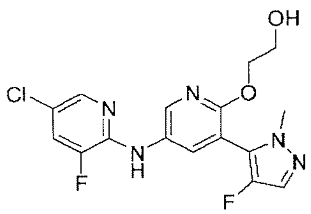 | 382 | 1.88 | 2 |
| I-140 | 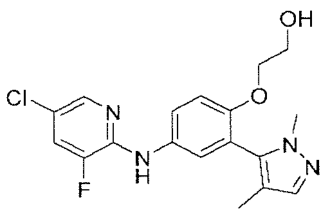 | 377 | 1.94 | 2 |

[Table 21]

| Compound No. | Structure | MS(m/z) | RT | LCMS method |
|---|---|---|---|---|
| I-141 | 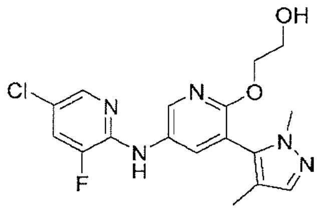 | 378 | 1.85 | 2 |
| I-142 | 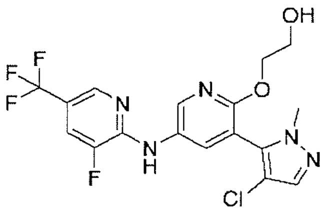 | 432 | 2.04 | 2 |
| I-143 | 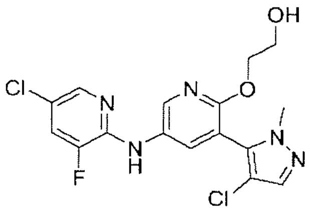 | 398 | 1.95 | 2 |
| I-144 | 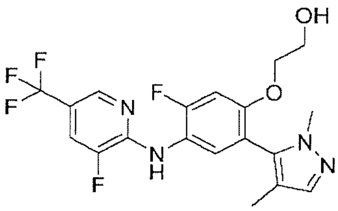 | 429 | 1.97 | 2 |
| I-145 | 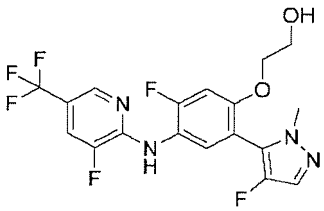 | 433 | 2.00 | 2 |
| I-146 | 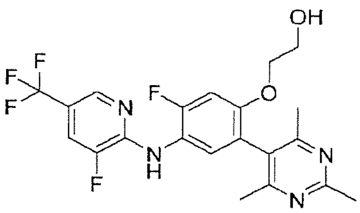 | 455 | 1.72 | 2 |
| I-147 | 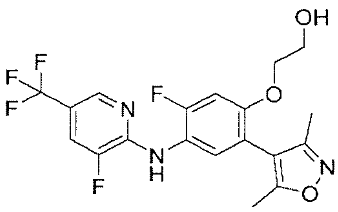 | 430 | 2.09 | 2 |

[Table 22]

| Compound No. | Structure | MS(m/z) | RT | LCMS method |
|---|---|---|---|---|
| I-148 | 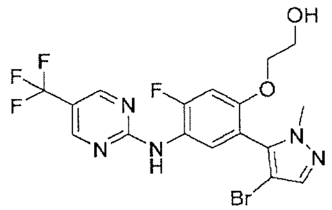 | 476 | 1.89 | 2 |
| I-149 | 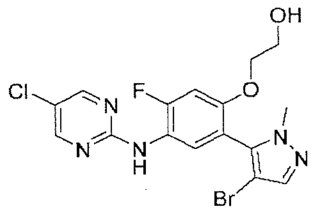 | 442 | 1.79 | 2 |
| I-150 | 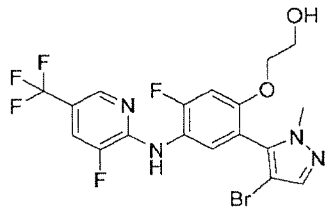 | 493 | 2.11 | 2 |
| I-151 | 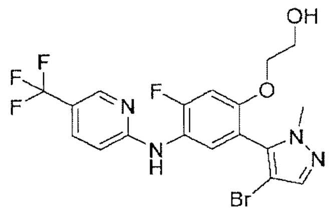 | 475 | 2.02 | 2 |
| I-152 | 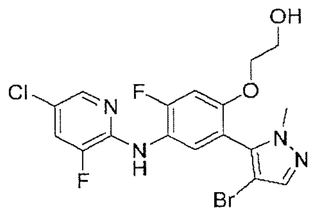 | 459 | 2.06 | 2 |
| I-153 | 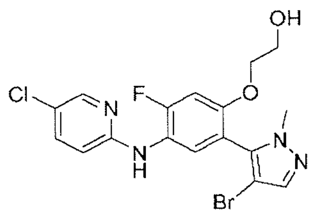 | 441 | 1.92 | 2 |
| I-154 | 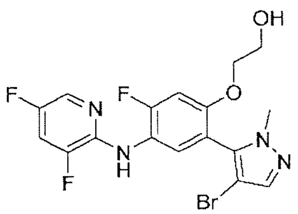 | 443 | 1.87 | 2 |

[Table 23]

| Compound No. | Structure | MS(m/z) | RT | LCMS method |
|---|---|---|---|---|
| I-155 | 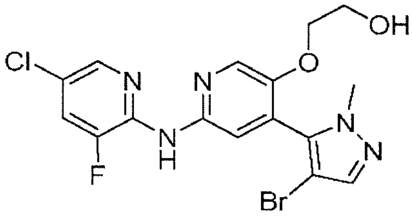 | 442 | 1.72 | 1 |
| I-156 | 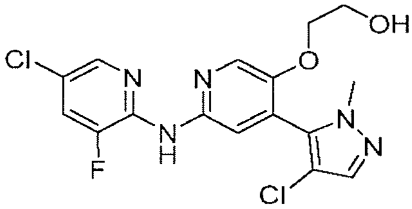 | 398 | 1.69 | 1 |
| I-157 | 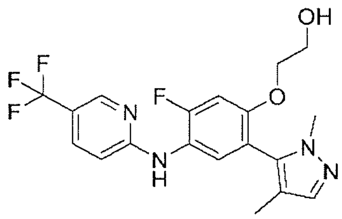 | 411 | 1.87 | 2 |
| I-158 | 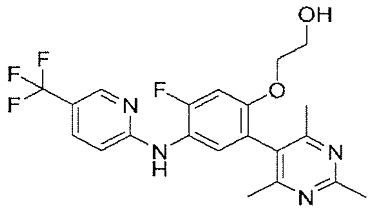 | 437 | 1.62 | 2 |
| I-159 | 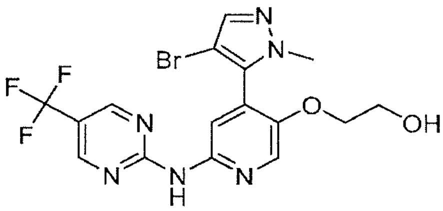 | 459 | 1.74 | 1 |

[Table 24]

| Compound No. | Structure | MS(m/z) | RT | LCMS method |
|---|---|---|---|---|
| I-160 | 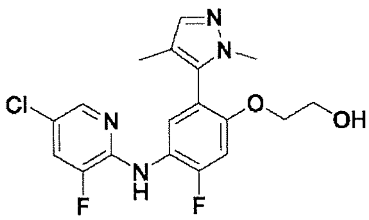 | 395 | 1.93 | 2 |

(continued)

| Compound No. | Structure | MS(m/z) | RT | LCMS method |
|---|---|---|---|---|
| I-161 | 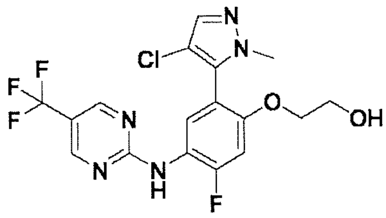 | 432 | 1.89 | 2 |
| I-162 | 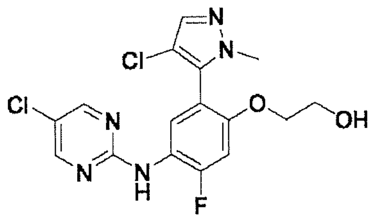 | 398 | 1.78 | 2 |
| I-163 | 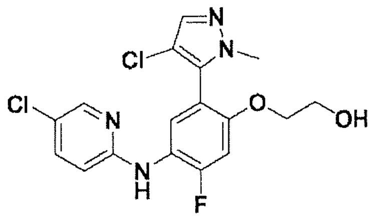 | 397 | 1.91 | 2 |
| I-164 | 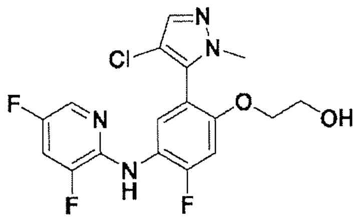 | 399 | 1.87 | 2 |
| I-165 | 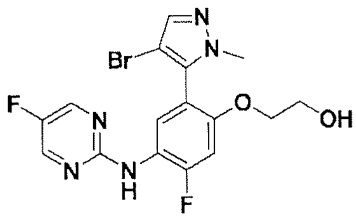 | 426 | 1.61 | 2 |
| I-166 | 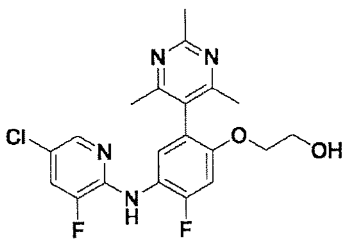 | 421 | 1.6 | 2 |

[Table 25]

| Compound No. | Structure | MS(m/z) | RT | LCMS method |
|---|---|---|---|---|
| I-167 | 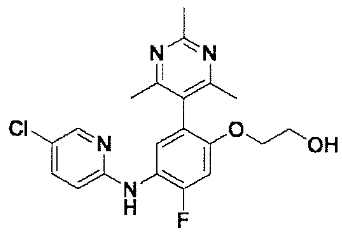 | 403 | 1.43 | 2 |
| I-168 | 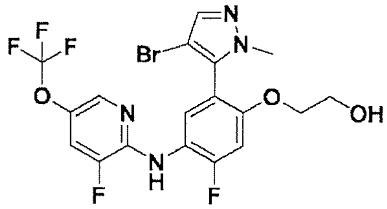 | 509 | 2.21 | 2 |
| I-169 | 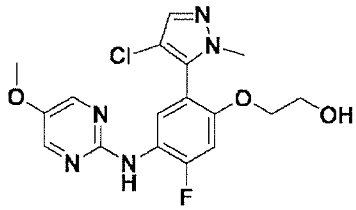 | 394 | 1.55 | 2 |
| I-170 | 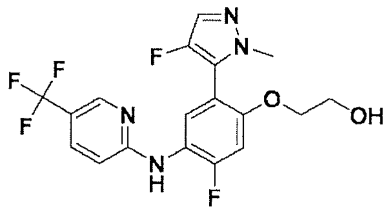 | 415 | 2.3 | 2 |
| I-171 | 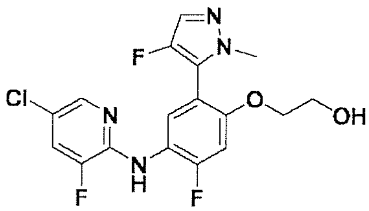 | 399 | 2.35 | 2 |
| I-172 | 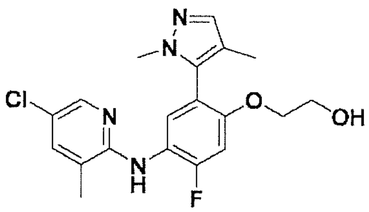 | 391 | 1.88 | 2 |
| I-173 | 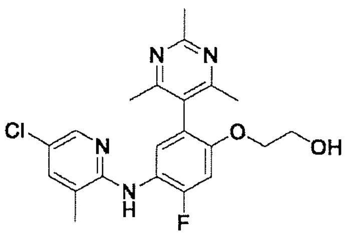 | 417 | 1.54 | 2 |

[Table 26]

| Compound No. | Structure | MS(m/z) | RT | LCMS method |
|---|---|---|---|---|
| I-174 | 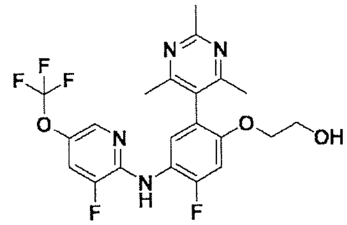 | 471 | 1.78 | 2 |
| I-175 | 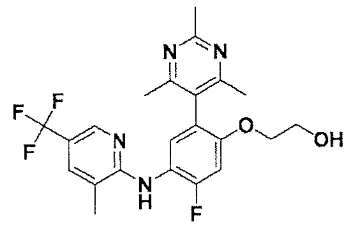 | 451 | 1.72 | 2 |
| I-176 | 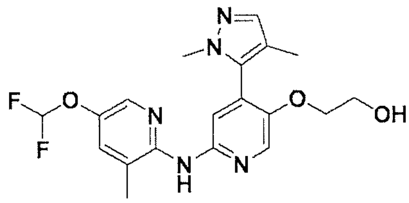 | 406 | 1.18 | 2 |
| I-177 | 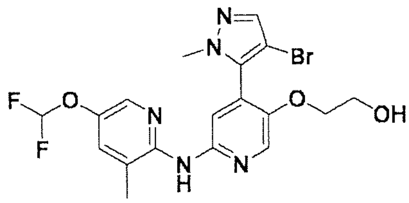 | 470 | 1.31 | 2 |
| I-178 | 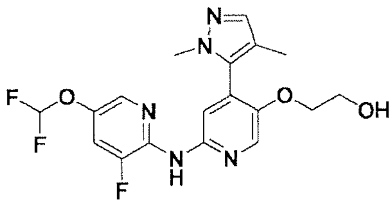 | 410 | 1.27 | 2 |
| I-179 | 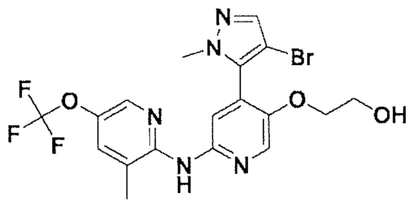 | 488 | 1.62 | 2 |
| I-180 | 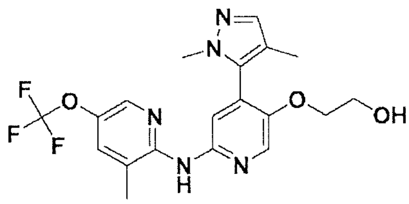 | 424 | 1.4 | 2 |

[Table 27]

| Compound No. | Structure | MS(m/z) | RT | LCMS method |
|---|---|---|---|---|
| I-181 | 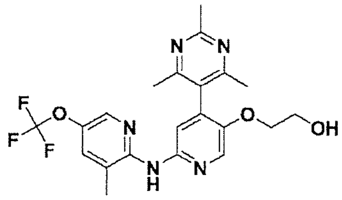 | 450 | 1.23 | 2 |
| I-182 | 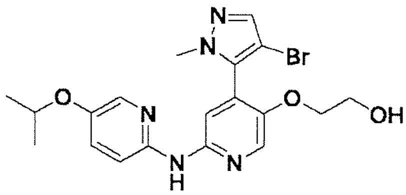 | 448 | 1.3 | 2 |
| I-183 | 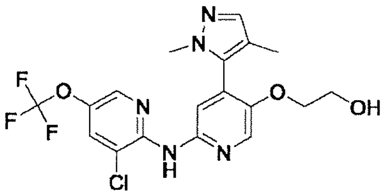 | 444 | 1.9 | 2 |
| I-184 | 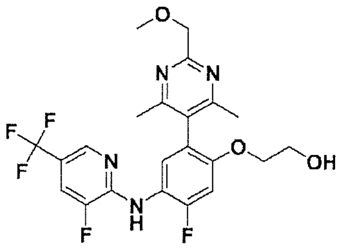 | 485 | 1.88 | 2 |
| I-185 | 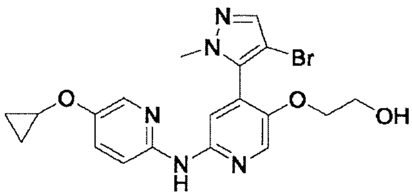 | 446 | 1.24 | 2 |
| I-186 | 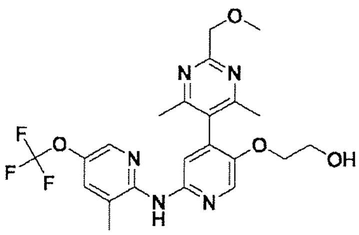 | 480 | 1.27 | 2 |
| I-187 | 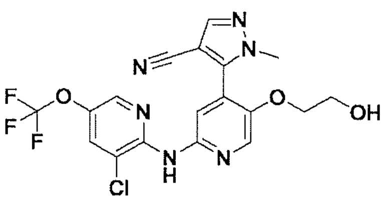 | 455 | 2.18 | 2 |

[Table 28]

| Compound No. | Structure | MS(m/z) | RT | LCMS method |
|---|---|---|---|---|
| I-188 | 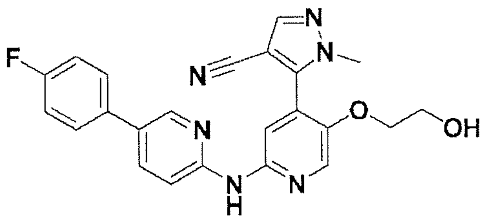 | 431 | 1.51 | 2 |
| I-189 | 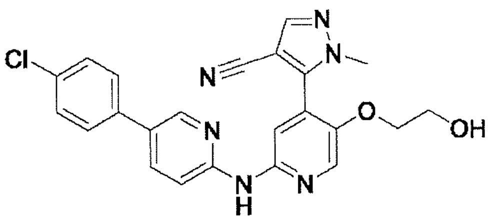 | 447 | 1.64 | 2 |
| I-190 | 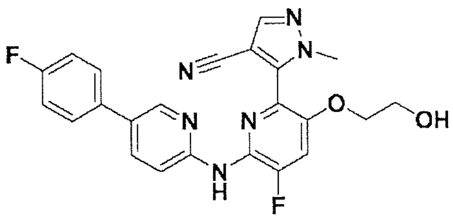 | 449.1 | 1.69 | 2 |
| I-191 | 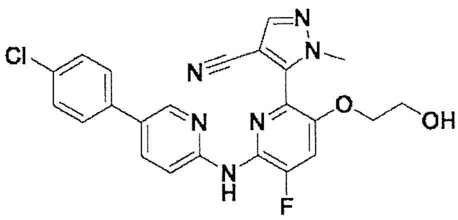 | 465.1 | 1.88 | 2 |
| I-192 | 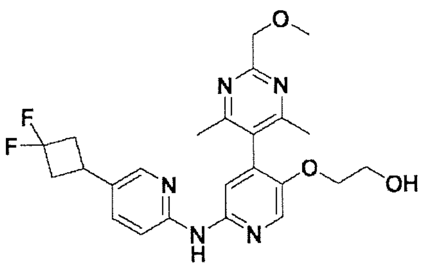 | 472 | 1.14 | 2 |
| I-193 | 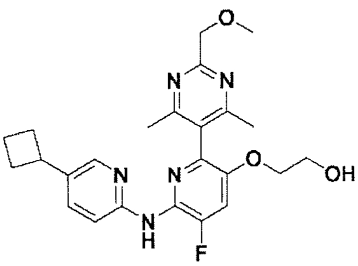 | 454 | 1.19 | 2 |

[Table 29]

| Compound No. | Structure | MS(m/z) | RT | LCMS method |
|---|---|---|---|---|
| I-194 | 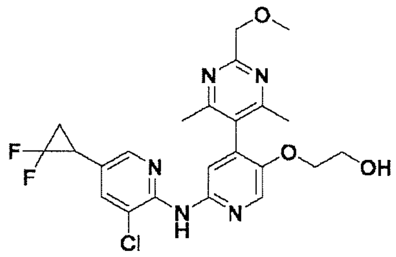 | 492 | 1.37 | 2 |
| I-195 | 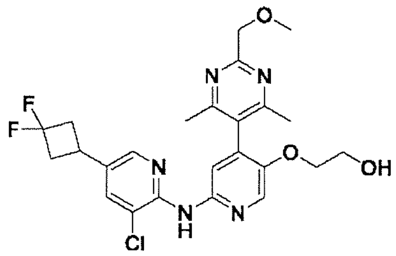 | 506 | 1.43 | 2 |
| I-196 | 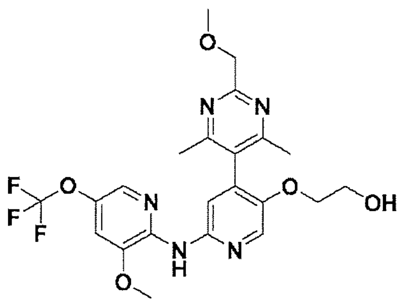 | 496.1 | 1.95 | 3 |
| I-197 | 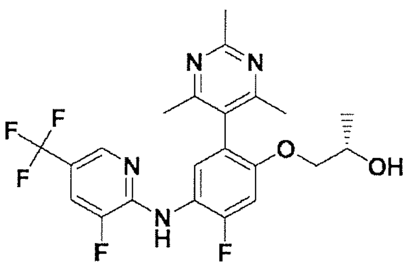 | 469 | 1.78 | 1 |
| I-198 | 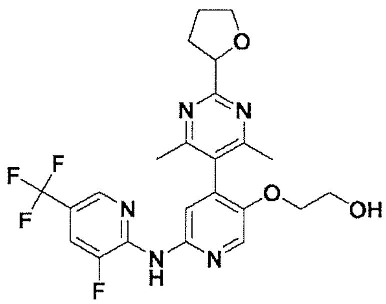 | 494 | 1.65 | 1 |

[Table 30]

| Compound No. | Structure | MS(m/z) | RT | LCMS method |
|---|---|---|---|---|
| I-199 | 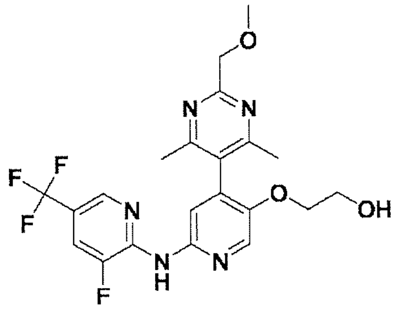 | 468 | 1.5 | 1 |
| I-200 | 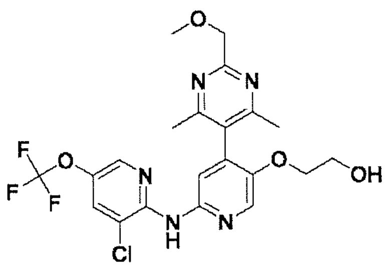 | 500 | 1.72 | 1 |
| I-201 | 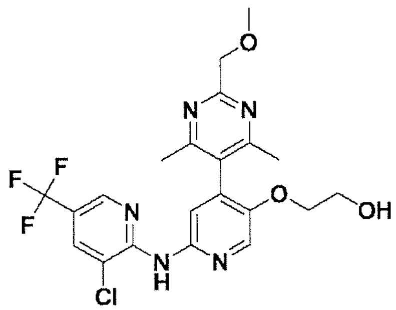 | 484 | 1.77 | 1 |
| I-202 | 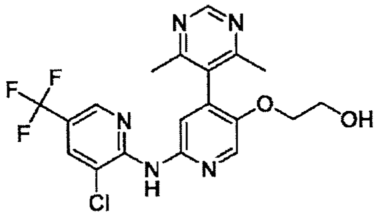 | 440 | 1.74 | 1 |
| I-203 | 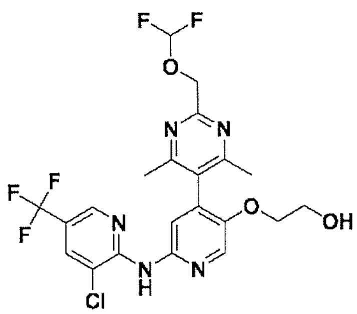 | 520 | 2.06 | 1 |

[Table 31]

| Compound No. | Structure | MS(m/z) | RT | LCMS method |
|---|---|---|---|---|
| I-204 | 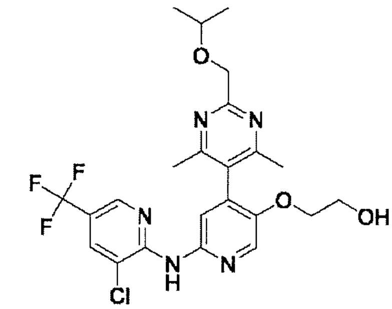 | 512 | 2.07 | 1 |
| I-205 | 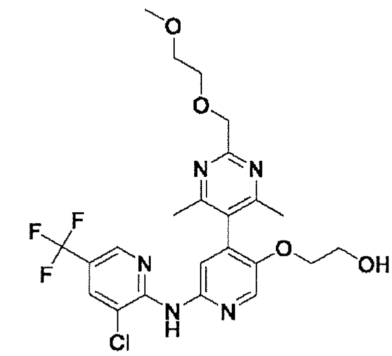 | 528 | 1.8 | 1 |
| I-206 | 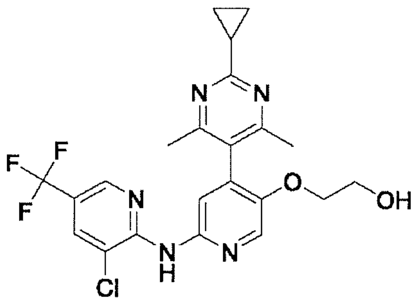 | 480 | 2.13 | 1 |
| I-207 | 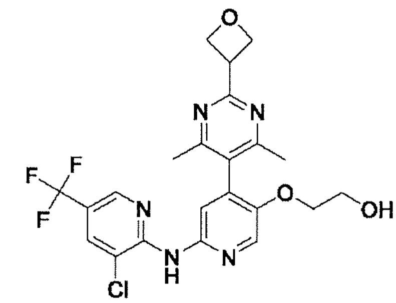 | 496 | 1.82 | 1 |
| I-208 | 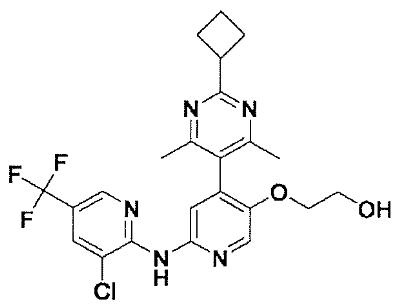 | 494 | 2.28 | 1 |

[Table 32]

| Compound No. | Structure | MS(m/z) | RT | LCMS method |
|---|---|---|---|---|
| I-209 | 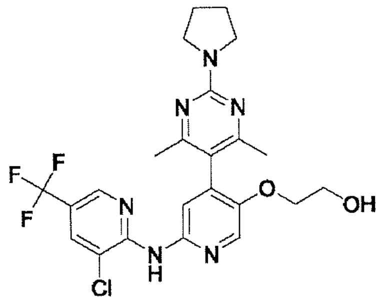 | 509 | 1.88 | 1 |
| I-210 | 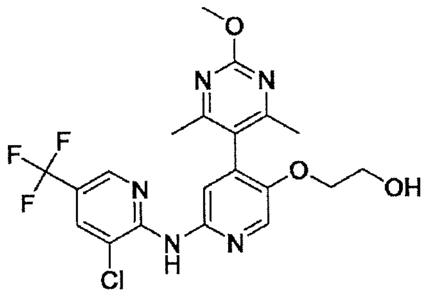 | 470 | 1.99 | 1 |
| I-211 | 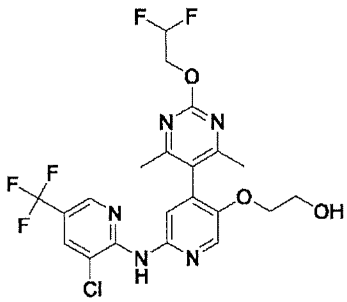 | 520 | 2.21 | 1 |
| I-212 | 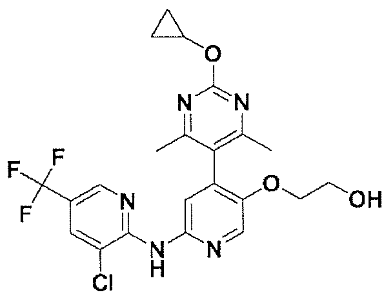 | 496 | 2.17 | 1 |
| I-213 | 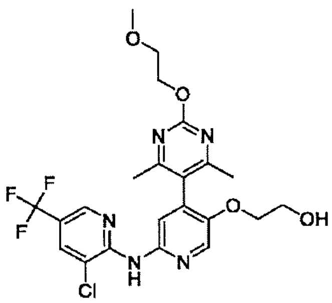 | 514 | 2 | 1 |

[Table 33]

| Compound No. | Structure | MS(m/z) | RT | LCMS method |
|---|---|---|---|---|
| I-214 | 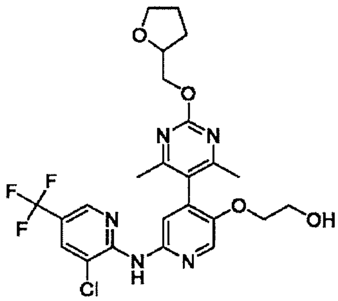 | 540 | 2.11 | 1 |
| I-215 | 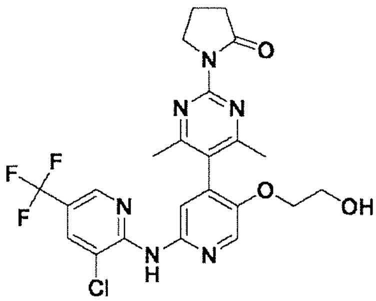 | 523 | 1.83 | 1 |
| I-216 | 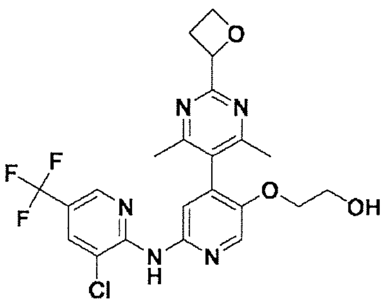 | 496 | 1.79 | 1 |
| I-217 | 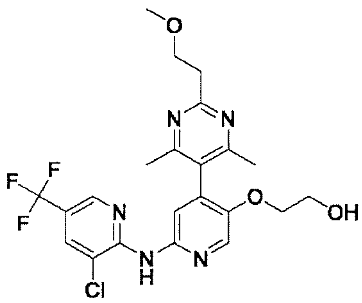 | 498 | 1.84 | 1 |
| I-218 | 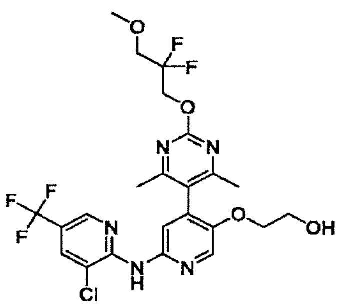 | 564 | 2.28 | 1 |

[Table 34]

| Compound No. | Structure | MS(m/z) | RT | LCMS method |
|---|---|---|---|---|
| I-219 | 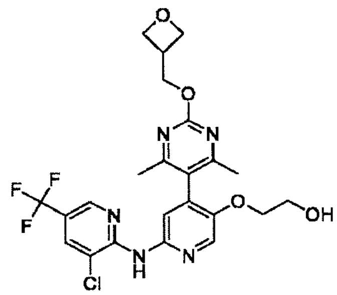 | 526 | 1.9 | 1 |
| I-220 | 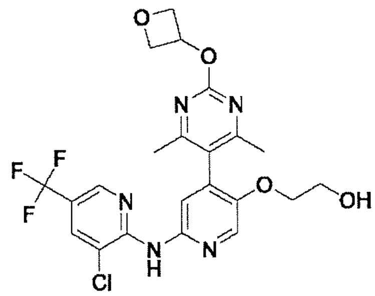 | 512 | 1.92 | 1 |
| I-221 | 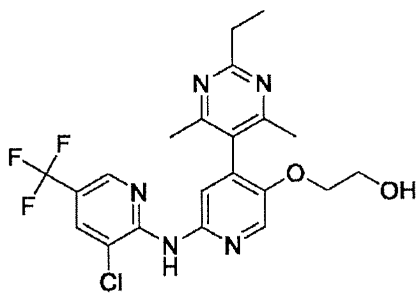 | 468 | 1.92 | 1 |
| I-222 | 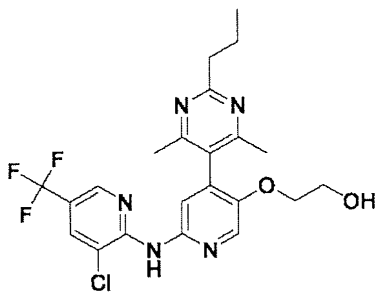 | 482 | 2.08 | 1 |
| I-223 | 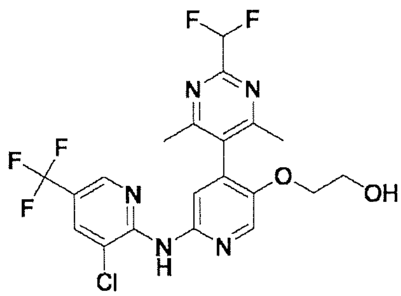 | 490 | 2.1 | 1 |

[Table 35]

| Compound No. | Structure | MS(m/z) | RT | LCMS method |
|---|---|---|---|---|
| I-224 | 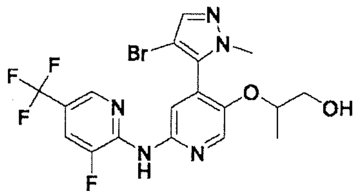 | 490 | 2.04 | 1 |
| I-225 | 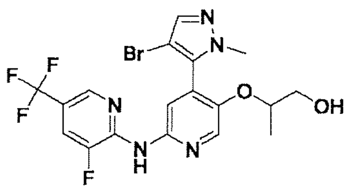 | 490 | 2.04 | 1 |
| I-226 | 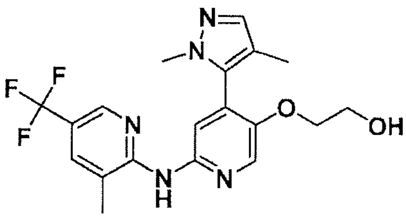 | 408 | 1.38 | 1 |
| I-227 | 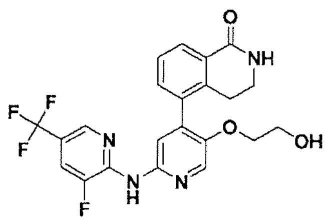 | 463 | 1.42 | 1 |
| I-228 | 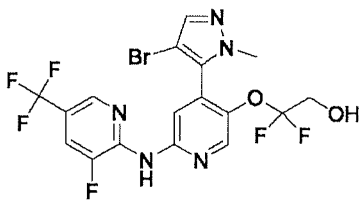 | 512 | 2.12 | 1 |
| I-229 | 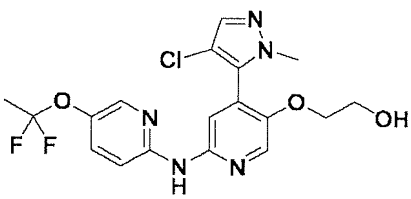 | 426 | 1.25 | 1 |
| I-230 | 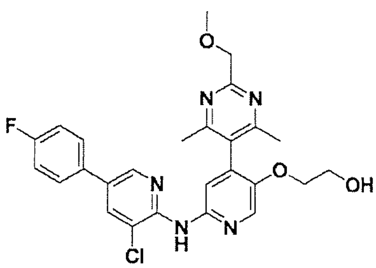 | 510 | 1.66 | 1 |

[Table 36]

| Compound No. | Structure | MS(m/z) | RT | LCMS method |
|---|---|---|---|---|
| I-231 | 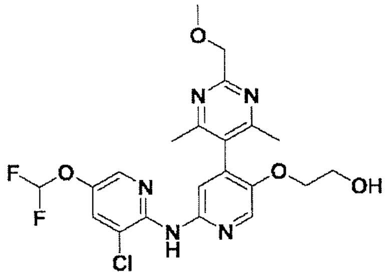 | 482 | 1.37 | 1 |
| I-232 | 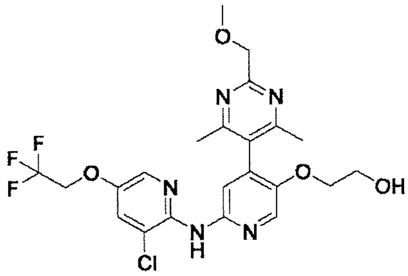 | 514 | 1.39 | 1 |
| I-233 | 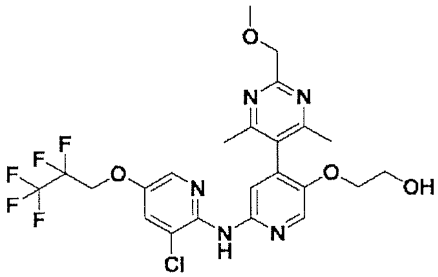 | 564 | 1.63 | 1 |
| I-234 | 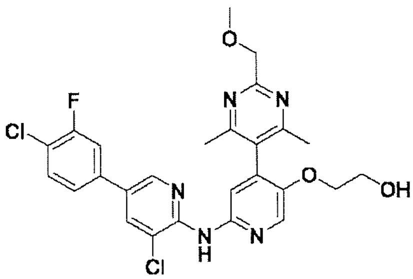 | 544 | 1.97 | 1 |
| I-235 | 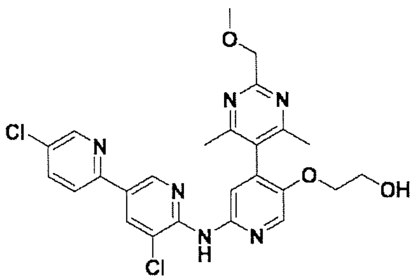 | 527 | 1.72 | 1 |

[Table 37]

| Compound No. | Structure | MS(m/z) | RT | LCMS method |
|---|---|---|---|---|
| I-236 | 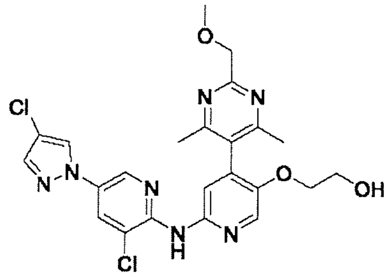 | 516 | 1.61 | 1 |
| I-237 | 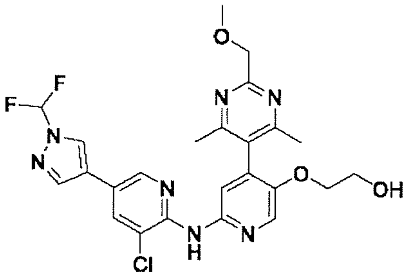 | 532 | 1.31 | 1 |
| I-238 | 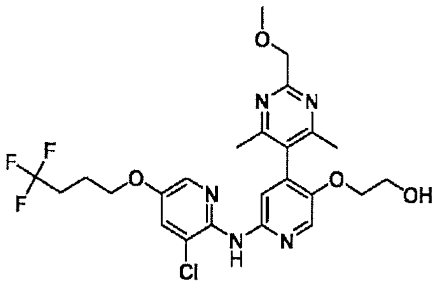 | 542 | 1.51 | 1 |
| I-239 | 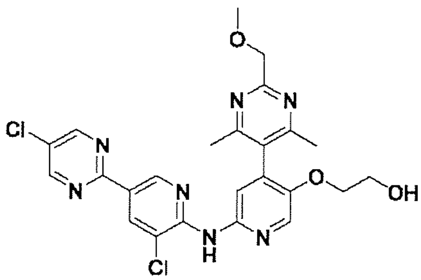 | 528 | 1.78 | 1 |
| I-240 | 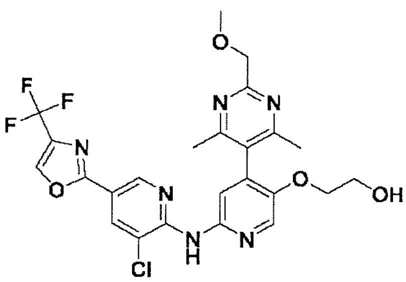 | 551 | 1.85 | 1 |

[Table 38]

| Compound No. | Structure | MS(m/z) | RT | LCMS method |
|---|---|---|---|---|
| I-241 | 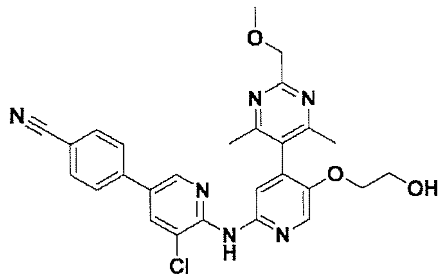 | 517 | 1.71 | 2 |
| I-242 | 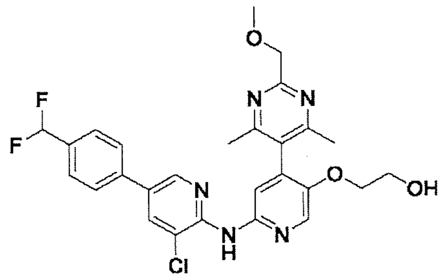 | 542 | 1.86 | 2 |
| I-243 | 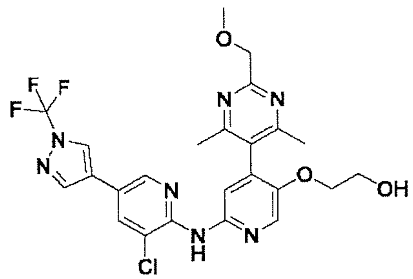 | 550 | 1.7 | 2 |
| I-244 | 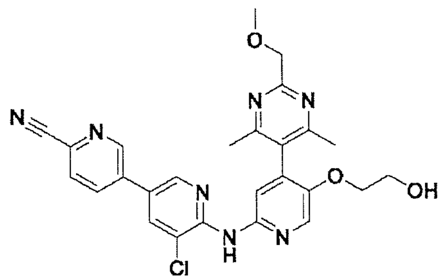 | 518 | 1.56 | 2 |
| I-245 | 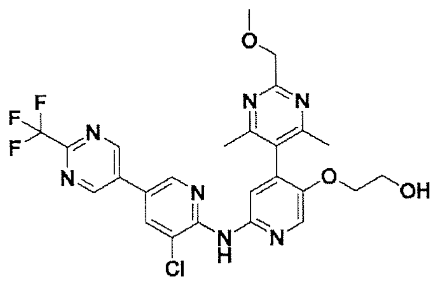 | 562 | 1.78 | 2 |

[Table 39]

| Compound No. | Structure | MS(m/z) | RT | LCMS method |
|---|---|---|---|---|
| I-246 | 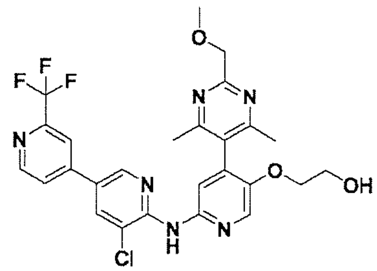 | 561 | 1.81 | 2 |
| I-247 | 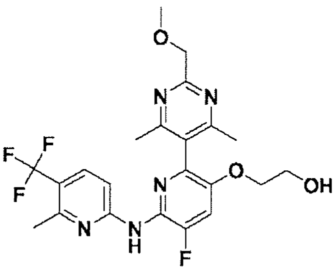 | 482 | 1.79 | 1 |
| I-248 | 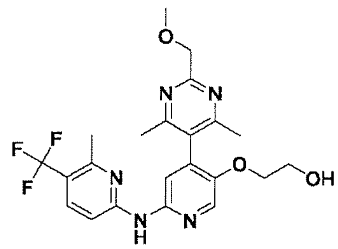 | 464 | 1.31 | 1 |
| I-249 | 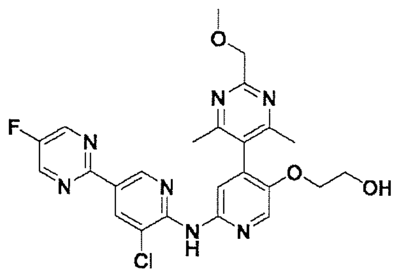 | 512 | 1.53 | 1 |
| I-250 | 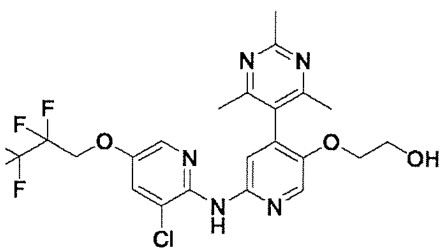 F’ | 534 | 1.6 | 1 |

(continued)

| Compound No. | Structure | MS(m/z) | RT | LCMS method |
|---|---|---|---|---|
| I-251 | 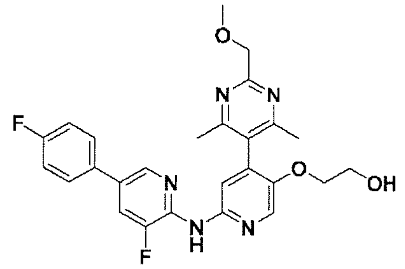 | 494 | 1.4 | 1 |

[Table 40]

| Compound No. | Structure | MS(m/z) | RT | LCMS method |
|---|---|---|---|---|
| I-252 | 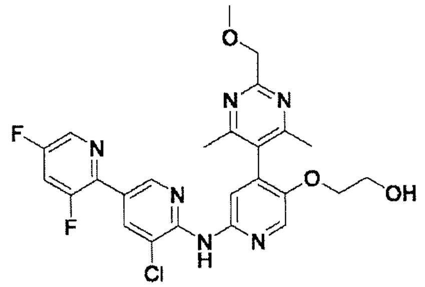 | 529 | 1.62 | 1 |
| I-253 | 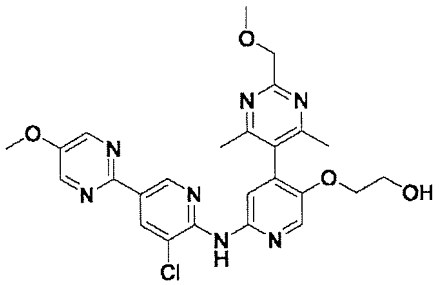 | 524 | 1.42 | 1 |
| I-254 | 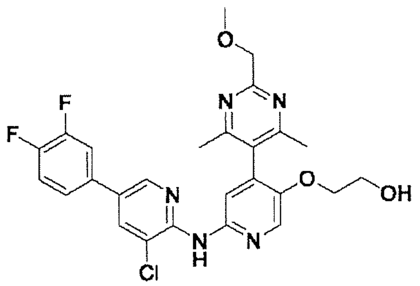 | 528 | 1.88 | 2 |
| I-255 | 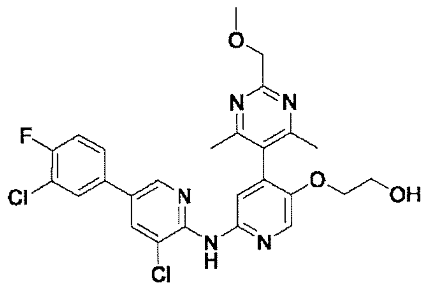 | 544 | 2 | 2 |

(continued)

| Compound No. | Structure | MS(m/z) | RT | LCMS method |
|---|---|---|---|---|
| I-256 | 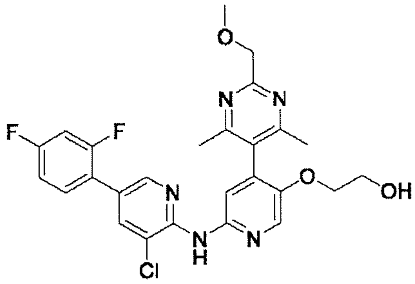 | 528 | 1.87 | 2 |

[Table 41]

| Compound No. | Structure | MS(m/z) | RT | LCMS method |
|---|---|---|---|---|
| I-257 | 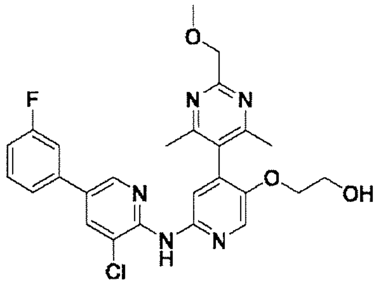 | 510 | 1.83 | 2 |
| I-258 | 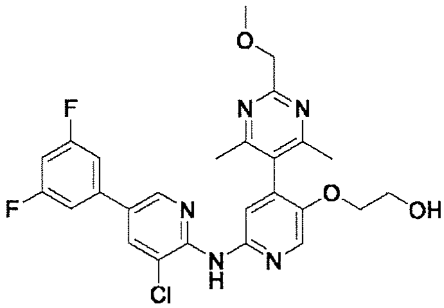 | 528 | 1.94 | 2 |
| I-259 | 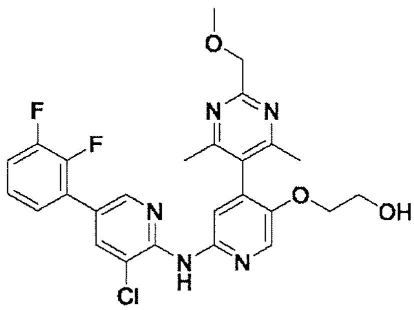 | 528 | 1.9 | 2 |
| I-260 | 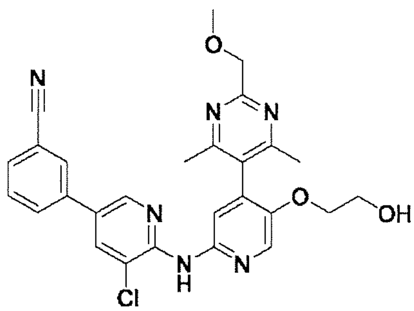 | 517 | 1.71 | 2 |

(continued)

| Compound No. | Structure | MS(m/z) | RT | LCMS method |
|---|---|---|---|---|
| I-261 | 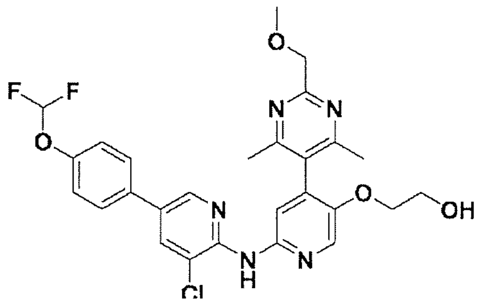 | 558 | 1.86 | 2 |

[Table 42]

| Compound No. | Structure | MS(m/z) | RT | LCMS method |
|---|---|---|---|---|
| I-262 | 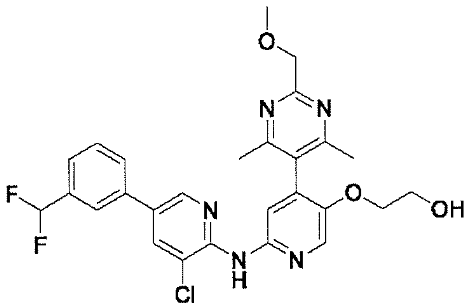 | 542 | 1.85 | 2 |
| I-263 | 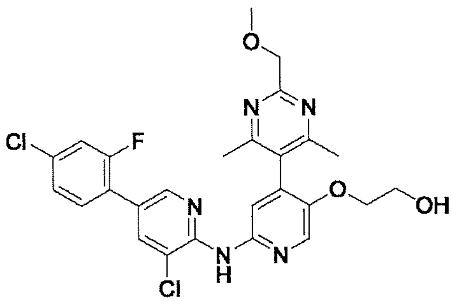 | 544 | 2.06 | 2 |
| I-264 | 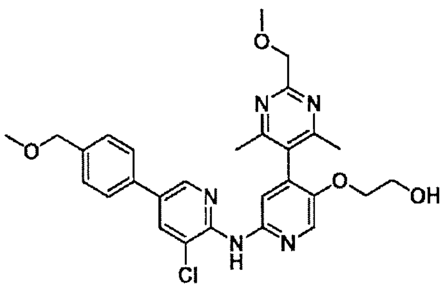 | 536 | 1.71 | 2 |
| I-265 | 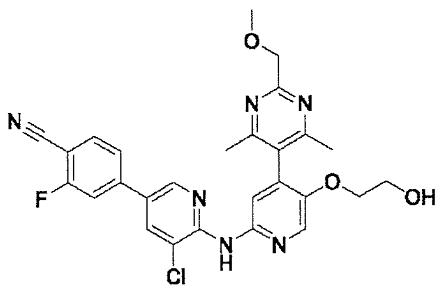 | 535 | 1.82 | 2 |

(continued)

| Compound No. | Structure | MS(m/z) | RT | LCMS method |
|---|---|---|---|---|
| I-266 | 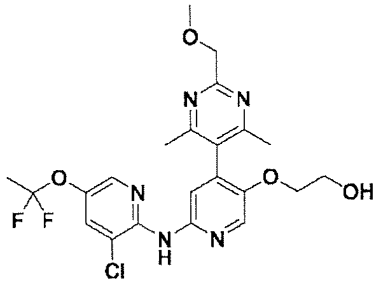 | 496 | 1.52 | 1 |

[Table 43]

| Compound No. | Structure | MS(m/z) | RT | LCMS method |
|---|---|---|---|---|
| I-267 | 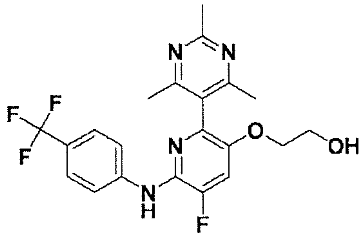 | 437 | 1.93 | 2 |
| I-268 | 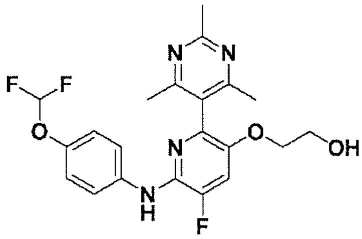 | 435 | 1.55 | 1 |
| I-269 | 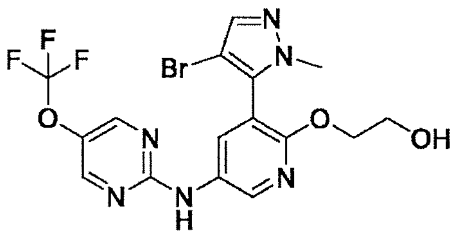 | 475 | 1.84 | 1 |
| I-270 | 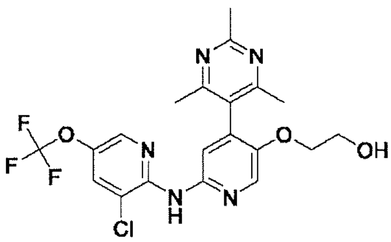 | 470 | 1.79 | 1 |
| I-271 | 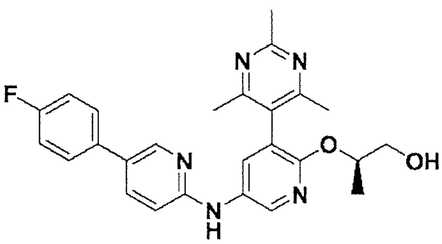 | 460 | 1.54 | 1 |

(continued)

| Compound No. | Structure | MS(m/z) | RT | LCMS method |
|---|---|---|---|---|
| I-272 | 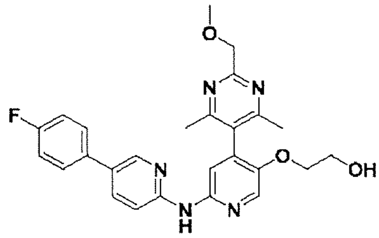 | 476 | 1.12 | 1 |

[Table 44]

| Compound No. | Structure | MS(m/z) | RT | LCMS method |
|---|---|---|---|---|
| I-273 | 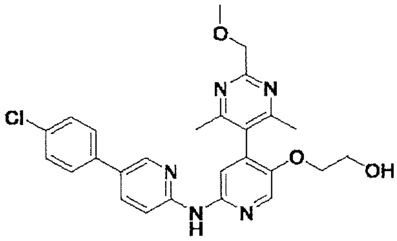 | 492 | 1.24 | 1 |
| I-274 | 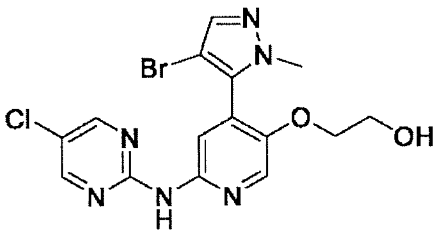 | 425 | 1.69 | 2 |
| I-275 | 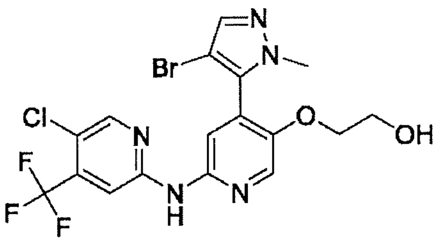 | 492 | 2.08 | 1 |
| I-276 | 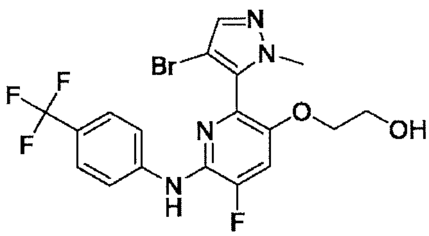 | 475 | 2.1 | 1 |
| I-277 | 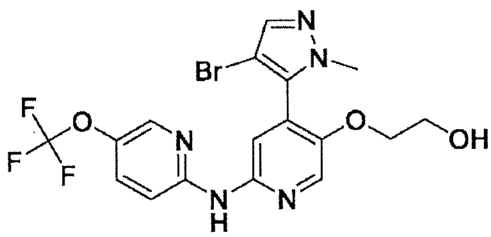 | 474 | 1.56 | 1 |

(continued)

| Compound No. | Structure | MS(m/z) | RT | LCMS method |
|---|---|---|---|---|
| I-278 | 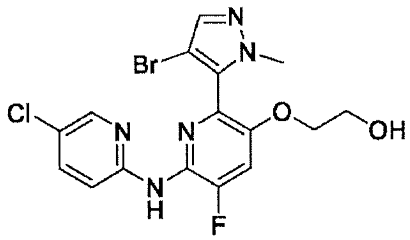 | 442 | 1.78 | 1 |

[Table 45]

| Compound No. | Structure | MS(m/z) | RT | LCMS method |
|---|---|---|---|---|
| 1-279 | 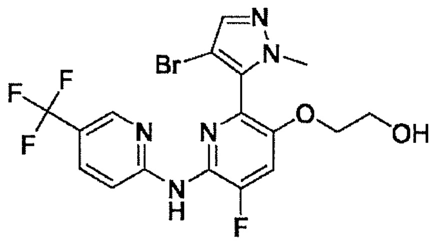 | 476 | 1.68 | 1 |
| I-280 | 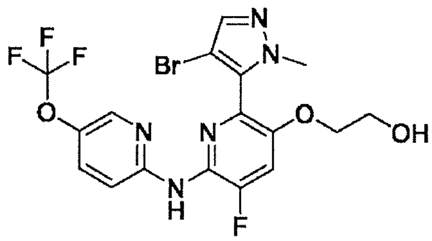 | 492 | 1.98 | 1 |
| I-281 | 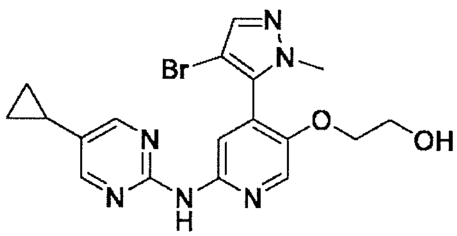 | 431 | 1.31 | 1 |
| I-282 | 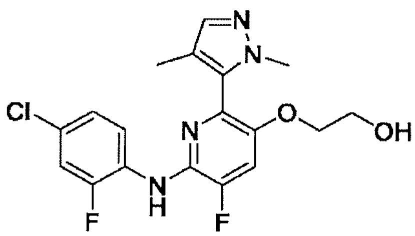 | 395 | 1.97 | 1 |
| I-283 | 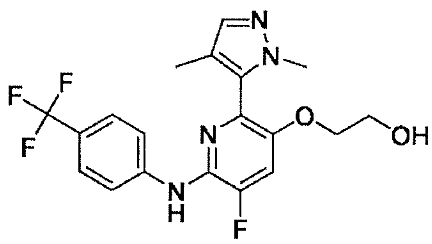 | 411 | 1.96 | 1 |

(continued)

| Compound No. | Structure | MS(m/z) | RT | LCMS method |
|---|---|---|---|---|
| I-284 | 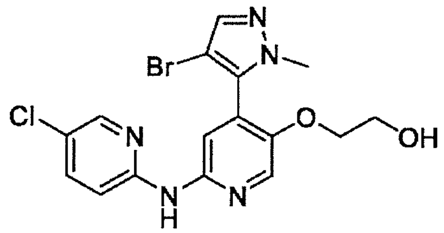 | 424 | 1.45 | 2 |

[Table 46]

| Compound No. | Structure | MS(m/z) | RT | LCMS method |
|---|---|---|---|---|
| I-285 | 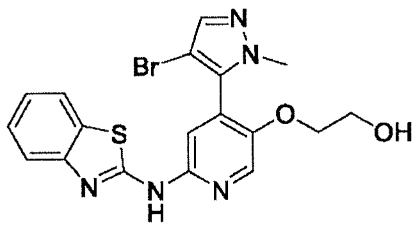 | 446 | 1.92 | 2 |
| I-286 | 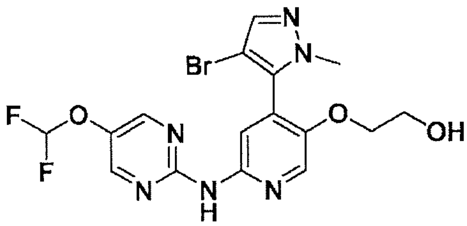 | 457 | 1.46 | 1 |
| I-287 | 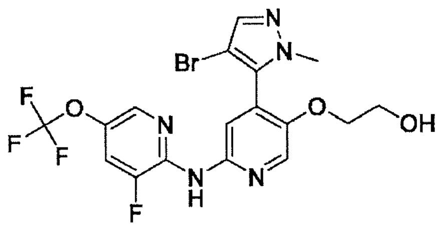 | 492 | 1.91 | 1 |
| I-288 | 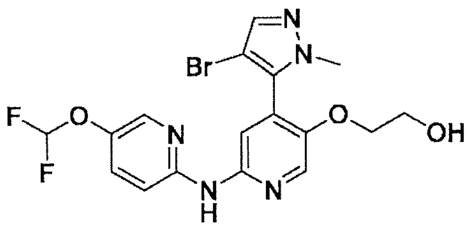 | 456 | 1.17 | 1 |
| I-289 | 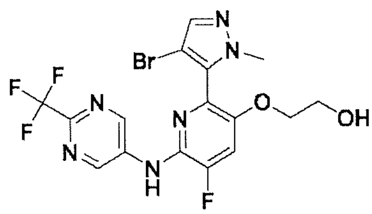 | 477 | 1.73 | 1 |

(continued)

| Compound No. | Structure | MS(m/z) | RT | LCMS method |
|---|---|---|---|---|
| I-290 | 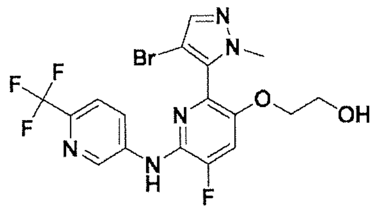 | 476 | 1.81 | 1 |
| I-291 | 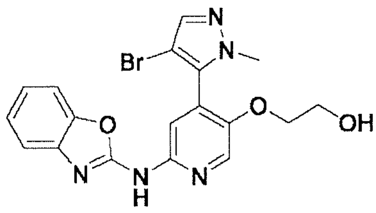 | 430 | 1.92 | 2 |

[Table 47]

| Compound No. | Structure | MS(m/z) | RT | LCMS method |
|---|---|---|---|---|
| I-292 | 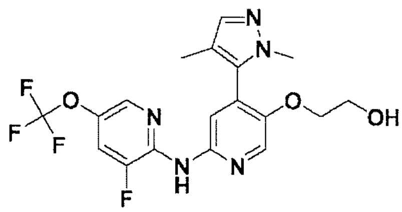 | 428 | 1.64 | 1 |
| I-293 | 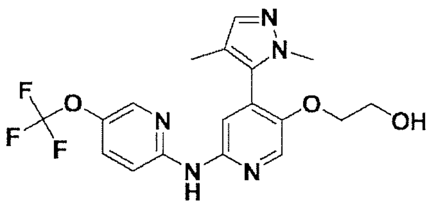 | 410 | 1.28 | 1 |
| I-294 | 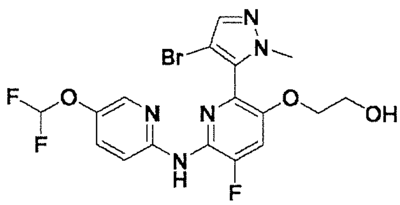 | 474 | 1.62 | 1 |
| I-295 | 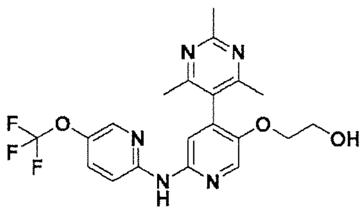 | 436 | 1.11 | 1 |
| I-296 | 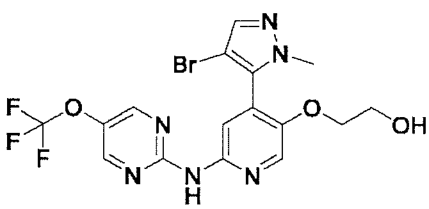 | 475 | 1.75 | 1 |

(continued)

| Compound No. | Structure | MS(m/z) | RT | LCMS method |
|---|---|---|---|---|
| I-297 | 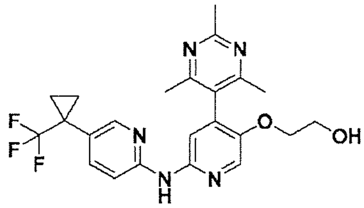 | 460 | 1.03 | 1 |
| I-298 | 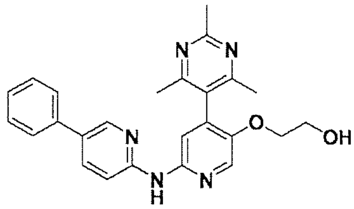 | 428 | 1.03 | 1 |

[Table 48]

| Compound No. | Structure | MS(m/z) | RT | LCMS method |
|---|---|---|---|---|
| I-299 | 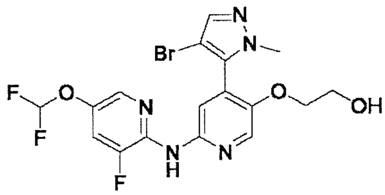 | 474 | 1.57 | 1 |
| I-300 | 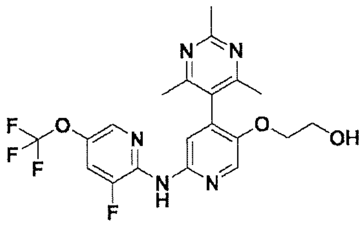 | 454 | 1.39 | 1 |
| I-301 | 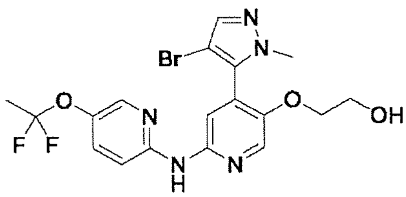 | 470 | 1.28 | 1 |
| I-302 | 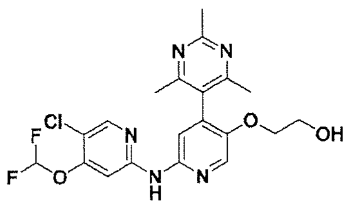 | 452 | 1.31 | 1 |
| I-303 | 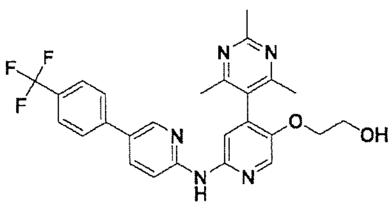 | 496 | 1.41 | 1 |

(continued)

| Compound No. | Structure | MS(m/z) | RT | LCMS method |
|---|---|---|---|---|
| I-304 | 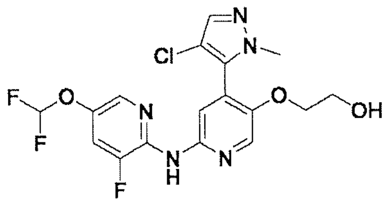 | 430 | 1.55 | 1 |
| I-305 | 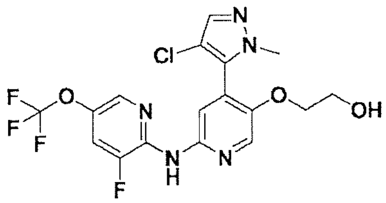 | 448 | 1.89 | 1 |

[Table 49]

| Compound No. | Structure | MS(m/z) | RT | LCMS method |
|---|---|---|---|---|
| I-306 | 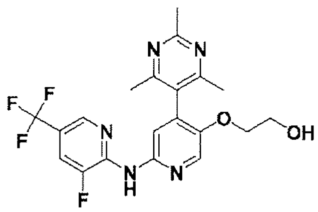 | 438 | 1.46 | 1 |
| I-307 | 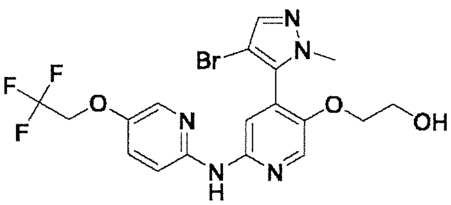 | 488 | 1.2 | 1 |
| I-308 | 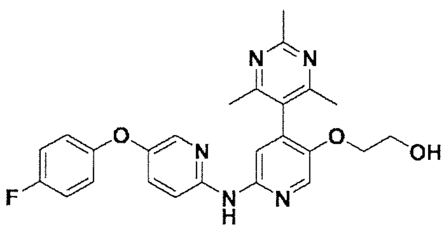 | 462 | 1.18 | 1 |
| I-309 | 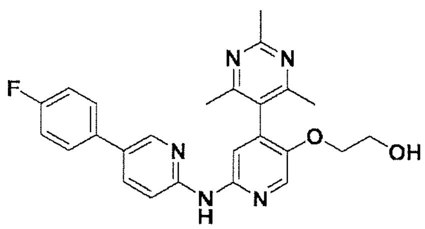 | 446 | 1.08 | 1 |

(continued)

| Compound No. | Structure | MS(m/z) | RT | LCMS method |
|---|---|---|---|---|
| I-310 | 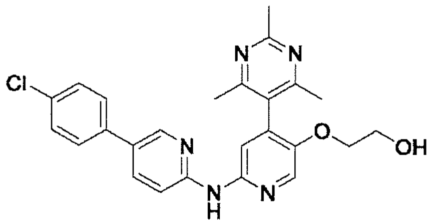 | 462 | 1.75 | 2 |
| I-311 | 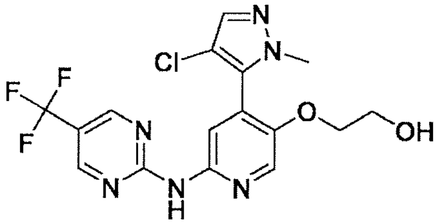 | 415 | 1.71 | 1 |
| I-312 | 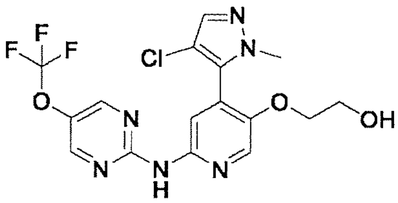 | 431 | 1.72 | 1 |

[Table 50]

| Compound No. | Structure | MS(m/z) | RT | LCMS method |
|---|---|---|---|---|
| I-313 | 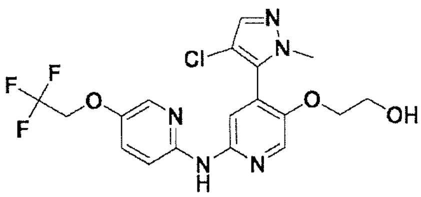 | 444 | 1.17 | 1 |
| I-314 | 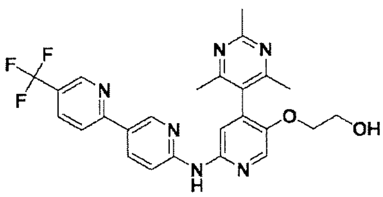 | 497 | 1.18 | 1 |
| I-315 | 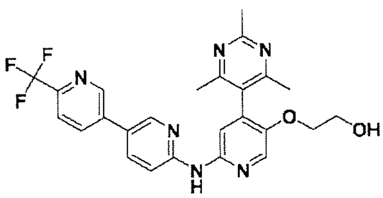 | 497 | 1.11 | 1 |
| I-316 | 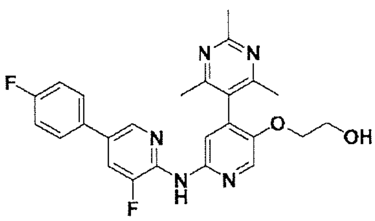 | 464 | 1.35 | 1 |

(continued)

| Compound No. | Structure | MS(m/z) | RT | LCMS method |
|---|---|---|---|---|
| I-317 | 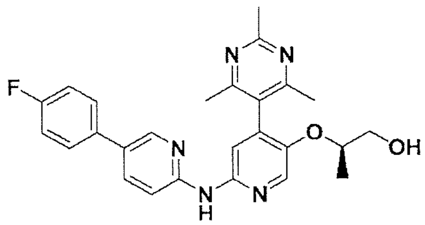 | 460 | 1.18 | 1 |
| I-318 | 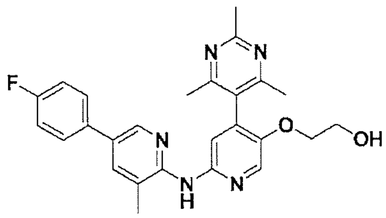 | 460 | 1.16 | 1 |
| I-319 | 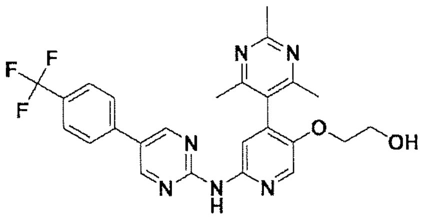 | 497 | 1.56 | 1 |

[Table 51]

| Compound No. | Structure | MS(m/z) | RT | LCMS method |
|---|---|---|---|---|
| I-320 | 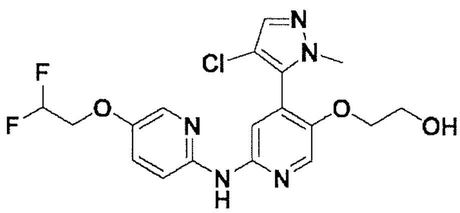 | 426 | 1.03 | 1 |
| I-321 | 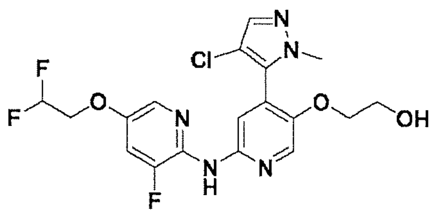 | 444 | 1.35 | 1 |
| I-322 | 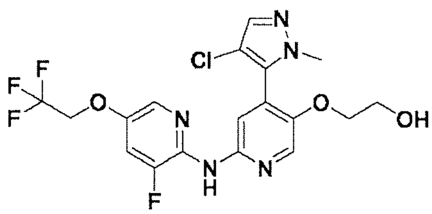 | 462 | 1.57 | 1 |

(continued)

| Compound No. | Structure | MS(m/z) | RT | LCMS method |
|---|---|---|---|---|
| I-323 | 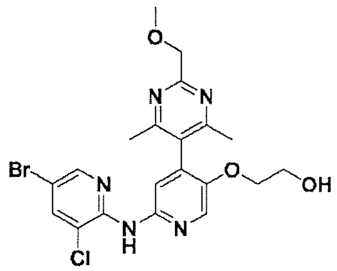 | 494 | 1.56 | 1 |
| I-324 | 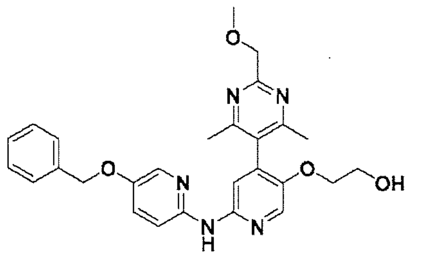 | 488 | 1.21 | 1 |
| I-325 | 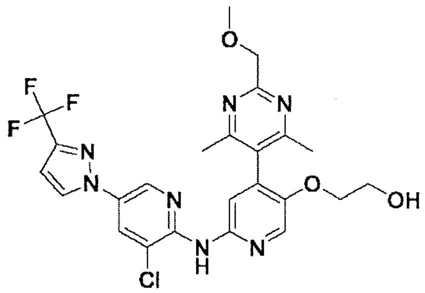 | 550 | 1.79 | 1 |

[Table 52]

| Compound No. | Structure | MS(m/z) | RT | LCMS method |
|---|---|---|---|---|
| I-326 | 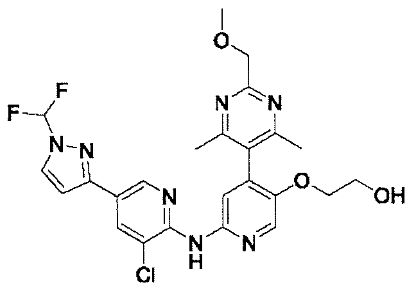 | 532 | 1.43 | 1 |
| I-327 | 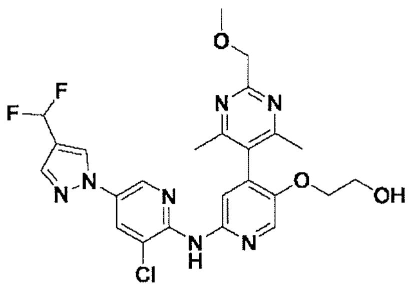 | 532 | 1.49 | 1 |

(continued)

| Compound No. | Structure | MS(m/z) | RT | LCMS method |
|---|---|---|---|---|
| I-328 | 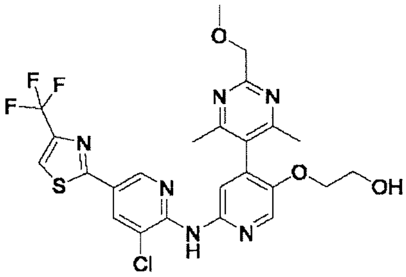 | 567 | 1.93 | 1 |
| I-329 | 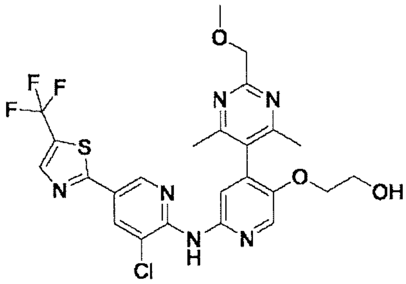 | 567 | 2.08 | 1 |
| I-330 | 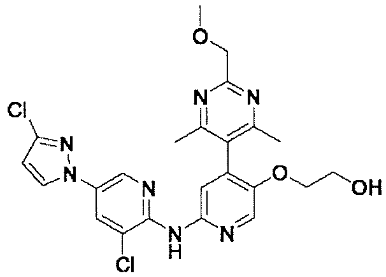 | 516 | 1.58 | 1 |

[Table 53]

| Compound No. | Structure | MS(m/z) | RT | LCMS method |
|---|---|---|---|---|
| I-331 | 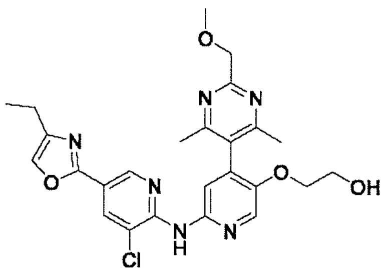 | 511 | 1.68 | 1 |
| I-332 | 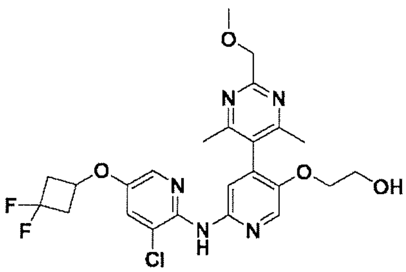 | 522 | 1.4 | 1 |

(continued)

| Compound No. | Structure | MS(m/z) | RT | LCMS method |
|---|---|---|---|---|
| I-333 | 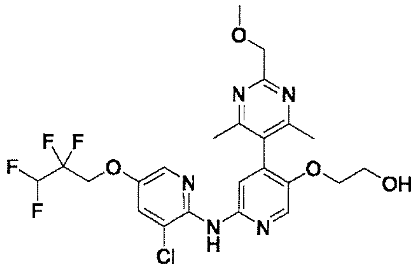 | 546 | 1.43 | 1 |
| I-334 | 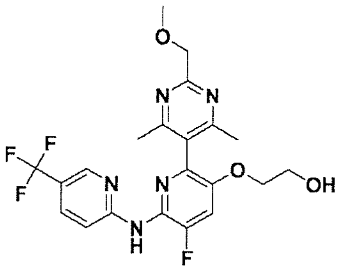 | 468 | 1.62 | 1 |
| I-335 | 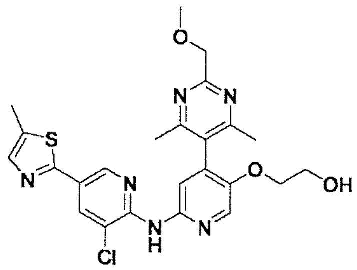 | 513 | 1.86 | 1 |
| I-336 | 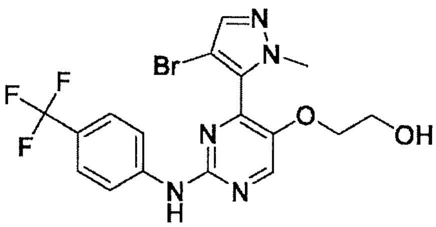 | 458 | 2.02 | 1 |

[Table 54]

| Compound No. | Structure | MS(m/z) | RT | LCMS method |
|---|---|---|---|---|
| I-337 | 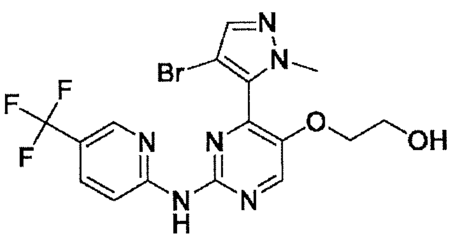 | 459 | 1.83 | 1 |

(continued)

| Compound No. | Structure | MS(m/z) | RT | LCMS method |
|---|---|---|---|---|
| I-338 | 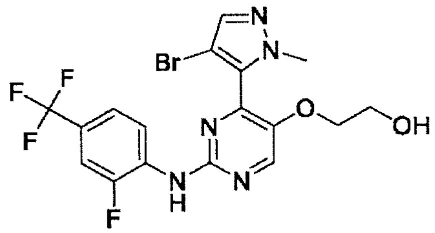 | 476 | 2.1 | 1 |
| I-339 | 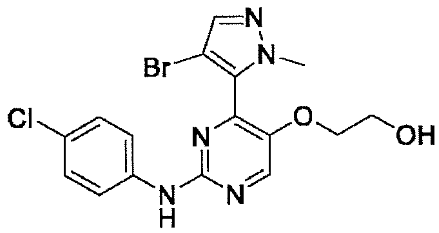 | 424 | 1.91 | 1 |
| I-340 | 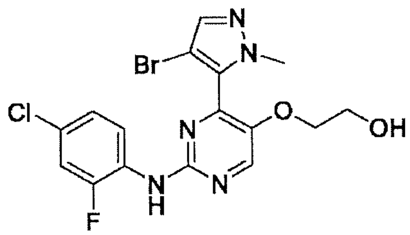 | 442 | 1.97 | 1 |
| I-341 | 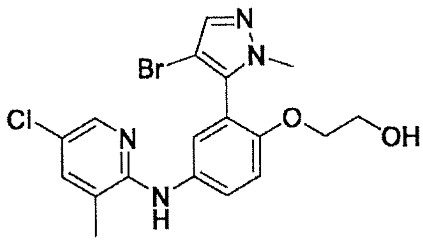 | 437 | 1.9 | 1 |
| I-342 | 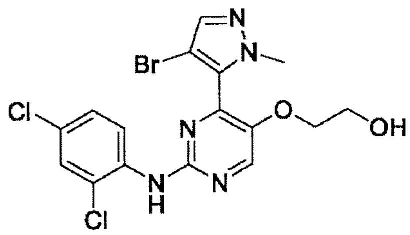 | 458 | 2.2 | 1 |

[Table 55]

| Compound No. | Structure | MS(m/z) | RT | LCMS method |
|---|---|---|---|---|
| I-343 | 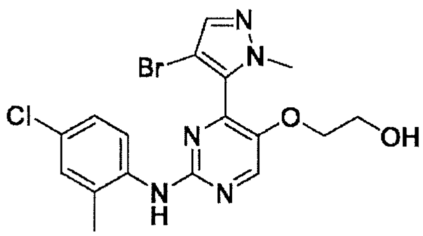 | 438 | 1.95 | 1 |

(continued)

| Compound No. | Structure | MS(m/z) | RT | LCMS method |
|---|---|---|---|---|
| I-344 | 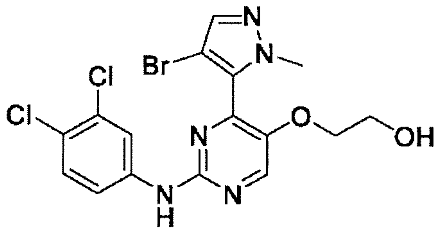 | 458 | 2.09 | 1 |
| I-345 | 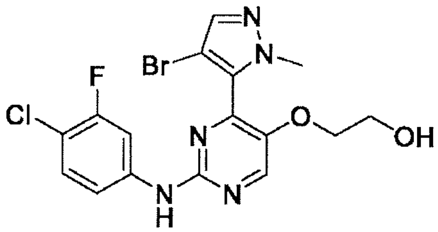 | 442 | 1.97 | 1 |
| I-346 | 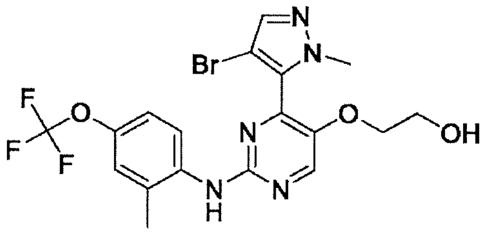 | 488 | 2.08 | 1 |
| I-347 | 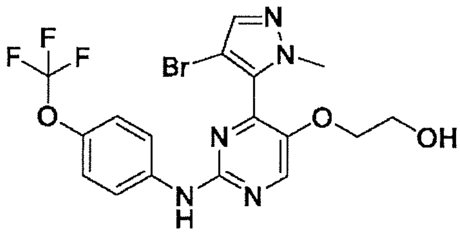 | 474 | 2.04 | 1 |
| I-348 | 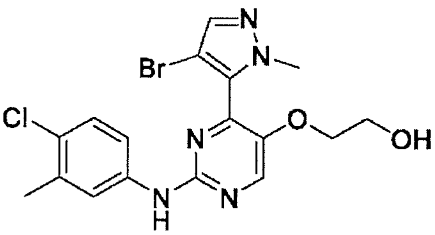 | 438 | 2.04 | 1 |

[Table 56]

| Compound No. | Structure | MS(m/z) | RT | LCMS method |
|---|---|---|---|---|
| I-349 | 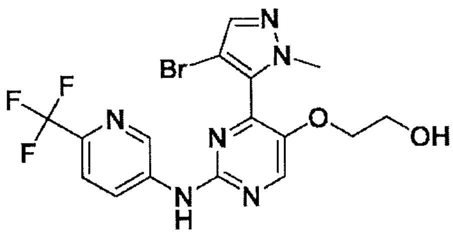 | 459 | 1.73 | 1 |

(continued)

| Compound No. | Structure | MS(m/z) | RT | LCMS method |
|---|---|---|---|---|
| I-350 | 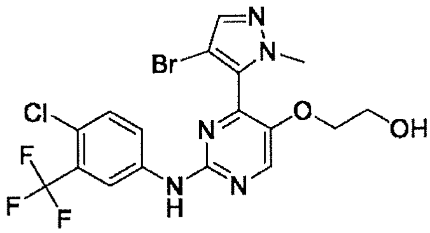 | 492 | 2.16 | 1 |
| I-351 | 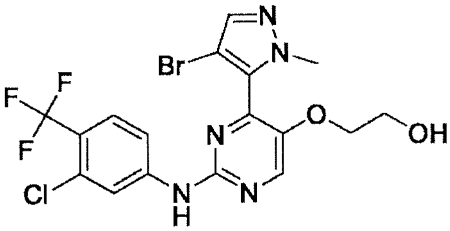 | 492 | 2.16 | 1 |
| I-352 | 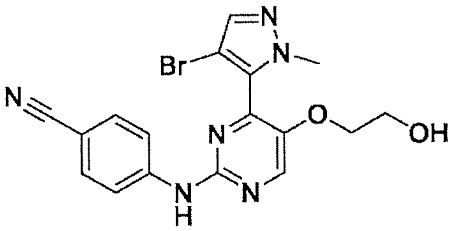 | 415 | 1.59 | 1 |
| I-353 | 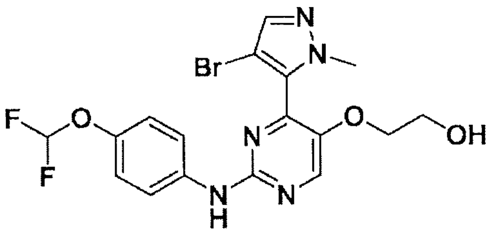 | 456 | 1.79 | 1 |
| I-354 | 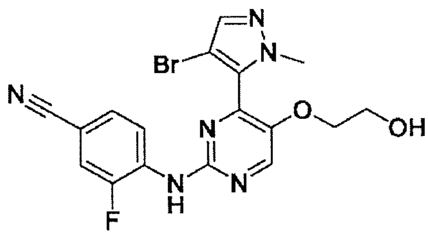 | 433 | 1.68 | 1 |

[Table 57]

| Compound No. | Structure | MS(m/z) | RT | LCMS method |
|---|---|---|---|---|
| I-355 | 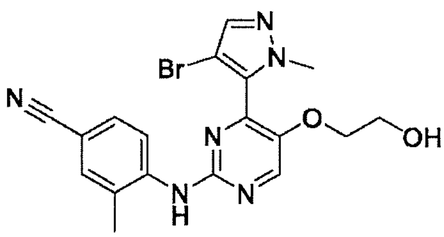 | 429 | 1.68 | 1 |

(continued)

| Compound No. | Structure | MS(m/z) | RT | LCMS method |
|---|---|---|---|---|
| I-356 | 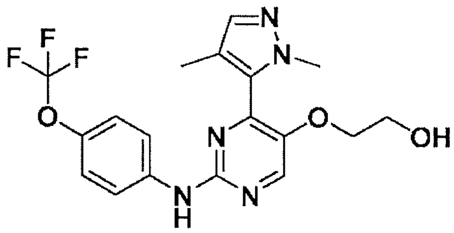 | 410 | 1.92 | 1 |
| I-357 | 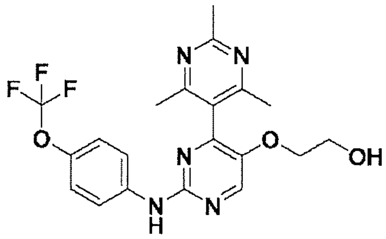 | 436 | 1.77 | 1 |
| I-358 | 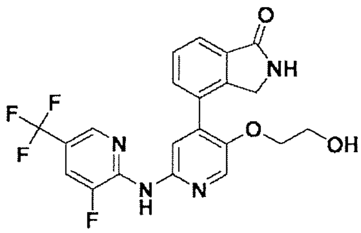 | 449 | 1.44 | 1 |
| I-359 | 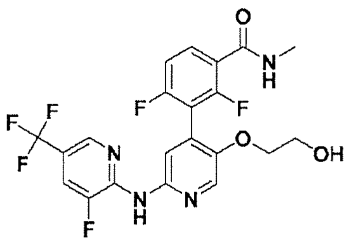 | 487 | 1.7 | 1 |
| I-360 | 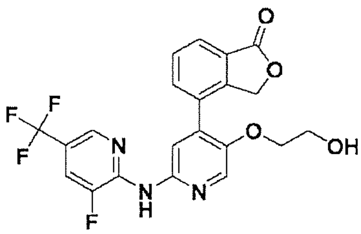 | 450 | 1.77 | 1 |

[Table 58]

| Compound No. | Structure | MS(m/z) | RT | LCMS method |
|---|---|---|---|---|
| I-361 | 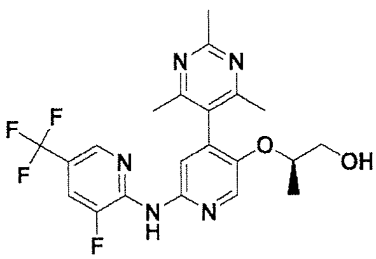 | 452 | 1.56 | 1 |

(continued)

| Compound No. | Structure | MS(m/z) | RT | LCMS method |
|---|---|---|---|---|
| I-362 | 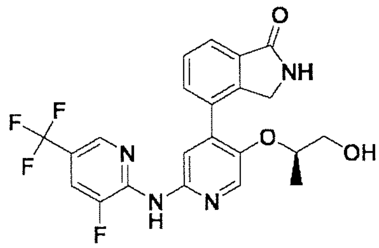 | 463 | 1.52 | 1 |
| I-363 | 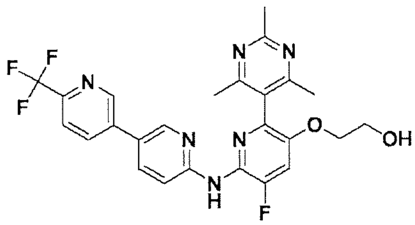 | 515 | 1.37 | 1 |
| I-364 | 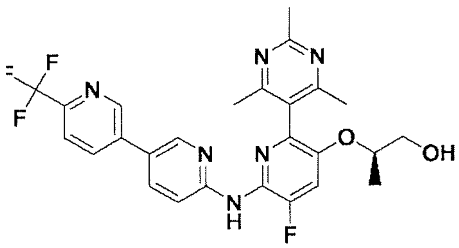 | 529 | 1.46 | 1 |
| I-365 | 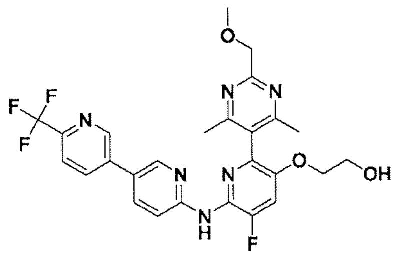 | 545 | 1.42 | 1 |
| I-366 | 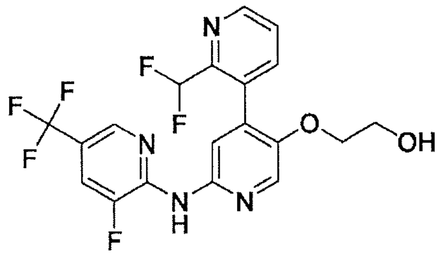 | 445 | 1.71 | 1 |

[Table 59]

| Compound No. | Structure | MS(m/z) | RT | LCMS method |
|---|---|---|---|---|
| I-367 | 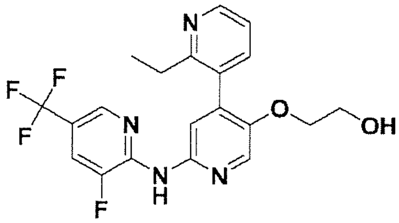 | 423 | 1.26 | 1 |

(continued)

| Compound No. | Structure | MS(m/z) | RT | LCMS method |
|---|---|---|---|---|
| I-368 | 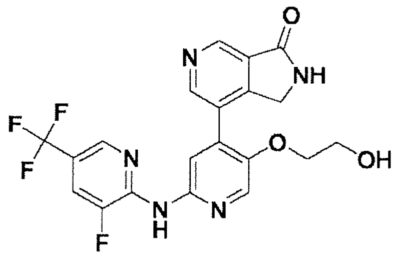 | 450 | 1.28 | 1 |
| I-369 | 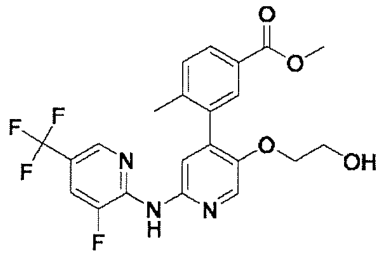 | 466 | 1.98 | 1 |
| I-370 | 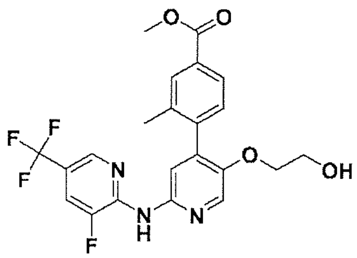 | 466 | 2.16 | 2 |
| I-371 | 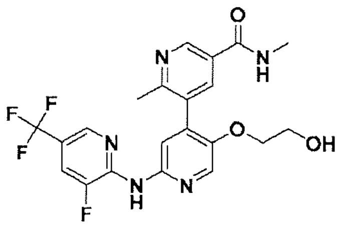 | 466 | 1.48 | 2 |
| I-372 | 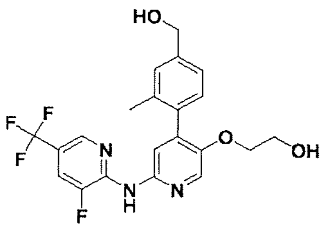 | 438 | 1.44 | 1 |

[Table 60]

| Compound No. | Structure | MS(m/z) | RT | LCMS method |
|---|---|---|---|---|
| I-373 | 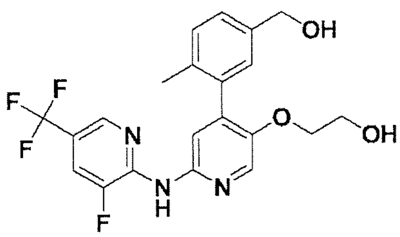 | 438 | 1.48 | 1 |

(continued)

| Compound No. | Structure | MS(m/z) | RT | LCMS method |
|---|---|---|---|---|
| I-374 | 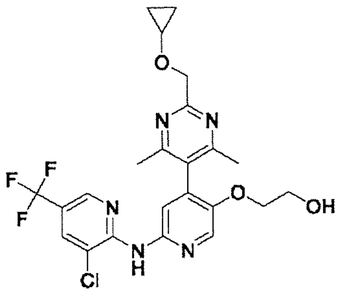 | 510 | 2.01 | 1 |
| I-375 | 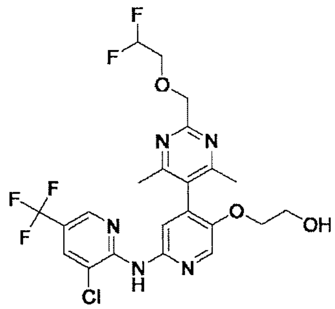 | 534 | 2.01 | 1 |
| I-376 | 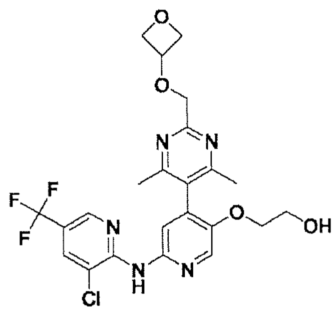 | 526 | 1.73 | 1 |
| I-377 | 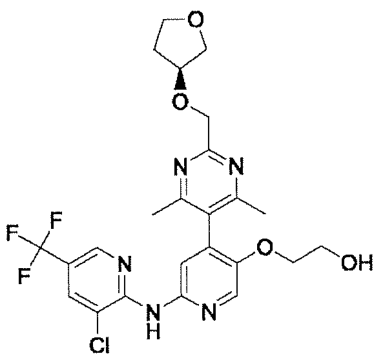 | 540 | 1.78 | 1 |

[Table 61]

| Compound No. | Structure | MS(m/z) | RT | LCMS method |
|---|---|---|---|---|
| I-378 | 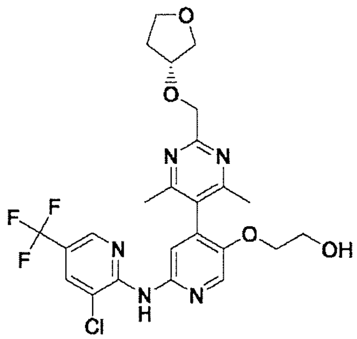 | 540 | 1.78 | 1 |
| I-379 | 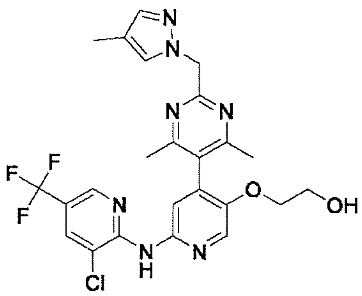 | 534 | 2.01 | 1 |
| I-380 | 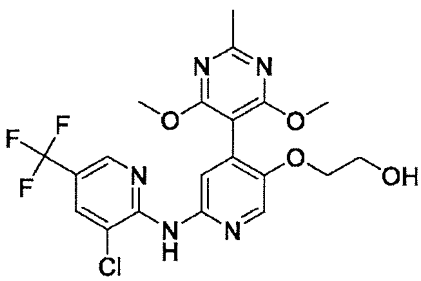 | 486 | 2.15 | 1 |
| I-381 | 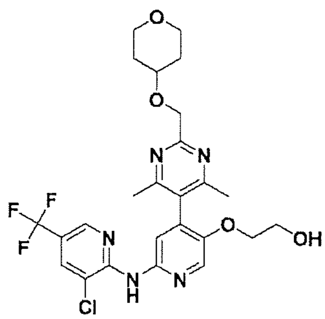 | 554 | 1.8 | 1 |
| I-382 | 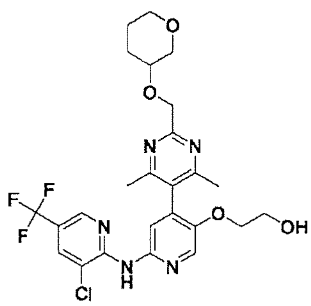 | 554 | 1.91 | 1 |

[Table 62]

| Compound No. | Structure | MS(m/z) | RT | LCMS method |
|---|---|---|---|---|
| I-383 | 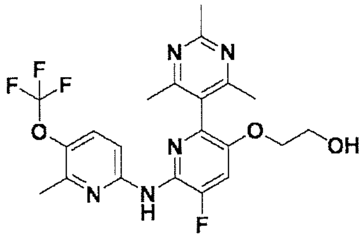 | 468.1 | 1.95 | 1 |
| I-384 | 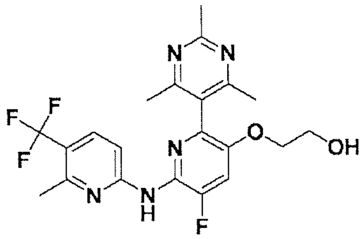 | 452 | 1.95 | 1 |
| I-385 | 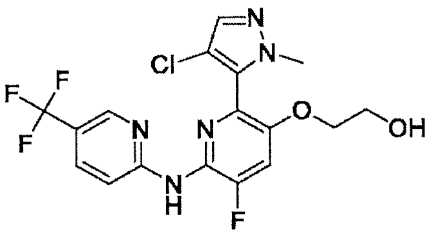 | 432 | 2.05 | 2 |
| I-386 | 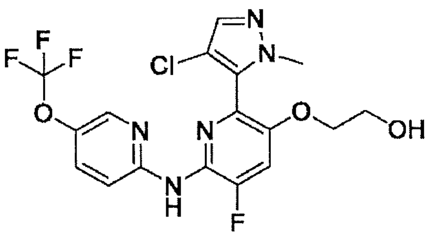 | 448 | 2.11 | 2 |
| I-387 | 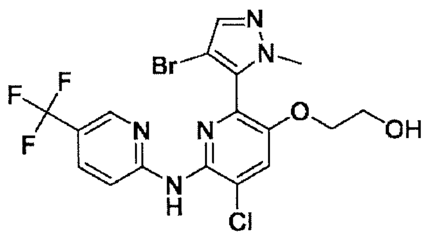 | 492 | 2.3 | 2 |
| I-388 | 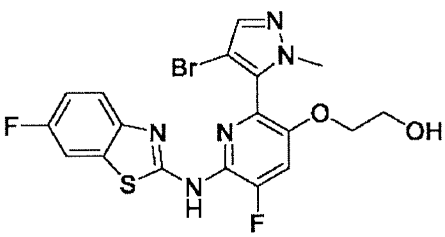 | 482 | 2.01 | 2 |

[Table 63]

| Compound No. | Structure | MS(m/z) | RT | LCMS method |
|---|---|---|---|---|
| I-389 | 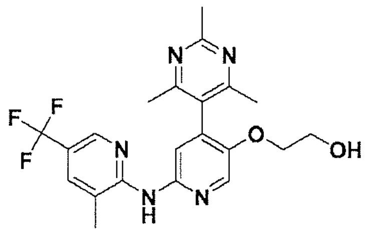 | 434.4 | 1.16 | 1 |
| I-390 | 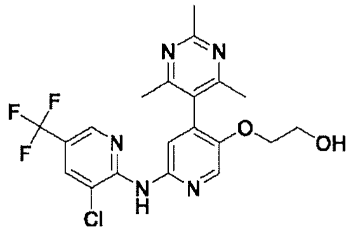 | 454.1 | 1.8 | 2 |
| I-391 | 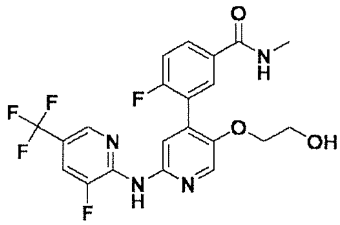 | 469 | 1.71 | 2 |
| I-392 | 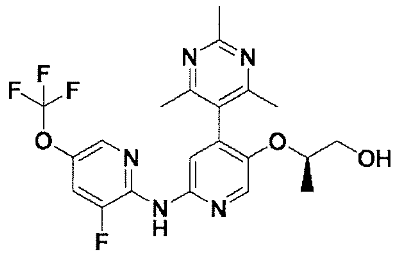 | 468 | 1.59 | 2 |
| I-393 | 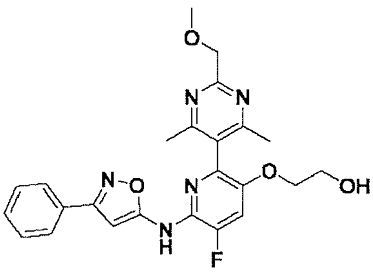 | 466 | 1.79 | 2 |
| I-394 | 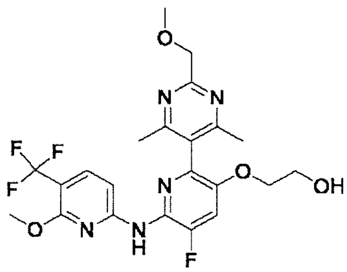 | 498 | 2.22 | 4 |

[Table 64]

| Compound No. | Structure | MS(m/z) | RT | LCMS method |
|---|---|---|---|---|
| I-395 | 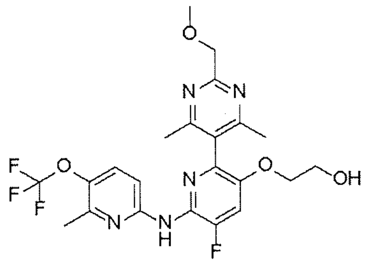 | 498 | 2.09 | 4 |
| I-396 | 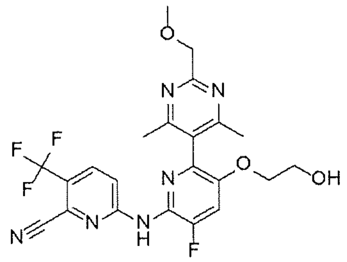 | 493 | 2.04 | 4 |
| I-397 | 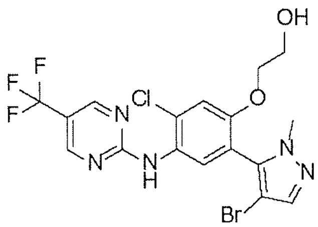 | 492 | 2.14 | 1 |
| I-398 | 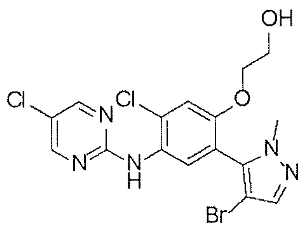 | 458 | 2.08 | 2 |
| I-399 | 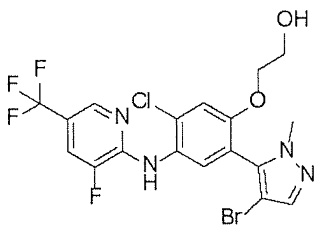 | 509 | 2.22 | 1 |
| I-400 | 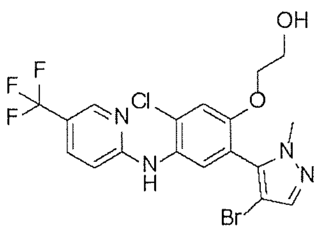 | 491 | 2.22 | 1 |

[Table 65]

| Compound No. | Structure | MS(m/z) | RT | LCMS method |
|---|---|---|---|---|
| I-401 | 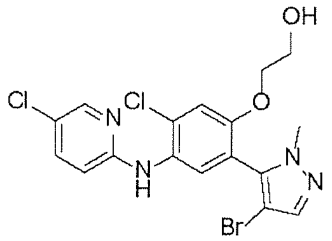 | 457 | 2.17 | 2 |
| I-402 | 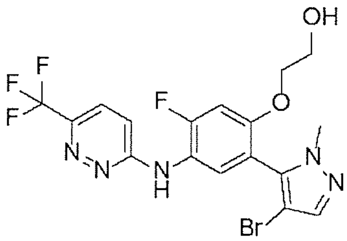 | 476 | 1.68 | 1 |
| I-403 | 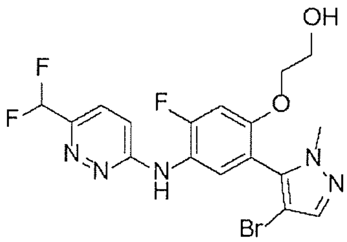 | 458 | 1.52 | 1 |
| I-404 | 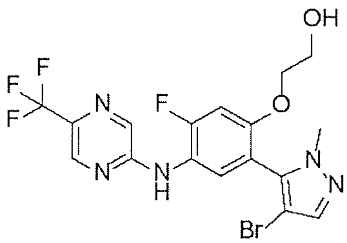 | 476 | 1.85 | 1 |
| I-405 | 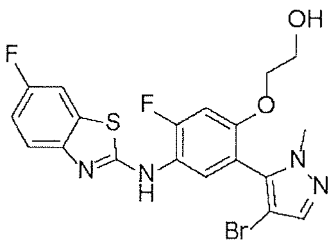 | 481 | 2.15 | 2 |
| I-406 | 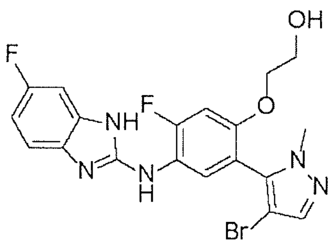 | 464 | 1.59 | 2 |

[Table 66]

| Compound No. | Structure | MS(m/z) | RT | LCMS method |
|---|---|---|---|---|
| I-407 | 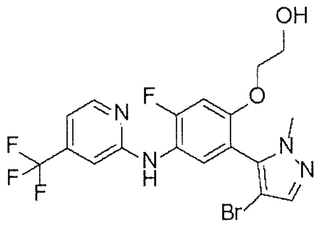 | 475 | 1.96 | 1 |
| I-408 | 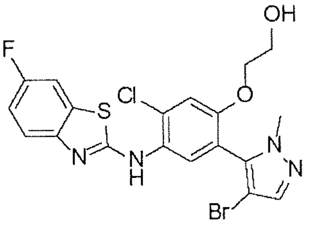 | 497 | 2.21 | 2 |
| I-409 | 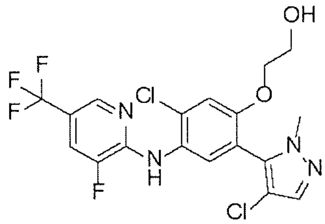 | 465 | 2.38 | 2 |
| I-410 | 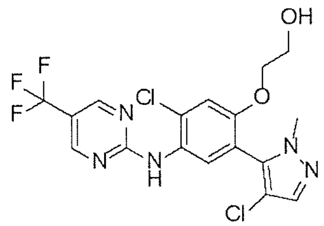 | 448 | 2.13 | 2 |

[Table 67]

| Compound No. | NMR |
|---|---|
| I-001 | $^1$H-NMR ($CDCl_3$) δ : 1.85 (t, J = 6.3Hz, 1H), 3.83-3.88 (m, 2H), 3.77 (s, 3H), 4.02-4.14 (m, 2H), 5.84 (s, 1H), 6.97 (d, J = 8.5Hz, 2H), 7.04 (d, J = 8.8Hz, 1H), 7.11 (d, J = 2.5Hz, 1H), 7.24-7.28 (m, 1H), 7.16 (d, J = 8.6Hz, 2H), 7.53 (s, 1H). |
| I-007 | $^1$H-NMR ($CDCl_3$) δ : 1.81 (t, J = 6.3Hz, 1H), 3.78 (s, 3H), 3.86-3.91 (m, 2H), 4.08-4.17 (m, 2H), 7.06 (d, J = 9.0Hz, 1H), 7.10 (d, J = 8.8Hz, 1H), 7.18 (br s, 1H), 7.30 (d, J = 2.5Hz, 1H), 7.51 (dd,J = 9.1, 2.6Hz, 1H), 7.52-7.55 (m, 1H), 7.55 (s, 1H). |
| I-008 | $^1$H-NMR ($CDCl_3$) δ : 1.75 (t, J = 6.0Hz, 1H), 1.99 (s, 3H), 3.73 (s, 3H), 3.79-3.84 (m, 2H), 3.99-4.14 (m, 2H), 6.77-6.82 (m, 1H), 7.05 (d, J = 8.8Hz, 1H), 7.39 (s, 1H), 7.46 (dd, J = 10.6, 1.9Hz, 1H), 7.50 (d, J = 2.8Hz, 1H), 7.69 (dd,J = 8.8, 2.8Hz, 1H), 8.25 (s, 1H). |
| I-009 | $^1$H-NMR ($CDCl_3$) δ : 1.76-1.82 (m, 1H), 3.81 (s, 3H), 3.81-3.88 (m, 2H), 4.07-4.10 (m, 2H), 6.30 (d, J = 2.0Hz, 1H), 6.79 (br d, J = 2.8Hz 1H), 7.04 (d, J = 9.0Hz, 1H), 7.46 (dd, J = 10.8, 1.8Hz, 1H), 7.54 (d, J = 2.0Hz, 1H), 7.59 (d, J = 2.8Hz, 1H), 7.68 (dd,J = 9.0, 2.8Hz, 1H), 8.25 (s, 1H). |
| I-010 | $^1$H-NMR ($CDCl_3$) δ: 1.76 (1H, t, *J* = 6.1 Hz), 2.23 (3H, s), 2.36 (3H, s), 3.86-3.91 (2H, m), 4.07 (2H, t, *J*= 4.5 Hz), 6.78 (1H, s), 7.01 (1H, d, *J* = 8.8 Hz), 7.46 (1H, d, *J* = 6.8 Hz), 7.47 (1H, s), 7.58 (1H, dd, *J* = 8.8, 2.6 Hz), 8.24 (1H, s). |

(continued)

| Compound No. | NMR |
|---|---|
| I-015 | $^1$H-NMR ($CDCl_3$) δ: 1.87 (1H, t, *J* = 6.3 Hz), 3.78 (3H, s), 3.84-3.89 (2H, m), 4.05-4.15 (2H, m), 6.82 (1H, d, J = 2.5 Hz), 7.07 (1H, d, *J* = 8.9 Hz), 7.47 (1H, dd, *J* = 10.8, 1.9 Hz), 7.52 (1H, s), 7.57 (1H, d, *J* = 2.8 Hz), 7.75 (1H, dd, *J* = 8.9, 2.8 Hz), 8.26 (1H, s). |
| I-027 | $^1$H-NMR ($CDCl_3$) δ: 3.15 (1H, brs), 3.57 (3H, s), 3.89 (2H, brs), 4.10 (2H, t, *J* = 4.1 Hz), 6.83 (1H, d, *J* = 2.8 Hz), 7.05 (1H, d, *J* = 8.9 Hz), 7.34 (1H, s), 7.46 (1H, dd, J = 10.8, 1.5 Hz), 7.56 (1H, d, *J* = 2.8 Hz), 7.75 (1H, dd, *J* = 8.9, 2.8 Hz), 8.25 (1H, s). |
| I-030 | $^1$H-NMR ($CDCl_3$) δ: 2.07 (6H, s), 3.72-3.77 (2H, m), 4.03 (2H, t, *J* = 4.6 Hz), 6.79 (1H, d, *J* = 3.8 Hz), 7.05 (1H, d, *J* = 8.8 Hz), 7.31 (1H, d, *J* = 2.8 Hz), 7.45 (1H, d, *J* = 10.5 Hz), 7.69 (1H, dd, J = 8.8, 2.8 Hz), 8.25 (1H, s), 8.36 (2H, s). |
| I-033 | $^1$H-NMR ($CDCl_3$) δ : 2.11 (t, J = 6.0Hz, 1H), 2.29 (s, 3H), 3.85-3.90 (m, 2H), 3.93 (s, 3H), 4.05-4.13 (m, 2H), 6.87 (br d, J = 2.8Hz, 1H), 7.07 (d, J = 9.0Hz, 1H), 7.47 (dd, J = 10.9, 2.0Hz, 1H), 7.58 (d, J = 2.8Hz, 1H), 7.68 (dd,J = 9.0, 2.8Hz, 1H), 8.24 (s, 1H). |
| I-034 | $^1$H-NMR ($CDCl_3$) δ: 2.08 (6H, s), 3.62-3.71 (2H, m), 3.99 (2H, t, *J* = 4.5 Hz), 6.77 (1H, brs), 7.04 (1H, d, *J* = 8.8 Hz), 7.10-7.20 (3H, m), 7.30 (1H, *d, J* = 2.8 Hz), 7.43 (1H, dd, *J* = 11.0, 1.8 Hz), 7.70 (1H, dd, *J* = 8.8, 2.8 Hz), 8.24 (1H, s). |
| I-039 | 1H-NMR (DMSO-D6) δ: 3.59-3.67 (2H, m), 3.70 (3H, s), 3.99-4.04 (2H, m), 4.82 (1H, t, J = 5.2 Hz), 7.20 (1H, d, J = 9.1 Hz), 7.25 (1H, t, J = 7.6 Hz), 7.38 (2H, t, J = 7.6 Hz), 7.51 (2H, d, J = 7.6 Hz), 7.60 (1H, d, J = 2.8 Hz), 7.62 (1H, s), 7.66 (1H, s), 7.73 (1H, dd, J = 9.1. 2.8 Hz). 10.32 (1H, s). |
| I-043 | 1H-NMR (CDCl3) δ: 1.93 (1H, t, J = 6.2 Hz), 3.78 (3H, s), 3.82-3.88 (2H, m), 4.02-4.12 (2H, m), 6.57 (1H, s), 7.04 (1H, d, J = 8.9 Hz), 7.44 (1H, dd, J = 10.2, 1.9 Hz), 7.51 (1H, d, J = 2.8 Hz), 7.54 (1H, s), 7.71 (1H, dd, J = 8.9, 2.8 Hz), 8.04 (1H, d, J = 1.9 Hz). |
| I-048 | 1H-NMR (CDCl3) δ: 0.60-0.62 (2H, m), 0.90-0.93 (2H, m), 1.78-1.85 (1H, m), 3.77 (3H, s), 3.83 (2H, t, J = 4.5 Hz), 4.01-4.11 (2H, m), 6.41 (1H, s), 6.72 (1H, d, J = 8.7 Hz), 7.01 (1H, d, J = 8.7 Hz), 7.19 (1H, dd, J = 8.7, 2.2 Hz), 7.23 (1H, d, J = 2.8 Hz), 7.42 (1H, dd, J = 8.7, 2.8 Hz), 7.53 (1H, s). 8.02 (1H, d. J = 2.2 Hz). |

[Table 68]

| Compound No. | NMR |
|---|---|
| I-052 | $^1$H-NMR ($CDCl_3$) δ: 1.70 (1H, t, *J* = 6.3 Hz), 3.74 (3H, s), 3.81-3.85 (2H, m), 4.03-4.14 (2H, m), 6.81 (1H, d, *J* = 3.1 Hz), 7.06 (1H, d, *J* = 9.0 Hz), 7.47 (1H, dd, *J* = 10.8, 1.9 Hz), 7.58 (1H, d, *J* = 2.6 Hz), 7.77 (1H, s), 7.78 (1H, dd, *J* = 8.9, 2.6 Hz), 8.25 (1H, s). |
| I-061 | $^1$H-NMR ($CDCl_3$) δ: 1.82 (1H, brs), 3.76 (3H, s), 3.80-3.86 (2H, m), 4.00-4.11 (2H, m), 5.63 (1H, s), 6.96 (2H, d, *J* = 8.8 Hz), 6.99-7.03 (2H, m), 7.10 (2H, d, *J* = 8.8 Hz), 7.19 (1H, dd, *J* = 8.8, 2.8 Hz), 7.53 (1H, s). |
| I-067 | $^1$H-NMR ($CDCl_3$) δ: 0.77-0.89 (2H, m), 0.96-1.04 (1H, m), 1.16-1.23 (1H, m), 1.90 (1H, t, *J* = 6.5 Hz), 3.48-3.55 (1H, m), 3.84-3.88 (2H, m), 4.09-4.12 (2H, m), 6.82 (1H, d, *J* = 3.4 Hz), 7.07 (1H, d, *J* = 8.9 Hz), 7.47 (1H, d, *J* = 10.8 Hz), 7.50 (1H, s), 7.61 (1H, d, J = 2.8 Hz), 7.76 (1H, dd, *J* = 8.9, 2.8 Hz), 8.25 (1H, s). |
| I-079 | $^1$H-NMR ($CDCl_3$) δ: 1.86 (1H, brs), 3.78 (3H, s), 3.80-3.85 (5H, m), 4.01-4.10 (2H, m), 6.33 (1H, brs), 7.00-7.04 (2H, m), 7.49 (1H, d, *J* = 2.8 Hz), 7.53 (1H, d, *J* = 8.3 Hz), 7.67 (1H, dd, *J* = 8.9, 2.8 Hz), 7.73 (1H, d, *J* = 2.5 Hz). |
| I-084 | 1H-NMR (DMSO-D6) δ: 3.66-3.71 (2H, m), 3.74 (4H, s), 3.74 (4H, s), 4.05-4.15 (2H, m), 7.35 (1H, d, J = 9.0 Hz), 7.39-7.43 (1H, m), 7.46-7.49 (1H, m), 7.50 (1H, d, J = 1.8 Hz), 7.64 (1H, s), 7.71-7.77 (1H, m). |
| I-088 | $^1$H-NMR ($CDCl_3$) δ : 2.03 (s, 3H), 3.77 (s, 3H), 3.82-3.85 (m, 2H), 4.02-4.12 (m, 2H), 6.77-6.82 (m, 1H), 7.37 (dd, J = 10.1, 2.1Hz, 1H), 7.41 (s, 1H), 7.53 (br d, J = 2.2Hz, 1H), 7.99 (d, J = 2.0Hz, 1H), 8.10 (s, 1H), 8.32 (s, 1H). |

(continued)

| Compound No. | NMR |
|---|---|
| I-094 | 1H-NMR (DMSO-D6) δ: 3.47-3.56 (2H, m), 3.61-3.66 (2H, m), 3.70 (3H, s), 3.95-4.04 (2H, m), 4.82 (1H, br s), 6.79 (1H, d, J = 8.9 Hz), 7.15 (1H, d, J = 8.7 Hz), 7.52 (1H, d, J = 8.7 Hz), 7.56 (1H, d, J = 2.6 Hz), 7.60 (1H, s), 7.75 (1H, dd, J = 8.9, 2.6 Hz). 8.07 (1H, s), 9.10 (1H. s). |
| I-095 | $^{1}$H-NMR (DMSO-$D_6$) δ: 3.51 (3H, s), 3.66 (2H, brs), 4.02 (2H, brs), 4.83 (1H, t, *J* = 5.2 Hz), 7.15 (1H, d, *J* = 9.0 Hz), 7.67 (1H, d, *J* = 2.8 Hz), 7.71 (1H, s), 7.83 (1H, dd, *J* = 9.0, 2.8 Hz), 7.96 (1H, dd, *J* = 11.4, 1.9 Hz), 8.30 (1H, s), 9.42 (1H, d, *J* = 1.9 Hz). |
| I-097 | $^{1}$H-NMR ($CDCl_3$) δ: 1.66 (1H, t, *J* = 6.4 Hz), 2.28 (6H, s), 2.72 (3H, s), 3.77-3.82 (2H, m), 4.05 (2H, t, J= 4.6 Hz), 6.80 (1H, d, J= 3.0 Hz), 7.04 (1H, d, J= 8.9 Hz), 7.39 (1H, d, *J* = 2.8 Hz), 7.45 (1H, dd, *J* = 10.9, 1.9 Hz), 7.68 (1H, dd, *J* = 8.9, 2.8 Hz), 8.24 (1H, s). |
| I-105 | $^{1}$H-NMR ($CDCl_3$) δ : 1.85 (t, J = 6.4Hz, 1H), 3.78 (s, 3H), 3.83-3.88 (m, 2H), 4.05-4.13 (m, 2H), 7.06 (d, J = 9.0Hz, 1H), 7.34 (br s, 1H), 7.54 (d, J = 2.8Hz, 1H), 7.55 (s, 1H), 7.67 (dd,J = 8.9, 2.9Hz, 1H), 8.61 (s, 2H). |
| I-107 | $^{1}$H-NMR ($CDCl_3$) δ: 2.29 (1H, t, *J* = 6.4 Hz), 2.39 (3H, s), 3.89 (3H, s), 3.93-3.97 (2H, m), 4.18 (2H, t, *J* = 4.3 Hz), 6.55 (1H, d, *J* = 2.6 Hz), 6.97 (1H, s), 7.48 (1H, dd, *J* = 10.6, 1.9 Hz), 7.79 (1H, s), 7.85 (1H, s), 8.19 (1H, s). |
| I-115 | $^{1}$H-NMR ($CDCl_3$) δ : 1.90 (t, J = 6.3Hz, 1H), 3.83 (s, 3H), 3.87-3.92 (m, 2H), 4.02-4.22 (m, 2H), 7.52 (dd, J = 10.4, 1.9Hz, 1H), 7.56 (s, 1H), 7.68-7.74 (m, 1H), 8.16 (s, 1H), 8.32 (s, 1H), 8.51 (s, 1H). |
| I-117 | $^{1}$H-NMR ($CDCl_3$) δ : 3.19 (t, J = 5.9Hz, 1H), 3.82 (s, 3H), 3.90-3.95 (m, 2H), 4.50-4.53 (m, 2H), 6.50-6.53 (m, 1H), 7.36 (dd,J = 10.3, 2.0Hz, 1H), 7.56 (s, 1H), 7.96 (d, J = 2.0Hz, 1H), 8.06 (d, J = 2.8Hz, 1H), 8.46 (d, J = 2.8Hz, 1H). |

[Table 69]

| Compound No. | NMR |
|---|---|
| I-133 | 1H-NMR ($CDCl_3$) δ : 0.63-0.68 (m, 2H), 0.95-1.01 (m, 2H), 1.74-1.82 (m, 1H), 3.33 (t, J = 5.8Hz, 1H), 3.81 (s, 3H), 3.89-3.94 (m, 2H), 4.48-4.51 (m, 2H), 6.97 (br s, 1H), 7.55 (s, 1H), 8.09 (d, J = 2.8Hz, 1H), 8.20 (s, 2H), 8.41 (d, J = 2.8Hz, 1H). |
| I-145 | $^{1}$H-NMR ($CDCl_3$) δ: 1.94 (1H, td, *J* = 6.3, 2.4 Hz), 3.79 (3H, s), 3.87-3.93 (2H, m), 4.09 (2H, t, *J* = 4.4 Hz), 6.89 (2H, m), 7.42 (1H, d, *J* = 4.5 Hz), 7.50 (1H, dd, *J* = 10.7, 1.6 Hz), 8.26 (1H, s), 8.37 (1H, d, *J* = 9.0 Hz). |
| I-147 | $^{1}$H-NMR ($CDCl_3$) δ: 1.71 (1H, t, *J* = 6.2 Hz), 2.23 (3H, s), 2.36 (3H, s), 3.87-3.92 (2H, m), 4.05 (2H, t, *J* = 4.5 Hz), 6.84-6.88 (2H, m), 7.49 (1H, dd, *J* = 10.6, 1.8 Hz), 8.19 (1H, d, *J* = 9.2 Hz), 8.24 (1H, s). |
| I-150 | $^{1}$H-NMR ($CDCl_3$) δ: 1.93 (1H, t, *J* = 6.3 Hz), 3.80 (3H, s), 3.85-3.90 (2H, m), 4.05-4.10 (2H, m), 6.89-6.93 (2H, m), 7.50 (1H, dd, *J* = 10.7, 1.7 Hz), 7.56 (1H, s), 8.26 (1H, s), 8.36 (1H, d, *J* = 9.2 Hz). |

[Table 70]

| Compound No. | NMR |
|---|---|
| I-186 | $^{1}$H-NMR ($CDCl_3$) δ : 1.58-1.61 (m, 1H), 2.34 (s, 3H), 2.35 (s, 6H), 3.61 (s, 3H), 3.78-3.82 (m, 2H), 4.09 (t, J = 4.4 Hz, 2H), 4.70 (s, 2H), 7.10 (s, 1H), 7.31 (s, 1H), 8.01 (s, 1H), 8.06 (s, 1H), 8.27 (s, 1H). |
| I-211 | $^{1}$H-NMR ($CDCl_3$) δ: 1.65 (t, *J* = 6.0 Hz, 1H), 2.28 (s, 6H), 3.84 (td, *J* = 5.2, 4.8 Hz, 2H), 4.14 (t, *J* = 4.8 Hz, 2H), 4.63 (td, *J* = 13.2, 4.3 Hz, 2H), 6.20 (tt, *J* = 55.5, 4.3 Hz, 1H), 7.82 (d, *J* = 2.0 Hz, 1H), 8.01 (s, 1H), 8.12 (s, 1H), 8.31 (s, 1H), 8.38 (br s, 1H). |
| I-228 | $^{1}$H-NMR ($CDCl_3$) δ : 2.18 (t, J = 7.2Hz, 1H), 3.82-3.88 (m, 5H), 7.54-7.60 (m, 2H), 7.90 (s, 1H), 8.36 (s, 1H), 8.43 (s, 1H), 8.63 (s, 1H). |

(continued)

| Compound No. | NMR |
|---|---|
| I-267 | $^1$H-NMR (DMSO-$D_6$) δ: 2.19 (s, 6H), 2.57 (s, 3H), 3.54-3.59 (m, 2H), 4.03 (t, J = 4.8 Hz, 2H), 4.79 (t, *J* = 5.2 Hz, 1H), 7.55 (d, *J* = 8.6 Hz, 2H), 7.74 (d, *J* = 8.6 Hz, 2H), 7.82 (d, *J* = 12.5 Hz, 1H), 9.18 (s, 1H). |
| I-268 | $^1$H-NMR ($CDCl_3$) δ: 2.00-2.05 (m, 1H), 2.28 (s, 6H), 2.71 (s, 3H), 3.78-3.83 (m, 2H), 3.99 (t, *J* = 4.4 Hz, 2H), 6.42 (t, J = 74.4 Hz, 1H), 6.52 (brs, 1H), 7.04 (d, *J* = 8.9 Hz, 2H), 7.24 (d, *J* = 11.7 Hz, 1H), 7.54 (d, *J* = 8.9 Hz, 2H). |
| I-359 | $^1$H-NMR ($CDCl_3$) δ: 1.82 (t, *J* = 6.3 Hz, 1H), 3.03 (d, *J* = 4.5 Hz, 3H), 3.84-3.89 (m, 2H), 4.18-4.21 (m, 2H), 6.70-6.74 (m, 1H), 7.15 (dd, *J* = 8.5, 8.5 Hz, 1H), 7.51 (dd, *J* = 10.7, 1.9 Hz, 1H), 7.70 (d, J = 2.8 Hz, 1H), 8.14 (s, 1H), 8.20 (ddd, *J* = 8.5, 8.5, 6.8 Hz, 1H), 8.31 (s, 1H), 8.52 (s, 1H). |
| I-378 | $^1$H-NMR ($CDCl_3$) δ: 1.74 (br s, 1H), 2.04-2.20 (m, 2H), 2.34 (s, 6H), 3.82-3.90 (m, 4H), 3.93-4.02 (m, 2H), 4.12-4.14 (m, 2H), 4.43-4.46 (m, 1H), 4.68-4.78 (m, 2H), 7.82 (s, 1H), 8.02 (s, 1H), 8.13 (s, 1H), 8.31 (s, 1H), 8.36 (s, 1H). |
| I-379 | $^1$H-NMR ($CDCl_3$) δ: 1.77 (br s, 1H), 2.13 (s, 3H), 2.29 (s, 6H), 3.79-3.83 (m, 2H), 4.12 (t, J = 4.4 Hz, 2H), 5.45 (s, 2H), 7.38 (s, 1H), 7.45 (s, 1H), 7.82 (d, J = 1.6 Hz, 1H), 8.01 (s, 1H), 8.12 (s, 1H), 8.30 (s, 1H), 8.37 (br s, 1H). |

Biological Test Examples for the compounds of the present invention will be described below. The compounds of the present invention can be tested essentially as described in the following text examples.

It is desirable that the compound represented by Formula (I) or (I-1) according to the present invention has serotonin 5-HT2A receptor antagonism and/or inverse agonism and 5-HT2C receptor antagonism and/or inverse agonism.

Specifically, in the evaluation method described below, the Ki value is preferably 5000 nM or less, more preferably 1000 nM or less, and even more preferably 100 nM or less.

Test Example 1: 5-HT2A Receptor Binding Inhibition Test

(Each Experimental Condition)

Cell membrane: 15 μg of Jump-In HEK cell membrane (expressing human recombinant 5-HT2A receptor) per well
Assay buffer: 50 mmol/L Tris-HCl (pH 7.4) containing 120 mmol/L NaCl, 1 mmol/L $MgCl_2 \cdot 6H_2O$, 5 mmol/L KCl, 0.1% BSA, and 2 mmol/L $CaCl_2$
Radioactive ligand: [$^3$H]-Ketanserin with a final concentration of around Kd value calculated by the following method
Non-specific ligand: Serotonin HCl with a final concentration of 500 μmol/L

The Kd value is calculated when the lot of the cell membrane is changed. 1 mmol/L non-specific binding calculating compound dissolved in DMSO or DMSO is previously dispensed to the microplate by 0.5 μL, and the cell membrane is diluted with an Assay buffer. The radioactive ligand solution is diluted step by step, and counts are checked with a liquid scintillator. The Assay buffer containing diluted cell membranes is dispensed by 50 μL/well into the microplate. The radioactive ligand solution is then dispensed by 50 μL/well into the microplate and the plate is sealed. The microplate is allowed to stand for 1.5 hours at room temperature (25°C). During this time, 50 mmol/L Tris-HCl (pH 7.4) is dispensed by 50 μL/well on the GF/B UniFilter plate and allowed to stand at 4°C for 1 hour or longer. Thereafter, filtration is performed with a Cell Harvester (PerkinElmer). The radioactive ligand solution is dispensed by 10 μL/well into empty wells of the GF/B UniFilter plate. After drying the GF/B UniFilter plate at room temperature, MicroScinti20 is dispensed by 50 μL/well into the GF/B UniFilter plate, and the plate is sealed. GF/B UniFilter plate is allowed to stand at room temperature overnight. The radioactivity of [$^3$H]-Ketanserin bound to the 5-HT2A receptor is measured using Microbeta2 (PerkinElmer) at a measurement time of 1 min/well. The Saturation curve is drawn from the measured value, and the Kd value is calculated from the slope of the Scatchard Plot.

(Binding Test of Compound According to Present Invention)

A compound solution dissolved in DMSO is dispensed to a microplate by 0.5 μL, and a cell membrane and a hot ligand are each diluted with an Assay buffer. The Assay buffer containing diluted cell membrane is then dispensed by 50 μL/well into the microplate. The radioactive ligand solution is then dispensed by 50 μL/well into the microplate and the plate is sealed. Thereafter, the microplate is allowed to stand at room temperature (25°C) for 1.5 hours. During this time, 50 mmol/L Tris-HCl (pH 7.4) is dispensed by 50 μL/well into the GF/B UniFilter plate and allowed to stand at 4°C for 1 hour or longer. Thereafter, filtration is performed with a Cell Harvester (PerkinElmer). After drying the GF/B UniFilter plate at room temperature, MicroScinti20 is dispensed by 50 μL/well into the GF/B UniFilter plate, and the plate is sealed. GF/B UniFilter plate is allowed to stand at room temperature overnight. The radioactivity of [$^3$H]-Ketanserin bound to the 5-HT2A receptor is measured using Microbeta2 (PerkinElmer) at a measurement time of 1 min/well. Non-specific binding is calculated from the radioactivity of [$^3$H]-Ketanserin in the presence of 500 μmol/L Serotonin HCl of unlabeled ligand, and total binding is calculated from the radioactivity of [$^3$H]-Ketanserin in the absence of the compound of the present invention (Vehicle). Finally, the Ki value is calculated from the dose-response curve.

(The binding activity of the compound according to the present invention is calculated from the following binding inhibition rate (%).)

$$\text{Inhibition rate (\%)} = [1-(c-a)/(b-a)] \times 100$$

a; average cpm of non-specific binding
b; average cpm of total binding
c; cpm in the presence of the test compound

The compounds of the present invention were tested essentially as described above. The test results of the human serotonin 5-HT2A receptor binding activity (h 5-HT2A Ki) of the compounds of the present invention are shown below.

(Results)

[Table 71]

| Compound No. | h 5-HT2A Ki (nM) |
|---|---|
| I-001 | 0.447 |
| I-002 | 0.375 |
| I-003 | 0.597 |
| I-004 | 0.42 |
| I-005 | 0.301 |
| I-006 | 0.317 |
| I-007 | 0.548 |
| I-008 | 0.344 |
| I-009 | 0.337 |

Test Example 1-2 5-HT2A Receptor Binding Inhibition Test

(Each Experimental Condition)

Cell membrane: 15 μg of Jump-In HEK cell membrane (expressing human recombinant 5-HT2A receptor) per well
Assay buffer: 50 mmol/L Tris-HCl (pH 7.4) containing 120 mmol/L NaCl, 1 mmol/L $MgCl_2 \cdot 6H_2O$, 5 mmol/L KCl, 0.1% BSA, and 2 mmol/L $CaCl_2$
Radioactive ligand: [$^3$H]-Ketanserin with a final concentration of around Kd value calculated by the following method

Non-specific ligand: Ketanserin at a final concentration of 500 μmol/L

The Kd value is calculated when the lot of the cell membrane is changed. 1 mmol/L non-specific binding calculating compound dissolved in DMSO or DMSO is previously dispensed to the microplate by 0.5 μL, and the cell membrane is diluted with an Assay buffer. The radioactive ligand solution is diluted step by step, and counts are checked with a liquid scintillator. The Assay buffer containing diluted cell membranes is dispensed by 50 μL/well into the microplate. The radioactive ligand solution is then dispensed by 50 μL/well into the microplate and the plate is sealed. The microplate is allowed to stand for 1.5 hours at room temperature (25°C). During this time, 50 mmol/L Tris-HCl (pH 7.4) is dispensed by 50 μL/well into the UniFilter plate and allowed to stand at 4°C for 1 hour or longer. Thereafter, filtration is performed with a Cell Harvester (PerkinElmer). The radioactive ligand solution is dispensed by 10 μL/well into empty wells of the UniFilter plate. After drying the UniFilter plate at room temperature, MicroScinti20 is dispensed by 50 μL/well into the UniFilter plate, and the plate is sealed. UniFilter plate is allowed to stand at room temperature overnight. The radioactivity of [$^3$H]-Ketanserin bound to the 5-HT2A receptor is measured using Microbeta2 (PerkinElmer) at a measurement time of 1 min/well. The Saturation curve is drawn from the measured value, and the Kd value is calculated from the slope of the Scatchard Plot.

(Binding Test of Compound According to Present Invention)

A compound solution dissolved in DMSO is dispensed to a microplate by 0.5 μL, and a cell membrane and a hot ligand are each diluted with an Assay buffer. The Assay buffer containing diluted cell membrane is then dispensed by 50 μL/well into the microplate. The radioactive ligand solution is then dispensed by 50 μL/well into the microplate and the plate is sealed. Thereafter, the microplate is allowed to stand at room temperature (25°C) for 1.5 hours. During this time, 50 mmol/L Tris-HCl (pH 7.4) is dispensed by 50 μL/well into the UniFilter plate and allowed to stand at 4°C for 1 hour or longer. Thereafter, filtration is performed with a Cell Harvester (PerkinElmer). After drying the UniFilter plate at room temperature, MicroScinti20 is dispensed by 50 μL/well into the UniFilter plate, and the plate is sealed. UniFilter plate is allowed to stand at room temperature overnight. The radioactivity of [$^3$H]-Ketanserin bound to the 5-HT2A receptor is measured using Microbeta2 (PerkinElmer) at a measurement time of 1 min/well. Non-specific binding is calculated from the radioactivity of [$^3$H]-Ketanserin in the presence of 500 μmol/L Ketanserin of unlabeled ligand, and total binding is calculated from the radioactivity of [$^3$H]-Ketanserin in the absence of the compound of the present invention (Vehicle). Finally, the Ki value is calculated from the dose-response curve.

(The binding activity of the compound according to the present invention is calculated from the following binding inhibition rate (%).)

$$\text{Inhibition rate (\%)} = [1-(c-a)/(b-a)] \times 100$$

a; average cpm of non-specific binding
b; average cpm of total binding
c; cpm in the presence of the test compound

The compounds of the present invention were tested essentially as described above. The test results of the human serotonin 5-HT2A receptor binding activity (h 5-HT2A Ki) of the compounds of the present invention are shown below.

(Results)

[Table 72]

| Compound No. | h 5-HT2A Ki (nM) | Compound No. | h 5-HT2A Ki (nM) | Compound No. | h 5-HT2A Ki (nM) |
|---|---|---|---|---|---|
| I-006 | 0.253 | I-059 | 0.45 | I-110 | 0.375 |
| I-008 | 0.338 | I-060 | 0.158 | I-111 | 2.98 |
| I-010 | 0.246 | I-061 | 0.254 | I-112 | 0.276 |
| I-011 | 0.133 | I-062 | 0.286 | I-113 | 0.295 |
| I-012 | 0.272 | I-063 | 0.337 | I-114 | 1.56 |
| I-013 | 0.332 | I-064 | 0.327 | I-115 | 0.265 |

(continued)

| Compound No. | h 5-HT2A Ki (nM) | Compound No. | h 5-HT2A Ki (nM) | Compound No. | h 5-HT2A Ki (nM) |
|---|---|---|---|---|---|
| I-014 | 0.413 | I-065 | 0.15 | I-116 | 0.325 |
| I-015 | 0.195 | I-066 | 0.222 | I-117 | 0.241 |
| I-016 | 0.284 | I-067 | 0.403 | I-118 | 0.262 |
| I-017 | 0.266 | I-068 | 0.29 | I-119 | 0.315 |
| I-018 | 0.235 | I-069 | 0.373 | I-120 | 0.903 |
| I-019 | 0.548 | I-070 | 0.302 | I-121 | 0.279 |
| I-020 | 1.12 | I-071 | 0.379 | I-122 | 0.485 |
| I-021 | 0.722 | I-072 | 0.412 | I-123 | 0.81 |
| I-022 | 0.428 | I-073 | 0.184 | I-124 | 0.259 |
| I-023 | 0.246 | I-074 | 0.236 | I-125 | 0.36 |
| I-024 | 0.607 | I-075 | 0.149 | I-126 | 0.654 |
| I-025 | 0.25 | I-076 | 0.211 | I-127 | 0.271 |
| I-026 | 0.894 | I-077 | 0.23 | I-128 | 0.258 |
| I-027 | 0.292 | I-078 | 0.263 | I-129 | 0.237 |
| I-028 | 0.209 | I-079 | 0.295 | I-130 | 0.256 |
| I-029 | 0.286 | I-080 | 0.445 | I-131 | 0.163 |
| I-030 | 0.31 | I-081 | 0.202 | I-132 | 0.236 |
| I-031 | 0.239 | I-082 | 0.184 | I-133 | 0.322 |
| I-032 | 0.734 | I-083 | 0.289 | I-134 | 0.621 |
| I-033 | 0.376 | I-084 | 0.442 | I-135 | 1.24 |
| I-034 | 0.77 | I-085 | 0.192 | I-136 | 0.167 |

[Table 73]

| Compound No. | h 5-HT2A Ki (nM) | Compound No. | h 5-HT2A Ki (nM) | Compound No. | h 5-HT2A Ki (nM) |
|---|---|---|---|---|---|
| I-035 | 2.54 | I-086 | 0.205 | I-137 | 0.205 |
| I-036 | 0.344 | I-087 | 0.241 | I-138 | 0.264 |
| I-037 | 0.289 | I-088 | 0.267 | I-139 | 0.431 |
| I-038 | 0.215 | I-089 | 0.412 | I-140 | 0.148 |
| I-039 | 0.319 | I-090 | 0.203 | I-141 | 0.296 |
| I-040 | 0.175 | I-091 | 0.197 | I-142 | 0.269 |
| I-041 | 0.155 | I-092 | 0.274 | I-143 | 0.259 |
| I-042 | 0.194 | I-093 | 0.283 | I-144 | 0.341 |
| I-043 | 0.188 | I-094 | 0.258 | I-145 | 0.202 |
| I-044 | 0.626 | I-095 | 0.372 | I-146 | 0.678 |
| I-045 | 0.235 | I-096 | 0.341 | I-147 | 0.259 |
| I-046 | 0.318 | I-097 | 0.509 | I-148 | 0.506 |
| I-047 | 0.207 | I-098 | 0.559 | I-149 | 0.365 |
| I-048 | 0.163 | I-099 | 0.224 | I-150 | 0.279 |
| I-049 | 0.168 | I-100 | 0.27 | I-151 | 0.292 |

(continued)

| Compound No. | h 5-HT2A Ki (nM) | Compound No. | h 5-HT2A Ki (nM) | Compound No. | h 5-HT2A Ki (nM) |
|---|---|---|---|---|---|
| I-050 | 0.395 | I-101 | 0.497 | I-152 | 0.354 |
| I-051 | 0.318 | I-102 | 0.265 | I-153 | 0.286 |
| I-052 | 0.389 | I-103 | 0.351 | I-154 | 0.332 |
| I-053 | 0.291 | I-104 | 0.39 | I-155 | 0.318 |
| I-054 | 0.632 | I-105 | 0.314 | I-156 | 0.315 |
| I-055 | 0.396 | I-106 | 0.409 | I-157 | 0.238 |
| I-056 | 1.3 | I-107 | 1.67 | I-158 | 0.907 |
| I-057 | 0.386 | I-108 | 0.478 | I-159 | 0.295 |
| I-058 | 0.418 | I-109 | 0.598 | | |

[Table 74]

| Compound No. | h 5-HT2A Ki (nM) | Compound No. | h 5-HT2A Ki (nM) | Compound No. | h 5-HT2A Ki (nM) |
|---|---|---|---|---|---|
| I-160 | 0.210 | I-190 | 0.493 | I-220 | 1.39 |
| I-161 | 0.361 | I-191 | 0.473 | I-221 | 0.996 |
| I-162 | 0.395 | I-192 | 2.31 | I-222 | 1.35 |
| I-163 | 0.216 | I-193 | 3.43 | I-223 | 0.877 |
| I-164 | 0.325 | I-194 | 8.31 | I-224 | 0.521 |
| I-165 | 0.389 | I-195 | 6.75 | I-225 | 0.396 |
| I-166 | 0.670 | I-196 | 4.46 | I-226 | 0.268 |
| I-167 | 0.511 | I-197 | 2.83 | I-227 | 1.21 |
| I-168 | 0.248 | I-198 | 5.3 | I-228 | 0.316 |
| I-169 | 0.738 | I-199 | 6.09 | I-229 | 0.280 |
| I-170 | 0.212 | I-200 | 1.49 | I-230 | 0.576 |
| I-171 | 0.160 | I-201 | 2.26 | I-231 | 3.78 |
| I-172 | 0.812 | I-202 | 0.828 | I-232 | 8.27 |
| I-173 | 1.42 | I-203 | 1.97 | I-233 | 4.42 |
| I-174 | 0.888 | I-204 | 2.35 | I-234 | 0.525 |
| I-175 | 1.92 | I-205 | 2.94 | I-235 | 0.925 |
| I-176 | 0.453 | I-206 | 1.3 | I-236 | 1.24 |
| I-177 | 0.331 | I-207 | 2.23 | I-237 | 7.43 |
| I-178 | 0.386 | I-208 | 1.02 | I-238 | 5.12 |
| I-179 | 0.278 | I-209 | 1.71 | I-239 | 2.3 |
| I-180 | 0.346 | I-210 | 0.804 | I-240 | 2.05 |
| I-181 | 0.984 | I-211 | 0.706 | I-241 | 0.799 |
| I-182 | 0.475 | I-212 | 1.22 | I-242 | 0.258 |
| I-183 | 0.271 | I-213 | 1.26 | I-243 | 3.58 |
| I-184 | 3.84 | I-214 | 1.66 | I-244 | 3.75 |
| I-185 | 0.420 | I-215 | 4.48 | I-245 | 5.85 |
| I-186 | 1.75 | I-216 | 1.81 | I-246 | 2.7 |

(continued)

| Compound No. | h 5-HT2A Ki (nM) | Compound No. | h 5-HT2A Ki (nM) | Compound No. | h 5-HT2A Ki (nM) |
|---|---|---|---|---|---|
| I-187 | 0.183 | I-217 | 1.74 | I-247 | 1.74 |
| I-188 | 0.181 | I-218 | 1.22 | I-248 | 0.794 |
| I-189 | 0.217 | I-219 | 1.25 | I-249 | 5.19 |

[Table 75]

| Compound No. | h 5-HT2A Ki (nM) | Compound No. | h 5-HT2A Ki (nM) | Compound No. | h 5-HT2A Ki (nM) |
|---|---|---|---|---|---|
| I-250 | 6.47 | I-280 | 0.312 | I-310 | 0.194 |
| I-251 | 0.325 | I-281 | 0.668 | I-311 | 0.432 |
| I-252 | 3.95 | I-282 | 0.223 | I-312 | 0.252 |
| I-253 | 2.74 | I-283 | 0.267 | I-313 | 0.198 |
| I-254 | 0.496 | I-284 | 0.301 | I-314 | 0.902 |
| I-255 | 0.520 | I-285 | 0.299 | I-315 | 1.21 |
| I-256 | 0.500 | I-286 | 0.600 | I-316 | 0.506 |
| I-257 | 0.395 | I-287 | 0.280 | I-317 | 0.453 |
| I-258 | 0.933 | I-288 | 0.387 | I-318 | 0.575 |
| I-259 | 0.672 | I-289 | 0.793 | I-319 | 0.650 |
| I-260 | 2.05 | I-290 | 0.438 | I-320 | 0.297 |
| I-261 | 0.608 | I-291 | 0.423 | I-321 | 0.305 |
| I-262 | 0.558 | I-292 | 0.385 | I-322 | 0.414 |
| I-263 | 0.577 | I-293 | 0.259 | I-323 | 2.14 |
| I-264 | 0.752 | I-294 | 0.395 | I-324 | 0.421 |
| I-265 | 0.972 | I-295 | 0.851 | I-325 | 1.6 |
| I-266 | 1.88 | I-296 | 0.349 | I-326 | 6.26 |
| I-267 | 0.465 | I-297 | 2.07 | I-327 | 2.4 |
| I-268 | 0.958 | I-298 | 0.303 | I-328 | 1.1 |
| I-269 | 0.295 | I-299 | 0.271 | I-329 | 0.896 |
| I-270 | 0.938 | I-300 | 1.33 | I-330 | 1.74 |
| I-271 | 0.400 | I-301 | 0.189 | I-331 | 1.91 |
| I-272 | 0.343 | I-302 | 1.08 | I-332 | 5.58 |
| I-273 | 0.309 | I-303 | 0.379 | I-333 | 9.02 |
| I-274 | 0.219 | I-304 | 0.169 | I-334 | 3.49 |
| I-275 | 0.273 | I-305 | 0.188 | I-335 | 1.18 |
| I-276 | 0.135 | I-306 | 1.27 | I-336 | 0.226 |
| I-271 | 0.186 | I-307 | 0.221 | I-337 | 0.313 |
| I-278 | 0.203 | I-308 | 0.575 | I-338 | 0.223 |
| I-279 | 0.306 | I-309 | 0.247 | I-339 | 0.217 |

[Table 76]

| Compound No. | h 5-HT2A Ki (nM) | Compound No. | h 5-HT2A Ki (nM) | Compound No. | h 5-HT2A Ki (nM) |
|---|---|---|---|---|---|
| I-340 | 0.212 | I-370 | 1.03 | I-400 | 0.338 |

(continued)

| Compound No. | h 5-HT2A Ki (nM) | Compound No. | h 5-HT2A Ki (nM) | Compound No. | h 5-HT2A Ki (nM) |
|---|---|---|---|---|---|
| I-341 | 0.303 | I-371 | 2.37 | I-401 | 0.211 |
| I-342 | 0.256 | I-372 | 0.847 | I-402 | 0.533 |
| I-343 | 0.184 | I-373 | 1.21 | I-403 | 0.565 |
| I-344 | 0.260 | I-374 | 0.955 | I-404 | 0.362 |
| I-345 | 0.257 | I-375 | 1.27 | I-405 | 0.274 |
| I-346 | 0.240 | I-376 | 2.24 | I-406 | 0.463 |
| I-347 | 0.262 | I-377 | 2.9 | I-407 | 0.225 |
| I-348 | 0.215 | I-378 | 2.43 | I-408 | 0.384 |
| I-349 | 0.254 | I-379 | 1.13 | I-409 | 0.420 |
| I-350 | 0.204 | I-380 | 0.527 | I-410 | 0.573 |
| I-351 | 0.242 | I-381 | 2.66 | | |
| I-352 | 0.202 | I-382 | 2.44 | | |
| I-353 | 0.179 | I-383 | 2.57 | | |
| I-354 | 0.319 | I-384 | 2.87 | | |
| I-355 | 0.398 | I-385 | 0.226 | | |
| I-356 | 0.163 | I-386 | 0.244 | | |
| I-357 | 0.635 | I-387 | 0.318 | | |
| I-358 | 0.603 | I-388 | 0.366 | | |
| I-359 | 0.952 | I-389 | 1.2 | | |
| I-360 | 1.22 | I-390 | 1.08 | | |
| I-361 | 2.4 | I-391 | 0.357 | | |
| I-362 | 0.924 | I-392 | 2.88 | | |
| I-363 | 2.32 | I-393 | 17.6 | | |
| I-364 | 3.34 | I-394 | 1.34 | | |
| I-365 | 4.65 | I-395 | 3.37 | | |
| I-366 | 2.19 | I-396 | 9.96 | | |
| I-367 | 1.97 | I-397 | 0.413 | | |
| I-368 | 2.13 | I-398 | 0.576 | | |
| I-369 | 1.72 | I-399 | 0.347 | | |

Test Example 2: 5-HT2C receptor binding inhibition test

(Each Experimental Condition)

Cell membrane: 0.5 μg of Jump-In HEK cell membrane (expressing human recombinant 5-HT2C receptor) per well
Assay buffer: 50 mmol/L Tris-HCl (pH 7.4) containing 120 mmol/L NaCl, 1 mmol/L $MgCl_2 \cdot 6H_2O$, 5 mmol/L KCl, 0.1% BSA, and 2 mmol/L $CaCl_2$
Radioactive ligand: $[^3H]$-Mesulergine with a final concentration of around Kd value calculated by the following method
Non-specific ligand: Serotonin HCl with a final concentration of 500 μmol/L

The Kd value is calculated when the lot of the cell membrane is changed. 1 mmol/L non-specific binding

calculating compound dissolved in DMSO or DMSO is previously dispensed to the microplate by 0.5 μL, and the cell membrane is diluted with an Assay buffer. The radioactive ligand solution is diluted step by step, and counts are checked with a liquid scintillator. The Assay buffer containing diluted cell membranes is dispensed by 50 μL/well into the microplate. The radioactive ligand solution is then dispensed by 50 μL/well into the microplate and the plate is sealed. The microplate is allowed to stand at 37°C for 2 hours. During this time, 50 mmol/L Tris-HCl (pH 7.4) is dispensed by 50 μL/well into the GF/B UniFilter plate and allowed to stand at 4°C for 1 hour or longer. Thereafter, filtration is performed with a Cell Harvester (PerkinElmer). The radioactive ligand solution is dispensed by 10 μL/well into empty wells of the GF/B UniFilter plate. After drying the GF/B UniFilter plate at room temperature, MicroScinti20 is dispensed by 50 μL/well into the GF/B UniFilter plate, and the plate is sealed. GF/B UniFilter plate is allowed to stand at room temperature overnight. The radioactivity of [$^3$H]-Mesulergine bound to the 5-HT2C receptor is measured using Microbeta2 (PerkinElmer) at a measurement time of 1 min/well. The Saturation curve is drawn from the measured value, and the Kd value is calculated from the slope of the Scatchard Plot.

(Binding Test of Compound According to Present Invention)

A compound solution dissolved in DMSO is dispensed to a microplate by 0.5 μL, and a cell membrane and a hot ligand are each diluted with an Assay buffer. The Assay buffer containing diluted cell membrane is then dispensed by 50 μL/well into the microplate. The radioactive ligand solution is then dispensed by 50 μL/well into the microplate and the plate is sealed. The microplate is then allowed to stand at 37°C for 2 hours. During this time, 50 mmol/L Tris-HCl (pH 7.4) is dispensed by 50 μL/well into the GF/B UniFilter plate and allowed to stand at 4°C for 1 hour or longer. Thereafter, filtration is performed with a Cell Harvester (PerkinElmer). After drying the GF/B UniFilter plate at room temperature, MicroScinti20 is dispensed by 50 μL/well into the GF/B UniFilter plate, and the plate is sealed. GF/B UniFilter plate is allowed to stand at room temperature overnight. The radioactivity of [$^3$H]-Mesulergine bound to the 5-HT2C receptor is measured using Microbeta2 (PerkinElmer) at a measurement time of 1 min/well. Non-specific binding is calculated from the radioactivity of [$^3$H]-Mesulergine in the presence of 500 μmol/L Serotonin HCl of unlabeled ligand, and total binding is calculated from the radioactivity of [$^3$H]-Mesulergine in the absence of the compound of the present invention (Vehicle). Finally, the Ki value is calculated from the dose-response curve.

(The binding activity of the compound according to the present invention is calculated from the following binding inhibition rate (%).)

$$\text{Inhibition rate (\%)} = [1-(c-a)/(b-a)] \times 100$$

a; average cpm of non-specific binding
b; average cpm of total binding
c; cpm in the presence of the test compound

The compounds of the present invention were tested essentially as described above. The test results of the human serotonin 5-HT2C receptor binding activity (h 5-HT2C Ki) of the compounds of the present invention are shown below.

(Results)

[Table 77]

| Compound No. | h 5-HT2C Ki (nM) |
|---|---|
| I-001 | 1.44 |
| I-002 | 19.2 |
| I-003 | 11.4 |
| I-004 | 0.387 |
| I-005 | 1.08 |
| I-006 | 0.223 |
| I-007 | 14.9 |
| I-008 | 1.05 |

(continued)

| Compound No. | h 5-HT2C Ki (nM) |
|---|---|
| I-009 | 8.7 |

Test Example 2-2: 5-HT2C receptor binding inhibition test

(Each Experimental Condition)

Cell membrane: 0.5 μg of Jump-In HEK cell membrane (expressing human recombinant 5-HT2C receptor) per well
Assay buffer: 50 mmol/L Tris-HCl (pH 7.4) containing 120 mmol/L NaCl, 1 mmol/L $MgCl_2 \cdot 6H_2O$, 5 mmol/L KCl, 0.1% BSA, and 2 mmol/L $CaCl_2$
Radioactive ligand: [$^3$H]-Mesulergine with a final concentration of around Kd value calculated by the following method
Non-specific ligand: Ketanserin at a final concentration of 500 μmol/L

The Kd value is calculated when the lot of the cell membrane is changed. 1 mmol/L non-specific binding calculating compound dissolved in DMSO or DMSO is previously dispensed to the microplate by 0.5 μL, and the cell membrane is diluted with an Assay buffer. The radioactive ligand solution is diluted step by step, and counts are checked with a liquid scintillator. The Assay buffer containing diluted cell membranes is dispensed by 50 μL/well into the microplate. The radioactive ligand solution is then dispensed by 50 μL/well into the microplate and the plate is sealed. The microplate is allowed to stand at 37°C for 2 hours. During this time, 50 mmol/L Tris-HCl (pH 7.4) is dispensed by 50 μL/well into the UniFilter plate and allowed to stand at 4°C for 1 hour or longer. Thereafter, filtration is performed with a Cell Harvester (PerkinElmer). The radioactive ligand solution is dispensed by 10 μL/well into empty wells of the UniFilter plate. After drying the UniFilter plate at room temperature, MicroScinti20 is dispensed by 50 μL/well into the UniFilter plate, and the plate is sealed. UniFilter plate is allowed to stand at room temperature overnight. The radioactivity of [$^3$H]-Mesulergine bound to the 5-HT2C receptor is measured using Microbeta2 (PerkinElmer) at a measurement time of 1 min/well. The Saturation curve is drawn from the measured value, and the Kd value is calculated from the slope of the Scatchard Plot.

(Binding Test of Compound According to Present Invention)

A compound solution dissolved in DMSO is dispensed to a microplate by 0.5 μL, and a cell membrane and a hot ligand are each diluted with an Assay buffer. The Assay buffer containing diluted cell membrane is then dispensed by 50 μL/well into the microplate. The radioactive ligand solution is then dispensed by 50 μL/well into the microplate and the plate is sealed. The microplate is then allowed to stand at 37°C for 2 hours. During this time, 50 mmol/L Tris-HCl (pH 7.4) is dispensed by 50 μL/well into the UniFilter plate and allowed to stand at 4°C for 1 hour or longer. Thereafter, filtration is performed with a Cell Harvester (PerkinElmer). After drying the UniFilter plate at room temperature, MicroScinti20 is dispensed by 50 μL/well into the UniFilter plate, and the plate is sealed. UniFilter plate is allowed to stand at room temperature overnight. The radioactivity of [$^3$H]-Mesulergine bound to the 5-HT2C receptor is measured using Microbeta2 (PerkinElmer) at a measurement time of 1 min/well. Non-specific binding is calculated from the radioactivity of [$^3$H]-Mesulergine in the presence of 500 μmol/L Ketanserin of unlabeled ligand, and total binding is calculated from the radioactivity of [$^3$H]-Mesulergine in the absence of the compound of the present invention (Vehicle). Finally, the Ki value is calculated from the dose-response curve.

(The binding activity of the compound according to the present invention is calculated from the following binding inhibition rate (%).)

$$\text{Inhibition rate (\%)} = [1-(c-a)/(b-a)] \times 100$$

a; average cpm of non-specific binding
b; average cpm of total binding
c; cpm in the presence of the test compound

The compounds of the present invention were tested essentially as described above. The test results of the human serotonin 5-HT2C receptor binding activity (h 5-HT2C Ki) of the compounds of the present invention are shown below.

(Results)

[Table 78]

| Compound No. | h 5-HT2C Ki (nM) | Compound No. | h 5-HT2C Ki (nM) | Compound No. | h 5-HT2C Ki (nM) |
|---|---|---|---|---|---|
| I-006 | 0.271 | I-059 | 30 | I-110 | 17.3 |
| I-008 | 0.822 | I-060 | 0.184 | I-111 | 24.2 |
| I-010 | 1.41 | I-061 | 1.31 | I-112 | 25.5 |
| I-011 | 4.55 | I-062 | 0.366 | I-113 | 3.09 |
| I-012 | 6.43 | I-063 | 2.09 | I-114 | 4.68 |
| I-013 | 7.21 | I-064 | 0.18 | I-115 | 0.818 |
| I-014 | 8.88 | I-065 | 5.02 | I-116 | 0.745 |
| I-015 | 0.248 | I-066 | 0.17 | I-117 | 0.305 |
| I-016 | 0.699 | I-067 | 0.196 | I-118 | 0.596 |
| I-017 | 0.415 | I-068 | 1.15 | 1-119 | 2.09 |
| I-018 | 1.89 | I-069 | 0.36 | I-120 | 3.86 |
| I-019 | 3.12 | I-070 | 0.213 | I-121 | 1.3 |
| I-020 | 5.38 | I-071 | 1.96 | I-122 | 1.38 |
| I-021 | 4.1 | I-072 | 4.64 | I-123 | 5.59 |
| I-022 | 48.4 | I-073 | 0.936 | I-124 | 2.32 |
| I-023 | 3.62 | I-074 | 0.843 | I-125 | 6.04 |
| I-024 | 7.35 | I-075 | 0.939 | I-126 | 4.42 |
| I-025 | 3.7 | I-076 | 3 | I-127 | 3.47 |
| I-026 | 7.89 | I-077 | 3.25 | I-128 | 0.452 |
| I-027 | 0.266 | I-078 | 1.1 | I-129 | 0.959 |
| I-028 | 4.96 | I-079 | 0.625 | I-130 | 3.26 |
| I-029 | 0.636 | I-080 | 2.74 | I-131 | 1.25 |
| I-030 | 0.881 | I-081 | 0.457 | I-132 | 5 |
| I-031 | 1.6 | I-082 | 0.358 | I-133 | 2.53 |
| I-032 | 8.32 | I-083 | 2.97 | I-134 | 13.2 |
| I-033 | 3.43 | I-084 | 0.297 | I-135 | 7.13 |
| I-034 | 3.57 | I-085 | 0.55 | I-136 | 0.277 |

[Table 79]

| Compound No. | h 5-HT2C Ki (nM) | Compound No. | h 5-HT2C Ki (nM) | Compound No. | h 5-HT2C Ki (nM) |
|---|---|---|---|---|---|
| I-035 | 2.35 | I-086 | 8.84 | I-137 | 0.772 |
| I-036 | 1.92 | I-087 | 6.37 | I-138 | 4.76 |
| I-037 | 1.4 | I-088 | 8.31 | I-139 | 7.73 |
| I-038 | 0.666 | I-089 | 3 | I-140 | 1.06 |
| I-039 | 0.245 | I-090 | 0.575 | I-141 | 4.07 |
| I-040 | 1.16 | I-091 | 8.99 | I-142 | 0.195 |
| I-041 | 8.83 | I-092 | 2.76 | I-143 | 0.274 |

(continued)

| Compound No. | h 5-HT2C Ki (nM) | Compound No. | h 5-HT2C Ki (nM) | Compound No. | h 5-HT2C Ki (nM) |
|---|---|---|---|---|---|
| I-042 | 8.35 | I-093 | 0.325 | I-144 | 2.49 |
| I-043 | 0.165 | I-094 | 0.202 | I-145 | 4.76 |
| I-044 | 15.1 | I-095 | 0.761 | I-14C | 3.82 |
| I-045 | 3.77 | I-096 | 1.66 | I-147 | 4.75 |
| I-046 | 1.33 | I-097 | 1.34 | I-148 | 0.906 |
| I-047 | 4.26 | I-098 | 3.18 | I-149 | 1.35 |
| I-048 | 0.313 | I-099 | 7.15 | I-150 | 0.239 |
| I-049 | 0.641 | I-100 | 6.82 | I-151 | 0.206 |
| I-050 | 1.69 | I-101 | 3.11 | I-152 | 0.52 |
| I-051 | 2.6 | I-102 | 7.3 | I-153 | 0.351 |
| I-052 | 0.368 | I-103 | 9.13 | I-154 | 1.1 |
| I-053 | 1.25 | I-104 | 6.13 | I-155 | 1.04 |
| I-054 | 1.03 | I-105 | 0.6 | I-156 | 1.78 |
| I-055 | 0.745 | I-106 | 7.28 | I-157 | 2.33 |
| I-056 | 1.04 | I-107 | 99.3 | I-158 | 3.5 |
| I-057 | 5.3 | I-108 | 6.23 | I-159 | 1.88 |
| I-058 | 0.456 | I-109 | 15.4 | | |

[Table 80]

| Compound No. | h 5-HT2C Ki (nM) | Compound No. | h 5-HT2C Ki (nM) | Compound No. | h 5-HT2C Ki (nM) |
|---|---|---|---|---|---|
| I-160 | 3.01 | I-190 | 9.08 | I-220 | 5.91 |
| I-161 | 1.13 | I-191 | 5.29 | I-221 | 2.23 |
| I-162 | 4.25 | I-192 | 9.15 | I-222 | 1.91 |
| I-163 | 1.26 | I-193 | 3.76 | I-223 | 9.21 |
| I-164 | 2.09 | I-194 | 6.01 | I-224 | 20.2 |
| I-166 | 9.78 | I-195 | 2.1 | I-225 | 0.712 |
| I-166 | 5.57 | I-196 | 5.17 | I-226 | 1.91 |
| I-167 | 5.37 | I-197 | 65.6 | I-227 | 26.7 |
| I-168 | 0.162 | I-198 | 9.63 | I-228 | 0.748 |
| I-169 | 9.97 | I-199 | 5.98 | I-229 | 2.22 |
| I-170 | 5.45 | I-200 | 1.9 | I-230 | 0.372 |
| I-171 | 5.81 | I-201 | 2.54 | I-231 | 5.1 |
| I-172 | 2.49 | I-202 | 9.12 | I-232 | 6.06 |
| I-173 | 5.11 | I-203 | 1.82 | I-233 | 1.76 |
| I-174 | 2.15 | I-204 | 2.7 | I-234 | 0.167 |
| I-175 | 3.19 | I-205 | 0.752 | I-235 | 0.984 |
| I-176 | 6.81 | I-206 | 1.78 | I-236 | 0.727 |
| I-177 | 0.630 | I-207 | 6.02 | I-237 | 7.37 |
| I-178 | 3.34 | I-208 | 1.67 | I-238 | 2.64 |

(continued)

| Compound No. | h 5-HT2C Ki (nM) | Compound No. | h 5-HT2C Ki (nM) | Compound No. | h 5-HT2C Ki (nM) |
|---|---|---|---|---|---|
| I-179 | 0.296 | I-209 | 5.63 | I-239 | 1.35 |
| I-180 | 1.86 | I-210 | 3.67 | I-240 | 1.54 |
| I-181 | 3.62 | I-211 | 1.89 | I-241 | 0.637 |
| I-182 | 4.75 | I-212 | 3.89 | I-242 | 0.337 |
| I-183 | 1.22 | I-213 | 4.07 | I-243 | 2.53 |
| I-184 | 2.98 | I-214 | 8.87 | I-244 | 2.87 |
| I-185 | 2.76 | I-215 | 9.55 | I-245 | 1.41 |
| I-186 | 1.73 | I-216 | 5.53 | I-246 | 5.25 |
| I-187 | 2.9 | I-217 | 2.66 | I-247 | 1.62 |
| I-188 | 1.87 | I-218 | 3.36 | I-248 | 4.78 |
| I-189 | 0.663 | I-219 | 6.28 | I-249 | 4.29 |

[Table 81]

| Compound No. | h 5-HT2C Ki (nM) | Compound No. | h 5-HT2C Ki (nM) | Compound No. | h 5-HT2C Ki (nM) |
|---|---|---|---|---|---|
| I-250 | 2.43 | I-280 | 1.58 | I-310 | 0.608 |
| I-251 | 0.393 | I-281 | 6.55 | I-311 | 6.41 |
| I-252 | 3.17 | I-282 | 6.19 | I-312 | 4.82 |
| I-253 | 4.42 | I-283 | 2.41 | I-313 | 1.38 |
| I-254 | 0.278 | I-284 | 2.07 | I-314 | 3.68 |
| I-255 | 0.360 | I-285 | 7.69 | I-315 | 1.05 |
| I-256 | 0.322 | I-286 | 3.22 | I-316 | 0.864 |
| I-257 | 0.316 | I-287 | 0.266 | I-317 | 6.92 |
| I-258 | 0.301 | I-288 | 0.780 | I-318 | 1.04 |
| I-259 | 0.623 | I-289 | 60.2 | I-319 | 2.02 |
| I-260 | 2.06 | I-290 | 17.6 | I-320 | 7.91 |
| I-261 | 0.482 | I-291 | 16.4 | I-321 | 3.63 |
| I-262 | 0.541 | I-292 | 2.4 | I-322 | 1.3 |
| I-263 | 0.318 | I-293 | 3.67 | I-323 | 2.81 |
| I-264 | 0.709 | I-294 | 2.37 | I-324 | 8.52 |
| I-265 | 0.470 | I-295 | 7.58 | I-325 | 1.17 |
| I-266 | 2.24 | I-296 | 3.02 | I-326 | 7.23 |
| I-267 | 0.786 | I-297 | 4.37 | I-327 | 2.69 |
| I-268 | 0.589 | I-298 | 3.15 | I-328 | 0.499 |
| I-269 | 2.85 | I-299 | 0.352 | I-329 | 0.282 |
| I-270 | 2.74 | I-300 | 7.17 | I-330 | 1.78 |
| I-271 | 3.92 | I-301 | 1.38 | I-331 | 1.66 |
| I-272 | 1.4 | I-302 | 6.21 | I-332 | 3.12 |
| I-273 | 0.395 | I-303 | 0.278 | I-333 | 4.19 |
| I-274 | 4.5 | I-304 | 0.644 | I-334 | 9.69 |

(continued)

| Compound No. | h 5-HT2C Ki (nM) | Compound No. | h 5-HT2C Ki (nM) | Compound No. | h 5-HT2C Ki (nM) |
|---|---|---|---|---|---|
| I-275 | 0.722 | I-305 | 0.585 | I-335 | 1.87 |
| I-276 | 0.267 | I-306 | 5.67 | I-336 | 0.748 |
| I-277 | 0.217 | I-307 | 0.715 | I-337 | 7.18 |
| I-278 | 3.56 | I-308 | 9.45 | I-338 | 0.765 |
| I-279 | 1.94 | I-309 | 1.37 | I-339 | 2.52 |

[Table 82]

| Compound No. | h 5-HT2C Ki (nM) | Compound No. | h 5-HT2C Ki (nM) | Compound No. | h 5-HT2C Ki (nM) |
|---|---|---|---|---|---|
| I-340 | 1.38 | I-370 | 86.8 | I-400 | 1.38 |
| I-341 | 0.140 | I-371 | 61.5 | I-401 | 0.849 |
| I-342 | 1.88 | I-372 | 9.14 | I-402 | 11 |
| I-343 | 2.42 | I-373 | 64.7 | I-403 | 29.9 |
| I-344 | 6.63 | I-374 | 1.8 | I-404 | 5.79 |
| I-345 | 3.12 | I-375 | 1.57 | I-405 | 0.293 |
| I-346 | 1.7 | I-376 | 2.74 | I-406 | 2.48 |
| I-347 | 1.08 | I-377 | 4.66 | I-407 | 3.64 |
| I-348 | 6.11 | I-378 | 1.51 | I-408 | 1.89 |
| I-349 | 47.7 | I-379 | 0.791 | I-409 | 2.12 |
| I-350 | 6.51 | I-380 | 7.28 | I-410 | 3.94 |
| I-351 | 1.77 | I-381 | 3.58 | | |
| I-352 | 5.99 | I-382 | 3.43 | | |
| I-353 | 0.975 | I-383 | 4.59 | | |
| I-354 | 8.53 | I-384 | 4.58 | | |
| I-355 | 8.2 | I-385 | 5.08 | | |
| I-356 | 7.79 | I-386 | 5.22 | | |
| I-357 | 3.07 | I-387 | 3.82 | | |
| I-358 | 5.78 | I-388 | 7.66 | | |
| I-359 | 4.57 | I-389 | 2.41 | | |
| I-360 | 54.8 | I-390 | 3.91 | | |
| I-361 | 18.3 | I-391 | 19 | | |
| I-362 | 25.2 | I-392 | 12.3 | | |
| I-363 | 2.76 | I-393 | 78.9 | | |
| I-364 | 12.2 | I-394 | 2.16 | | |
| I-365 | 1.98 | I-395 | 1.84 | | |
| I-366 | 59.1 | I-396 | 17.2 | | |
| I-367 | 64.9 | I-397 | 1.8 | | |
| I-368 | 51.2 | I-398 | 5.26 | | |
| I-369 | 58.2 | I-399 | 0.860 | | |

Test Example 3: hERG Test

For the purpose of evaluating risk of an electrocardiogram QT interval prolongation of the compound of the present invention, effects of the compound are studied by evaluating the activity of potassium channel using CHO cells expressing human ether-a-go-go related gene (hERG) channel.

Evaluation is performed using a FluxORII Green Potassium Ion Channel Assay kit (Invitrogen: Molecular Probes).

Cells (8000 cells/well/40 μL) are seeded in a 384 assay plate, and are incubated overnight (37°C, 5% $CO_2$). After replacing the medium with a Wash buffer (lxHBSS, 20 mM HEPES) with a microplate washer, a fluorescent indicator is added to the medium and allowed to incubate (37°C, 5% $CO_2$) for 1 hour for incorporation of the fluorescent indicator into cells.

A cell plate is placed on a cell-based kinetic assay system FLIPR (Molecular Devices, LLC.), and the compound is added to the cells so as to have a desired concentration and reacted for 10 minutes. When a mixed solution of potassium and thallium as a stimulator is added thereto, the potassium channel is opened, and thallium that flowed into the cells binds to the fluorescent indicator, so that the intracellular fluorescence signal increases, and the potassium channel current is detected as a fluorescence signal. For the inhibition rate at each concentration, the signal intensity when E-4031 is added to cells at a final concentration of 10.3 μmol/L is defined as an inhibition rate of 100%, and the signal intensity when DMSO is added to cells at a final concentration of 0.5% is defined as an inhibition rate of 0%. Then, the inhibition rate is calculated from the signal intensity at each concentration. $IC_{50}$ is calculated from the inhibition rate at each concentration.

The compounds of the present invention were tested essentially as described above. The results are shown in the following.

(Results)

Compound I-062: $IC_{50}$> 52.0 μM
Compound I-063: $IC_{50}$> 52.0 μM

Test Example 3-2: hERG Test

For the purpose of evaluating risk of an electrocardiogram QT interval prolongation of the compound of the present invention, effects of the compound are studied by evaluating the activity of potassium channel using CHO cells expressing human ether-a-go-go related gene (hERG) channel.

Evaluation is performed using a FluxORII Green Potassium Ion Channel Assay kit (Invitrogen: Molecular Probes).

Cells (4000 cells/well/40 μL) are seeded in a 384 assay plate, and are incubated overnight (37°C, 5% $CO_2$). After replacing the medium with a Wash buffer (lxHBSS, 20 mM HEPES) with a microplate washer, a fluorescent indicator is added to the medium and allowed to incubate (37°C, 5% $CO_2$) for 1 hour for incorporation of the fluorescent indicator into cells.

A cell plate is placed on a cell-based kinetic assay system FLIPR (Molecular Devices, LLC.), and the compound is added to the cells so as to have a desired concentration and reacted for 10 minutes. When a mixed solution of potassium and thallium as a stimulator is added thereto, the potassium channel is opened, and thallium that flowed into the cells binds to the fluorescent indicator, so that the intracellular fluorescence signal increases, and the potassium channel current is detected as a fluorescence signal. For the inhibition rate at each concentration, the signal intensity when E-4031 is added to cells at a final concentration of 10.3 μmol/L is defined as an inhibition rate of 100%, and the signal intensity when DMSO is added to cells at a final concentration of 0.5% is defined as an inhibition rate of 0%. Then, the inhibition rate is calculated from the signal intensity at each concentration. $IC_{50}$ is calculated from the inhibition rate at each concentration.

The compounds of the present invention were tested essentially as described above. The results are shown in the following.

(Results)

Compound I-006: $IC_{50}$> 52.0 μM
Compound I-127: $IC_{50}$> 52.0 μM
Compound I-200: $IC_{50}$> 52.0 μM

Compound I-228: $IC_{50}$= 50.6 μM
Compound I-267: $IC_{50}$> 52.0 μM
Compound I-271: $IC_{50}$> 52.0 μM
Compound I-359: $IC_{50}$> 52.0 μM

Test Example 4: Bioavailability evaluation

Materials and methods for experiments

(1) Experimental animals: Mice or rats are used.
(2) Rearing condition: Mice or rats are allowed free access to solid feed and sterilized tap.
(3) Setting of dosage and grouping: Oral administration and intravenous administration are performed with the predetermined dosage. Grouping is set as below. The dosage is changed per compound as necessary.

Oral administration 2 to 60 μmol/kg or 1 to 30 mg/kg (n = 2 to 3)
Intravenous administration 1 to 30 μmol/kg or 0.5 to 10 mg/kg (n = 2 to 3)

(4) Preparation of administration solution: Oral administration is performed as solution or suspension. Intravenous administration is performed after solubilization.
(5) Routes of administration: Oral administration is performed mandatory into the stomach by oral sonde. Intravenous administration is performed from caudal vein by syringes with needle.
(6) Evaluation items: Blood is collected serially and concentration of a compound according to the present invention in plasma is measured by LC/MS/MS.
(7) Statistical analysis: About transition of concentration of a compound according to the present invention in plasma, the area under the plasma concentration versus time curve (AUC) is calculated by moment analysis method, and bioavailability (BA) of a compound according to the present invention is calculated from the dosage ratio and the AUC ratio between the oral administration group and the intravenous administration group. The dilution concentration or the dilution solvent are changed as necessary.

The compounds of the present invention can be tested essentially as described above.

Test Example 5: Clearance evaluation test

Materials and methods for experiments

(1) Experimental animals: SD rats are used.
(2) Rearing condition: SD rats are allowed free access to solid feed and sterilized tap water.
(3) Setting of dosage and grouping: Intravenous administration is performed with the predetermined dosage. Grouping is set as below.
Intravenous administration 1 μmol/kg (n = 2)
(4) Preparation of administration solution: The test sample is solubilized using a solvent of dimethyl sulfoxide/propylene glycol = 1/1 and administered.
(5) Administration method: Intravenous administration is performed from caudal vein by syringes with needle.
(6) Evaluation items: Blood is collected serially and concentration of a compound according to the present invention in plasma is measured by LC/MS/MS.
(7) Statistical analysis: About transition of concentration of a compound according to the present invention in plasma, total clearance (CLtot) of a compound according to the present invention is calculated by the moment analysis method. The dilution concentration or the dilution solvent are changed as necessary.

The compounds of the present invention can be tested essentially as described above.

Test Example 6: Metabolic stability test

Using commercially available pooled human liver microsomes, a compound of the present invention is reacted

for a constant time, and a remaining rate is calculated by comparing a reacted sample and an unreacted sample, thereby, a degree of metabolism in liver is assessed.

Reaction is performed in 0.2 mL buffer (50 mmol/L Tris-HCl, pH 7.4, 150 mmol/L potassium chloride, 10 mmol/L magnesium chloride) containing 0.5 mg human liver microsome protein/mL (oxidation reaction) in presence of 1 mmol/L NADPH at 37°C for 0 or 30 minutes. After the reaction, 70 μL of reaction solution is added 140 μL of methanol/acetonitrile = 1/1 (v/v) solution, mixed, and centrifuged at 3000 rpm for 15 minutes. The compound of the present invention in the centrifugal supernatant is analyzed by LC/MS/MS or solid phase extraction (SPE)/MS, and a remaining amount of the compound of the present invention after the reaction was calculated, letting a compound amount at 0-minute reaction time to be 100%. Hydrolysis reaction is performed in the absence of NADPH, and glucuronidation reaction is performed in the presence of 5 mmol/L UDP-glucuronic acid instead of NADPH. Then, the same operation is carried out. The dilution concentration or the dilution solvent are changed as necessary.

The compounds of the present invention can be tested essentially as described above.

Test Example 7: P-gp substrate test

The compound of the present invention is added to one side of a Transwell (registered trademark, CORNING) in which human MDR1-expressing cells or parental cells have been monolayer cultured, and allowed to react for a certain time. For MDR1-expressing cells and parent cells, the membrane permeability coefficients from the Apical side to the Basolateral side (A → B) and from the Basolateral side to the Apical side (B → A) are calculated, and Efflux Ratio (ER; ratio of membrane permeability coefficients of B → A and A → B) value of the MDR1-expressing cells and the parent cells are calculated.

The Efflux Ratio (ER value) of the MDR1-expressing cells and the parent cells are compared to determine whether the compound according to the present invention is a P-gp substrate or not.

The compounds of the present invention can be tested essentially as described above.

Test Example 8: CYP3A4 (MDZ) MBI test

CYP3A4 (MDZ) MBI test is a test of investigating MBI potential on CYP3A4 by the enhancement of inhibitory degree of a metabolic reaction caused by the compound of the present invention. CYP3A4 inhibition is evaluated using pooled human liver microsomes by 1-hydroxylation reaction of midazolam (MDZ) as a marker reaction.

The reaction conditions are as follows: substrate, 10 μmol/L MDZ; pre-reaction time, 0 or 30 minutes; substrate reaction time, 2 minutes; reaction temperature, 37°C; pooled human liver microsomes, at pre-reaction 0.5 mg/mL, at reaction 0.05 mg/mL (at 10-fold dilution); concentrations of the compound of the present invention, at pre-reaction 0.83, 5, 10, 20 μmol/L (4 points).

Pooled human liver microsomes and a solution of the compound of the present invention in K-Pi buffer (pH 7.4) as a pre-reaction solution are added to a 96-well plate at the composition of the pre-reaction. A part of pre-reaction solution is transferred to another 96-well plate, and diluted 10-fold by K-Pi buffer containing a substrate. NADPH as a cofactor is added to initiate a reaction as a marker reaction (without preincubation). After a predetermined time of the reaction, methanol/acetonitrile=1/1 (V/V) solution is added to stop the reaction. NADPH is also added to the remaining pre-reaction solution to initiate the pre-reaction (with pre-reaction), and after the pre-reaction for a predetermined time, a part is transferred to and diluted 10-fold by K-Pi buffer containing a substrate to initiate the reaction as a marker reaction. After a predetermined reaction time, the reaction is stopped by adding a methanol/acetonitrile = 1/1 (V/V) solution. Each plate where the indicator reaction has been performed is centrifuged at 3000 rpm for 15 minutes. Then, midazolam 1-hydroxide in the centrifuged supernatant is quantified by LC/MS/MS.

The sample adding DMSO as a solvent to a reaction system instead of a solution dissolving the compound of the present invention is adopted as a control (100 %). Remaining activity (%) is calculated at each concentration of the compound of the present invention compared to a control, and IC value is calculated by reverse-presumption by a logistic model using a concentration and an inhibition rate. Shifted IC value is calculated as "IC of preincubation at 0 minutes/ IC of preincubation at 30 minutes". When a shifted IC is 1.5 or more, this is defined as positive. When a shifted IC is 1.0 or less, this is defined as negative.

The compounds of the present invention were tested essentially as described above. The results are shown in the following.

(Results)

Compound I-073: Negative

Compound I-109: Negative
Compound I-127: Negative
Compound I-200: Negative
Compound I-247: Negative
Compound I-376: Negative
Compound I-378: Negative

Test Example 9: Solubility test

The solubility of the compounds of the present invention is determined under 1% DMSO addition conditions. A 10 mmol/L compound solution is prepared in DMSO, and 2 μL of the solution of the compound of the present invention is added to 198 μL of 1st fluid for dissolution test (water is added to 2.0 g sodium chloride and 7.0 mL hydrochloric acid to 1000 mL) or 2nd fluid for dissolution (1.7 g potassium dihydrogen phosphate and 1.775 g disodium hydrogen phosphate anhydrous are dissolved in water to 1000 mL) in Description of the Japanese Pharmacopoeia, 18th Edition. After shaking at room temperature for 3 hours, the mixture is suction filtered. The filtrate is diluted 100 times with methanol/acetonitrile/-water = 1/1/2 (V/V/V), and the concentration in the filtrate is measured using LC/MS/MS by absolute calibration curve method. The compounds of the present invention can be tested essentially as described above.

Description of the Japanese Pharmacopoeia, 18th Edition

1st Fluid for dissolution test

2.0 g sodium chloride is dissolved in 7.0 mL of hydrochloric acid and water to 1000 mL. This solution is colorless clear and its pH is about 1.2.

2nd Fluid for dissolution test

1 volume of water is added to 1 volume of phosphate buffer at pH 6.8.

Phosphate buffer at pH 6.8

3.40 g of potassium dihydrogen phosphate and 3.55 g of disodium hydrogen phosphate anhydrous are dissolved in water to 1000 mL.

Test Example 10: MK801-induced hyperactivity inhibition test Six to ten-week-old Wistar male rats are used. For preparation of a test compound administration solution, the test compound is dissolved in 30 mmol/L HCl as a vehicle, and for preparation of an MK801 administration solution, MK801 is dissolved in physiological saline as a vehicle. Using SCANET manufactured by Melquest Ltd., a data collection program SCL-40, and a transparent plastic cage, the MK801-induced hyperactivity inhibition test is performed as follows.

In a breeding room, a compound administration solution (solvent or test compound solution) is administered subcutaneously or orally into the rat, and rat is returned to the breeding cage. After 30 minutes, animals are carried into the laboratory and habituated to the laboratory. After 15 minutes, the rat is gently taken out, and the MK801 dosing solution (solvent or MK801 lysis solution) is administered intraperitoneally or subcutaneously, and the rat is returned to the breeding cage. The rats are taken out 15 minutes after intraperitoneal administration, and placed gently into the SCANET, and measurement of the amount of motor activity is started. The measurement is ended 30 minutes after the start of the measurement, and the amounts of motor activity of the individuals for 30 minutes are summed.

Analysis of the test results is performed as follows. Student-T test (significance level: two-sided 5%) is performed in the test compound administration group and the vehicle administration group. When significant suppression of the amount of motor activity is shown in the test compound administration group as compared with the vehicle administration group, it is determined that the test compound has an antipsychotic effect.

The compounds of the present invention can be tested essentially as described above.

The formulation examples shown below are only for illustrative purposes and are by no means intended to limit the scope of the invention.

The compound of the present invention can be administered as a pharmaceutical composition by any conventional route, particularly enterally, for example, orally, for example, in the form of a tablet or a capsule; parenterally, for example, in the form of an injectable preparation or a suspension; and topically, for example, in the form of a lotion, a gel, an ointment or a cream, or in a transnasal form or a suppository form. A pharmaceutical composition comprising the compound of the present invention in a free form or in the form of a pharmaceutically acceptable salt together with at least one pharmaceutically acceptable carrier or diluent can be manufactured by a mixing, granulating, or coating method in a

conventional manner. For example, oral compositions can be a tablet, a granular preparation, or a capsule, each containing an excipient, a disintegrating agent, a binder, a lubricating agent, and the like, as well as an active ingredient and the like. Compositions for injection can be solutions or suspensions, may be sterilized, and may contain preservatives, stabilizers, buffering agents, and the like.

INDUSTRIAL APPLICABILITY

Since the compound according to the present invention has serotonin 5-HT2A receptor antagonism and/or inverse agonism and serotonin 5-HT2C receptor antagonism and/or inverse agonism, the compound is considered to be useful as a therapeutic agent and/or prophylactic agent for a disease or condition associated with a serotonin 5-HT2A receptor and/or serotonin 5-HT2C receptor.

## Claims

1. A compound represented by Formula (I):

[Chemical formula 1]

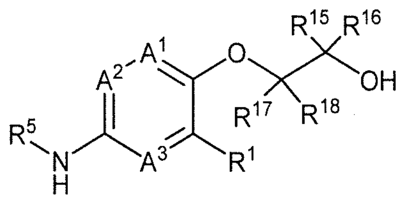

(I)

wherein

$R^1$ is substituted or unsubstituted 6-membered aromatic heterocyclyl or substituted or unsubstituted 5-membered aromatic heterocyclyl (provided that substituted or unsubstituted tetrazolyl and substituted or unsubstituted tetrazolonyl are excluded), substituted or unsubstituted 6-membered aromatic carbocyclyl, substituted or unsubstituted bicyclic 9-membered non-aromatic aromatic heterocyclyl, or substituted or unsubstituted bicyclic 10-membered non-aromatic heterocyclyl;
$A^1$ is $CR^2$ or N;
$A^2$ is $CR^3$ or N;
$A^3$ is $CR^4$ or N;
$R^2$, $R^3$, and $R^4$ are the following (i) or (ii);

(i) $R^2$, $R^3$, and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;
(ii) when $A^1$ is $CR^2$, $A^2$ is $CR^3$, $A^3$ is $CR^4$ or N, $R^2$ and $R^3$ are taken together with a carbon atom to which $R^2$ and $R^3$ are bonded to form a substituted or unsubstituted aromatic carbocycle, a substituted or unsubstituted non-aromatic carbocycle, a substituted or unsubstituted aromatic heterocycle, or a substituted or unsubstituted non-aromatic heterocycle, and $R^4$ each independently is hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy;

$R^5$ is substituted or unsubstituted aromatic heterocyclyl, substituted or unsubstituted aromatic carbocyclyl,

substituted or unsubstituted non-aromatic heterocyclyl, or substituted or unsubstituted non-aromatic carbocyclyl; $R^{15}$ and $R^{16}$ are each independently a hydrogen atom or substituted or unsubstituted alkyl; or $R^{15}$ and $R^{16}$ may be taken together with a carbon atom to which $R^{15}$ and $R^{16}$ are bonded to form a substituted or unsubstituted non-aromatic carbocycle; $R^{17}$ and $R^{18}$ are each independently a hydrogen atom, halogen, or substituted or unsubstituted alkyl; or $R^{17}$ and $R^{18}$ may be taken together with a carbon atom to which $R^{17}$ and $R^{18}$ are bonded to form a substituted or unsubstituted non-aromatic carbocycle, or a pharmaceutically acceptable salt thereof.

**2.** The compound according to claim 1, wherein $R^{15}$ and $R^{16}$ are each a hydrogen atom, and $R^{17}$ and $R^{18}$ are each independently a hydrogen atom, halogen, or alkyl, or a pharmaceutically acceptable salt thereof.

**3.** The compound according to claim 1, wherein $R^{15}$, $R^{16}$, $R^{17}$, and $R^{18}$ are each a hydrogen atom, or a pharmaceutically acceptable salt thereof.

**4.** The compound according to any one of claims 1 to 3, wherein $R^1$ is a group represented by Formula:

[Chemical formula 2]

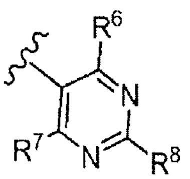, 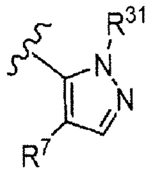, 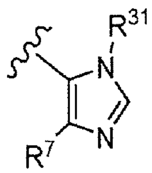, 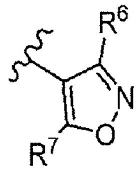, 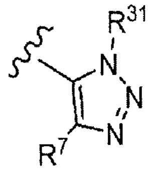,

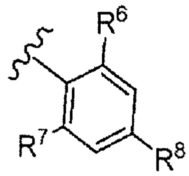, 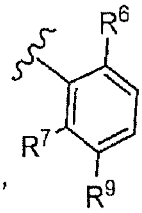, 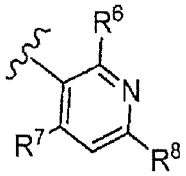, 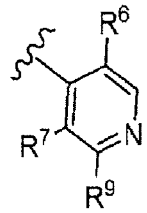, 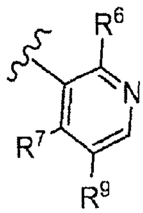,

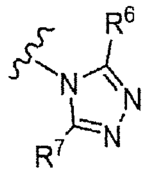, 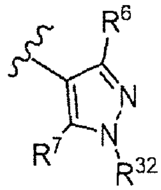, 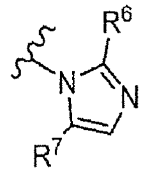, 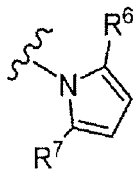, 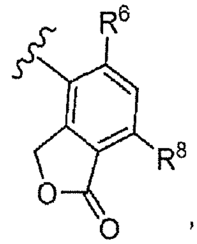,

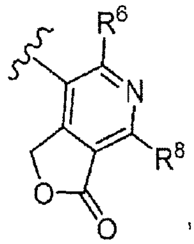, 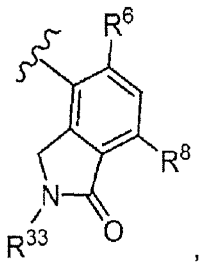, 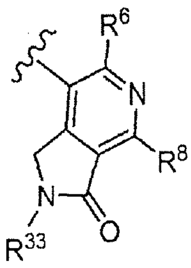, 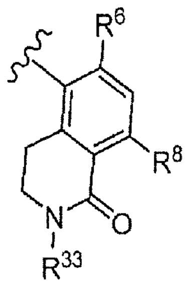

or 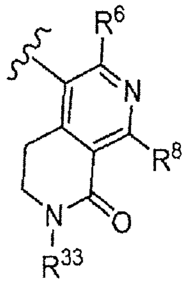

wherein

$R^6$ and $R^7$ are each independently a hydrogen atom, halogen, substituted or unsubstituted alkyl, substituted or unsubstituted alkyloxy, substituted or unsubstituted alkenyl, substituted or unsubstituted alkynyl, cyano, or substituted or unsubstituted non-aromatic carbocyclyl (provided that when $R^6$ is a hydrogen atom, $R^7$ is halogen, substituted or unsubstituted alkyl, substituted or unsubstituted alkyloxy, substituted or unsubstituted alkenyl, substituted or unsubstituted alkynyl, cyano, or substituted or unsubstituted non-aromatic carbocyclyl);

$R^8$ and $R^9$ are each independently a hydrogen atom, halogen, substituted or unsubstituted alkyl, substituted or unsubstituted alkyloxy, substituted or unsubstituted alkyloxycarbonyl, substituted or unsubstituted carbamoyl, cyano, substituted or unsubstituted non-aromatic carbocyclyloxy, substituted or unsubstituted non-aromatic heterocyclyloxy, substituted or unsubstituted non-aromatic carbocyclyl, or substituted or unsubstituted non-aromatic heterocyclyl;

$R^{31}$ is substituted or unsubstituted alkyl, or substituted or unsubstituted non-aromatic carbocyclyl; and

$R^{32}$ and $R^{33}$ are each independently a hydrogen atom, substituted or unsubstituted alkyl, or substituted or unsubstituted non-aromatic carbocyclyl, or a pharmaceutically acceptable salt thereof.

**5.** The compound according to any one of claims 1 to 4, wherein $R^1$ is a group represented by Formula:

[Chemical formula 3]

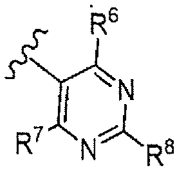
,
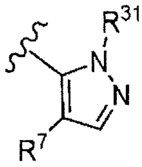
or
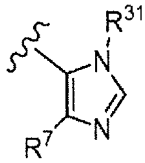

wherein

$R^6$ and $R^7$ are each independently halogen, substituted or unsubstituted alkyl, substituted or unsubstituted alkyloxy, substituted or unsubstituted alkenyl, substituted or unsubstituted alkynyl, or cyano;
$R^8$ is a hydrogen atom, substituted or unsubstituted alkyl, substituted or unsubstituted alkyloxy, substituted or unsubstituted non-aromatic carbocyclyloxy, substituted or unsubstituted non-aromatic heterocyclyloxy, substituted or unsubstituted non-aromatic carbocyclyl, or substituted or unsubstituted non-aromatic heterocyclyl; and
$R^{31}$ is substituted or unsubstituted alkyl or substituted or unsubstituted non-aromatic carbocyclyl, or a pharmaceutically acceptable salt thereof.

**6.** The compound according to any one of claims 1 to 5, wherein $A^1$ is $CR^2$ or N;

$A^2$ is $CR^3$ or N;
$A^3$ is $CR^4$ or N;
$R^2$, $R^3$, and $R^4$ are the following (i-1):

(i-1) $R^2$ and $R^3$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy, and $R^4$ is a hydrogen atom,
or a pharmaceutically acceptable salt thereof.

**7.** The compound according to any one of claims 1 to 6, wherein $A^1$ is $CR^2$ or N;

$A^2$ is $CR^3$ or N;
$A^3$ is $CR^4$ or N;
$R^2$, $R^3$, and $R^4$ are following (i-2):

(i-2) $R^2$ is a hydrogen atom, halogen, or substituted or unsubstituted alkyl, and $R^3$ and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
or a pharmaceutically acceptable salt thereof.

**8.** The compound according to any one of claims 1 to 7, wherein $A^1$ is $CR^2$ or N;

$A^2$ is $CR^3$ or N;

$A^3$ is $CR^4$ or N;
$R^2$, $R^3$, and $R^4$ are the following (i-3):

(i-3) $R^2$, $R^3$, and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
or a pharmaceutically acceptable salt thereof.

**9.** The compound according to any one of claims 1 to 8, wherein $A^1$ is $CR^2$ or N;

$A^2$ is $CR^3$ or N;
$A^3$ is $CR^4$ or N;
$R^2$, $R^3$, and $R^4$ are the following (i-4):

(i-4) $R^3$ is a hydrogen atom, halogen, or substituted or unsubstituted alkyl, and $R^2$ and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
or a pharmaceutically acceptable salt thereof.

**10.** The compound according to any one of claims 1 to 9, wherein $A^1$ is $CR^2$ or N;

$A^2$ is $CR^3$ or N;
$A^3$ is $CR^4$ or N;
$R^2$, $R^3$, and $R^4$ are the following (i-5):

(i-5) $R^3$ is a hydrogen atom or halogen, $R^2$, and $R^4$ are each independently a hydrogen atom, halogen, cyano, substituted or unsubstituted carbamoyl, substituted or unsubstituted alkyl, substituted or unsubstituted alkyloxy, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, substituted or unsubstituted aromatic carbocyclyloxy, substituted or unsubstituted non-aromatic carbocyclyloxy, substituted or unsubstituted aromatic heterocyclyloxy, or substituted or unsubstituted non-aromatic heterocyclyloxy,
or a pharmaceutically acceptable salt thereof.

**11.** The compound according to any one of claims 1 to 10, wherein

(i') $A^1$ is CH, $A^2$ is N, and $A^3$ is CH; or
(ii') $A^1$ is CH, $A^2$ is $CR^3$, $R^3$ is a hydrogen atom, halogen, or substituted or unsubstituted alkyl, and $A^3$ is N, or a pharmaceutically acceptable salt thereof.

**12.** The compound according to any one of claims 1 to 11, wherein $R^5$ is substituted or unsubstituted aromatic heterocyclyl or substituted or unsubstituted aromatic carbocyclyl, or a pharmaceutically acceptable salt thereof.

**13.** The compound according to any one of claims 1 to 11, wherein $R^5$ is substituted or unsubstituted 6-membered aromatic heterocyclyl or substituted or unsubstituted phenyl.

**14.** The compound according to any one of claims 1 to 13, wherein $R^5$ is a group represented by Formula:

[Chemical formula 4]

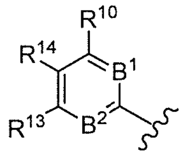

wherein

$R^{10}$ and $R^{13}$ are each independently a hydrogen atom, cyano, substituted or unsubstituted alkyloxy, or substituted or unsubstituted alkyl;
$R^{14}$ is a hydrogen atom, halogen, substituted or unsubstituted alkyl, substituted or unsubstituted alkyloxy, substituted or unsubstituted non-aromatic carbocyclyl, substituted or unsubstituted non-aromatic heterocyclyl, substituted or unsubstituted aromatic carbocyclyl, substituted or unsubstituted aromatic heterocyclyl, substituted or unsubstituted non-aromatic carbocyclyloxy, substituted or unsubstituted aromatic carbocyclyloxy, substituted or unsubstituted alkyloxycarbonyl, substituted or unsubstituted alkylcarbonyl, or cyano;
$B^1$ is $CR^{11}$ or N;
$B^2$ is $CR^{12}$ or N; and
$R^{11}$ and $R^{12}$ are each independently a hydrogen atom, halogen, methyloxy, methyl, or halomethyl, or a pharmaceutically acceptable salt thereof.

**15.** The compound according to claim 14, wherein (i'') $B^1$ is N, and $B^2$ is $CR^{12}$; or (ii") $B^1$ and $B^2$ are each N, or a pharmaceutically acceptable salt thereof.

**16.** The compound according to claim 14 or 15, wherein $R^{14}$ is a hydrogen atom, halogen, substituted or unsubstituted alkyl, substituted or unsubstituted alkyloxy, substituted or unsubstituted aromatic carbocyclyl, substituted or unsubstituted aromatic heterocyclyl, substituted or unsubstituted non-aromatic carbocyclyloxy, or substituted or unsubstituted non-aromatic carbocyclyl, or a pharmaceutically acceptable salt thereof.

**17.** The compound according to claim 1, wherein the compound is selected from the group consisting of compounds I-006, I-062, I-063, I-073, I-127, I-200, I-211, I-247, I-257, I-266, I-267, I-271, I-359, I-376, and I-378, or a pharmaceutically acceptable salt thereof.

**18.** A pharmaceutical composition comprising the compound according to any one of claims 1 to 17 or a pharmaceutically acceptable salt thereof.

**19.** The pharmaceutical composition according to claim 18, wherein the pharmaceutical composition is a serotonin 5-HT2A receptor antagonist and/or inverse agonist.

**20.** The pharmaceutical composition according to claim 18, wherein the pharmaceutical composition is an antagonist and/or inverse agonist of serotonin 5-HT2A receptor and serotonin 5-HT2C receptor.

**21.** A method for treating and/or preventing a disease associated with a serotonin 5-HT2A receptor, the method comprising administering the compound according to any one of claims 1 to 17 or a pharmaceutically acceptable salt thereof.

**22.** A method for treating and/or preventing a disease associated with serotonin 5-HT2A and 5-HT2C receptors, the method comprising administering the compound according to any one of claims 1 to 17 or a pharmaceutically acceptable salt thereof.

**23.** The compound according to any one of claims 1 to 17 or a pharmaceutically acceptable salt thereof, for use in treating and/or preventing a disease associated with a serotonin 5-HT2A receptor antagonist and/or inverse agonist.

**24.** The compound according to any one of claims 1 to 17 or a pharmaceutically acceptable salt thereof, for use in treating and/or preventing a disease associated with an antagonist and/or agonist of a serotonin 5-HT2A receptor and a serotonin 5-HT2C receptor.

## INTERNATIONAL SEARCH REPORT

International application No. **PCT/JP2023/043865**

**A. CLASSIFICATION OF SUBJECT MATTER**

***C07D 401/12***(2006.01)i; ***A61K 31/44***(2006.01)i; ***A61K 31/415***(2006.01)i; ***A61K 31/423***(2006.01)i; ***A61K 31/427***(2006.01)i; ***A61K 31/428***(2006.01)i; ***A61K 31/433***(2006.01)i; ***A61K 31/437***(2006.01)i; ***A61K 31/444***(2006.01)i; ***A61K 31/497***(2006.01)i; ***A61K 31/501***(2006.01)i; ***A61K 31/506***(2006.01)i; ***A61K 31/4184***(2006.01)i; ***A61K 31/4375***(2006.01)i; ***A61K 31/4439***(2006.01)i; ***A61K 31/4725***(2006.01)i; ***A61K 31/4985***(2006.01)i; ***A61P 43/00***(2006.01)i; ***C07D 213/74***(2006.01)i; ***C07D 231/16***(2006.01)i; ***C07D 401/04***(2006.01)i; ***C07D 401/14***(2006.01)i; ***C07D 403/04***(2006.01)i; ***C07D 403/12***(2006.01)i; ***C07D 405/14***(2006.01)i; ***C07D 407/14***(2006.01)i; ***C07D 413/12***(2006.01)i; ***C07D 413/14***(2006.01)i; ***C07D 417/12***(2006.01)i; ***C07D 417/14***(2006.01)i; ***C07D 471/04***(2006.01)i; ***C07D 487/04***(2006.01)i; ***C07D 513/04***(2006.01)i

FI: C07D401/12 CSP; A61K31/415; A61K31/4184; A61K31/423; A61K31/427; A61K31/428; A61K31/433; A61K31/437; A61K31/4375; A61K31/44; A61K31/4439; A61K31/444; A61K31/4725; A61K31/497; A61K31/4985; A61K31/501; A61K31/506; A61P43/00 111; C07D213/74; C07D231/16; C07D401/04; C07D401/14; C07D403/04; C07D403/12; C07D405/14; C07D407/14; C07D413/12; C07D413/14; C07D417/12; C07D417/14; C07D471/04 113; C07D487/04 144; C07D513/04

According to International Patent Classification (IPC) or to both national classification and IPC

**B. FIELDS SEARCHED**

Minimum documentation searched (classification system followed by classification symbols)

C07D401/12; A61K31/44; A61K31/415; A61K31/423; A61K31/427; A61K31/428; A61K31/433; A61K31/437; A61K31/444; A61K31/497; A61K31/501; A61K31/506; A61K31/4184; A61K31/4375; A61K31/4439; A61K31/4725; A61K31/4985; A61P43/00; C07D213/74; C07D231/16; C07D401/04; C07D401/14; C07D403/04; C07D403/12; C07D405/14; C07D407/14; C07D413/12; C07D413/14; C07D417/12; C07D417/14; C07D471/04; C07D487/04; C07D513/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2004/058722 A1 (ARENA PHARMCEUTICALS. INC.) 15 July 2004 (2004-07-15) claims, examples | 1-24 |
| Y | JP 2009-537543 A (ARENA PHARMACEUTICALS, INC.) 29 October 2009 (2009-10-29) claims, compound number 68, 76, examples 1.32, 1.35, 4 | 1-24 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 February 2024** | **20 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2023/043865**

**C. DOCUMENTS CONSIDERED TO BE RELEVANT**

| Category* | Citation of document, with indication, where appropriate, of the relevant passages | Relevant to claim No. |
|---|---|---|
| Y | JP 2009-533442 A (ARENA PHARMACEUTICALS, INC.) 17 September 2009 (2009-09-17) claims, examples | 1-24 |
| A | JP 2008-520695 A (ARENA PHARMACEUTICALS, INC.) 19 June 2008 (2008-06-19) entire text | 1-24 |
| A | JP 2015-519307 A (RIGEL PHARMACEUTICALS, INC.) 09 July 2015 (2015-07-09) claims, compounds 77-87 | 1-24 |
| A | JP 2016-502545 A (RIGEL PHARMACEUTICALS, INC.) 28 January 2016 (2016-01-28) claims, examples 135-146 | 1–24 |
| A | JP 2016-519657 A (RIGEL PHARMACEUTICALS, INC.) 07 July 2016 (2016-07-07) claims, examples 59-69 | 1-24 |
| A | JP 2017-518282 A (ARENA PHARMACEUTICALS, INC.) 06 July 2017 (2017-07-06) claims, intermediate G38, fluoro compound E25, example 51, aniline N31 | 1-24 |
| A | JP 2015-520157 A (NOVARTIS AG) 16 July 2015 (2015-07-16) claims, examples | 1-24 |

Form PCT/ISA/210 (second sheet) (July 2022)

**INTERNATIONAL SEARCH REPORT**
**Information on patent family members**

International application No.
**PCT/JP2023/043865**

| Patent document cited in search report | Publication date (day/month/year) | Patent family member(s) | Publication date (day/month/year) |
|---|---|---|---|
| WO 2004/058722 A1 | 15 July 2004 | US 2006/0229335 A1 | |
| | | AU 2003297441 A | |
| JP 2009-537543 A | 29 October 2009 | US 2009/0186895 A1 claims, compound number 68, 76, examples 1.32, 1.35, 4 | |
| | | JP 2014-28824 A | |
| | | US 2012/0295938 A1 | |
| | | US 2014/0163032 A1 | |
| | | US 2015/0174102 A1 | |
| | | US 2017/0166533 A1 | |
| | | US 2020/0247755 A1 | |
| | | WO 2007/136680 A2 | |
| | | EP 2027119 A2 | |
| | | EP 3395816 A1 | |
| | | CA 2646076 A | |
| | | CN 101479261 A | |
| | | AU 2007254244 A | |
| | | CN 103396412 A | |
| | | CN 104761498 A | |
| JP 2009-533442 A | 17 September 2009 | WO 2007/120600 A2 claims, examples | |
| | | EP 2004627 A2 | |
| JP 2008-520695 A | 19 June 2008 | US 2007/0207994 A1 entire text | |
| | | US 2007/0244086 A1 | |
| | | US 2011/0105456 A1 | |
| | | US 2013/0237540 A1 | |
| | | US 2016/0304463 A1 | |
| | | US 2019/0263757 A1 | |
| | | WO 2006/055734 A2 | |
| | | EP 1833799 A2 | |
| | | EP 1978019 A1 | |
| | | CA 2588513 A | |
| | | NO 20072986 A | |
| | | CN 101084195 A | |
| | | AR 51675 A | |
| | | AT 407927 T | |
| | | NI 200700127 A | |
| | | DK 1833799 T | |
| | | HK 1102818 A | |
| | | HR 20080592 T | |
| | | SI 1833799 T | |
| | | ES 2314750 T | |
| | | GT 200500336 A | |
| | | BR PI 0518446 A | |
| | | PT 1833799 E | |
| | | EA 200701101 A | |
| | | ZA 200703885 A | |
| | | IL 182945 A | |
| | | PE 11302006 A | |
| | | NZ 554817 A | |

Form PCT/ISA/210 (patent family annex) (July 2022)

**INTERNATIONAL SEARCH REPORT**
**Information on patent family members**

International application No.
**PCT/JP2023/043865**

| Patent document cited in search report | Publication date (day/month/year) | Patent family member(s) | Publication date (day/month/year) |
|---|---|---|---|
| | | RS 50698 B | |
| | | AU 2005307744 A | |
| | | MA 29081 B | |
| | | MX 2007005959 A | |
| | | CR 9098 A | |
| | | HR P 20080592 T | |
| | | TW 201231052 A | |
| | | CN 102977029 A | |
| | | CN 103012378 A | |
| | | MY 147684 A | |
| | | TW 200618798 A | |
| | | KR 10-2013-0027039 A | |
| | | UA 91350 C | |
| | | KR 10-2007-0086357 A | |
| | | CY 1108508 T | |
| | | TN SN 07188 A | |
| | | SA 5260357 B | |
| | | PL 1833799 T | |
| | | PE 20061130 A | |
| JP 2015-519307 A | 09 July 2015 | WO 2013/152198 A1<br>claims, compounds 77-87 | |
| | | JP 2016-519657 A | |
| | | US 2013/0331374 A1 | |
| | | US 2016/0257681 A1 | |
| | | US 2018/0030050 A1 | |
| | | US 2014/0275055 A1 | |
| | | US 2016/0058760 A1 | |
| | | US 2017/0360786 A1 | |
| | | US 2019/0060310 A1 | |
| | | WO 2014/151900 A1 | |
| | | EP 2833889 A1 | |
| | | EP 2970275 A1 | |
| | | CA 2867760 A | |
| | | ES 2658395 T | |
| | | CA 2904610 A | |
| JP 2016-502545 A | 28 January 2016 | WO 2014/089112 A1<br>claims, examples 135-146 | |
| | | US 2014/0163022 A1 | |
| | | US 2015/0306100 A1 | |
| | | EP 2928891 A1 | |
| | | CA 2892677 A | |
| JP 2016-519657 A | 07 July 2016 | WO 2014/151900 A1<br>claims, examples 59-69 | |
| | | JP 2015-519307 A | |
| | | US 2013/0331374 A1 | |
| | | US 2014/0275055 A1 | |
| | | US 2016/0058760 A1 | |
| | | US 2016/0257681 A1 | |
| | | US 2017/0360786 A1 | |
| | | US 2018/0030050 A1 | |

Form PCT/ISA/210 (patent family annex) (July 2022)

**INTERNATIONAL SEARCH REPORT**
**Information on patent family members**

International application No.
**PCT/JP2023/043865**

| Patent document cited in search report | Publication date (day/month/year) | Patent family member(s) | Publication date (day/month/year) |
|---|---|---|---|
| | | US 2019/0060310 A1 | |
| | | WO 2013/152198 A1 | |
| | | EP 2833889 A1 | |
| | | EP 2970275 A1 | |
| | | CA 2867760 A | |
| | | CA 2904610 A | |
| | | ES 2658395 T | |
| JP 2017-518282 A | 06 July 2017 | WO 2015/175632 A1 claims, intermediate G38, fluoro compound E25, example 51, aniline N31 | |
| | | US 2017/0166598 A1 | |
| | | EP 3143015 A1 | |
| | | CN 106687457 A | |
| JP 2015-520157 A | 16 July 2015 | WO 2013/171641 A1 claims, examples | |
| | | US 2015/0141427 A1 | |
| | | US 2016/0185733 A1 | |
| | | EP 2900637 A1 | |
| | | AU 2013261129 A | |
| | | CA 2870339 A | |
| | | CN 104334529 A | |
| | | KR 10-2015-0014452 A | |
| | | EA 201492092 A | |
| | | MX 2014013375 A | |
| | | PT 2900637 T | |
| | | ES 2646777 T | |
| | | BR 112014027584 A | |
| | | PL 2900637 T | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005012254 A
- WO 2006078610 A
- WO 2007136703 A
- WO 2007136680 A
- WO 2022093850 A
- WO 2022093849 A
- WO 2007120600 A
- WO 2006055734 A
- WO 2004058722 A
- WO 2004028450 A
- WO 0129008 A
- WO 2013171641 A
- WO 2008027483 A
- WO 2007136875 A
- WO 9952927 A
- WO 03062206 A
- WO 2019165158 A
- WO 2013152198 A
- WO 2014089112 A
- WO 2014151900 A
- WO 200501225 A


### Non-patent literature cited in the description


- *Nature Reviews Neurology*, 2014, vol. 10, 620-633
- *Progress in Neurology and Psychiatry*, 2018, vol. 22 (1), 30-35
- *Movement Disorders*, 2009, vol. 24 (11), 1641-1649
- *Parkisonism and Related Disorders*, 2009, vol. 15 (3), S105-S109
- *Neurology*, 2004, vol. 63 (2), 293-300
- *JAMA Neurology*, 2016, vol. 73 (5), 535-541
- *The Lancet*, 2014, vol. 383, 533-540
- *Journal of Pharmacology and Experimental Therapeutics*, 2006, vol. 317 (2), 910-918
- *CNS Spectrums*, 2016, vol. 21, 271-275
- *Bioorganic & Medicinal Chemistry Letters*, 2009, vol. 19, 5486-5489
- *Journal of Medicinal Chemistry*, 2010, vol. 53, 4412-4421
- *Journal of Medicinal Chemistry*, 2010, vol. 53, 1923-1936
- *Journal of Medicinal Chemistry*, 2010, vol. 53, 5696-5706
- Isotopes in the Physical and Biomedical Sciences. 1987, vol. 1
- Design of Prodrugs. Elsevier, 1985